# EUROPEAN PATENT APPLICATION

(11) **EP 4 813 980 A1**
(43) Date of publication of application: **30.09.2026**
(21) Application number: 24893565.2
(22) Date of filing: 22.11.2024
(51) Int. Cl.: C07D 401/14, C07D 471/04, C07D 471/02, C07D 487/04, A61K 31/4375, A61K 31/407, A61P 35/00, A61P 3/00, A61P 31/12, A61P 29/00

(54) **EP300/CBP REGULATOR, AND PREPARATION METHOD THEREFOR AND USE THEREOF**

(30) Priority: 24.11.2023 WO PCT/CN2023/134158; 28.02.2024 WO PCT/CN2024/078870
(71) Applicant: Miracure Biotechnology Limited, Haidian, Beijing 100192 (CN)
(72) Inventor: LI, Xiaolin, Beijing 100192 (CN); QI, Longwu, Beijing 100192 (CN); XU, Shusen, Beijing 100192 (CN)
(74) Representative: Cabinet Becker et Associés
(86) International application number: PCT/CN2024/133759
(87) International publication number: WO 2025/108411

(57) **Abstract**

The present disclosure relates to an EP300/CBP regulator as represented by formula (III), and a preparation method therefor and the use thereof. The structure of formula (III) is as follows.

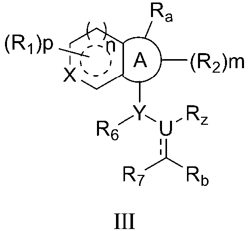

## Description

This application claims the benefit of priority to the following prior applications filed by the applicant:
Patent Application No. PCT/CN2023/134158, entitled "EP300/CBP REGULATOR, AND USE TEHREOF" filed with China Intellectual Property Administration on November 24, 2023;
Patent Application No. PCT/CN2024/078870, entitled "BRIDGED EP300/CBP DEGRADER, AND PREPARATION METHOD THEREFOR AND USE TEHREOF" filed with China Intellectual Property Administration on February 28, 2024.

The entire contents of the prior applications are incorporated herein by reference.

### TECHNICAL FIELD

The present disclosure belongs to the field of medicine, and specifically relates to an EP300/CBP regulator, a preparation method therefor and a use thereof.

### BACKGROUND

Histone acetyltransferase (HAT) and histone deacetylase (HDAC) can affect histone acetylation. Recruitment and normal functioning of HAT and HDAC are key regulatory steps in gene expression and the cell cycle, and functional defects in these enzymes may lead to a variety of diseases including tumors. E1A-binding protein EP300 (EP300) and its closely related analog, cAMP response element-binding protein (CBP), are widely expressed lysine acetyltransferases that acetylate conserved lysine residues in histone by transferring acetyl groups from acetyl-CoA to the ε-N-acetylated lysine.

EP300/CBP is highly expressed and activated in many different tumors. EP300/CBP is closely related to a variety of tumor diseases and is a highly promising target for tumor therapy. Mounting evidence indicates that most oncogenic transcription factors, such as MYC, NF-κB, β-catenin, E2F1, and nuclear receptors, use EP300 and CBP as co-activators. Therefore, depletion of EP300 and/or CBP may affect tumor growth by impairing the function of these oncogenic transcription factors. In addition, EP300 has been reported to modulate immune cell function and is also an important transcriptional co-activator for STAT and NF-κB family transcription factors. Accordingly, EP300/CBP regulators have attracted increasing attention from researchers.

The bromodomain of EP300/CBP is a domain of approximately 110 amino acids that may recognize acetylated lysine residues. The bromodomain actes as "readers" of lysine acetylation, responsible for transducing signals carried by acetylated lysine residues and translating the signals into normal or abnormal phenotypes. The bromodomain of EP300/CBP has a wide range of functions, ranging from histone acetyltransferase activity and chromatin remodeling to the mediation of transcriptional co-activation. Extensive evidence has demonstrated that the bromodomain plays important roles in malignant tumor progression, and regulators targeting the characteristic bromodomain of EP300/CBP hold significant development value and clinical significance in multiple tumors.

### SUMMARY OF THE INVENTION

To address the problems in the prior art, in a first aspect, the present disclosure provides a compound represented by formula (III), and a racemate,a stereoisomer, a tautomer, an isotopic derivative, a N-oxide, a solvate, a polymorph, a metabolite, an ester, or a prodrug, or a pharmaceutically acceptable salt thereof: 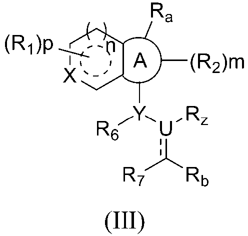 wherein: 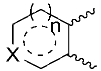
denotes aromatic aryl, heteroaryl, or non-aromatic heterocyclyl;
ring A is selected from phenyl and 5- to 6-membered heteroaryl;
X is selected from NR₁ and CH;
Y is selected from N and CH;
when 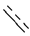 is a single bond, U is Z₁; Z₁ is selected from N and CH; and one of Y and Z₁ is N;
when 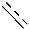 is a double bond, U is C;
each R₁ is the same or different, and is each independently selected from H, oxo (=O), halogen, CN, NH₂, COOH, OH, and the following groups unsubstituted or optionally substituted with 1, 2 or more R₁a: C₁₋₁₂ alkyl, C₁₋₁₂ alkoxy, -(C=O)R, C₆₋₁₄ aryl, 5- to 14- membered heteroaryl; wherein R is selected from C₁₋₁₂ alkyl, C₁₋₁₂ alkoxy, NH₂-, C₁₋₁₂ alkyl-NH-, N,N-di(C₁₋₁₂ alkyl)amino, and C₃₋₁₂ cycloalkyl;
each R₁a is the same or different, and is each independently selected from oxo (=O), halogen, CN, NH₂, COOH, OH, C₁₋₁₂ alkyl, and C₁₋₁₂ alkoxy;
each R₂ is the same or different, and is each independently selected from H, oxo (=O), halogen, CN, NH₂, COOH, OH, C₁₋₁₂ alkyl, and C₁₋₁₂ alkoxy;
Ra is selected from -link-E group, H, halogen, CN, NH₂, COOH, OH, and the following groups unsubstituted or optionally substituted with 1, 2 or more Ra₁: C₆₋₁₄ aryl, 5- to14-membered heteroaryl, 3- to 14- membered heterocyclyl, C₃₋₁₂ cycloalkyl, and C₁₋₁₂ alkyl;
each Ra₁ is the same or different, and is each independently selected from H, oxo (=O), halogen, COOH, OH, and the following groups unsubstituted or optionally substituted with 1, 2 or more Ra₂: NH₂, C₁₋₁₂ alkyl, C₂₋₁₂ alkenyl, C₁₋₁₂ alkoxy, C₁₋₁₂ alkylthio, C₁₋₁₂alkyl-NH-, C₂₋₁₂ alkenyl-NH-, N,N-di(C₁₋₁₂ alkyl)amino, C₁₋₁₂ alkyl-C(=O)-, C₂₋₁₂ alkenyl-C(=O)-, C₂₋₁₂ alkynyl-C(=O)-, C₁₋₁₂ alkoxy-C(=O)-, C₁₋₁₂ alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, C₃₋₁₂ cycloalkyl, C₆₋₁₄ aryl-C(=O)-, 5- to 14- membered heteroaryl-C(=O)-, 3- to 14- membered heterocyclyl-C(=O)-, C₃₋₁₂ cycloalkyl-C(=O)-, C₁₋₁₂ alkyl-S(=O)₂-, C₁₋₁₂ alkyl-S(=O)-,C₂₋₁₂ alkenyl-S(=O)-, C₁₋₁₂ alkylS(=O)(=NH)-, C₃₋₁₂ cycloalkyl-S(=O)₂-, C₂₋₁₂ alkenyl-S(=O)₂-, C₁₋₁₂ alkyl-C(=O)-NH-, C₂₋₁₂ alkenyl-C(=O)-NH-, C₂₋₁₂ alkynyl-C(=O)-NH-, C₁₋₁₂alkyl-S(=O)₂-NH- and C₂₋₁₂ alkenyl-S(=O)₂-NH-;
each Ra₂ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
R₆ is selected from H, halogen, CN, NH₂, COOH, OH, and the following groups unsubstituted or optionally substituted with 1, 2 or more R₆a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, and N,N-di(C₁₋₁₂ alkyl)aminocarbonyl;
each R₆a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
R₇ is selected from H, halogen, CN, NH₂, COOH, OH, and the following groups unsubstituted or optionally substituted with 1, 2 or more R₇a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, and N,N-di(C₁₋₁₂ alkyl)aminocarbonyl;
each R₇a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
alternatively, R₆ and R₇ together with the atoms to which they are attached form the following groups unsubstituted or optionally substituted with 1, 2 or more Rsa, Rc, Rd or Re: C₆₋₁₄ aryl, 5- to 14- membered heteroaryl and 3- to 14- membered heterocyclyl; the 5- to 14-membered heteroaryl and 3- to 14- membered heterocyclyl contains at least one N atom;
each Rsa is the same or different, and is each independently selected from oxo (=O), halogen, CN, NH₂, COOH, OH, and the following groups unsubstituted or optionally substituted with 1, 2 or more Rsb: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
each Rsb is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
Rb, Rc, Rd, Re and Rz are the same or different, and are each independently selected from oxo (=O) and the following groups unsubstituted or optionally substituted with 1, 2 or more Rb₁: C₆₋₁₄ aryl and 5- to 14- membered heteroaryl; alternatively, two adjacent groups of Rb, Rc, Rd, Re and Rz together with the atoms to which they are attached form the following groups unsubstituted or optionally substituted by 1, 2 or more Rb₂: C₆₋₁₄ aryl and 5- to 14- membered heteroaryl;
each of Rb₁ and Rb₂ is the same or different, and is each independently selected from H, halogen, CN, NH₂, COOH, OH, and the following groups unsubstituted or optionally substituted by 1, 2 or more Rb₃: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14- membered heteroaryl, 3-to 14- membered heterocyclyl, and C₃₋₁₂ cycloalkyl; each Rb₃ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
alternatively, Rb and Rz together with the atoms to which they are attached form 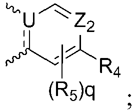
Z₂ is selected from CR₃ and N;
R₃ is selected from H, halogen, CN, NH₂, COOH, OH, and the following groups unsubstituted or optionally substituted by 1, 2 or more R₃a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl;
each R₃a is the same or different, and is each independently selected from oxo (=O), halogen, CN, NH₂, COOH, OH, and the following groups unsubstituted or optionally substituted with 1, 2 or more R₃b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
each R₃b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
R₄ is selected from -link-E group, H, halogen, CN, NH₂, COOH, OH, and the following groups unsubstituted or optionally substituted by 1, 2 or more R₄a: C₁₋₁₂ alkyl, C₁₋₁₂ alkoxy, C₁₋₁₂ alkyl-NH-, N,N-di(C₁₋₁₂ alkyl)amino, C_{1 -12} alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl;
each R₄a is the same or different, and is each independently selected from oxo (=O), halogen, CN, NH₂, COOH, OH, and the following groups unsubstituted or optionally substituted with 1, 2 or more R₄b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
each R₄b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
and at least one of Ra and R₄ is a -link-E group, wherein -link-E group in Ra is -link₁-E₁ and -link-E group in R₄ is - link₂-E₂;
link1₁ and link₂ are the same or different, and are each independently selected from -Cy₁-L₁-Cy₂-L₂-Q-;
Cy₁ is selected from the following groups unsubstituted or optionally substituted with 1, 2 or more R_{Cy1}: 3- to 14- membered heterocyclylene, C₃₋₁₂ cycloalkylene, and 5- to 14- membered heteroarylene;
Cy₂ is absent or is selected from the following groups unsubstituted or optionally substituted with 1, 2 or more R_{Cy2}: 3- to 14- membered heterocyclylene, C₃₋₁₂ cycloalkylene, C₆₋₁₄ arylene, and 5- to 14- membered heteroarylene;
L₁ is absent or is selected from N(R_{L}), and the following groups unsubstituted or optionally substituted with 1, 2 or more R_{L1}: C₁₋₁₂ alkylene and C₁₋₁₂ alkyleneoxy; R_{L} is selected from H and C₁₋₁₂ alkyl;
L₂ is absent or is selected from the following groups unsubstituted or optionally substituted with 1, 2 or more R_{L2}: C₁₋₁₂ alkylene and (C₁₋₁₂ alkyleneoxy)t; t is selected from 0, 1, 2, 3, 4, 5, 6, 7, and 8;
Q is absent or is selected from O, N(R_{q}), ethynylene; R_{q} is selected from H and C₁₋₁₂ alkyl;
R_{Cy1} and R_{Cy2} are the same or different, and are each independently selected from halogen, oxo (=O), C₁₋₁₂ alkyl, and C₁₋₁₂ alkoxy;
R_{L1} and R_{L2} are the same or different, and are each independently selected from halogen, oxo (=O), C₁₋₁₂ alkyl, and C₁₋₁₂ alkoxy;
the end at Cy₁ is connected to 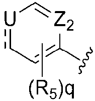 and the end at Q is connected to E₁ or E₂;
E₁ and E₂ are the same or different, and are each independently selected from 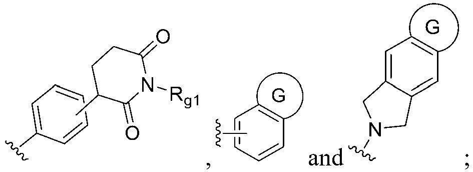 ring G is selected from 5- to 6- membered heterocyclyl containing N and substituted with 1, 2 or more R_{g}; each R_{g} is the same or different, and is each independently selected from H, oxo (=O), C₁₋₁₂ alkyl, and 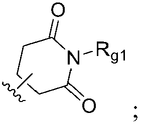 and ring G contains at least one 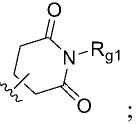 R_{g1} is selected from H, hydroxy C₁₋₁₂ alkyl, (R_{g11})(R_{g12})-N-C₁₋₁₂ alkyl-C(O)O-C₁₋₁₂ alkyl; R_{g11} and R_{g12} are the same or different, and are each independently selected from H and C₁₋₁₂ alkyl; 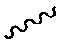 indicates a connection site;
each R₅ is the same or different, and is each independently selected from oxo (=O), halogen, CN, NH₂, COOH, OH, and the following groups unsubstituted or optionally substituted with 1, 2 or more R₅a: C₁₋₁₂ alkyl and C₁₋₁₂ alkoxy;
each R₅a is the same or different, and is each independently selected from oxo (=O), halogen, CN, NH₂, COOH, OH, C₁₋₁₂ alkyl, and C₁₋₁₂ alkoxy;
n is selected from 0, 1, and 2;
m is selected from 0, 1, 2, and 3;
p is selected from 0, 1, 2, 3, 4, and 5;
q is selected from 0, 1, and 2.

According to an embodiment of the present disclosure, the compound represented by formula (III) has the structure represented by formula (I) below: 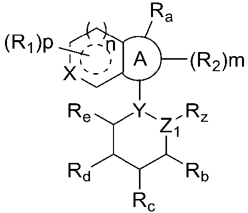 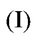 wherein: 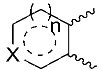
denotes aromatic aryl, heteroaryl, or non-aromatic heterocyclyl;
ring A is selected from phenyl and 5- to 6- membered heteroaryl;
X is selected from NR₁ and CH;
Y is selected from N and CH;
Z₁ is selected from N, C, and CH; and one of Y and Z₁ is N;
each R₁ is the same or different, and is each independently selected from H, oxo (=O), halogen, CN, NH₂, COOH, OH, and the following groups unsubstituted or optionally substituted with 1, 2 or more R₁a: C₁₋₁₂ alkyl, C₁₋₁₂ alkoxy, -(C=O)R, C₆₋₁₄ aryl, 5- to 14- membered heteroaryl; wherein R is selected from C₁₋₁₂ alkyl, C₁₋₁₂ alkoxy, NH₂-, C₁₋₁₂ alkyl-NH-, N,N-di(C₁₋₁₂ alkyl)amino, and C₃₋₁₂ cycloalkyl;
each R₁a is the same or different, and is each independently selected from oxo (=O), halogen, CN, NH₂, COOH, OH, C₁₋₁₂ alkyl, and C₁₋₁₂ alkoxy;
each R₂a is the same or different, and is each independently selected from H, oxo (=O), halogen, CN, NH₂, COOH, OH, C₁₋₁₂ alkyl, and C₁₋₁₂ alkoxy;
Ra is selected from H, halogen, CN, NH₂, COOH, OH, and the following groups unsubstituted or optionally substituted with 1, 2 or more Ra₁: C₆₋₁₄ aryl, 5- to 14- membered heteroaryl, 3- to 14- membered heterocyclyl, C₃₋₁₂ cycloalkyl, and C₁₋₁₂ alkyl;
each Ra₁ is the same or different, and is each independently selected from H, oxo (=O), halogen, COOH, OH, and the following groups unsubstituted or optionally substituted with 1, 2 or more Ra₂: NH₂, C₁₋₁₂ alkyl, C₂₋₁₂ alkenyl, C₁₋₁₂ alkoxy, C₁₋₁₂ alkylthio, C₁₋₁₂alkyl-NH-, C₂₋₁₂ alkenyl-NH-, N,N-di(C₁₋₁₂ alkyl)amino, C₁₋₁₂ alkyl-C(=O)-, C₂₋₁₂ alkenyl-C(=O)-, C₂₋₁₂ alkynyl-C(=O)-, C₁₋₁₂ alkoxy-C(=O)-, C₁₋₁₂ alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, C₃₋₁₂ cycloalkyl, C₆₋₁₄ aryl-C(=O)-, 5- to 14- membered heteroaryl-C(=O)-, 3- to 14- membered heterocyclyl-C(=O)-, C₃₋₁₂ cycloalkyl-C(=O)-, C₁₋₁₂ alkyl-S(=O)₂-, C₁₋₁₂ alkyl-S(=O)-, C₂₋₁₂ alkenyl-S(=O)-, C₁₋₁₂ alkyl-S(=O)(=NH)-, C₃₋₁₂ cycloalkyl-S(=O)₂-, C₂₋₁₂ alkenyl-S(=O)₂-, C₁₋₁₂ alkyl-C(=O)-NH-, C₂₋₁₂ alkenyl-C(=O)-NH-, C₁₋₁₂ alkyl-S(=O)₂-NH-, and C₂₋₁₂ alkenyl-S(=O)₂-NH-;
each Ra₂ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
Rb, Rc, Rd, Re and Rz are the same or different, and are each independently selected from oxo (=O) and the following groups unsubstituted or optionally substituted with 1, 2 or more Rb₁: C₆₋₁₄ aryl and 5- to 14- membered heteroaryl; alternatively, two adjacent groups of Rb, Rc, Rd, Re and Rz together with the atoms to which they are attached form the following groups unsubstituted or optionally substituted by 1, 2 or more Rb₂: C₆₋₁₄ aryl, 5- to 14- membered heteroaryl, and C₃₋₁₂ cycloalkyl;
each of Rb₁ and Rb₂ is the same or different, and is each independently selected from H, halogen, CN, NH₂, COOH, OH, and the following groups unsubstituted or optionally substituted by 1, 2 or more Rb₃: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14- membered heteroaryl, 3-to 14- membered heterocyclyl, and C₃₋₁₂ cycloalkyl; each Rb₃ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
n= 0, 1, 2;
m = 0, 1, 2, 3;
p= 0, 1, 2, 3, 4, 5.

According to an embodiment of the present disclosure, Y is N; Z is selected from C and CH.

According to an embodiment of the present disclosure, Y is CH; Z is N.

According to an embodiment of the present disclosure, ring A is selected from phenyl, pyrazolyl, and imidazolyl.

According to an embodiment of the present disclosure, ring A is selected from

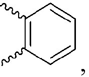

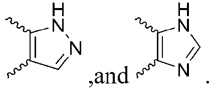

According to an embodiment of the present disclosure, 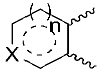 is selected from phenyl and 5- to 6- membered heteroaryl; 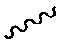 indicates a connection site.

According to an embodiment of the present disclosure, 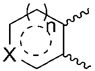 is selected from tetrahydropyrrolyl, piperidinyl, and phenyl.

According to an embodiment of the present disclosure, 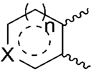 is selected from

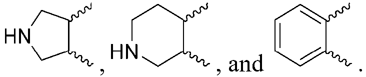

According to an embodiment of the present disclosure, 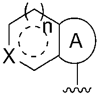 is selected from 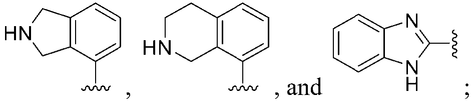 where 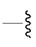 is connected to

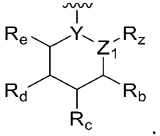

According to an embodiment of the present disclosure, 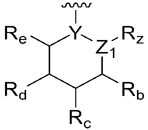 is selected from 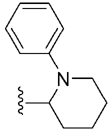 unsubstituted or optionally substituted with 1, 2 or more Rb₁, and 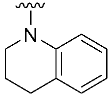 unsubstituted or optionally substituted with 1, 2 or more Rb₂.

According to an embodiment of the present disclosure, 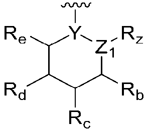 is selected from

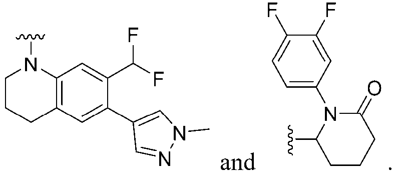

According to an embodiment of the present disclosure, each R₁ is the same or different, and is each independently selected from -(C=O)C₁₋₆ alkyl, -(C=O)-NH-C₁₋₆ alkyl, and 5- to 6-membered heteroaryl substituted with C₁₋₆ alkyl.

According to an embodiment of the present disclosure, each R₁ is the same or different, and is each independently selected from -(C=O)CH₃, -(C=O)-NH-CH₃, and 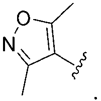 According to an embodiment of the present disclosure, Ra is selected from the following groups unsubstituted or optionally substituted by 1, 2 or more Ra₁: 3- to 8- membered heterocyclyl and C₃₋₈ cycloalkyl.

According to an embodiment of the present disclosure, Ra is selected from the following groups unsubstituted or optionally substituted by 1, 2 or more Ra₁: cyclohexyl, piperidinyl, and piperazinyl.

According to an embodiment of the present disclosure, each Ra₁ is the same or different, and is each independently selected from the following groups unsubstituted or optionally substituted by 1, 2 or more Ra₂: C₁₋₆ alkyl, C₁₋₆ alkyl-C(=O)-, C₂₋₆ alkenyl-C(=O)-, C₂₋₆ alkynyl-C(=O)-, C₁₋₆ alkyl-C(=O)-NH-, C₂₋₆ alkenyl-C(=O)-NH-, C₂₋₆ alkynyl-C(=O)-NH-, C₁₋₆ alkylS(=O)₂-, C₂₋₆ alkenyl-S(=O)₂-, C₁₋₆ alkyl-S(=O)₂-NH-, C₂₋₆ alkenyl-S(=O)₂-NH-, and 3- to 6-membered heterocyclyl.

According to an embodiment of the present disclosure, each Ra₂ is the same or different, and is each independently selected from halogen, oxo (=O), and (C₁₋₁₂alkyl)₂-NH-.

According to an embodiment of the present disclosure, each Ra₁ is the same or different, and is each independently selected from methyl,

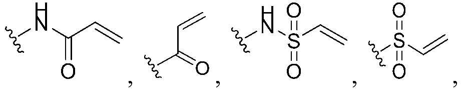

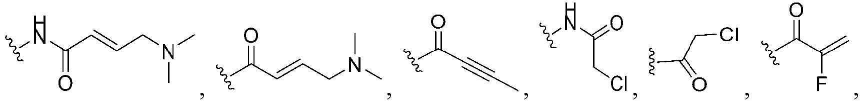

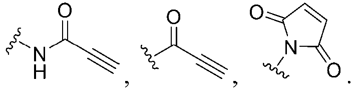

According to an embodiment of the present disclosure, each Rb is the same or different, and is each independently selected from oxo (=O) and C₆₋₁₀ aryl unsubstituted or substituted optionally by 1, 2 or more Rb₁; alternatively, the two Rb together with the atoms to which they are attached form C₆₋₁₀ aryl unsubstituted or optionally substituted by 1, 2 or more Rb₂.

According to an embodiment of the present disclosure, each Rb is the same or different, and is each independently selected from oxo (=O), phenyl unsubstituted or optionally substituted by 1, 2 or more Rb₁; alternatively, the two Rb together with the atoms to which they are attached form phenyl unsubstituted or optionally substituted by 1, 2 or more Rb₂.

According to an embodiment of the present disclosure, each Rb₁ is the same or different, and is each independently selected from halogen, e.g., F.

According to an embodiment of the present disclosure, each Rb₂ is the same or different, and is each independently selected from CN, halogenated C₁₋₆ alkyl, C₃₋₆ alkyl, and C₁₋₆ alkyl-5-to 6- membered heteroaryl.

According to an embodiment of the present disclosure, each Rb₂ is the same or different, and is each independently selected from CN, difluoromethyl, cyclopropyl, and

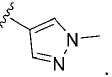

According to an embodiment of the present disclosure, formula I is further selected from the structures shown below: 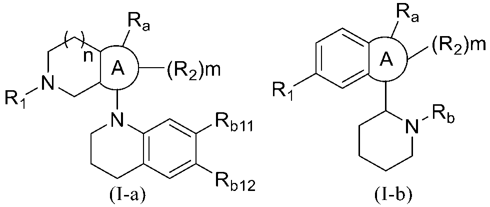 wherein ring A, R₁, R₂, Ra, Rb, m, and n are each independently as defined herein; Rb₁₁ and Rb₁₂ are the same or different, and are each independently as defined for Rb₂.

According to an embodiment of the present disclosure, formula I is further selected from the structures shown below: 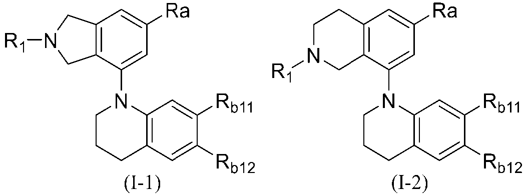 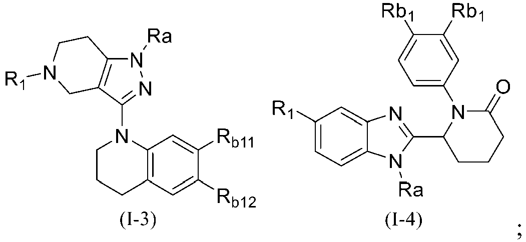 wherein R₁, Ra, and Rb₁ are each independently as defined herein; Rb₁₁ and Rb₁₂ are the same or different, and are each independently as defined for Rb₂.

According to an embodiment of the present disclosure, the compound represented by formula (III) has the structures represented by formula (II) below: 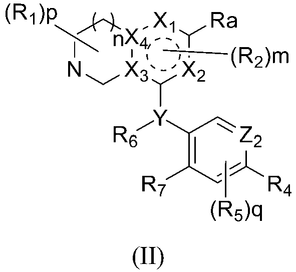 wherein:
n= 0, 1, 2;
m = 0, 1, 2, 3;
p= 0, 1, 2, 3, 4, 5;
q= 0, 1, 2;
X₁, X₂, X₃ and X₄ are the same or different, and is each independently selected from C and N;
Y is selected from C and N;
Z₂ is selected from CR₃ and N;
each R₁ is the same or different, and is each independently selected from oxo (=O), halogen, CN, NH₂, COOH, OH, and the following groups unsubstituted or optionally substituted with 1, 2 or more R₁a: C₁₋₁₂ alkyl, C₁₋₁₂ alkoxy, and -(C=O)R;
wherein R is selected from C₁₋₁₂ alkyl, C₁₋₁₂ alkoxy, NH₂-, C₁₋₁₂ alkyl-NH-, N,N-di(C₁₋₁₂ alkyl)amino, and C₃₋₁₂ cycloalkyl;
each R₁a is the same or different, and is each independently selected from oxo (=O), halogen, CN, NH₂, COOH, OH, C₁₋₁₂ alkyl, and C₁₋₁₂ alkoxy;
each R₂ is the same or different, and is each independently selected from oxo (=O), halogen, CN, NH₂, COOH, OH, C₁₋₁₂ alkyl, and C₁₋₁₂ alkoxy;
Ra is selected from -link-E group, H, halogen, CN, NH₂, COOH, OH, and the following groups unsubstituted or optionally substituted with 1, 2 or more Ra₁: C₆₋₁₄ aryl, 5- to 14-membered heteroaryl, 3- to 14- membered heterocyclyl, C₃₋₁₂ cycloalkyl, and C₁₋₁₂ alkyl;
each Ra₁ is the same or different, and is each independently selected from H, oxo (=O), halogen, COOH, OH, and the following groups unsubstituted or optionally substituted with 1, 2 or more Ra₂: NH₂, C₁₋₁₂ alkyl, C₁₋₁₂ alkoxy, C₁₋₁₂ alkylthio, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, C₃₋₁₂ cycloalkyl, C₆₋₁₄ aryl-C(=O)-, 5- to 14- membered heteroaryl-C(=O)-, 3- to 14- membered heterocyclyl-C(=O)-, C₃₋₁₂ cycloalkyl-C(=O)-, C₁₋₁₂ alkyl-S(=O)₂-, C₁₋₁₂alkylS(=O)-, C₁₋₁₂ alkyl-S(=O)(=NH)-, C₃₋₁₂ cycloalkyl-S(=O)₂-, C₁₋₁₂ alkyl-C(=O)-NH-, and C₁₋₁₂ alkyl-S(=O)₂-NH-;
each Ra₂ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
R₃ is selected from H, halogen, CN, NH₂, COOH, OH, and the following groups unsubstituted or optionally substituted by 1, 2 or more R₃a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl;
each R₃a is the same or different, and is each independently selected from oxo (=O), halogen, CN, NH₂, COOH, OH, and the following groups unsubstituted or optionally substituted with 1, 2 or more R₃b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl;
each R₃b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
R₄ is selected from -link-E group, H, halogen, CN, NH₂, COOH, OH, and the following groups unsubstituted or optionally substituted by 1, 2 or more R₄a: C₁₋₁₂ alkyl, C₁₋₁₂ alkoxy, C₁₋₁₂ alkyl-NH-, N,N-di(C₁₋₁₂ alkyl)amino, C_{1 -12} alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl;
each R₄a is the same or different, and is each independently selected from oxo (=O), halogen, CN, NH₂, COOH, OH, and the following groups unsubstituted or optionally substituted with 1, 2 or more R₄b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
each R₄b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
and at least one of Ra and R₄ is a -link-E group, wherein -link-E group in Ra is -link₁-E₁ and -link-E group in R₄ is -link₂-E₂;
link1₁ and link₂ are the same or different, and are each independently selected from -Cy₁-L₁-Cy₂-L₂-Q-;
Cy₁ is selected from the following groups unsubstituted or optionally substituted with 1, 2 or more R_{Cy1}: 3- to 14- membered heterocyclylene, C₃₋₁₂ cycloalkylene, and 5- to 14- membered heteroarylene;
Cy₂ is absent or is selected from the following groups unsubstituted or optionally substituted with 1, 2 or more R_{Cy2}: 3- to 14- membered heterocyclylene, C₃₋₁₂ cycloalkylene, C₆₋₁₄ arylene, and 5- to 14- membered heteroarylene;
L₁ is absent or is selected from N(R_{L}), and the following groups unsubstituted or optionally substituted with 1, 2 or more R_{L1}: C₁₋₁₂ alkylene and C₁₋₁₂ alkyleneoxy; R_{L} is selected from H and C₁₋₁₂ alkyl;
L₂ is absent or is selected from the following groups unsubstituted or optionally substituted with 1, 2 or more R_{L2}: C₁₋₁₂ alkylene and (C₁₋₁₂ alkyleneoxy)t; t is selected from 0, 1, 2, 3, 4, 5, 6, 7, and 8;
Q is absent or is selected from O, N(R_{q}), ethynylene; R_{q} is selected from H and C₁₋₁₂ alkyl;
R_{Cy1} and R_{Cy2} are the same or different, and are each independently selected from halogen, oxo (=O), C₁₋₁₂ alkyl, and C₁₋₁₂ alkoxy;
R_{L1} and R_{L2} are the same or different, and are each independently selected from halogen, oxo (=O), C₁₋₁₂ alkyl, and C₁₋₁₂ alkoxy;
the end at Cy₁ is connected to 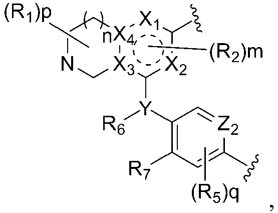 and the end at Q is connected to E₁ or E₂;
E₁ and E₂ are the same or different, and are each independently selected from 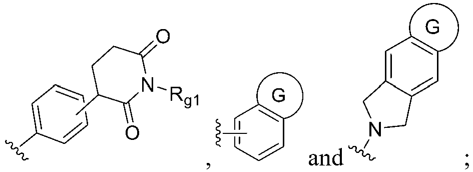 ring G is selected from 5- to 6- membered heterocyclyl containing N and substituted with 1, 2 or more R_{g}; each R_{g} is the same or different, and is each independently selected from H, oxo (=O), C₁₋₁₂ alkyl, and 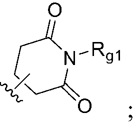 and ring G contains at least one 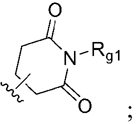 R_{g1} is selected from H, hydroxy C₁₋₁₂ alkyl, (R_{g11})(R_{g12})-N-C₁₋₁₂ alkyl-C(O)O-C₁₋₁₂ alkyl; R_{g11} and R_{g12} are the same or different, and are each independently selected from H and C₁₋₁₂ alkyl; 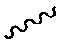 indicates a connection site;
each R₅ is the same or different, and is each independently selected from oxo (=O), halogen, CN, NH₂, COOH, OH, and the following groups unsubstituted or optionally substituted with 1, 2 or more R₅a: C₁₋₁₂ alkyl and C₁₋₁₂ alkoxy;
each R₅a is the same or different, and is each independently selected from oxo (=O), halogen, CN, NH₂, COOH, OH, C₁₋₁₂ alkyl, and C₁₋₁₂ alkoxy;
R₆ is selected from H, halogen, CN, NH₂, COOH, OH, and the following groups unsubstituted or optionally substituted with 1, 2 or more R₆a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, and N,N-di(C₁₋₁₂ alkyl)aminocarbonyl;
each R₆a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl;
R₇ is selected from H, halogen, CN, NH₂, COOH, OH, and the following groups unsubstituted or optionally substituted with 1, 2 or more R₇a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl -NHC(=O)-, and N,N-di(C₁₋₁₂ alkyl)aminocarbonyl;
each R₇a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
alternatively, R₆ and R₇ together with the atoms to which they are attached form the following groups unsubstituted or optionally substituted with 1, 2 or more Rsa: C₆₋₁₄ aryl, 5- to 14- membered heteroaryl and 3- to 14- membered heterocyclyl; the 5- to 14- membered heteroaryl and 3- to 14- membered heterocyclyl contain at least one N atom;
each Rsa is the same or different, and is each independently selected from oxo (=O), halogen, CN, NH₂, COOH, OH, and the following groups unsubstituted or optionally substituted with 1, 2 or more Rsb: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl-NHC(=O)-, and N,N-di(C₁₋₁₂ alkyl)aminocarbonyl;
each Rsb is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino.

In some embodiments, 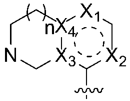 in formula (II) is selected from the following structures: 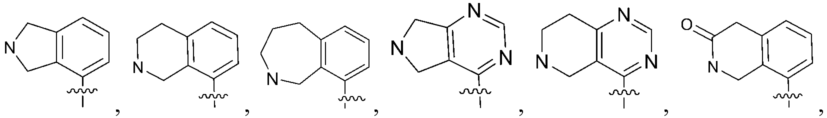 these structures have substituents R₁ and R₂ as defined hereinbefore.

In some embodiments, each R₁ is the same or different, and is each independently selected from oxo (=O), C₁₋₆ alkyl, C₁₋₆ alkoxy, and -(C=O)R; wherein R is selected from C₁₋₆ alkyl, C₁₋₆ alkoxy, NH₂-, C₁₋₆ alkyl-NH-, and C₃₋₆ cycloalkyl.

In some embodiments, R₁ is a substituent on the N atom constituting the ring, exemplarily, such as 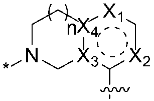 (* represents the substitution site of R₁).

In some embodiments, R₁ is -(C=O)CH₃, -C(=O)OCH₃, or -C(=O)NH-CH₃.

In some embodiments, Ra is selected from -link₁-E₁ group and 5-to 8- membered heterocyclyl; the 5-to 8- membered heterocyclyl is, for example,

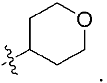

In some embodiments, R₃ is halogenated C₁₋₆ alkyl;

In some embodiments, R₃ is -CHF₂.

In some embodiments, R₄ is selected from -link₂-E₂ group and 5- to 6- membered heteroaryl unsubstituted or optionally substituted with 1, 2 or more R₄a (e.g., pyrazolyl); each R₄a is the same or different, and is each independently selected from C₁₋₆ alkyl and C₁₋₆ alkoxy; preferably, R₄ is

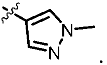

In some embodiments, R₆ and R₇ together with the atoms to which they are attached form the following groups unsubstituted or optionally substituted with 1, 2 or more Rsa: 5- to 6-membered heterocyclyl and C₆₋₁₀ aryl, for example piperidinyl or phenyl.

In some embodiments, R₆ and R₇ together with the atoms to which they are attached form the following structures unsubstituted or optionally substituted with 1, 2 or more Rsa:

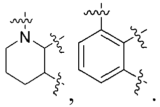

In some embodiments, 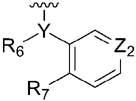 in formula (II) is selected from the following structures:

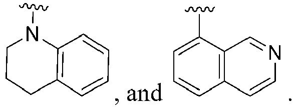

In some embodiments, Cy₁ is selected from the following groups unsubstituted or optionally substituted with 1, 2 or more R_{Cy1}: 5-to 8- membered heterocyclylene, C₃₋₈ cycloalkylene, and 5-to 10- membered heteroarylene.

In some embodiments, Cy₁ is selected from piperidinylene, cyclohexylene, and pyridinylene; for example

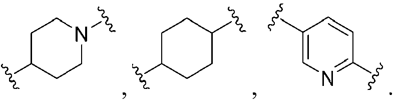

In some embodiments, Cy₂ is absent or is selected from the following groups unsubstituted or optionally substituted with 1, 2 or more R_{Cy2}: 5-to 8- membered heterocyclylene, C₃₋₈ cycloalkylene, C₆₋₁₀ arylene, and 5-to 10- membered heteroarylene.

In some embodiments, Cy₂ is absent or is selected from piperidinylene, morpholinylene, piperazinylene, phenylene, pyridinylene, and triazolylene; for example,

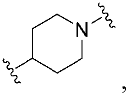

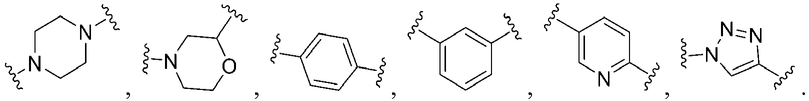

In some embodiments, L₁ is absent or is C₁₋₁₀ alkylene unsubstituted or optionally substituted with 1, 2, or more R_{L1}.

In some embodiments, L₁ is absent or is selected from NH, N(CH₃), methylene, ethylene,

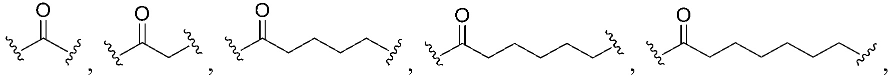

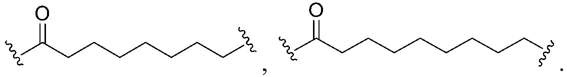

In some embodiments, L₂ is absent or is C₁₋₆ alkylene, (C₁₋₆ alkyleneoxy)t; t is selected from 1, 2, 3, and 4.

In some embodiments, L₂ is absent or is selected from methylene, ethylene, propylene, ethyleneoxy,

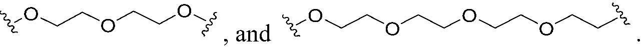

In some embodiments, Q is absent or is selected from O, NH, and ethynylene.

In some embodiments, link₁ and link₂ are the same or different, and is each independently selected from

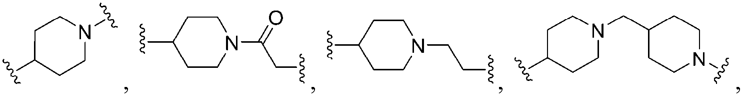

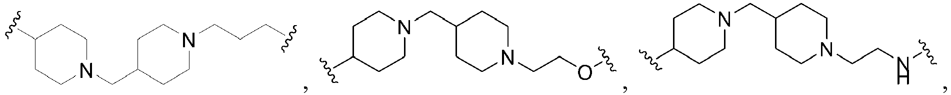

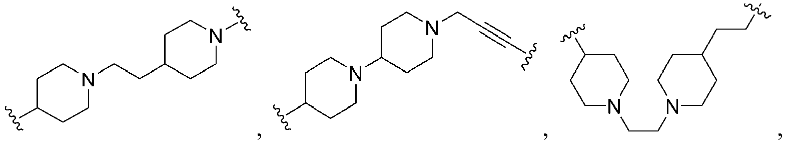

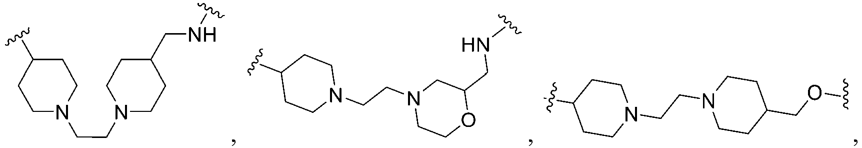

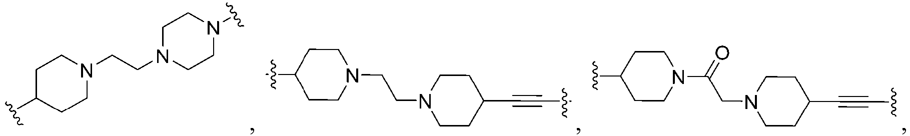

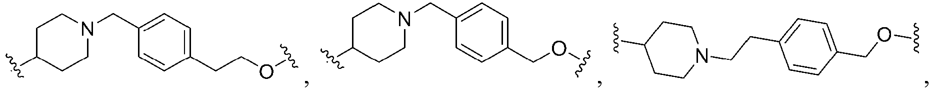

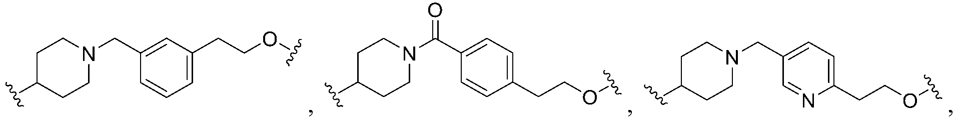

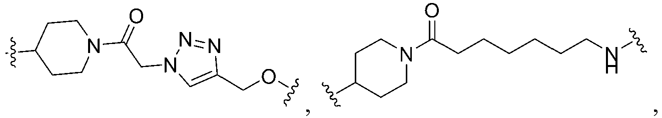

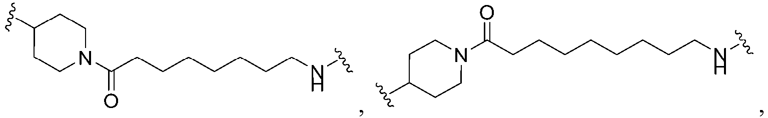

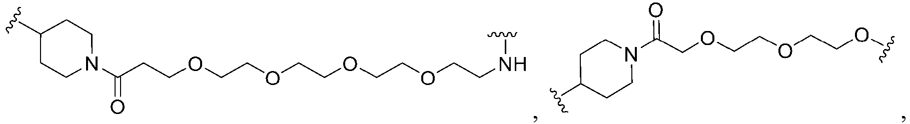

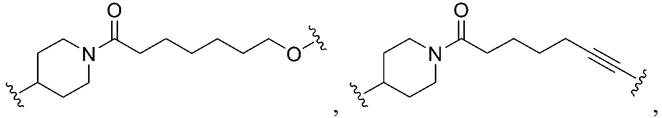

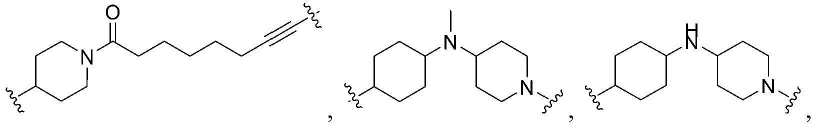

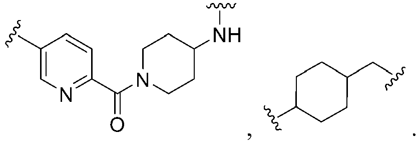

In some embodiments, ring G is selected from tetrahydropyrrolyl and imidazolidinyl substituted with 1, 2 or more R_{g}; for example

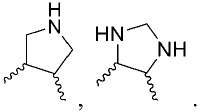

In some embodiments, ring G is selected from

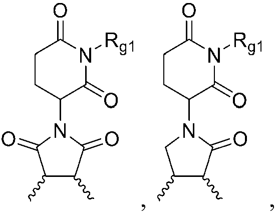

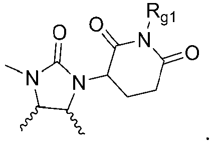

In some embodiments, each R_{g} is the same or different, and is each independently selected from H, oxo (=O), C₁₋₆ alkyl, and

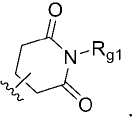

In some embodiments, R_{g1} is selected from H, hydroxyC₁₋₆ alkyl, (C₁₋₆ alkyl)(C₁₋₆ alkyl)-N-C₁₋₆ alkylene-C(O)O-C₁₋₆ alkylene, H₂N-C₁₋₆ alkylene-C(O)O-C₁₋₆ alkylene.

In some embodiments, R_{g1} is selected from H, hydroxymethyl,

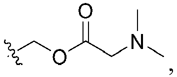

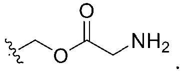

In some embodiments, E₁ and E₂ are the same or different, and are each independently selected from:

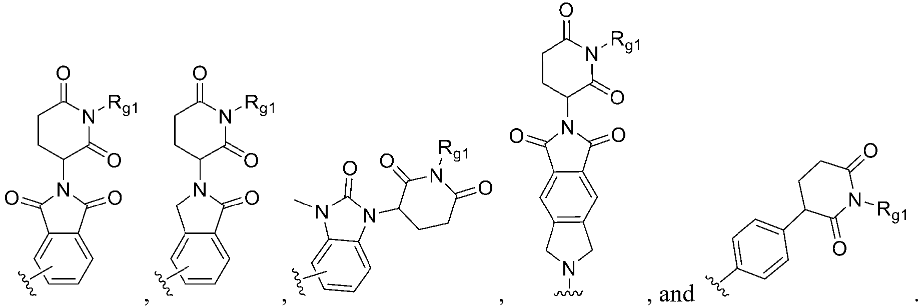

In some embodiments, E₁ and E₂ are the same or different, and are each independently selected from:

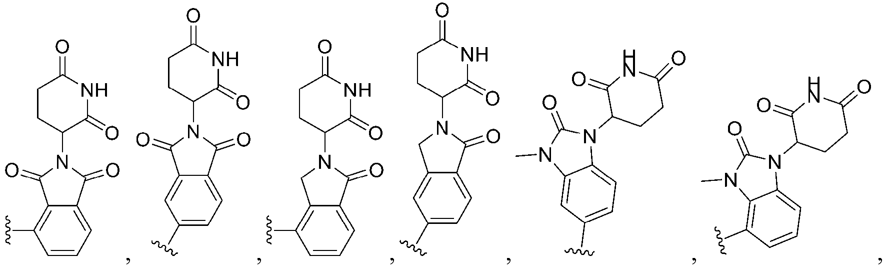

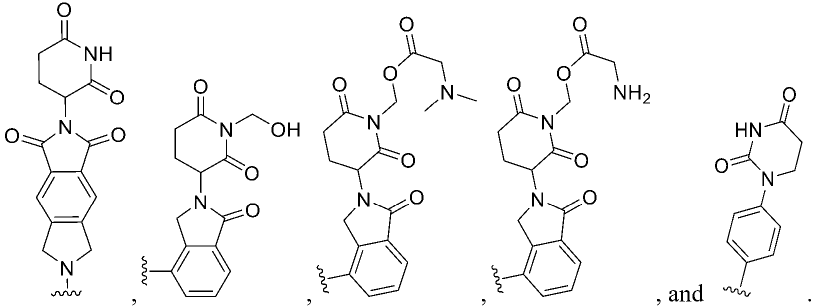

In some embodiments, formula (III) is selected from the following structures: 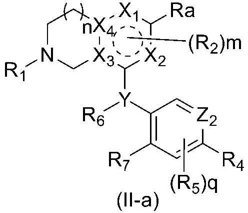 wherein R₁, R₂, Ra, R₄, R₅, R₆, R₇, X₁, X₂, X₃, X₄, Y, Z₂, m, n, and q are as defined herein. In some embodiments, formula (III) is further selected from the following structures: 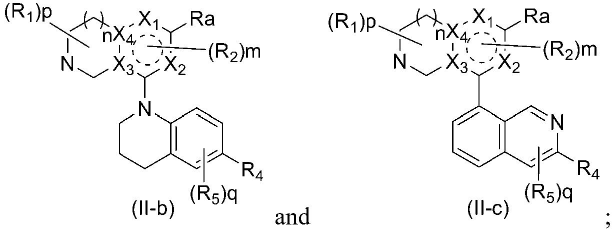 wherein R₁, R₂, Ra, R₄, R₅, X₁, X₂, X₃, X₄, lm, n, p, and q are as defined herein.

In some embodiments, formula (III) is further selected from the following structures: 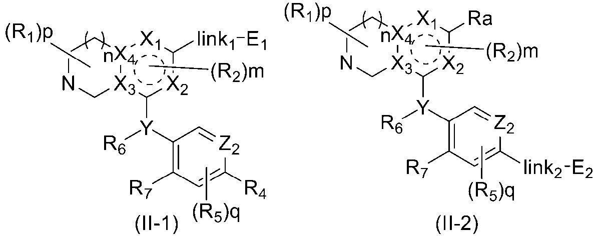 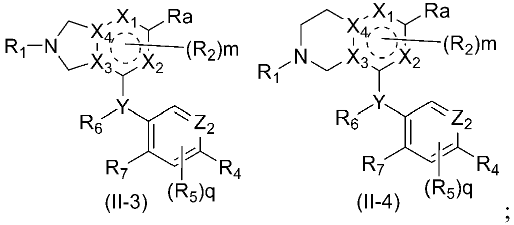 wherein R₁, R₂, Ra, R₄, R₅, R₆, R₇, X₁, X₂, X₃, X₄, Y, Z₂, link₁, link₂, E₁, E₂, m, n, p and q are as defined herein.

In some embodiments, exemplary specific compounds of the compound represented by formula (III) are as follows:

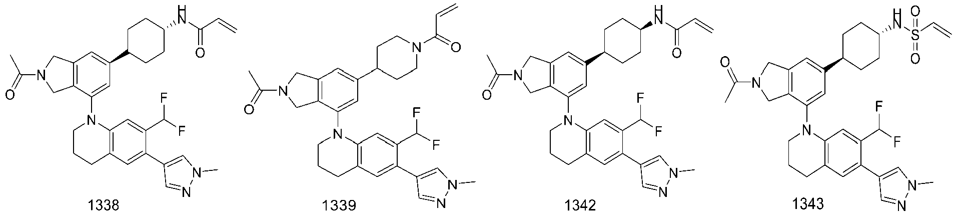

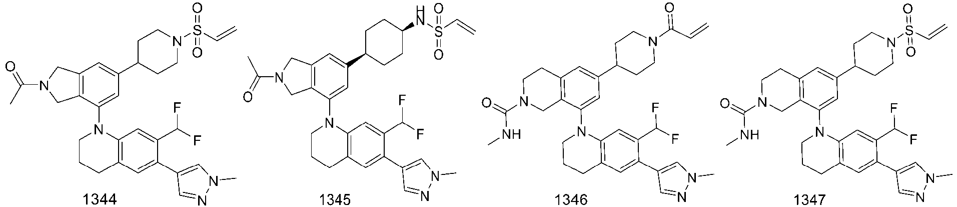

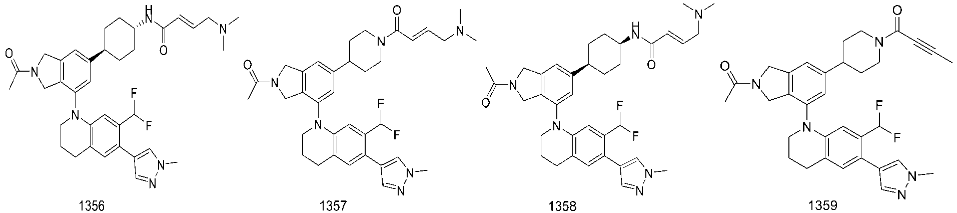

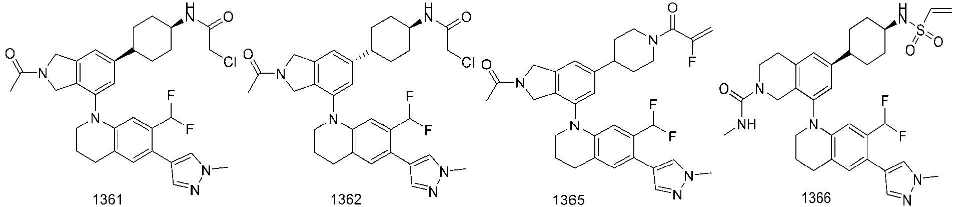

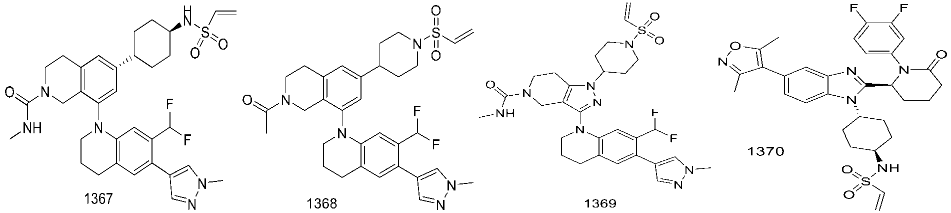

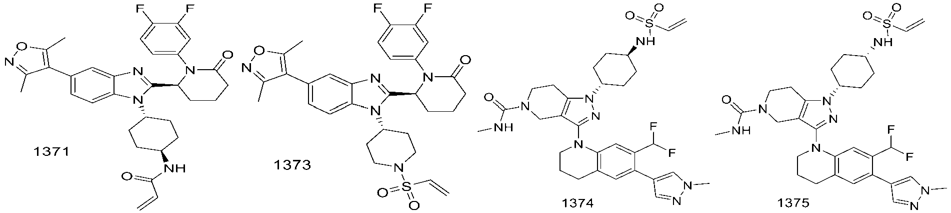

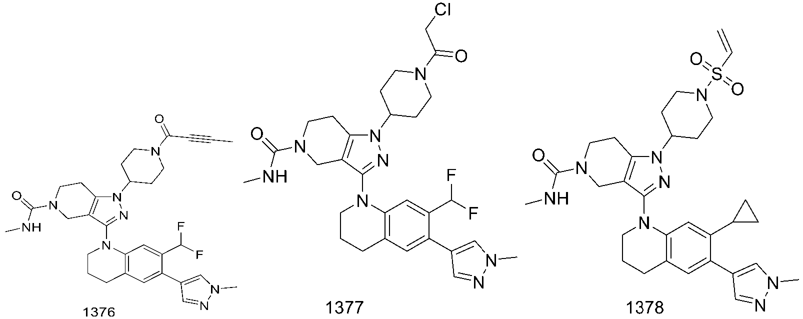

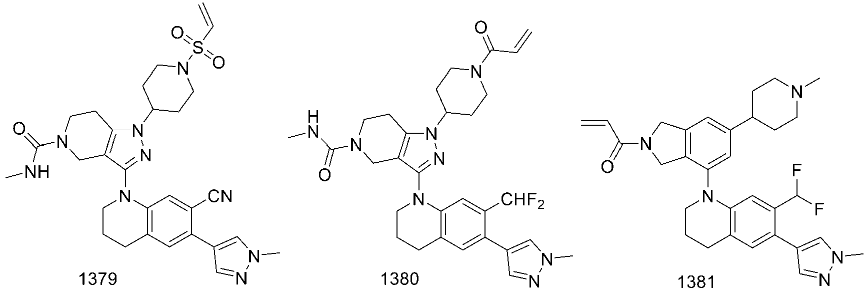

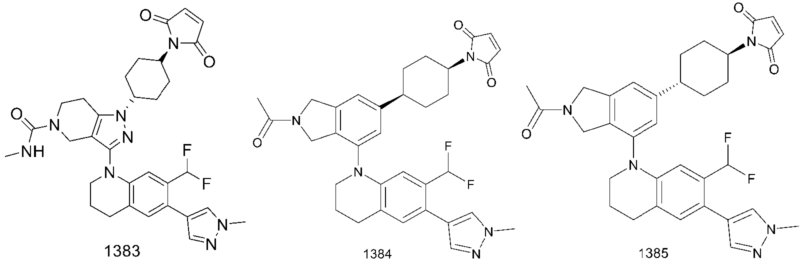

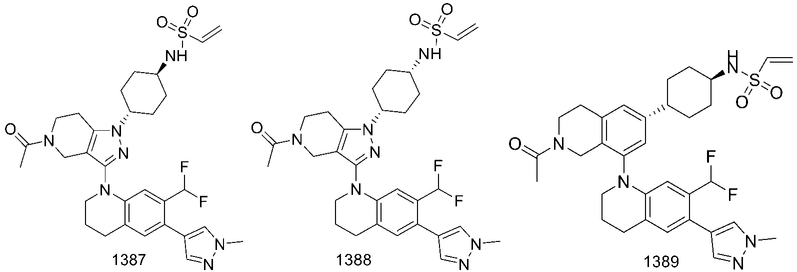

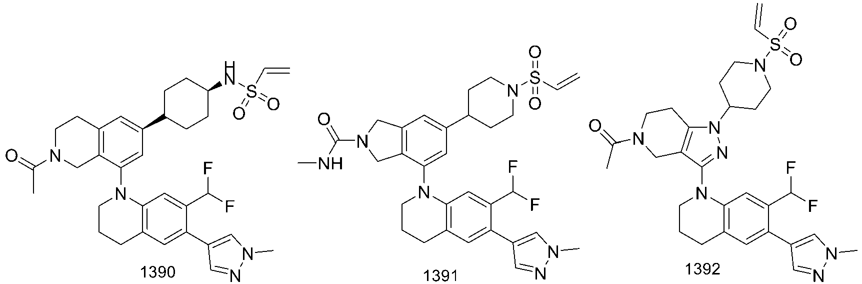

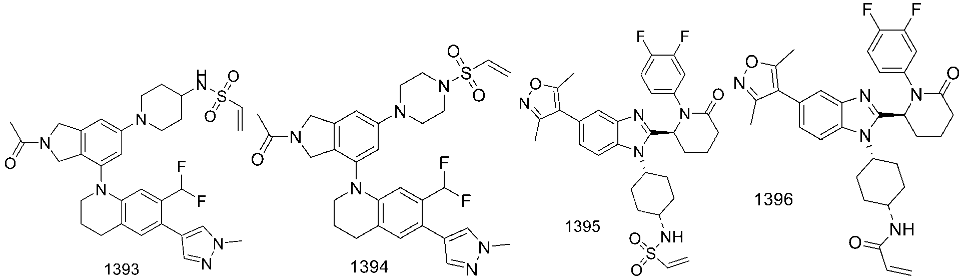

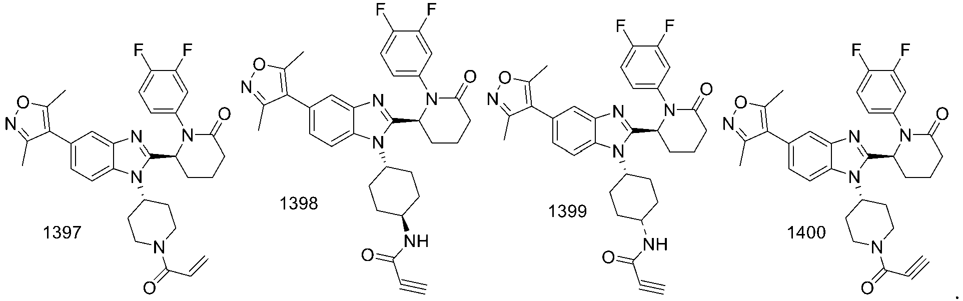

In some embodiments, exemplary specific compounds of the compound represented by formula (III) are as follows:

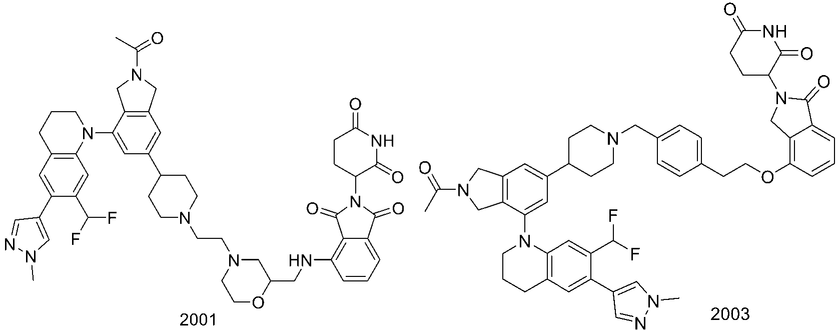

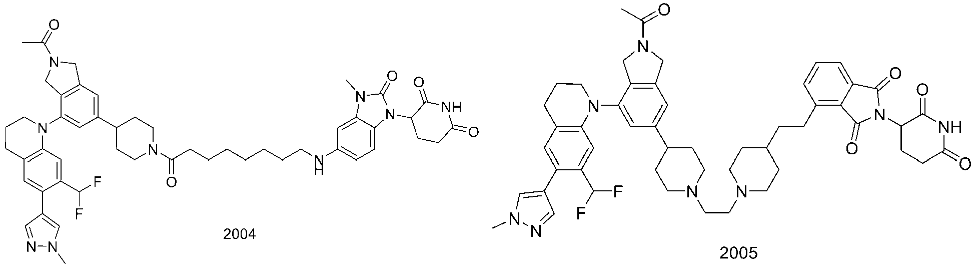

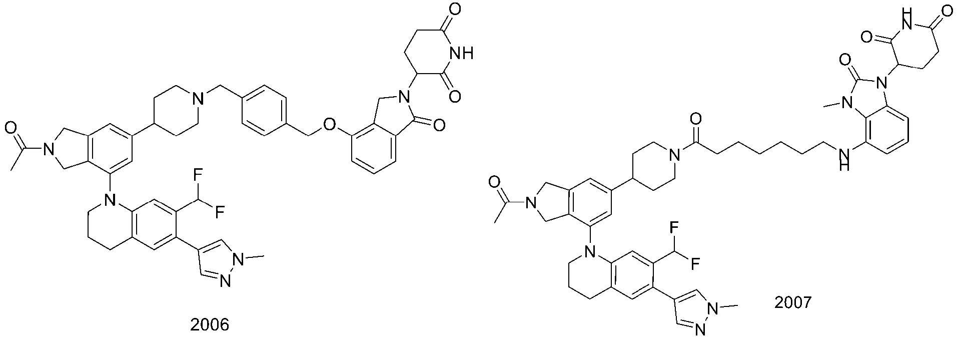

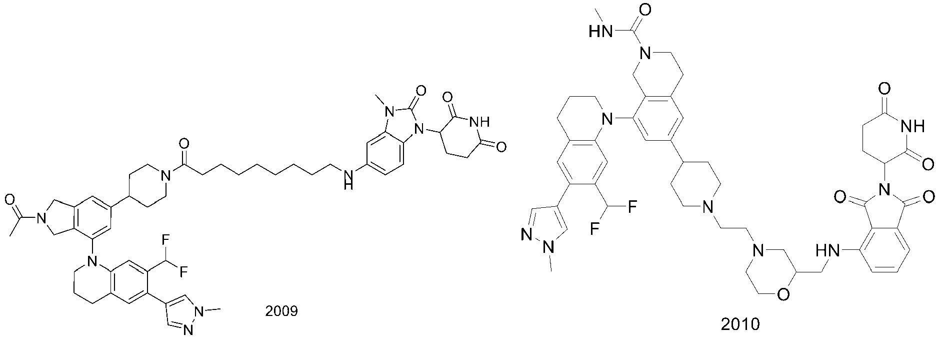

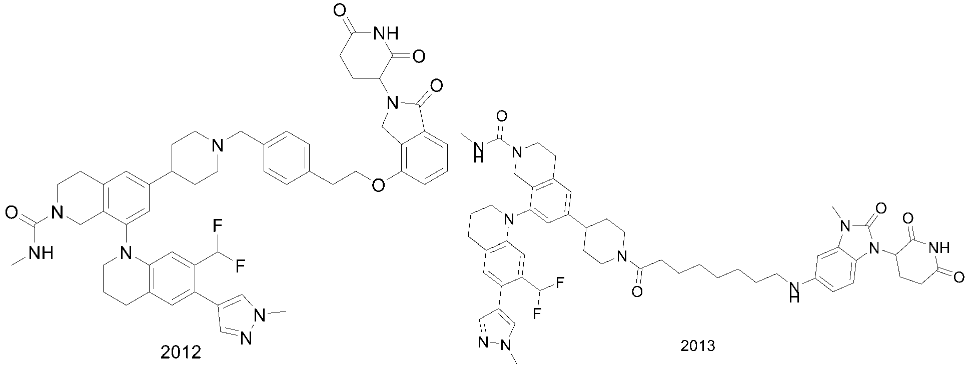

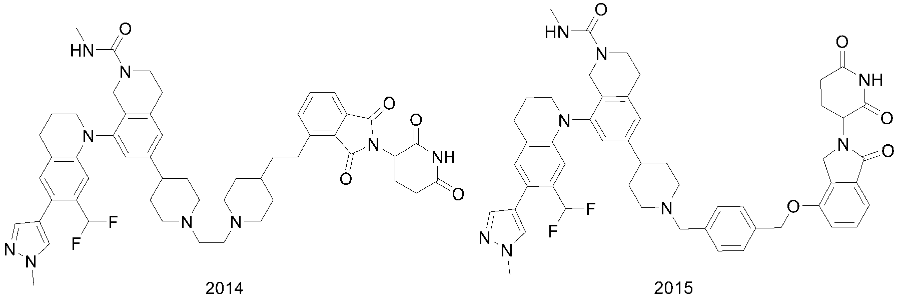

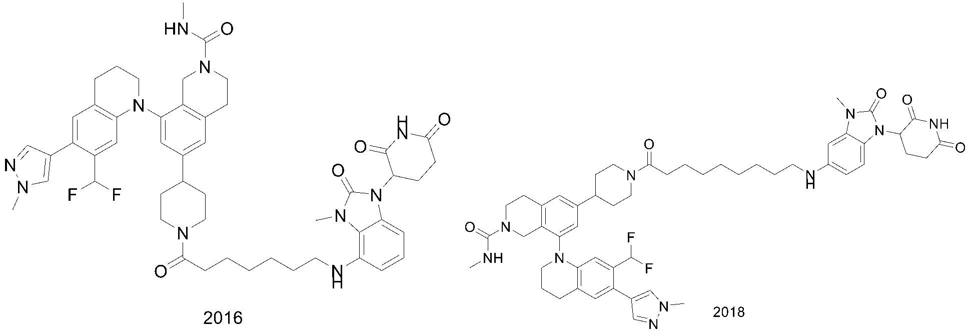

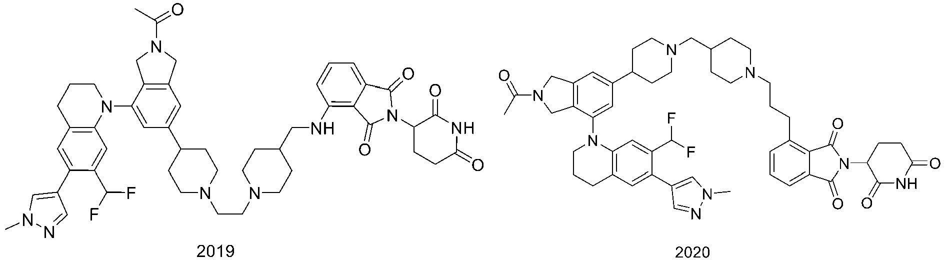

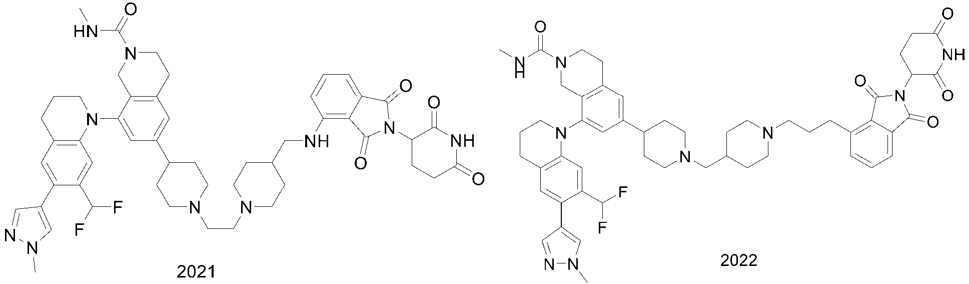

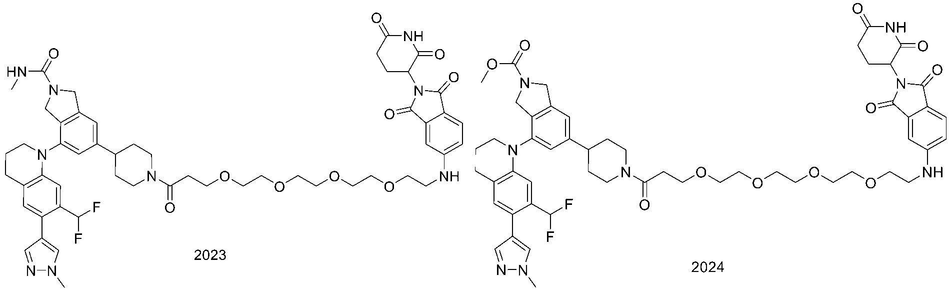

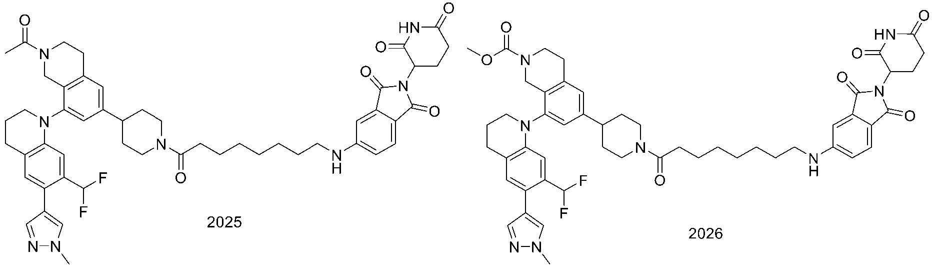

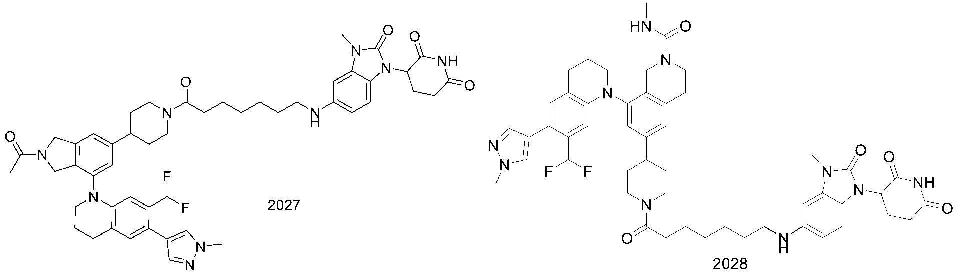

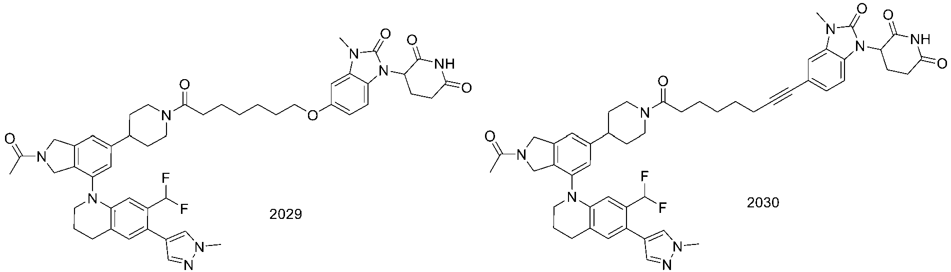

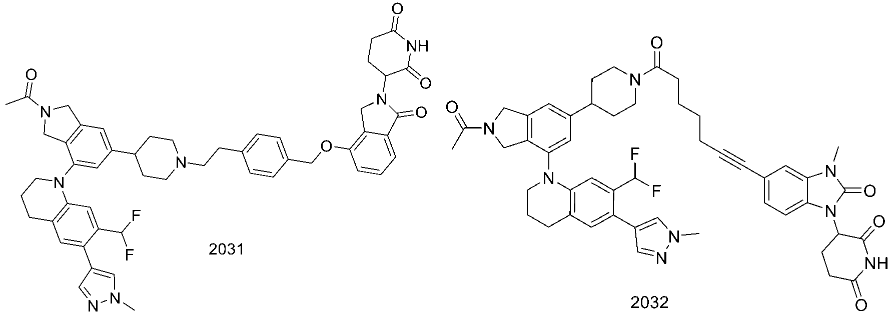

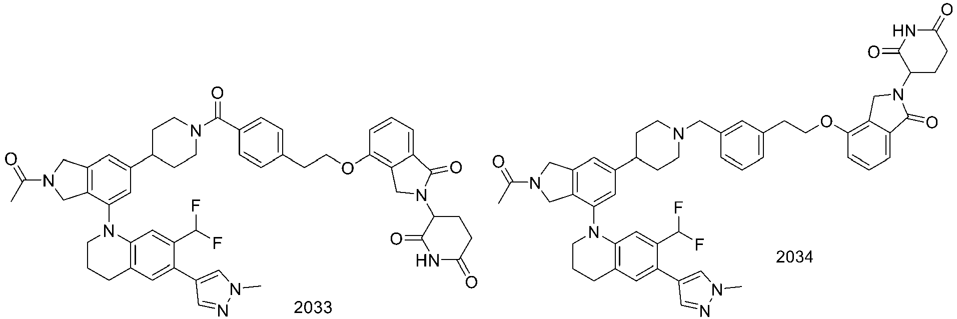

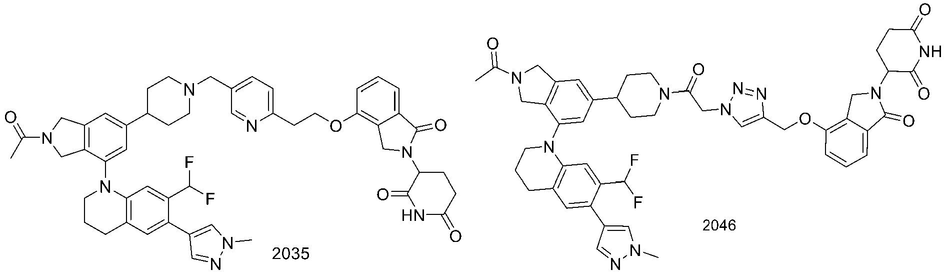

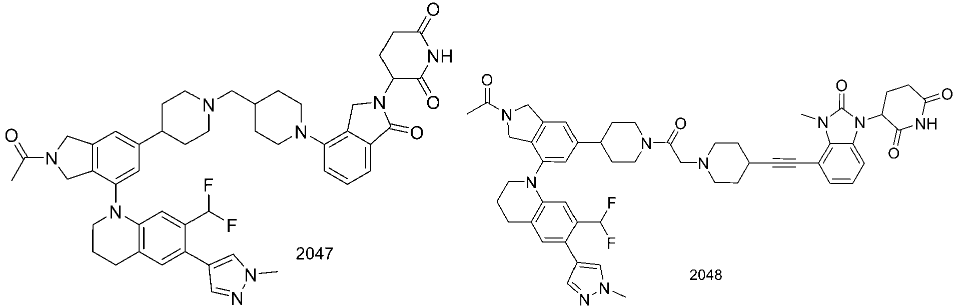

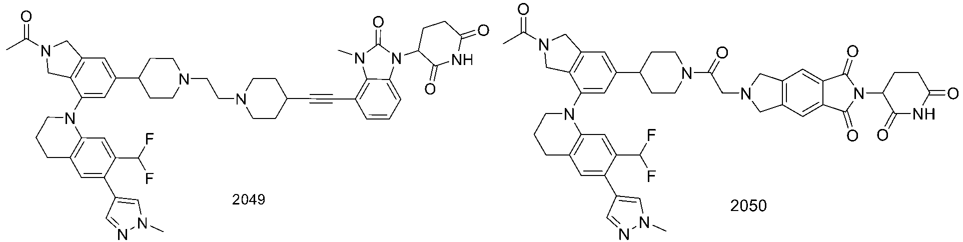

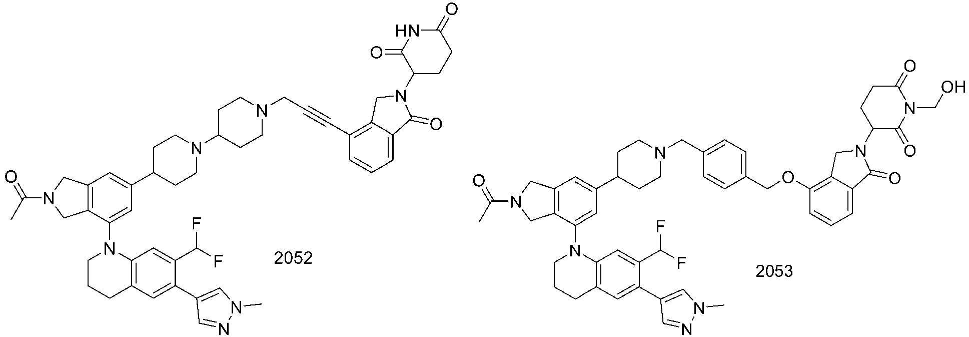

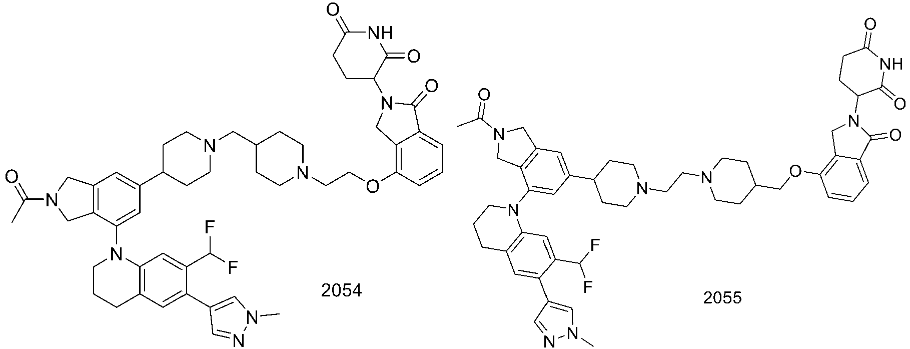

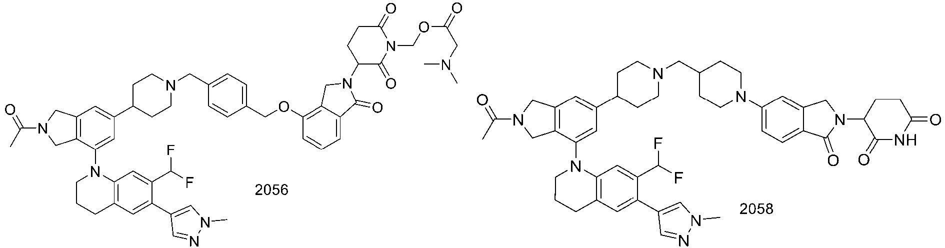

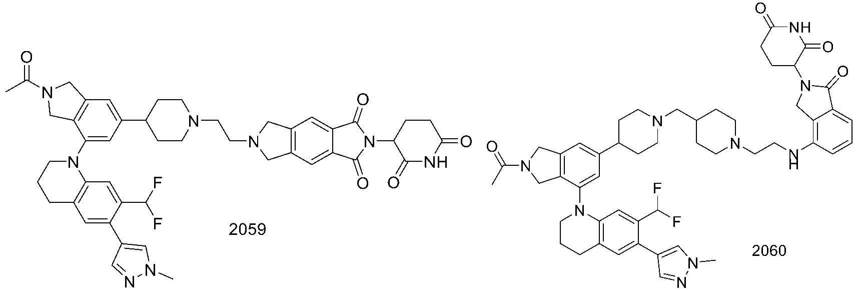

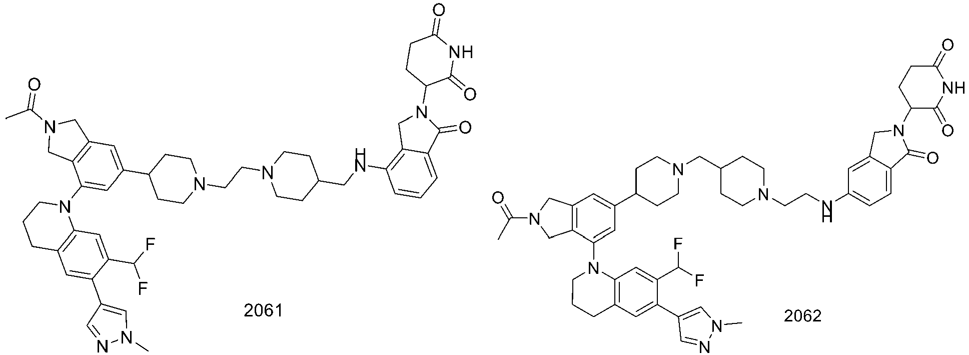

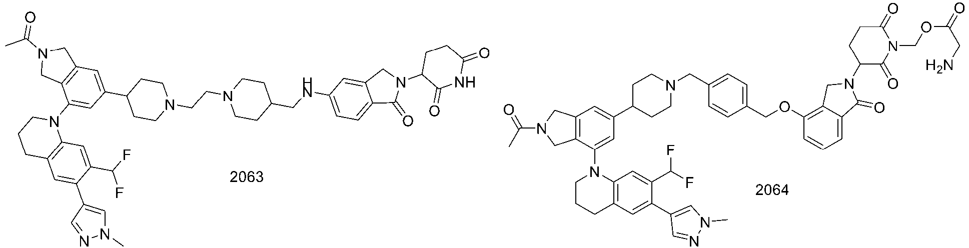

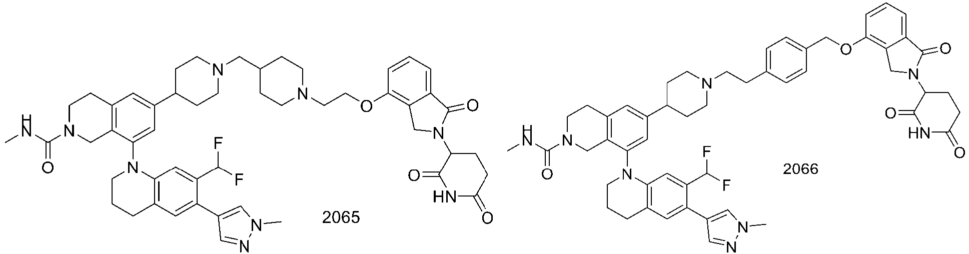

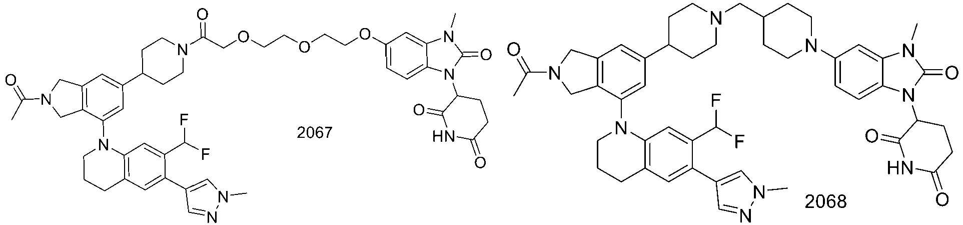

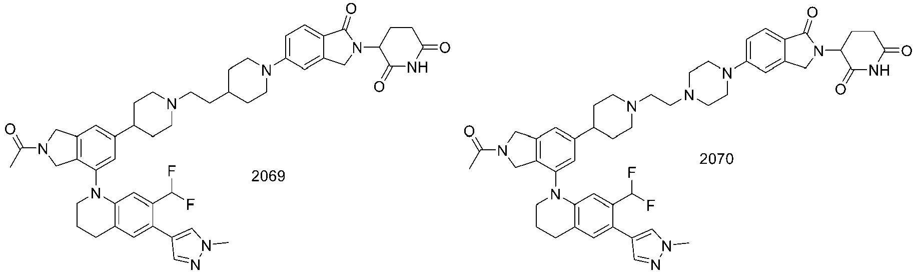

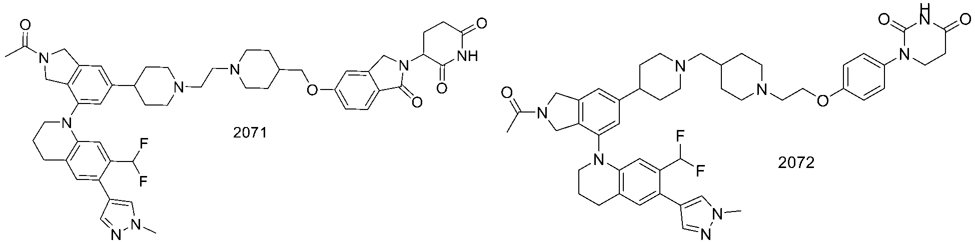

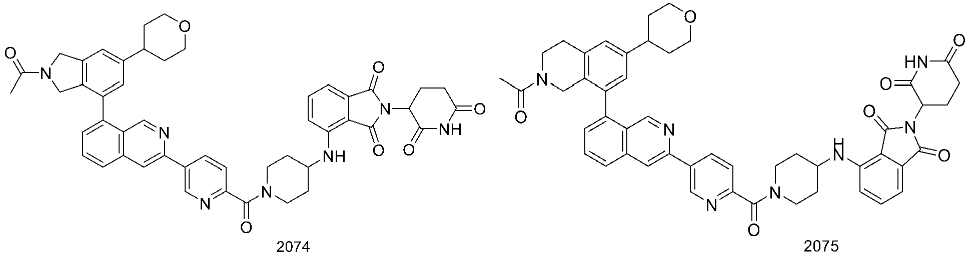

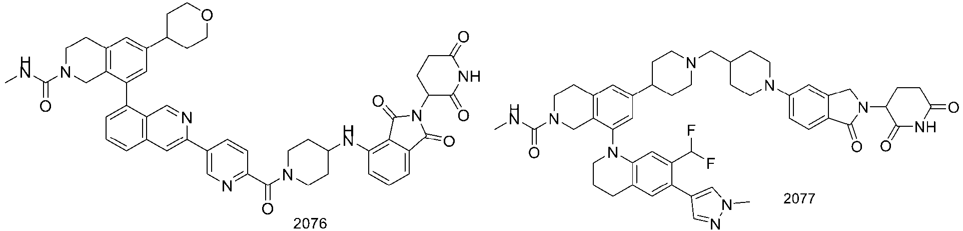

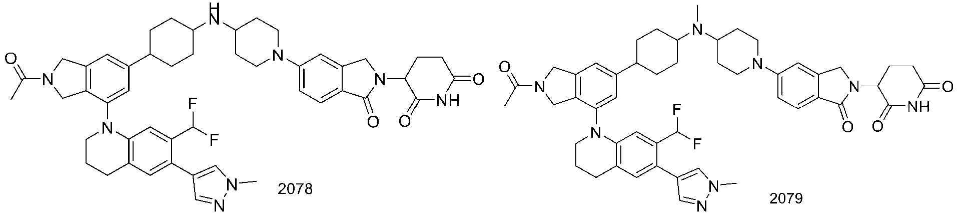

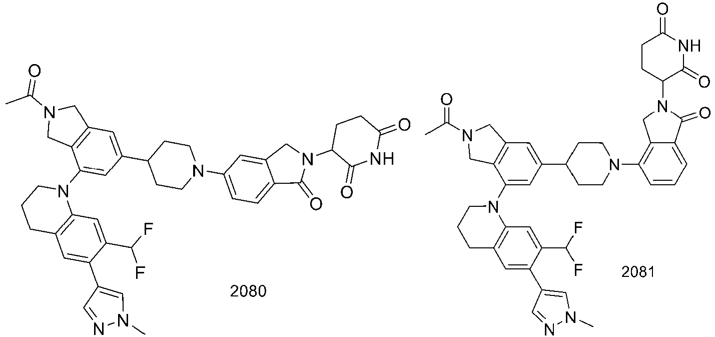

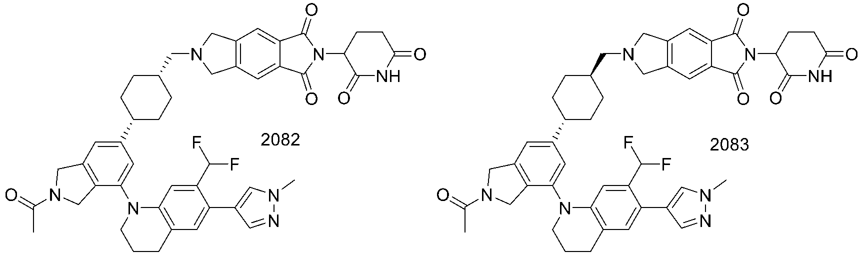

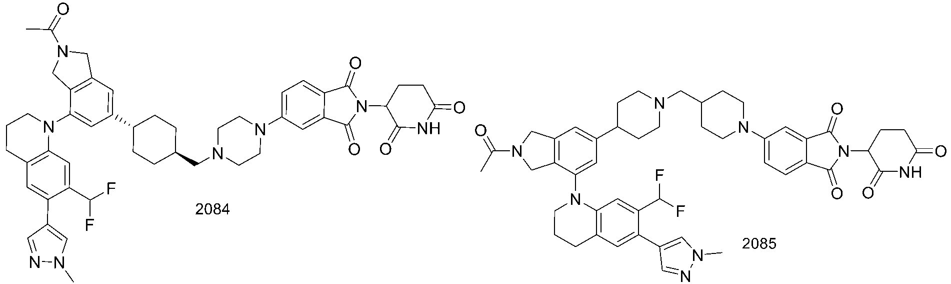

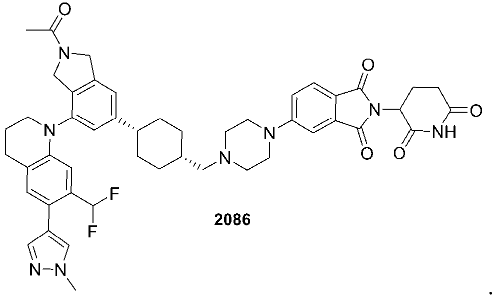

In another aspect, the present disclosure provides a pharmaceutical composition, comprising the compound represented by formula (III), and a racemate, a stereoisomer, a tautomer, an isotopic derivative, a N-oxide, a solvate, a polymorph, a metabolite, an ester, a prodrug, or a pharmaceutically acceptable salt thereof.

In yet another aspect, the present disclosure also provides the use of the compound represented by formula (III), and a racemate, a stereoisomer, a tautomer, an isotopic derivative, a N-oxide, a solvate, a polymorph, a metabolite, an ester, a prodrug, or a pharmaceutically acceptable salt thereof, and a pharmaceutical composition comprising the same, in the preparation of a medicine for preventing or treating an EP300 and/or CBP-mediated disease or condition.

According to an embodiment of the present disclosure, the preventing and/or treating an EP300 and/or CBP mediated disease or condition is achieved by degradation of EP300 and/or CBP proteins with the compound represented by formula (III), and a racemate, a stereoisomer, a tautomer, an isotopic derivative, a N-oxide, a solvate, a polymorph, a metabolite, an ester, a prodrug, or a pharmaceutically acceptable salt thereof, and a pharmaceutical composition comprising the same.

In yet another aspect, the present disclosure also provides a method for preventing and/or treating an EP300 and/or CBP-mediated disease or condition comprising administering to a patient in need thereof a therapeutically effective amount of the compound represented by formula (III), and a racemate, a stereoisomer, a tautomer, an isotopic derivative, a N-oxide, a solvate, a polymorph, a metabolite, an ester, a prodrug, or a pharmaceutically acceptable salt thereof, and a pharmaceutical composition comprising the same.

The EP300 and/or CBP-mediated disease or condition described in the present disclosure is selected from cancer, heart diseases, metabolic diseases, inflammatory diseases, fibrotic diseases, and viral infections. The cancer includes, but are not limited to, prostate cancer, breast cancer, bladder cancer, lung cancer, melanoma, colorectal cancer, gastric cancer, ovarian cancer, cervical cancer, bladder cancer, laryngeal cancer, multiple myeloma, liver cancer, lymphoma, and leukemia. The prostate cancer may be, for example, castration-resistant prostate cancer (CRPC). The lung cancer may be, for example, non-small cell lung cancer or small cell lung cancer. The lymphoma may be selected from non-Hodgkin's lymphoma, diffuse large B-cell lymphoma, and others.

The compounds of the present disclosure can be used in combination with other drugs or other therapies.

In some embodiments, the compounds described hereinbefore in the present disclosure are used in combination with radiation therapy.

In some embodiments, the present disclosure also provides the compounds in combination with immunoregulators for use in the treatment of diseases (e.g., multiple myeloma) associated with the EP300 and/or CBP-mediated disease or condition.

In some embodiments, the immunoregulator comprises tumor necrosis factor; interferons α, β, and γ; IL-2 and other cytokines; F42K and other cytokine analogs; or MIP-1, MIP-1β, MCP-1, RANTES, and other chemokines.

In some embodiments, the compounds described hereinbefore in the present disclosure are used in combination with a second therapeutic agent, which may be selected from drugs conventionally used for the treatment of cancer, heart diseases, metabolic diseases, inflammatory diseases, fibrotic diseases and viral infections. In some embodiments, the classes of the second therapeutic agent may include androgen receptor antagonists, such as enzalutamide, and inhibitors of CYP17A1 (17α-hydroxylase/C17,20-lyase), such as abiraterone; cytotoxic chemotherapeutic agents, such as docetaxel; the classes of therapeutic agents for the treatment of lung cancer include cytotoxic chemotherapeutic agents, such as cisplatin, carboplatin, docetaxel; the classes of therapeutic agents for the treatment of bladder cancer include cytotoxic chemotherapeutic agents, such as gemcitabine, cisplatin, or immunotherapy, such as Bacillus Calmette-Guérin (BCG). The classes of second therapeutic agent may further be selected from immune checkpoint inhibitors, such as pembrolizumab, nivolumab, atezolizumab, ipilimumab; PARP (poly(ADP-ribose) polymerase) inhibitors, such as olaparib; and CDK4/6 (cyclin-dependent kinase 4 and 6) inhibitors. In some embodiments, the second therapeutic agent may be selected for use in combination with KRAS inhibitors and the like for the treatment of various tumors such as lung cancer, rectal cancer, pancreatic cancer, liver cancer and hematological tumor.

Accordingly, the present disclosure provides a combined composition comprising a compound represented by formula III, or a racemate, a stereoisomer, a tautomer, an isotopic derivative, a N-oxide, a solvate, a polymorph, a metabolite, an ester, a prodrug, or a pharmaceutically acceptable salt thereof, and the second therapeutic agent.

The present disclosure also provides a method for the combined prevention and/or treatment of an EP300 and/or CBP-mediated disease or condition comprising administering to a patient in need thereof a therapeutically effective amount of the compound represented by formula (III), and a racemate, a stereoisomer, a tautomer, an isotopic derivative, a N-oxide, a solvate, a polymorph, a metabolite, an ester, a prodrug, or a pharmaceutically acceptable salt thereof, and a second therapeutic agent.

The pharmaceutically acceptable salts of the compounds of the present disclosure may be inorganic or organic salts. If such compounds possess a basic center, they may form acid addition salts; if such compounds possess an acidic center, they may form base addition salts; if such compounds contain both an acidic center (e.g., carboxyl group) and a basic center (e.g., amino group), they may also form internal salts.

The compounds of the present disclosure may exist in specific geometric or stereoisomeric forms. For example, cis and trans isomers, (-)- and (+)-enantiomers, (R)- and (S)-enantiomers, diastereoisomers, (D)-isomers, (L)-isomers, racemic mixtures and other mixtures, as well as enantiomerically or diastereomerically enriched mixtures, all such mixtures are within the scope of the present disclosure. Additional asymmetric carbon atoms may be present in substituents such as alkyl. All such isomers and mixtures thereof are included within the scope of the present disclosure.

The compounds and intermediates of the present disclosure may also exist in different tautomeric forms, and all such forms are encompassed within the scope of the present disclosure. "Tautomers" refer to structural isomers of different energies that are interconvertible via a low energy barrier. For example, proton tautomers (also known as proton transfer tautomers) include interconversions via migration of a proton, such as keto-enol isomerization, imine-enamine isomerization, and lactam-lactim isomerization. All tautomeric forms of all compounds in the present disclosure are within the scope of the present disclosure. The name of a compound named in a single manner does not exclude any tautomer.

The present disclosure also encompasses isotopically labeled compounds of the present disclosure that have the same structures as those described herein, but in which one or more atoms are replaced by atoms having an atomic weight or mass number different from that ordinarily found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl etc., respectively. All changes in isotopic composition of the compounds of the present disclosure, whether radioactive or not, are included within the scope of the present disclosure.

Unless otherwise indicated, when a position is specifically designated as deuterium (D), such position is understood to have deuterium at an abundance that is at least 1000 times greater than the natural abundance of deuterium (which is 0.015%) (i.e., at least 10% deuterium incorporation). The compounds in the examples may have deuterium at an abundance of at least 1000 times, at least 2000 times, at least 3000 times, at least 4000 times, at least 5000 times, at least 6000 times, or higher than the natural abundance of deuterium. Each available hydrogen atom attached to a carbon atom may be independently replaced by a deuterium atom. Those skilled in the art can synthesize deuterated forms of the compounds by referring to the relevant literature. Commercially available deuterated starting materials may be used in preparing deuterated forms of the compounds, or they may be synthesized using conventional techniques with deuterated reagents including, but not limited to, deuterated borane, tri-deuterate borane in tetrahydrofuran, deuterated lithium aluminum hydride, deuterated iodoethane, deuterated iodomethane, and the like.

The term "EP300- and/or CBP-mediated disease or condition" as used in the present disclosure refers to a disease or condition in which the biological function of EP300, CBP, or both EP300 and CBP affects the development and/or progression of the disease or condition, and/or in which modulation of EP300, CBP, or both EP300 and CBP alters the development, progression, and/or symptoms. EP300- or CBP-mediated diseases or conditions include those for which inhibition of EP300, inhibition of CBP, or inhibition of both EP300 and CBP provides a therapeutic benefit, for example, wherein treatment with an EP300 or CBP inhibitor (including the compounds described herein) provides a therapeutic benefit to a subject suffering from or at risk of the disease or condition. EP300- or CBP-mediated diseases or conditions are intended to include cancers having loss-of-function mutations in CBP or EP300, or cancers in which EP300 or CBP activation is present. EP300- or CBP-mediated diseases or conditions are also intended to include androgen receptor-expressing cancers.

A "therapeutically effective amount" of the present disclosure refers to an amount of an active compound or pharmaceutical agent that elicits a biological or medical response in a tissue, system, animal, individual, or human that is sought by a researcher, veterinarian, physician, or other clinician, and includes one or more of the following: (1) preventing a disease: for example, preventing disease, disorder, or condition in an individual who is susceptible to the disease, disorder, or condition but has not yet experienced or developed the pathology or symptoms of the disease; (2) inhibiting a disease: for example, inhibiting disease, disorder, or condition in an individual who is experiencing or exhibiting the pathology or symptoms of the disease, disorder, or condition (i.e., preventing further development of the pathology and/or symptoms); (3) alleviating a disease: for example, alleviating disease, disorder, or condition in an individual who is experiencing or exhibiting the pathology or symptoms of the disease, disorder, or condition (i.e., reversing the pathology and/or symptoms). With respect to a drug or pharmacologically active agent, a "therapeutically effective amount" refers to a sufficient amount of the drug or agent that is non-toxic but achieves the desired effect. The determination of an effective amount varies from person to person, depending on the age and general condition of the recipient as well as the particular active substance. An appropriate effective amount in an individual case may be determined by those skilled in the art through routine experimentation.

### Beneficial effects

The present disclosure provides a class of novel compounds having the structure represented by formula (III), which exhibit favorable EP300/CBP regulatory activities (e.g., inhibitory or degradation activities) and promising pharmaceutical prospects.

### BRIEF DESCRIPTION OF THE DRAWINGS

Figure 1 shows the degradation assay of the target protein P300 by the compound of the present disclosure.
Figure 2 shows the degradation assay of the target protein and its corresponding downstream proteins by the compound of the present disclosure.

### Definition and Explanation of Terms

Unless otherwise specified, the definitions of groups and terms described in the description and claims of the present application, including definitions thereof as examples, exemplary definitions, preferred definitions, definitions recited in tables, definitions of specific compounds in the examples, and the like, may be arbitrarily combined and coupled with each other. Such combined group definitions and compound structures shall be understood to fall within the scope recited in the specification and/or claims of the present application.

Unless stated to the contrary, the terms used in the description and claims have the following meanings.

The term "alkyl" refers to a saturated aliphatic hydrocarbon group, which is a straight or branched chain group containing 1 to 12 carbon atoms (e.g., 1, 2, 3, 4, 5, 6, 7, 8, 9, 10, 11, and 12), preferably an alkyl group containing 1 to 6 carbon atoms (C₁₋₆ alkyl). Non-limiting examples of alkyl include methyl, ethyl, n-propyl, isopropyl, n-butyl, isobutyl, *tert*-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. Alkyl may be substituted or unsubstituted.

The term "alkenyl" should be understood to preferably denote a straight or branched monovalent hydrocarbon group containing one or more double bonds and having 2, 3, 4, 5, 6, 7, 8, 9, 10, 11, or 12 carbon atoms (C₂₋₁₂ alkenyl), more preferably "C₂₋₈ alkenyl". "C₂₋₈ alkenyl" should be understood to preferably denote a straight or branched monovalent hydrocarbon group containing one or more double bonds and having 2, 3, 4, 5, 6, 7, or 8 carbon atoms, e.g., having 2, 3, 4, 5, or 6 carbon atoms (i.e., C₂₋₆ alkenyl), or having 2 or 3 carbon atoms (i.e., C₂₋₃ alkenyl). It is understood that where the alkenyl contains more than one double bond, the double bonds may be isolated or conjugated to each other. The alkenyl is, for example, ethenyl, allyl, (E)-2-methylethenyl, (Z)-2-methylethenyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylethenyl, 1-isopropylethenyl.

The term "alkynyl" should be understood to preferably denote a straight or branched monovalent hydrocarbon group containing one or more triple bonds and having 2, 3, 4, 5, 6, 7, 8, 9, 10, 11, or 12 carbon atoms (C₂₋₁₂ alkynyl), e.g., having 2, 3, 4, 5, 6, 7, or 8 carbon atoms (i.e., "C₂₋₈ alkynyl"), having 2, 3, 4, 5, or 6 carbon atoms (i.e., "C₂₋₆ alkynyl"), or having 2 or 3 carbon atoms ("C₂₋₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "alkoxy" refers to -O-(alkyl), wherein alkyl is as defined herein. Non-limiting examples of alkoxy include methoxy, ethoxy, propoxy, and butoxy. Alkoxy may be substituted or unsubstituted.

The term "cycloalkyl" refers to a saturated or partially unsaturated monocyclic or polycyclic cyclic hydrocarbon substituent. The cycloalkyl ring preferably contains 3 to 12 or 3 to 8 (e.g., 3, 4, 5, 6, 7, and 8) carbon atoms, more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl includes spiro, fused, and bridged cycloalkyl groups.

The term "spirocycloalkyl" refers to a polycyclic group sharing one carbon atom (referred to as a spiro atom) between individual rings in the system, which may contain one or more double bonds. It is preferably a 6- to 12-membered ring, more preferably a 7- to 10-membered ring (e.g., 7-, 8-, 9- or 10-membered). Spirocycloalkyl groups are classified as monospirocycloalkyl, dispirocycloalkyl or polyspirocycloalkyl according to the number of spiro atoms shared between rings, with monospirocycloalkyl and dispirocycloalkyl being preferred. More preferred are 3-/5-membered, 3-/6-membered, 4-/4-membered, 4-/5-membered, 4-/6-membered, 5-/5-membered or 5-/6-membered monospirocycloalkyl groups. Non-limiting examples of spirocycloalkyl include:

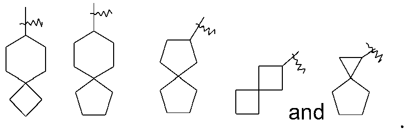

The term "fused cycloalkyl" refers to an all-carbon polycyclic group in which each ring in the system shares an adjacent pair of carbon atoms with other rings in the system, where one or more rings may contain one or more double bonds. It is preferably a 6- to 12-membered ring, more preferably a 7- to 10-membered ring (e.g., 7-, 8-, 9- or 10-membered). Fused cycloalkyl groups can be classified as bicyclic, tricyclic, tetracyclic or polycyclic fused cycloalkyl groups according to the number of constituent rings, preferably bicyclic or tricyclic, more preferably 3-/4-membered, 3-/5-membered, 3-/6-membered, 4-/4-membered, 4-/5-membered, 4-/6-membered, 5-/4-membered, 5-/5-membered, 5-/6-membered, 6-/3-membered, 6-/4-membered, 6-/5-membered and 6-/6-membered bicycloalkyl groups. Non-limiting examples of fused cycloalkyl include:

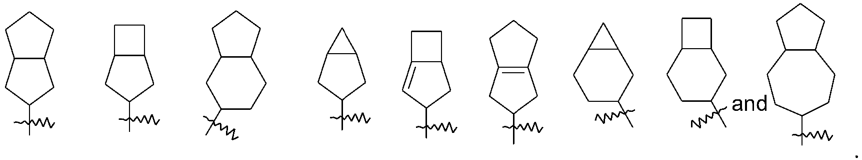

The term "bridged cycloalkyl" refers to an all-carbon polycyclic group in which any two rings share two carbon atoms that are not directly bonded to each other, which may contain one or more double bonds. It is preferably a 6- to 12-membered ring, more preferably a 7- to 10-membered ring (e.g., 7-, 8-, 9- or 10-membered). Bridged cycloalkyl groups can be classified as bicyclic, tricyclic, tetracyclic or polycyclic bridged cycloalkyl groups according to the number of constituent rings, preferably bicyclic, tricyclic or tetracyclic, and more preferably bicyclic or tricyclic. Non-limiting examples of bridged cycloalkyl include:

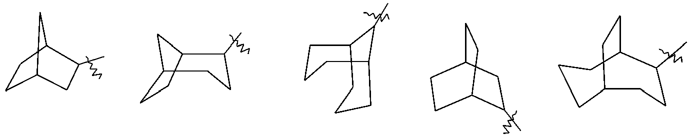

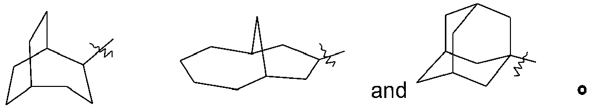

The cycloalkyl ring includes a cycloalkyl as described herein (including monocyclic, spiro, fused and bridged rings) fused to an aryl, heteroaryl or heterocycloalkyl ring, wherein the ring attached to the parent structure is a cycloalkyl. Non-limiting examples include: 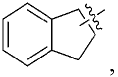 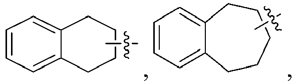 and the like; preferably 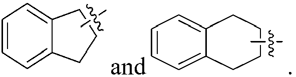 The cycloalkyl may be substituted or unsubstituted.

The term "heterocyclyl" refers to a saturated or partially unsaturated monocyclic or polycyclic cyclic substituent containing 3 to 14 ring atoms, wherein one or more ring atoms are heteroatoms selected from nitrogen, oxygen and sulfur, and the sulfur may be optionally oxo (i.e., forming a sulfoxide or sulfone), but excluding ring moieties of -O-O-, -O-S- or -S-S-, with the remaining ring atoms being carbon. Preferably, it contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably, it contains 3 to 8 ring atoms (e.g., 3, 4, 5, 6, 7 and 8), of which 1 to 3 (e.g., 1, 2 and 3) are heteroatoms; more preferably, it contains 3 to 6 ring atoms, of which 1 to 3 are heteroatoms; most preferably, it contains 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl and the like. Polycyclic heterocyclyl includes spiro, fused and bridged heterocyclyl groups.

The term "spiroheterocyclyl" refers to a polycyclic heterocyclic group sharing one atom (referred to as a spiro atom) between individual monocyclic rings in the system, wherein one or more ring atoms are heteroatoms selected from nitrogen, oxygen and sulfur, and the sulfur may be optionally oxo (i.e., forming a sulfoxide or sulfone), with the remaining ring atoms being carbon. It may contain one or more double bonds. It is preferably a 6- to 14-membered ring, more preferably a 7- to 10-membered ring (e.g., 7-, 8-, 9- or 10-membered). Spiroheterocyclyl groups are classified as monospiroheterocyclyl, dispiroheterocyclyl or polyspiroheterocyclyl according to the number of spiro atoms shared between rings, with monospiroheterocyclyl and dispiroheterocyclyl being preferred. More preferred are 3-/5-membered, 3-/6-membered, 4-/4-membered, 4-/5-membered, 4-/6-membered, 5-/5-membered or 5-/6-membered monospiroheterocyclyl groups. Non-limiting examples of spiroheterocyclyl include:

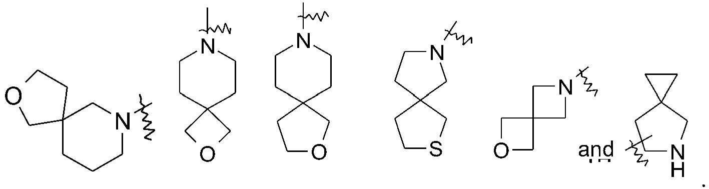

The term "fused heterocyclyl" refers to a polycyclic heterocyclic group in which each ring in the system shares an adjacent pair of atoms with other rings in the system. One or more rings may contain one or more double bonds, wherein one or more ring atoms are heteroatoms selected from nitrogen, oxygen and sulfur, and the sulfur may be optionally oxo (i.e., forming a sulfoxide or sulfone), with the remaining ring atoms being carbon. It is preferably a 6- to 14-membered ring, more preferably a 7- to 10-membered ring (e.g., 7-, 8-, 9- or 10-membered). Fused heterocyclyl groups can be classified as bicyclic, tricyclic, tetracyclic or polycyclic fused heterocyclyl groups according to the number of constituent rings, preferably bicyclic or tricyclic, and more preferably 3-/4-membered, 3-/5-membered, 3-/6-membered, 4-/4-membered, 4-/5-membered, 4-/6-membered, 5-/4-membered, 5-/5-membered, 5-/6-membered, 6-/3-membered, 6-/4-membered, 6-/5-membered and 6-/6-membered bicyclic fused heterocyclyl groups. Non-limiting examples of fused heterocyclyl include:

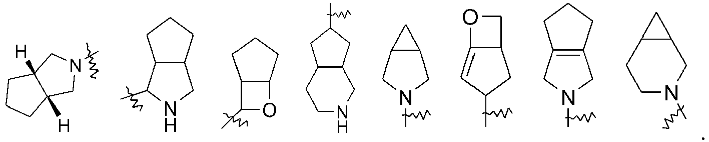

The term "bridged heterocyclyl" refers to a polycyclic heterocyclic group in which any two rings share two atoms that are not directly bonded to each other. It may contain one or more double bonds, wherein one or more ring atoms are heteroatoms selected from nitrogen, oxygen and sulfur, and the sulfur may be optionally oxo (i.e., forming a sulfoxide or sulfone), with the remaining ring atoms being carbon. It is preferably a 6- to 14-membered ring, more preferably a 7- to 10-membered ring (e.g., 7-, 8-, 9- or 10-membered). Bridged heterocyclyl groups can be classified as bicyclic, tricyclic, tetracyclic or polycyclic bridged heterocyclyl groups according to the number of constituent rings, preferably bicyclic, tricyclic or tetracyclic, and more preferably bicyclic or tricyclic. Non-limiting examples of bridged heterocyclic groups include:

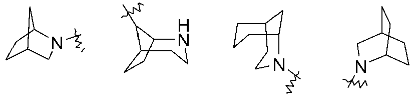

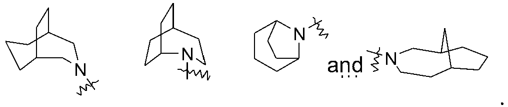

The heterocyclyl ring includes a heterocyclyl as described herein (including monocyclic, spiro heterocyclic, fused heterocyclic and bridged heterocyclic) fused to an aryl, heteroaryl or cycloalkyl ring, wherein the ring attached to the parent structure is a heterocyclyl. Non-limiting examples thereof include: 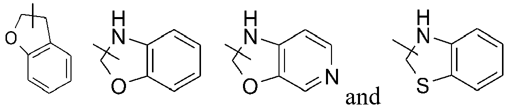 etc. The heterocyclyl may be substituted or unsubstituted.

The term "aryl" refers to a 6- to 14-membered all-carbon monocyclic or fused polycyclic group (fused polycyclic rings are rings sharing adjacent pairs of carbon atoms) having a conjugated π-electron system, preferably a 6- to 10-membered ring, such as phenyl and naphthyl. The aryl ring includes an aryl ring as described herein fused to a heteroaryl, heterocyclyl or cycloalkyl ring, wherein the ring attached to the parent structure is an aryl ring. Non-limiting examples thereof include: 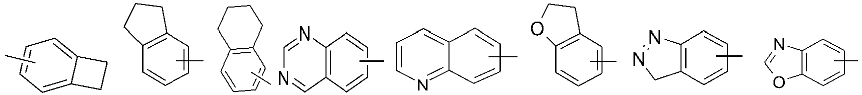 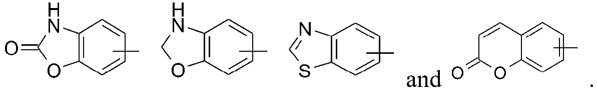 Aryl may be substituted or unsubstituted.

The term "heteroaryl" refers to a heteroaromatic system containing 1 to 4 (e.g., 1, 2, 3 and 4) heteroatoms and 5 to 14 ring atoms, wherein the heteroatoms are selected from oxygen, sulfur and nitrogen. Heteroaryl is preferably a 5- to 10-membered ring (e.g., 5-, 6-, 7-, 8-, 9- or 10-membered), more preferably a 5- or 6-membered ring, such as furyl, thienyl, pyridyl, pyrrolyl, N-alkylpyrrolyl, pyrimidinyl, pyrazinyl, pyridazinyl, imidazolyl, pyrazolyl, triazolyl, tetrazolyl and the like. The heteroaryl ring includes a heteroaryl ring as described herein fused to an aryl, heterocyclyl or cycloalkyl ring, wherein the ring attached to the parent structure is a heteroaryl ring. Non-limiting examples thereof include: 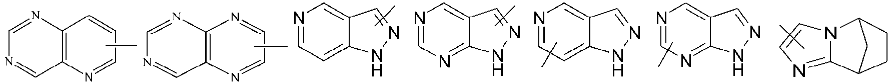 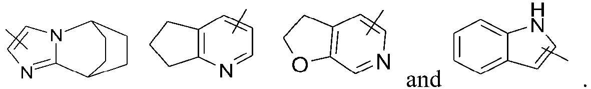 Heteroaryl may be substituted or unsubstituted.

Those skilled in the art will appreciate that the cycloalkyl, heterocyclyl, aryl, and heteroaryl mentioned above include residues derived by removing one hydrogen atom from a parent ring atom, or residues derived by removing two hydrogen atoms from the same or two different ring atoms of the parent ring, namely "divalent cycloalkyl", "divalent heterocyclyl", "arylene", and "heteroarylene". Those skilled in the art will understand that when a linking group is clearly required in a compound structure, the Markush variables listed for this group should be understood as linking groups. For example, if the structure requires a linking group and the definition of the Markush group for this variable lists "alkyl" or "aryl", it should be understood that such "alkyl" or "aryl" represents a linking alkylene group or arylene group, respectively. Therefore, when used as a linking group, the suffix "-yl" is equivalent to "-ylene". For instance, "alkyl" is equivalent to "alkylene", and "aryl" is equivalent to "arylene".

The term "halogen" means F, Cl, Br or I.

"Optional" or "optionally" means that the subsequently described event or circumstance may, but does not necessarily, occur, and includes instances where the event or circumstance occurs and instances where it does not. For example, "heterocycloalkyl optionally substituted with alkyl" means that alkyl may, but is not required to, be present, and includes the situation where the heterocycloalkyl is substituted with alkyl and the situation where the heterocycloalkyl is not substituted with alkyl.

The term "more" includes three, four, five and more.

The pharmaceutically acceptable salts of the compounds of the present disclosure may be inorganic or organic salts. If such compounds possess a basic center, they may form acid addition salts; if such compounds possess an acidic center, they may form base addition salts; if such compounds contain both an acidic center (e.g., carboxyl group) and a basic center (e.g., amino group), they may also form internal salts.

The compounds of the present disclosure may exist in specific geometric or stereoisomeric forms. For example, cis and trans isomers, (-)- and (+)-enantiomers, (R)- and (S)-enantiomers, diastereoisomers, (D)-isomers, (L)-isomers, racemic mixtures and other mixtures, as well as enantiomerically or diastereomerically enriched mixtures, all such mixtures are within the scope of the present disclosure. Additional asymmetric carbon atoms may be present in substituents such as alkyl. All such isomers and mixtures thereof are included within the scope of the present disclosure.

The compounds and intermediates of the present disclosure may also exist in different tautomeric forms, and all such forms are encompassed within the scope of the present disclosure. "Tautomers" refer to structural isomers of different energies that are interconvertible via a low energy barrier. For example, proton tautomers (also known as proton transfer tautomers) include interconversions via migration of a proton, such as keto-enol isomerization, imine-enamine isomerization, and lactam-lactim isomerization. All tautomeric forms of all compounds in the

present disclosure are within the scope of the present disclosure. The name of a compound named in a single manner does not exclude any tautomer.

The present disclosure also encompasses isotopically labeled compounds of the present disclosure that have the same structures as those described herein, but in which one or more atoms are replaced by atoms having an atomic weight or mass number different from that ordinarily found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl etc., respectively. All changes in isotopic composition of the compounds of the present disclosure, whether radioactive or not, are included within the scope of the present disclosure.

Unless otherwise indicated, when a position is specifically designated as deuterium (D), such position is understood to have deuterium at an abundance that is at least 1000 times greater than the natural abundance of deuterium (which is 0.015%) (i.e., at least 10% deuterium incorporation). The compounds in the examples may have deuterium at an abundance of at least 1000 times, at least 2000 times, at least 3000 times, at least 4000 times, at least 5000 times, at least 6000 times, or higher than the natural abundance of deuterium. Each available hydrogen atom attached to a carbon atom may be independently replaced by a deuterium atom. Those skilled in the art can synthesize deuterated forms of the compounds by referring to the relevant literature. Commercially available deuterated starting materials may be used in preparing deuterated forms of the compounds, or they may be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

The term "regulator" as used in the present disclosure refers to a compound that alters (i.e., increases or decreases) the activity of a target biomolecule, such as an enzyme. Typically, a regulator will be a small molecule.

The term "modulate" refers to the ability of a compound to increase or decrease the function and/or expression of a target (such as EP300 or CBP), wherein such function may include transcriptional regulatory activity and/or binding. Modulation can occur in vitro or in vivo. As used herein, modulation includes direct or indirect inhibition, antagonism, partial antagonism, degradation, activation, agonism, or partial agonism of a function or characteristic associated with EP300 or CBP, and/or direct or indirect upregulation or downregulation of EP300 or CBP expression. In another embodiment, modulation is direct. An inhibitor, antagonist, or degrader is a compound that (e.g., binds to) partially or fully block stimulation, reduce, prevent, inhibit, delay activation, inactivate, desensitize, or downregulate signal transduction. An activator or agonist is a compound that (e.g., binds to) stimulate, increase, turn on, activate, promote, enhance activation, activate, sensitize, or upregulate signal transduction. Accordingly, "EP300 & CBP regulators" may further encompass "EP300 & CBP inhibitors", "EP300 & CBP degraders", and "EP300 & CBP agonists".

The term "EP300- and/or CBP-mediated disease or condition" as used in the present disclosure refers to a disease or condition in which the biological function of EP300, CBP, or both EP300 and CBP affects the development and/or progression of the disease or condition, and/or in which modulation of EP300, CBP, or both EP300 and CBP alters the development, progression, and/or symptoms. EP300- or CBP-mediated diseases or conditions include those for which inhibition of EP300, inhibition of CBP, or inhibition of both EP300 and CBP provides a therapeutic benefit, for example, wherein treatment with an EP300 or CBP inhibitor (including the compounds described herein) provides a therapeutic benefit to a subject suffering from or at risk of the disease or condition. EP300- or CBP-mediated diseases or conditions are intended to include cancers having loss-of-function mutations in EP300 or CBP, or cancers in which EP300 or CBP activation is present. EP300- or CBP-mediated diseases or conditions are also intended to include androgen receptor-expressing cancers.

A "therapeutically effective amount" of the present disclosure refers to an amount of an active compound or pharmaceutical agent that elicits a biological or medical response in a tissue, system, animal, individual, or human that is sought by a researcher, veterinarian, physician, or other clinician, and includes one or more of the following: (1) preventing a disease: for example, preventing disease, disorder, or condition in an individual who is susceptible to the disease, disorder, or condition but has not yet experienced or developed the pathology or symptoms of the disease; (2) inhibiting a disease: for example, inhibiting disease, disorder, or condition in an individual who is experiencing or exhibiting the pathology or symptoms of the disease, disorder, or condition (i.e., preventing further development of the pathology and/or symptoms); (3) alleviating a disease: for example, alleviating a disease, disorder, or condition in an individual who is experiencing or exhibiting the pathology or symptoms of the disease, disorder, or condition (i.e., reversing the pathology and/or symptoms). With respect to a drug or pharmacologically active agent, a "therapeutically effective amount" refers to a sufficient amount of the drug or agent that is non-toxic but achieves the desired effect. The determination of an effective amount varies from person to person, depending on the age and general condition of the recipient as well as the particular active substance. An appropriate effective amount in an individual case may be determined by those skilled in the art through routine experimentation.

### DETAILED DESCRIPTION OF THE EMBODIMENTS

The technical solutions of the present disclosure will be described in further detail below with reference to specific examples. It should be understood that the following examples are only for illustrating and explaining the present disclosure exemplarily, and shall not be construed as limiting the protection scope of the present disclosure. All technologies implemented based on the foregoing content of the present disclosure fall within the scope intended to be protected by the present disclosure.

Unless otherwise stated, the raw materials and reagents used in the following examples are all commercially available products or can be prepared by known methods.

### Example 1-1

### Synthesis of 7-(difluoromethyl)-6-(1-methyl-1H-pyrazol-4-yl)-1,2,3,4-tetrahydroquinoline:

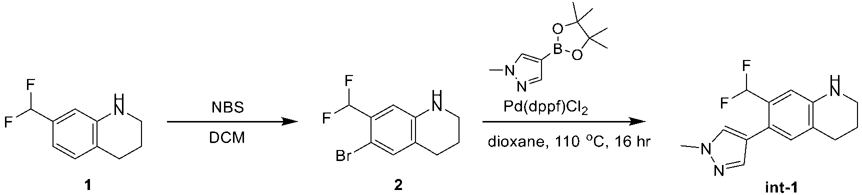

To a solution of 7-(difluoromethyl)-1,2,3,4-tetrahydroquinoline (18 g, 98.2 mmol, 1 eq) in DCM (180 mL) was added NBS (17.5 g, 98.2 mmol, 1 eq) at 0 °C. The mixture was stirred at 25 °C for 16 h. The reaction mixture was quenched by addition of ice water (200 mL), extracted with DCM (150 mL × 2), dried over Na₂SO₄, filtered, and concentrated under reduced pressure. The residue was purified by column chromatography (petroleum ether/EtOAc = 0 to 10%) to afford 6-bromo-7-(difluoromethyl)-1,2,3,4-tetrahydroquinoline (16 g, 61.1 mmol, 62.1% yield) as a yellow solid.

MS (ESI): C₁₀H₁₁F₂N; found 262.6 (M+H)⁺.

¹H NMR (400MHz, CDCl₃) δ = 7.18 (s, 1H), 7.04 (s, 1H), 6.63 (s, 1H), 4.09 - 3.82 (m, 1H), 3.28 - 3.18 (m, 2H), 2.66 (br t, J=6.3 Hz, 2H), 1.88 - 1.79 (m, 2H)

A mixture of 6-bromo-7-(difluoromethyl)-1,2,3,4-tetrahydroquinoline (11 g, 41.9 mmol, 1 eq), 1-methyl-4-(4,4,5,5-tetramethyl-1,3,2-dioxaborolan-2-yl)-pyrazole (8.7 g, 41.9 mmol, 1 eq), cyclopentyl(diphenyl)phosphine; palladium dichloride; a mixture of iron (3.0g, 4.2mmol, 0.1 eq) and K₂CO₃ (11.6g, 83.9mmol, 2 eq) in dioxane (80 mL) and H₂O (20 mL) were stirred at 110 °C under N₂ atmosphere for 12 h. The reaction mixture was concentrated under reduced pressure, and the residue was purified by column chromatography (petroleum ether/EtOAc = 0 to 40%) to afford 7-(difluoromethyl)-6-(1-methyl-1H-pyrazol-4-yl)-1,2,3,4-tetrahydroquinoline (Compound Int-1, 10 g, 37.9 mmol, 90.5% yield) as a brown solid.

MS (ESI): C₁₄H₁₅F₂N₃; found 263.9 (M+H)⁺.
¹H NMR (400MHz, DMSO-d6) δ = 7.68 (s, 1H), 7.43 (s, 1H), 6.90 (s, 1H), 6.73 (s, 1H), 6.01 (s, 1H), 3.84 (s, 3H), 3.24 - 3.14 (m, 2H), 2.68 (br t, J=6.0 Hz, 2H), 1.79 (quin, J=5.9 Hz, 2H)

### Examples 1-2

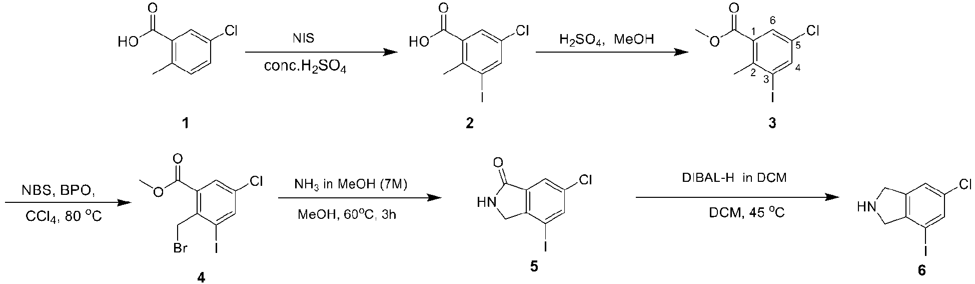

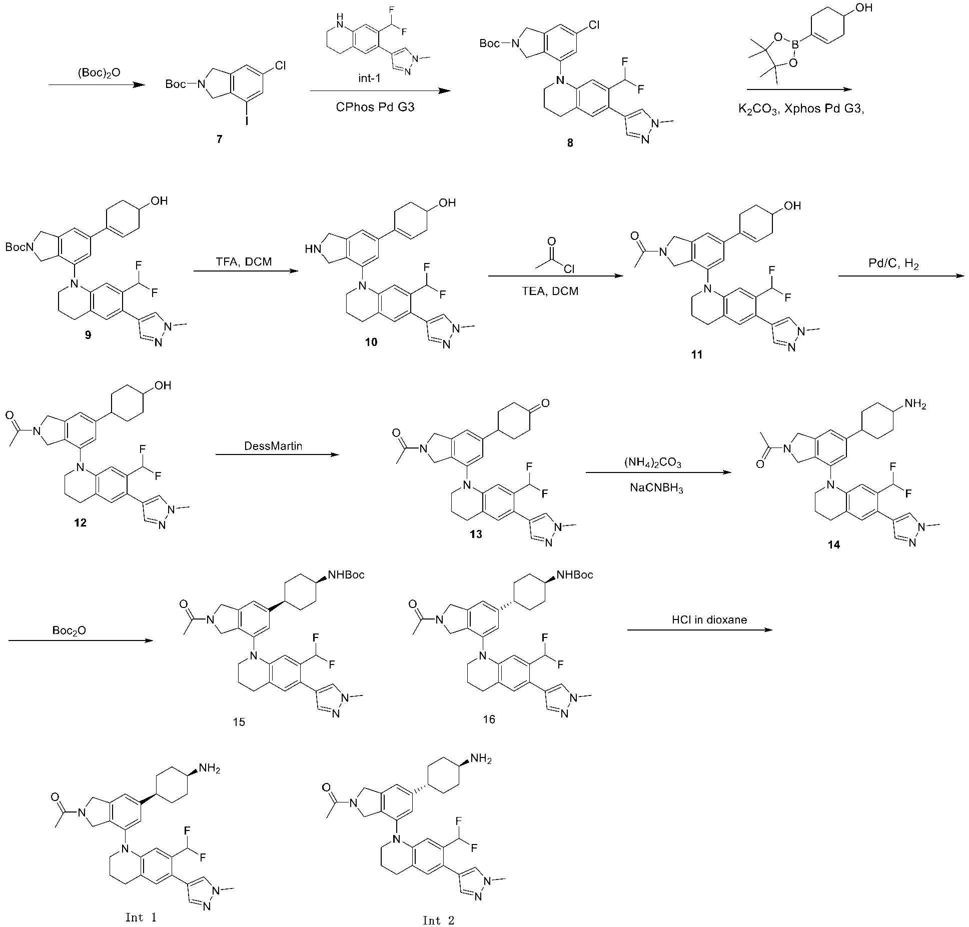

To a solution of 5-chloro-2-methylbenzoic acid (50 g, 293.1 mmol, 1 eq) in concentrated H₂SO₄ (350 mL) was added dropwise a solution of NIS (76 g, 331 mmol, 98% purity, 1.13 eq) in H₂SO₄ (30 mL) at 0 °C. The mixture was stirred at 25 °C for 16 h. The reaction mixture was poured into ice water (500 mL), stirred for 30 min, then filtered to afford 5-chloro-3-iodo-2-methylbenzoic acid (68.5 g, 231 mmol, 78.8% yield) as a brown solid.

MS (ESI): C₈H₆ClIO₂; found 294.8 (M-H)⁺.

To a solution of 5-chloro-3-iodo-2-methylbenzoic acid (68.5 g, 231 mmol, 1 eq) in MeOH (350 mL) was added concentrated H₂SO₄(12 mL). The mixture was stirred at 60 °C for 16 h. The reaction mixture was concentrated under reduced pressure. The residue was diluted with DCM (300 mL) and the combined organic phases were washed with NaHCO₃ aqueous solution (200 mL) and brine (200 mL). It was dried over anhydrous Na₂SO₄, filtered and concentrated in vacuum to afford methyl 5-chloro-3-iodo-2-methylbenzoate (58.5 g, 188.4 mmol, 81.5% yield) as a yellow oil.

¹H NMR (400MHz, CHLOROFORM-d) δ = 7.98 (d, J=2.0 Hz, 1H), 7.76 (d, J=1.8 Hz, 1H), 3.92 (s, 4H), 2.64 (s, 3H).

To a solution of methyl 5-chloro-3-iodo-2-methylbenzoate (55 g, 177.12 mmol, 1eq) in CCl₄ (350 ml) was added NBS (33 g, 185.41 mmol, 1.05eq) and BPO (4.5 g, 18.58 mmol, 1e-1eq). The mixture was stirred at 80 °C for 17 h. The reaction mixture was filtered and concentrated under reduced pressure. The residue was purified by column chromatography (petroleum ether/EtOAc = 100/0 to 10/1) to afford methyl 2-(bromomethyl)-5-chloro-3-iodobenzoate (40 g, 103.1 mmol, 57% yield) as a colorless oil.

¹H NMR (400MHz, CHLOROFORM-d) δ = 7.97 (d, J=2.2 Hz, 1H), 7.84 (d, J=2.2 Hz, 1H), 5.01 (s, 2H), 3.89 (s, 3H).

Methyl 2-(bromomethyl)-5-chloro-3-iodobenzoate (40 g, 102.5 mmol, 1 eq) was added to a solution of NH₃/MeOH (150 ml). The mixture was stirred at 60 °C for 2.5 h. The reaction mixture was concentrated under reduced pressure to remove MeOH. The residue was washed 3 times with EA (150 mL) to afford 6-chloro-4-iodoisoindol-1-one (20.6 g, 70.4 mmol, 68.5% yield) as a white solid.

MS (ESI): C₈H₆ClINO; found 295.8 (M+H)⁺.
¹H NMR (400MHz, DMSO-d6) δ = 8.96 (br s, 1H), 8.09 (s, 1H), 7.71 (s, 1H), 4.18 (s, 2H)

To a solution of 6-chloro-4-iodoisoindol-1-one (17 g, 57.92 mmol, 1 eq) in DCM (150 mL) was added DIBALH (1 M, 202.73 mL, 3.5 eq) dropwise at 0 °C under nitrogen. The mixture was stirred at 45 °C for 54 h. The reaction mixture was quenched by addition of 15% NaOH (100 mL) at 0 °C, then filtered and washed with EtOAc. The filtrate was washed with brine (200 mL), dried over Na₂SO₄, filtered, and concentrated under reduced pressure. The residue was purified by flash silica gel chromatography (flash silica gel column, eluent 0-10% MeOH/DCM) to afford 6-chloro-4-iodo-2,3-dihydro-1H-isoindole hydrochloride (5.5 g, 19.6 mmol, 33.9% yield) as a brown solid.

¹H NMR (400MHz, DMSO-d6) δ = 7.64 (s, 1H), 7.37 (s, 1H), 4.20 (s, 2H), 3.93 (s, 2H).

To a solution of 6-chloro-4-iodo-2,3-dihydro-1H-isoindole hydrochloride (11.5 g, 0.0364 mol) in THF (220 mL) and H₂O (109 mL) were added NaHCO₃ (9.17 g, 0.109 mol) and Boc₂O (8.74 g, 0.0400 mol) at 25 °C. The reaction mixture was stirred at 25 °C for 16 h. The mixture was extracted with EtOAc (100 mL × 2). The combined organic phases were dried over Na₂SO₄ and filtered. The filtrate was concentrated under reduced pressure. The residue was purified by silica gel chromatography eluting with 0-5% EtOAc in PE to afford tert-butyl (6-chloro-4-iodo-1,3-dihydroisoindol-2-yl)carboxylate (9.90 g, 70% yield) as a white solid. LC-MS: (ESI) m/z[m+H-56]+234.0.

To a solution of 7-(difluoromethyl)-6-(1-methylpyrazol-4-yl)-1,2,3,4-tetrahydroquinoline (14.0 g, 0.0532 mol) and tert-butyl (6-chloro-4-iodo-1,3-dihydroisoindol-2-yl)carboxylate (24.3 g, 0.0638 mol) in 1,4-dioxane (1.0 L) were added BuONa (15.3 g, 0.160 mol) and XPhos Pd G3 (4.50 g, 0.00530 mol) at 25 °C. The reaction mixture was heated to 90 °C and stirred under N₂ for 16 h. The reaction mixture was concentrated under reduced pressure. The mixture was added to water (500mL). The aqueous phase was extracted with EtOAc (200mL×3). The combined organic phases were dried over Na₂SO₄ and filtered. The filtrate was concentrated under reduced pressure. The residue was purified by silica gel chromatography eluting with 0-25% THF in PE to afford tert-butyl {6-chloro-4-[7-(difluoromethyl)-6-(1-methylpyrazol-4-yl)-3,4-dihydro-2H-quinolin-1-yl]-1,3-dihydroisoindol-2-yl}carboxylate (22.3 g, 80% yield) as a white solid. LC-MS: (ESI) m/z [M+H] ⁺ 515.2.

¹H NMR (400 MHz, DMSO-*d₆*) δ 7.76 (s, 1H), 7.50 (s, 1H), 7.32 (m, 2H), 7.16 (s, 1H), 6.78 (t, *J =* 55.2 Hz, 1H), 6.42 (d, *J =* 10.0 Hz, 1H), 4.63 (s, 2H), 4.36 - 4.19 (m, 2H), 3.86 (s, 3H), 3.55 (s, 2H), 2.87 (s, 2H), 1.99 (s, 2H), 1.49 - 1.33 (m, 9H).

To a solution of tert-butyl 6-chloro-4-(7-(difluoromethyl)-6-(1-methyl-1H-pyrazol-4-yl)-3,4-dihydroquinolin-1(2H)-yl)isoindoline-2-carboxylate (700 mg, 1.36 mmol) in dioxane (5.0 mL) and H₂O (0.5 mL) were added 4-(4,4,5,5-tetramethyl-1,3,2-dioxaborolan-2-yl)cyclohex-3-en-1-ol (609.2 mg, 2.72 mmol), K₂CO₃ (563.6 mg, 4.08 mmol), and XPhos Pd G3 (114.9 mg, 0.14 mmol). The mixture was then stirred at 100 °C for 2 h. LCMS showed consumption of starting material and detection of the desired product. The residue was purified by flash column chromatography eluting with 0-50% EtOAc in PE, followed by flash column chromatography eluting with 0-5% MeOH in DCM to afford tert-butyl 4-(7-(difluoromethyl)-6-(1-methyl-1H-pyrazol-4-yl)-3,4-dihydroquinolin-1(2H)-yl)-6-(4-hydroxycyclohex-1-en-1-yl)isoindoline-2-carboxylate as a yellow solid.

To a solution of tert-butyl 4-(7-(difluoromethyl)-6-(1-methyl-1H-pyrazol-4-yl)-3,4-dihydroquinolin-1(2H)-yl)-6-(4-hydroxycyclohex-1-en-1-yl)isoindoline-2-carboxylate (700 mg, 1.21 mmol) in DCM (3.0 mL) was added TFA (1.0 mL). The mixture was concentrated under reduced pressure to afford 4-(7-(7-(difluoromethyl)-6-(1-methyl-1H-pyrazol-4-yl)-3,4-dihydroquinolin-1(2H)-yl)isoindolin-5-yl)cyclohex-3-en-1-ol (600 mg, 98% yield) as a yellow solid.

To a solution of 4-(7-(7-(difluoromethyl)-6-(1-methyl-1H-pyrazol-4-yl)-3,4-dihydroquinolin-1(2H)-yl)isoindolin-5-yl)cyclohex-3-en-1-ol (600 mg, 1.3 mmol) in DCM (4.0 mL) were added 25% TEA (382.2 mg, 3.8 mmol) and acetyl chloride (197.7 mg, 2.5 mmol). The reaction was quenched by addition of water (50mL), and extracted with EtOAc (50mL×3). The combined organic layers were washed with brine (50mL×3), and dried over anhydrous Na₂SO₄. The residue was purified by flash column chromatography eluting with 0-50% EtOAc in PE, followed by flash column chromatography eluting with 0-5% methanol in DCM to afford 1-(4-(7-(difluoromethyl)-6-(1-methyl-1H-pyrazol-4-yl)-3,4-dihydroquinolin-1(2H)-yl)-6-(4-hydroxycyclohex-1-en-1-yl)isoindolin-2-yl)ethan-1-one (600 mg, 92% yield) as a yellow solid.

To a solution of 1-(4-(7-(difluoromethyl)-6-(1-methyl-1H-pyrazol-4-yl)-3,4-dihydroquinolin-1(2H)-yl)-6-(4-hydroxycyclohex-1-en-1-yl)isoindolin-2-yl)ethan-1-one (600 mg, 1.2 mmol) in THF (4.0 mL) was added Pd/C (60% in oil) (123.1 mg, 0.12 mmol) under H₂ atmosphere (15 PSI) at 25 °C. After filtration, the filtrate was concentrated under reduced pressure to afford 1-(4-(7-(difluoromethyl)-6-(1-methyl-1H-pyrazol-4-yl)-3,4-dihydroquinolin-1(2H)-yl)-6-(4-hydroxycyclohexyl)isoindolin-2-yl)ethan-1-one (600 mg, 99% yield) as a yellow solid.

To a solution of 1-(4-(7-(difluoromethyl)-6-(1-methyl-1H-pyrazol-4-yl)-3,4-dihydroquinolin-1(2H)-yl)-6-(4-hydroxycyclohexyl)isoindolin-2-yl)ethan-1-one (130 mg, 0.25 mmol) in DCM (2.0 mL) was added Dess Martin (158.9 mg, 0.37 mmol) at 25 °C. The mixture was then stirred at 25 °C for 2 h. The mixture was quenched by addition of saturated aqueous NaHCO₃ (20 mL) and extracted with DCM (3 × 20 mL). The combined organic layers were dried over Na₂SO₄. After filtration, the filtrate was purified by flash column chromatography eluting with 0-50% EtOAc in PE, followed by flash column chromatography eluting with 0-5% methanol in dichloromethane to afford 4-(2-acetyl-7-(7-(difluoromethyl)-6-(1-methyl-1H-pyrazol-4-yl)-3,4-dihydroquinolin-1(2H)-yl)isoindolin-5-yl)cyclohexan-1-one (100 mg, 77% yield) as a white solid.

To a solution of 4-(2-acetyl-7-(7-(difluoromethyl)-6-(1-methyl-1H-pyrazol-4-yl)-3,4-dihydroquinolin-1(2H)-yl)isoindolin-5-yl)cyclohexan-1-one (200 mg, 0.39 mmol) in MeOH (3.0 mL) was added ammonium carbonate (74.1 mg, 0.77 mmol) at 25 °C. The mixture was stirred at 25 °C for 1 h. Then NaCNBH₃ (48.5 mg, 0.77 mmol) was added to the mixture. The mixture was stirred at 25 °C for 1 h. LCMS showed consumption of starting material and detection of the desired product. The crude product was purified by preparative HPLC, eluting with CH₃CN in 0.1% FA/water from 38% to 45% over 8 min to afford 1-(6-(4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one (30.4 mg, 15% yield) as a white solid.

To a solution of 1-(6-(4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one (215 mg, 0.414 mmol) and Boc₂O (181 mg, 0.828 mmol) in DCM (6 mL) was added TEA (41.9 mg, 1.24 mmol) at 25 °C. The reaction mixture was stirred at 25 °C under N₂ for 1 h. The mixture was concentrated under reduced pressure. The residue was purified by SFC (Daicel CHIRALCEL IB-N, 250 mm × 30 mm I.D., 10 µm; mobile phase: CO₂/MeOH [0.2% NH₃ (7 M in MeOH)] = 50/40; 80 g/min; 35 °C) to afford tert-butyl ((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (37.2 mg, 14.5% yield) and tert-butyl ((1r,4r)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (105.3 mg, 41% yield) as a white solid.

To a solution of tert-butyl ((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (37.2 mg, 0.0601 mmol) in 1,4-dioxane (5 mL) was added HCl in 1,4-dioxane (4 N, 5 mL) at 25 °C. The reaction mixture was stirred at 25 °C for 1 h. The solution in water was dried by lyophilization to afford 1-(6-((1s,4s)-4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one hydrochloride (28.5 mg, 84% yield) as a white solid.

To a solution of *tert*-butyl ((1r,4r)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (105.3 mg, 0.170 mmol) in 1,4-dioxane (5 mL) was added HCl in 1,4-dioxane (4 N, 5 mL) at 25 °C. The reaction mixture was stirred at 25 °C for 1 h. The solution in water was dried by lyophilization to afford 1-(6-((1r,4r)-4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one hydrochloride (93.2 mg, 98% yield) as a white solid.

### Example 1- 3 (Synthesis of 1338)

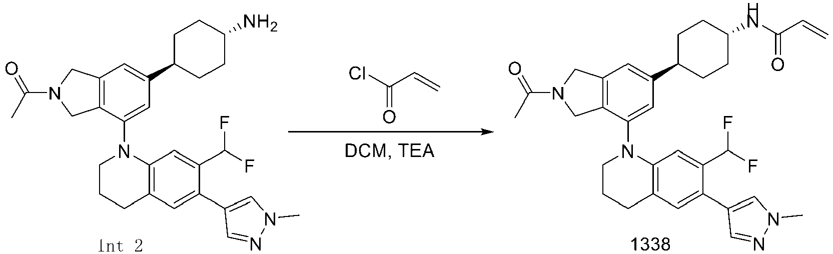

To a solution of 1-(6-((1r,4r)-4-aminocyclohexyl)-4-(7-(difluoromethyl)-6-(1-methyl-1H-pyrazol-4-yl)-3,4-dihydroquinolin-1(2H)-yl)isoindolin-2-yl)-1-one (20.0 mg, 0.0385 mmol) in DCM (3.0 mL) was added TEA (11.7 mg, 0.116 mmol) and acryloyl chloride (6.97 mg, 0.0770 mmol) at 25°C. The reaction mixture was stirred at 25°C for 10 min. The mixture was concentrated under reduced pressure, and the residue was purified by preparative HPLC (CH₃CN from 30% to 50% over 8 min) to afford N-(((1r,4r)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)acrylamide (Compound 1338, 11.2 mg, 50% yield) as a yellow solid.

¹H NMR (400 MHz, DMSO-*d₆*) δ 8.06 - 7.98 (m, 1H), 7.75 (s, 1H), 7.49 (s, 1H), 7.21 - 7.09 (m, 3H), 6.74 (t, *J* = 55.2 Hz, 1H), 6.46 - 6.36 (m, 1H), 6.27 - 6.16 (m, 1H), 6.12 - 6.02 (m, 1H), 5.61 - 5.50 (m, 1H), 4.91 - 4.80 (m, 1H), 4.75 - 4.50 (m, 2H), 4.34 - 4.20 (m, 1H), 3.86 (s, 3H), 3.75 - 3.63 (m, 1H), 3.61 - 3.50 (m, 2H), 2.93 - 2.84 (m, 2H), 2.59 - 2.53 (m, 1H), 2.08 - 1.97 (m, 5H), 1.95 - 1.80 (m, 4H), 1.65 - 1.50 (m, 2H), 1.40 - 1.25 (m, 2H).

### Example 1- 4 (Synthesis of 1344)

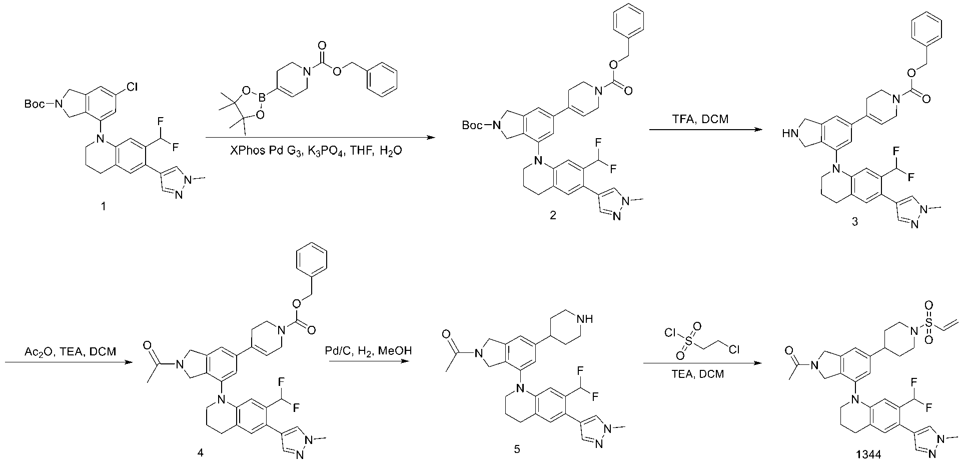

To a solution of tert-butyl {6-chloro-4-[7-(difluoromethyl)-6-(1-methylpyrazol-4-yl)-3,4-dihydro-2H-quinolin-1-yl]-1,3-dihydroisoindol-2-yl}carboxylate (2 g, 0.0039 mol) and benzyl 4-(4,4,5,5-tetramethyl-1,3,2-dioxaborolan-2-yl)-3,6-dihydro-2H-pyridine-1-carboxylate (2 g, 0.0058 mol) in THF/H₂O (33 mL) were added XPhos Pd G3 (0.33 g, 0.0003 mol) and K₃PO₄ (2.5 g, 0.012 mol). The mixture was stirred at 60 °C for 2 h, then concentrated under reduced pressure. The residue was purified by silica gel chromatography, eluting with 0% to 50% EtOAc in petroleum ether over 15 min to afford tert-butyl 6-(1-((benzyloxy)carbonyl)-1,2,3,6-tetrahydropyridin-4-yl)-4-(7-(difluoromethyl)-6-(1-methyl-1H-pyrazol-4-yl)-3,4-dihydroquinolin-1(2H)-yl)isoindoline-2-carboxylate (2.6 g, 95% yield) as a yellow solid. LCMS: m/z[m+H]+696.3.

A solution of tert-butyl 6-(1-((benzyloxy)carbonyl)-1,2,3,6-tetrahydropyridin-4-yl)-4-(7-(difluoromethyl)-6-(1-methyl-1H-pyrazol-4-yl)-3,4-dihydroquinolin-1(2H)-yl)isoindoline-2-carboxylate (2.6 g, 0.0037 mol) in DCM/TFA (20 mL) was stirred at 25 °C for 0.1 h. The mixture was concentrated under reduced pressure to afford benzyl 4-{7-[7-(difluoromethyl)-6-(1-methylpyrazol-4-yl)-3,4-dihydro-2H-quinolin-1-yl]-2,3-dihydro-1H-isoindol-5-yl}-3,6-dihydro-2H-pyridine-1-carboxylate (2.2 g, 95% yield) as a yellow solid. LCMS: m/z[m+H]+596.3.

To a solution of benzyl 4-{7-[7-(difluoromethyl)-6-(1-methylpyrazol-4-yl)-3,4-dihydro-2H-quinolin-1-yl]-2,3-dihydro-1H-isoindol-5-yl}-3,6-dihydro-2H-pyridine-1-carboxylate (2.2 g, 0.0037 mol) and TEA (3.7 g, 0.037 mol) in DCM (20 mL) was added acetyl chloride (1.5 g, 0.019 mol). The mixture was stirred at 25 °C for 0.1 h. LCMS showed consumption of starting material and detection of the desired MS. The mixture was concentrated under reduced pressure. The residue was purified by silica gel chromatography, eluting with 0% to 80% EtOAc in petroleum ether over 15 min to afford Compound 4 (2.3 g, 92% yield) as a yellow solid. MS: m/z [M+H]+ 596.2.

To a solution of benzyl 4-(2-acetyl-7-(7-(difluoromethyl)-6-(1-methyl-1H-pyrazol-4-yl)-3,4-dihydroquinolin-1(2H)-yl)isoindol-5-yl)-3,6-dihydropyridine-1(2H)-carboxylate (2.3 g, 0.0036 mol) in MeOH (20 mL) was added Pd/C (0.77 g, 0.0072 mol). The mixture was stirred at 25 °C for 12 h, filtered and concentrated under reduced pressure. The residue was purified by prep-HPLC, eluting with 0-100% CH₃CN aqueous solution over 15 min to afford 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (Compound 5, 1.4 g, 72% yield) as a white solid. LCMS: m/z[m+H]+506.3.

To a solution of 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (100 mg, 0.20 mmol) and TEA (40 mg, 0.40 mmol) in DCM (2 ml) was added 2-chloroethanesulfonyl chloride (39 mg, 0.24 mmol). The mixture was stirred at 25 °C for 0.1 h. The mixture was concentrated under reduced pressure and purified by prep-HPLC, eluting with 45% to 75% CH₃CN in water over 8 min to afford 1-(4-(7-(difluoromethyl)-6-(1-methyl-1H-pyrazol-4-yl)-3,4-dihydroquinolin-1(2H)-yl)-6-(1-(vinylsulfonyl)piperidin-4-yl)isoindol-2-yl)ethan-1-one (1344, 33 mg, 27% yield) as a white solid. MS: m/z [M+H]+ 596.2. ¹H NMR (400 MHz, DMSO-*d₆*) δ 7.75 (s, 1H), 7.49 (s, 1H), 7.19-7.12 (m, 3H), 6.89 - 6.59 (m, 2H), 6.40 (d, *J* = 27.6 Hz, 1H), 6.20 - 6.08 (m, 2H), 4.86 (s, 1H), 4.63 (d, *J* = 18.4 Hz, 2H), 4.28 (s, 1H), 3.85 (s, 3H), 3.72 - 3.50 (m, 4H), 2.87 (s, 2H), 2.79 - 2.63 (m, 3H), 2.07 - 1.95 (m, 5H), 1.88-1.85 (m, 2H), 1.71-1.64 (m, 2H).

### Example 1- 5 (Synthesis of 1391)

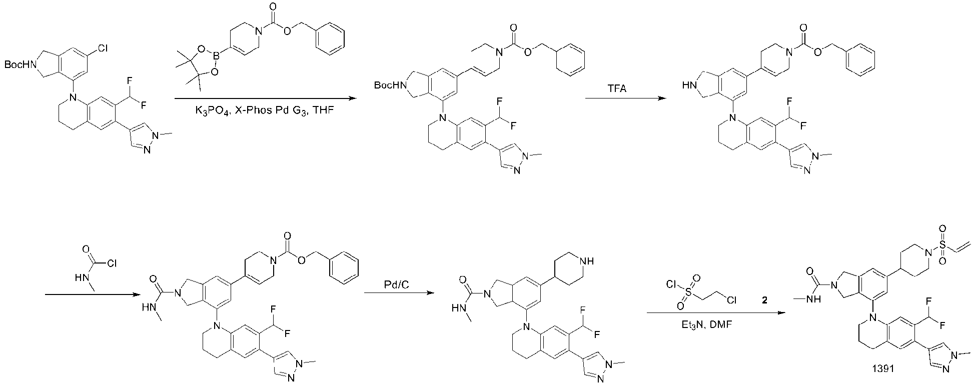

To a solution of *tert*-butyl 6-chloro-4-[7-(difluoromethyl)-6-(1-methylpyrazol-4-yl)-3,4-dihydro-2H-quinolin-1-yl]-1,3-dihydroisoindole-2-carboxylate (600 mg, 1.2 mmol) and benzyl 4-(4,4,5,5-tetramethyl-1,3,2-dioxaborolan-2-yl)-3,6-dihydro-2H-pyridine-1-carboxylate (800 mg, 2.3 mmol) in THF/H₂O (30 mL) were added XPhos Pd G3 (197 mg, 0.23 mmol) and K₃PO₄ (742 mg, 3.5 mmol). The mixture was stirred at 60 °C for 1 h. The mixture was concentrated under reduced pressure, and the residue was purified by silica gel chromatography, eluting with 0% to 30% EtOAc in petroleum ether over 20 min, to afford *tert*-butyl 6-{1-[(benzyloxy)carbonyl]-3,6-dihydro-2H-pyridin-4-yl}-4-[7-(difluoromethyl)-6-(1-methylpyrazol-4-yl)-3,4-dihydro-2H-quinolin-1-yl]-1,3-dihydroisoindole-2-carboxylate (480 mg, 53% yield) as a yellow gum.

A mixture of *tert*-butyl 6-{1-[(benzyloxy)carbonyl]-3,6-dihydro-2H-pyridin-4-yl}-4-[7-(difluoromethyl)-6-(1-methylpyrazol-4-yl)-3,4-dihydro-2H-quinolin-1-yl]-1,3-dihydroisoindole-2-carboxylate (500 mg, 0.72 mmol) in DCM/TFA = 2:1 (30 mL) was stirred at 25 °C for 1 h. The mixture was concentrated under reduced pressure to afford benzyl 4- { 7-[7-(difluoromethyl)-6-(-1-methylpyrazol-4-yl)-3,4-dihydro-2H-quinolin-1-yl]-2,3-dihydro-1H-isoindol-5-yl}-3,6-dihydro-2H-pyridine-1-carboxylate (450 mg, 95% yield) as a yellow gum.

To a solution of benzyl 4-{7-[7-(difluoromethyl)-6-(1-methylpyrazol-4-yl)-3,4-dihydro-2H-quinolin-1-yl]-2,3-dihydro-1H-isoindol-5-yl}-3,6-dihydro-2H-pyridine-1-carboxylate (450 mg, 0.76 mmol) and TEA (382 mg, 3.8 mmol) in DCM (20 mL) was added N-methylcarbamoyl chloride (106 mg, 1.1 mmol). The mixture was stirred at 25 °C for 1 h. The mixture was concentrated under reduced pressure, and the residue was purified by silica gel chromatography, eluting with 0% to 70% EtOAc in petroleum ether over 12 min, to afford benzyl 4-{7-[7-(difluoromethyl)-6-(1-methylpyrazol-4-yl)-3,4-dihydro-2H-quinolin-1-yl]-2-(methylcarbamoyl)-1,3-dihydroisoindol-5-yl}-3,6-dihydro-2H-pyridine-1-carboxylate (500 mg, 91% yield) as a yellow gum.

To a mixture of benzyl 4-{7-[7-(difluoromethyl)-6-(1-methylpyrazol-4-yl)-3,4-dihydro-2H-quinolin-1-yl]-2-(methylcarbamoyl)-1,3-dihydroisoindol-5-yl}-3,6-dihydro-2H-pyridine-1-carboxylate (500 mg, 0.77 mmol) in MeOH (10 mL) was added Pd/C (408 mg, 3.8 mmol). The mixture was stirred under H₂ at 25 °C for 3 h. The mixture was filtered and concentrated under reduced pressure to afford 4-[7-(difluoromethyl)-6-(1-methylpyrazol-4-yl)-3,4-dihydro-2H-quinolin-1-yl]-N-methyl-6-(piperidin-4-yl)-1,3-dihydroisoindole-2-carboxamide (350 mg, 79% yield) as a yellow gum.

To a solution of 4-[7-(difluoromethyl)-6-(1-methylpyrazol-4-yl)-3,4-dihydro-2H-quinolin-1-yl]-N-methyl-6-(piperidin-4-yl)-1,3-dihydroisoindole-2-carboxamide (100 mg, 0.19 mmol) and TEA (58.32 mg, 0.57 mmol) in DCM (5 mL) was added 2-chloroethanesulfonyl chloride (46.98 mg, 0.28 mmol). The mixture was stirred at 25 °C for 30 min. The mixture was concentrated under reduced pressure, and the residue was purified by pre-HPLC, eluting with 45% to 65% CH₃CN in H₂O over 18 min, to afford 4-(7-(difluoromethyl)-6-(1-methyl-1H-pyrazol-4-yl)-3,4-dihydroquinolin-1(2H)-yl)-N-methyl-6-(1-(vinylsulfonyl)piperidin-4-yl)isoindoline-2-carboxamide (25.8 mg, 20.93% yield) as a yellow solid.

1H NMR (400 MHz, DMSO) δ 7.74 (s, 1H), 7.49 (s, 1H), 7.37 - 7.05 (m, 3H), 6.96 - 6.55 (m, 2H), 6.39 - 6.21 (m, 2H), 6.19 - 6.07 (m, 2H), 4.61 (d, J = 9.2 Hz, 2H), 4.30 (s, 2H), 3.86 (s, 3H), 3.82 - 3.50 (m, 4H), 2.90 (d, J = 20.6 Hz, 2H), 2.77 - 2.54 (m, 6H), 2.16 - 1.82 (m, 4H), 1.76 - 1.62 (m, 2H).

### Example 1- 6 (Synthesis of 1345)

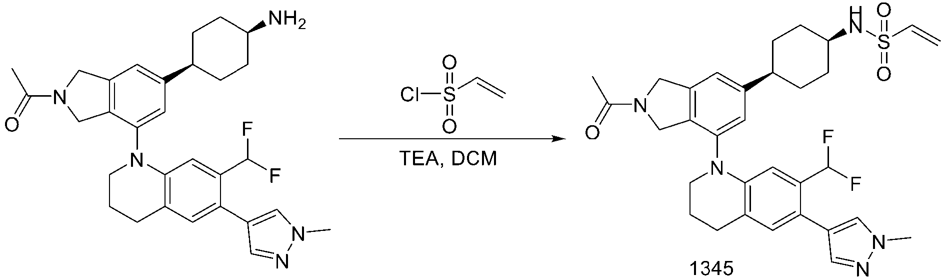

To a solution of 1-(6-((1s,4s)-4-aminocyclohexyl)-4-(7-(difluoromethyl)-6-(1-methyl-1H-pyrazol-4-yl)-3,4-dihydroquinolin-1(2H)-yl)isoindolin-2-yl)-1-one (15.0 mg, 0.0289 mmol) in DCM (3.0 mL) was added TEA (8.77 mg, 0.0867 mmol) and vinylsulfonyl chloride (7.32 mg, 0.0578 mmol) at 25°C. The reaction mixture was stirred at 25°C for 10 min. The mixture was concentrated under reduced pressure, and the residue was purified by preparative HPLC (30% to 50% CH₃CN over 8 min) to afford N-((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)ethenesulfonamide (3.40 mg, 19% yield) as a white solid.

¹H NMR (400 MHz, DMSO-*d₆*) δ 7.78 - 7.70 (m, 1H), 7.55 - 7.43 (m, 2H), 7.25 - 7.15 (m, 2H), 7.15 - 7.08 (m, 1H), 6.92 - 6.58 (m, 2H), 6.43 - 6.34 (m, 1H), 6.08 - 6.00 (m, 1H), 5.99 - 5.88 (m, 1H), 5.00 - 4.81 (m, 1H), 4.79 - 4.50 (m, 2H), 4.40 - 4.15 (m, 1H), 3.86 (s, 3H), 3.62 - 3.50 (m, 2H), 3.49 - 3.43 (m, 1H), 2.93 - 2.84 (m, 2H), 2.58 - 2.54 (m, 1H), 2.07 - 1.97 (m, 5H), 1.91 - 1.70 (m, 4H), 1.69 - 1.48 (m, 4H).

### Example 1-7 (Synthesis of 1366 and 1367)

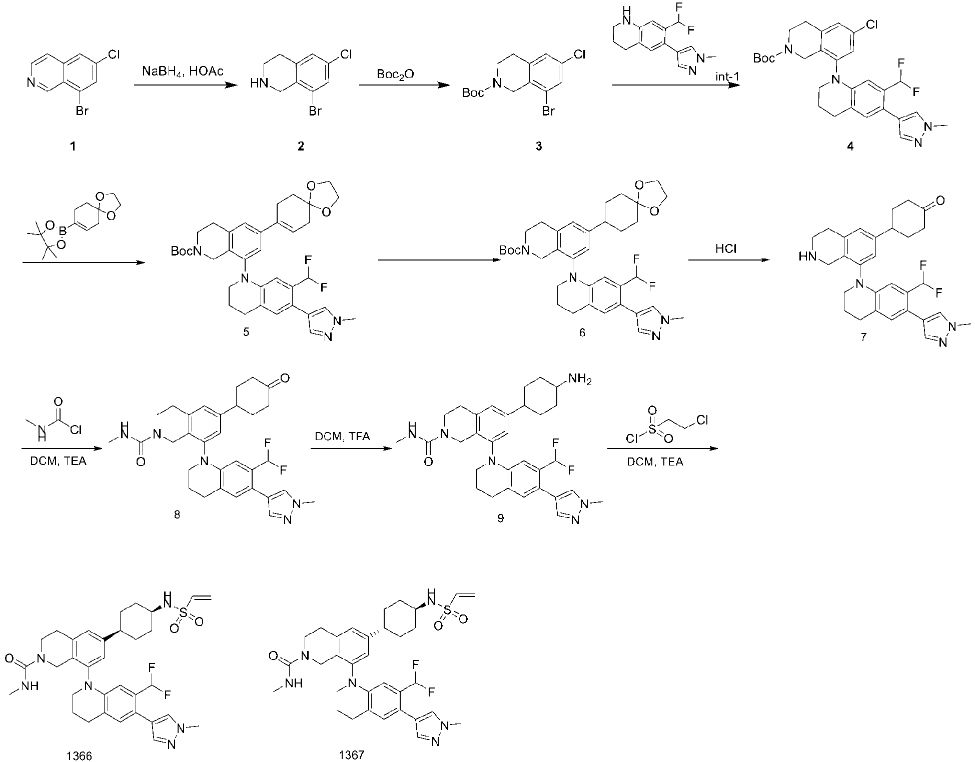

A solution of 8-bromo-6-chloroisoquinoline (8.6 g, 35.46 mmol) in AcOH (80 mL) was cooled to 0 °C. NaBH₄ (2.68 g, 70.93 mmol) was then added in batches. The mixture was stirred at 25 °C for 0.5 h. The mixture was poured into H₂O to quench the reaction, then the pH was adjusted to 8 with NH₄Cl. Extraction with EtOAc (200 mL) afforded Compound 2 (8.48 g, 34.40 mmol, 97.0% yield, crude product) as a yellow solid, which was confirmed by HNMR.

¹H NMR (400 MHz, CD₃Cl) δ 7.42 (s, 1 H), 7.09 (s, 1 H), 4.00 (s, 2H), 3.15-3.18(m, 2H), 2.85-2.88 (m, 2H).

To a solution of 8-bromo-6-chloro-1,2,3,4-tetrahydroisoquinoline (8.48 g, 34.40 mmol) in DCM (100 mL) were added TEA (6.96 g, 68.79 mmol) and Boc₂O (11.26 g, 51.60 mmol), and the mixture was stirred at 25 °C for 12 h. The mixture was washed with brine (50 mL × 2) to afford a crude product, which was purified by silica gel column chromatography (petroleum ether/EtOAc, 100% to 70%) to afford *tert*-butyl 8-bromo-6-chloro-3,4-dihydroisoquinoline-2(1*H*)-carboxylate (6.7 g, 19.33 mmol, 56.2% yield) as a yellow oil, and confirmed by NMR.

¹H NMR (400 MHz, CD₃Cl) δ 7.42 (s, 1 H), 7.10 (s, 1 H), 4.49 (s, 2H), 3.61-3.63(m, 2H), 2.80-2.82 (m, 2H), 1.50 (s, 9H).

A mixture of *tert*-butyl 8-bromo-6-chloro-3,4-dihydroisoquinoline-2(1*H*)-carboxylate (29.3 g, 84.52 mmol), 7-(difluoromethyl)-6-(1-methyl-1*H*-pyrazol-4-yl)-1,2,3.3,4-tetrahydroquinoline (13.35 g, 50.71 mmol), t-BuONa (24.37 g, 253.57 mmol), CPhos Pd G3 (6.82 g, 8.45 mmol) and dioxane (300 mL) was degassed and purged with N₂ three times. The mixture was then stirred under N₄ at 100 °C for 2 h. Extraction with EtOAc (200 mL) afforded a crude residue, which was purified by silica gel column chromatography (petroleum ether/EtOAc, 100% to 70%) to afford *tert*-butyl 6-chloro-8-(7-(difluoromethyl)-6-(1-methyl-1*H*-pyrazol-4-yl)-3,4-dihydroquinolin-1(2*H*)-yl)-3,4-dihydroisoquinoline-2(1*H*)-carboxylate as a yellow oil (22.6 g, 42.72 mmol, 50.5%).

LCMS (ESI+) : m/z 529.3 (M+H)+, Rt: 2.38 min.

To a solution of *tert*-butyl 6-chloro-8-(7-(difluoromethyl)-6-(1-methyl-1*H*-pyrazol-4-yl)-3,4-dihydroquinolin-1(2*H*)-yl)-3,4-dihydroisoquinoline-2(1*H*)-carboxylate (2.00 g, 3.80 mmol) and 2-{1,4-dioxaspiro[4.5]dec-7-en-8-yl}-4,4,5,5-tetramethyl-1,3,2-dioxaborolane (1.52 g, 5.70 mmol) in THF (20 mL) and H₂O (2.0 mL) were added K₃PO₄ (2.42 g, 11.4 mmol) and XPhos Pd G3 (0.320 g, 0.380 mmol) at 25 °C. The reaction mixture was stirred under N₂ at 60 °C for 2 h. The reaction was quenched with water (50 mL), followed by extraction with EtOAc (80 mL × 2). The combined organic layers were dried over Na₂SO₄ and filtered. The filtrate was concentrated under reduced pressure. The residue was purified by silica gel chromatography eluting with 0% to 50% THF in PE to afford *tert*-butyl 8-(7-(difluoromethyl)-6-(1-methyl-1*H*-pyrazol-4-yl)-3,4-dihydroquinolin-1(2*H*)-yl)-6-(1,4-dioxaspiro[4.5]dec-7-en-8-yl)-3,4-dihydroisoquinoline-2(1*H*)-carboxylate as a yellow solid (2.00 g, 79% yield). LC-MS: (ESI) m/z [M+H] ⁺ 633.3.

To a solution of *tert*-butyl 8-(7-(difluoromethyl)-6-(1-methyl-1*H*-pyrazol-4-yl)-3,4-dihydroquinolin-1(2*H*)-yl)-6-(1,4-dioxaspiro[4.5]dec-7-en-8-yl)-3,4-dihydroisoquinoline-2(1*H*)-carboxylate (2.00 g, 3.20 mmol) in MeOH (20 mL) was added Pd/C (0.680 g, 6.40 mmol), and the mixture was stirred at 25 °C under H₂ balloon pressure for 48 h. The solid was removed by filtration and the filtrate was concentrated to afford tert-butyl 8-(7-(difluoromethyl)-6-(1-methyl-1*H*-pyrazol-4-yl)-3,4-dihydroquinolin-1(2*H*)-yl)-6-(1,4-dioxaspiro[4.5]decan-8-yl)-3,4-dihydroisoquinoline-2(1*H*)-carboxylate (1.70 g, 78% yield) as a white solid. LC-MS: (ESI) m/z [M+H] + 635.3.

To a solution of *tert*-butyl 8-(7-(difluoromethyl)-6-(1-methyl-1*H*-pyrazol-4-yl)-3,4-dihydroquinolin-1(2*H*)-yl)-6-(1,4-dioxaspiro[4.5]decan-8-yl)-3,4-dihydroisoquinoline-2(1*H*)-carboxylate (1.90 g, 3.00 mmol) in DCM (8.0 mL) was added TFA (8.0 mL) at 25 °C, and the reaction mixture was stirred at 25 °C for 1 h. The mixture was concentrated under reduced pressure to afford 4-(8-(7-(difluoromethyl)-6-(1-methyl-1*H*-pyrazol-4-yl)-3,4-dihydroquinolin-1(2*H*)-yl)-1,2,3,4-tetrahydroisoquinolin-6-yl)cyclohexanone (1.40 g, crude) as a yellow solid, which was used directly in the next step without further purification. LC-MS: (ESI) m/z [M+H] ⁺ 491.3.

To a solution of 4-(8-(7-(difluoromethyl)-6-(1-methyl-1*H*-pyrazol-4-yl)-3,4-dihydroquinolin-1(2*H*)-yl)-1,2,3,4-tetrahydroisoquinolin-6-yl)cyclohexanone (1.40 g, 29.0 mmol) and *N*-methylcarbamoyl chloride (0.680 g, 7.25 mmol) in DCM (14 mL) was added TEA (0.880 g, 8.70 mol) at 25 °C. The reaction mixture was stirred at 25 °C for 10 min. The mixture was quenched with water (50 mL), and extracted with DCM (30 mL × 2). The combined organic phases were dried over Na₂SO₄ and filtered. The filtrate was concentrated under reduced pressure. The residue was purified by silica gel chromatography eluting with 0% to 4% methanol in DCM to afford 8-(7-(difluoromethyl)-6-(1-methyl-1*H*-pyrazol-4-yl)-3,4-dihydroquinolin-1(2*H*)-yl)-N-methyl-6-(4-oxocyclohexyl)-3,4-dihydroisoquinoline-2(1*H*)-carboxamide (1.30 g, 72% yield) as a yellow solid. LC-MS: (ESI) m/z [M+H] ⁺ 548.2.

To a solution of 8-(7-(difluoromethyl)-6-(1-methyl-1*H*-pyrazol-4-yl)-3,4-dihydroquinolin-1(2*H*)-yl)-*N*-methyl-6-(4-oxocyclohexyl)-3,4-dihydroisoquinoline-2(1*H*)-carboxamide (1.10 g, 2.00 mmol) in MeOH (20 mL) was added NH₄Cl (0.320 g, 6.00 mmol) and NaCNBH₃ (0.380 g, 6.00 mmol) at 25 °C. The reaction mixture was stirred at 25 °C for 16 h, quenched with water (50 mL), and extracted with EtOAc (50 mL × 2). The combined organic phases were dried over Na₂SO₄ and filtered, and the filtrate was concentrated under reduced pressure and purified by reverse-phase chromatography (ACN/H₂O with 0.1% FA, 5% to 35%) to afford 6-(4-aminocyclohexyl)-8-(7-(difluoromethyl)-6-(1-methyl-1*H*-pyrazol-4-yl)-3,4-dihydroquinolin-1(2*H*)-yl)-*N*-methyl-3,4-dihydroisoquinoline-2(1*H*)-carboxamide (0.462 g, 40% yield) as a white solid. LC-MS: (ESI) m/z [M+H] ⁺ 549.2.

To a solution of 6-(4-aminocyclohexyl)-8-(7-(difluoromethyl)-6-(1-methyl-1*H*-pyrazol-4-yl)-3,4-dihydroquinolin-1(2*H*)-yl)-*N*-methyl-3,4-dihydroisoquinoline-2(1H)-carboxamide (100 mg, 0.182 mmol) in DCM (6.0 mL) were added 2-chloroethanesulfonyl chloride (44.6 mg, 0.273 mmol) and TEA (55.3 mg, 0.547 mmol) at 25 °C. The reaction mixture was stirred at 25 °C for 10 min. LCMS showed consumption of the starting material and formation of the desired product. The mixture was concentrated. The residue was purified by Prep-HPLC (column: Xbridge-C18 150 × 19 mm, 5 µm; mobile phase: ACN-H₂O, 45-75% MeCN in 0.1% FA/H₂O, 30 mL/min) to afford 8-(7-(difluoromethyl)-6-(1-methyl-1*H*-pyrazol-4-yl)-3,4-dihydroquinolin-1(2*H*)-yl)-*N*-methyl-6-((1*s*,4*s*)-4-(vinylsulfonamido)cyclohexyl)-3,4-dihydroisoquinoline-2(1*H*)-carboxamide (8.20 mg, 7% yield) as a white solid.

Meanwhile, 8-(7-(difluoromethyl)-6-(1-methyl-1*H*-pyrazol-4-yl)-3,4-dihydroquinolin-1(2*H*)-yl)-*N*-methyl-6-((1*r*,4*r*)-4-(vinylsulfonamido)cyclohexyl)-3,4-dihydroisoquinoline-2(1*H*)-carboxamide (26.3 mg, 22% yield) was obtained as a white solid.

### Example 1-8 (Synthesis of 1346)

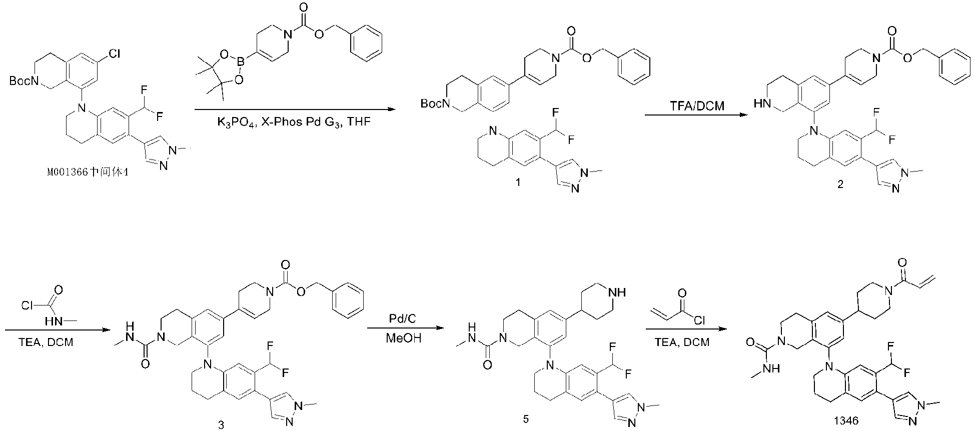

To a solution of tert-butyl 6-chloro-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (300 mg, 0.567 mmol) and benzyl 4-(4,4,5,5-tetramethyl-1,3,2-dioxaborolan-2-yl)-3,6-dihydro-2H-pyridine-1-carboxylate (292 mg, 0.851 mol) in THF (10 mL) and H₂O (1.0 mL) were added K₃PO₄ (360 mg, 1.70 mmol) and XPhos-Pd G3 (47.9 mg, 0.0567 mmol) at 25 °C. The reaction mixture was stirred at 60 °C under N₂ for 2 h. The mixture was quenched with water (100 mL), then extracted with EtOAc (100 mL × 2). The combined organic phases were dried over Na₂SO₄ and filtered. The filtrate was concentrated under reduced pressure. The residue was purified by silica gel chromatography eluting with 0% to 5% methanol in DCM to afford tert-butyl 6-(1-((benzyloxy)carbonyl)-1,2,3,6-tetrahydropyridin-4-yl)-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (308 mg, 77% yield) as a yellow solid. LC-MS: (ESI) m/z [M+H] ⁺ 710.3.

To a solution of tert-butyl 6-(1-((benzyloxy)carbonyl)-1,2,3,6-tetrahydropyridin-4-yl)-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (308 mg, 0.434 mmol) in DCM (3.0 mL) was added TFA (3.0 mL) at 25 °C. The reaction mixture was stirred at 25 °C for 1 h, then concentrated under reduced pressure to afford benzyl 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-3,6-dihydropyridine-1(2H)-carboxylate (270 mg, crude) as a brown oil. It was used directly in the next step without further purification. LC-MS: (ESI) m/z [M+H] ⁺ 610.4.

To a solution of benzyl 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-3,6-dihydropyridine-1(2H)-carboxylate (265 mg, 0.435 mmol) in DCM (8.0 mL) was added TEA (132 mg, 0.869 mmol) at 25 °C. The reaction mixture was stirred at 25 °C for 10 min, then concentrated under reduced pressure to afford benzyl 4-(8-(7-(difluoromethyl)-6-(1-methyl-1H-pyrazol-4-yl)-3,4-dihydroquinolin-1(2H)-yl)-2-(methylcarbamoyl)-1,2,3,4-tetrahydroisoquinolin-6-yl)-3,6-dihydropyridine-1(2H)-carboxylate (260 mg, 85% yield) as a white solid. LC-MS (ESI): m/z [M+H]⁺ 627.3.

To a solution of benzyl 4-(8-(7-(difluoromethyl)-6-(1-methyl-1H-pyrazol-4-yl)-3,4-dihydroquinolin-1(2H)-yl)-2-(methylcarbamoyl)-1,2,3,4-tetrahydroisoquinolin-6-yl)-3,6-dihydropyridine-1(2H)-carboxylate (260 mg, 0.390 mmol) in MeOH (10 mL) was added Pd/C (41.5 mg, 0.390 mol). The mixture was stirred under H₂ balloon pressure at 25 °C for 16 h. The mixture was filtered to remove solids and concentrated to afford 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (160 mg, 73% yield) as a yellow solid. LC-MS (ESI): m/z [M+H]⁺ 535.3.

To a solution of 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (20.0 mg, 0.0374 mmol) in DCM (3.0 mL) were added TEA (11.4 mg, 0.112 mmol) and prop-2-enoyl chloride (6.77 mg, 0.0748 mmol) at 25 °C. The reaction mixture was stirred at 25 °C for 30 min, then concentrated under reduced pressure. The residue was purified by Prep-HPLC (30% to 50% CH₃CN over 8 min) to afford 6-(1-acryloylpiperidin-4-yl)-8-(7-(difluoromethyl)-6-(1-methyl-1H-pyrazol-4-yl)-3,4-dihydroquinolin-1(2H)-yl)-N-methyl-3,4-dihydroisoquinoline-2(1H)-carboxamide (Compound 1346, 3.40 mg, 15% yield) as a white solid.

¹H NMR (400 MHz, DMSO-*d₆*) δ 7.71 (s, 1H), 7.47 (s, 1H), 7.10 - 7.00 (m, 3H), 6.86 - 6.77 (m, 1H), 6.70 - 6.54 (m, 1H), 6.51 - 6.44 (m, 1H), 6.17 (s, 1H), 6.13 - 6.05 (m, 1H), 5.69 - 5.62 (m, 1H), 4.60 - 4.50 (m, 1H), 4.46 - 4.37 (m, 2H), 4.18 - 4.06 (m, 3H), 3.86 (s, 3H), 3.20 - 3.02 (m, 2H), 2.94 - 2.74 (m, 6H), 2.72 - 2.63 (m, 2H), 2.55 - 2.53 (m, 3H), 2.53 - 2.52 (m, 1H), 2.12 - 1.97 (m, 2H), 1.85 - 1.75 (m, 2H), 1.58 - 1.39 (m, 2H). ¹⁹F NMR (400 MHz, DMSO-*d₆*) δ - 108.048. LC-MS: m/z [M-40+H] ⁺ 549.3.

### Example 1-9 (Synthesis of 1369)

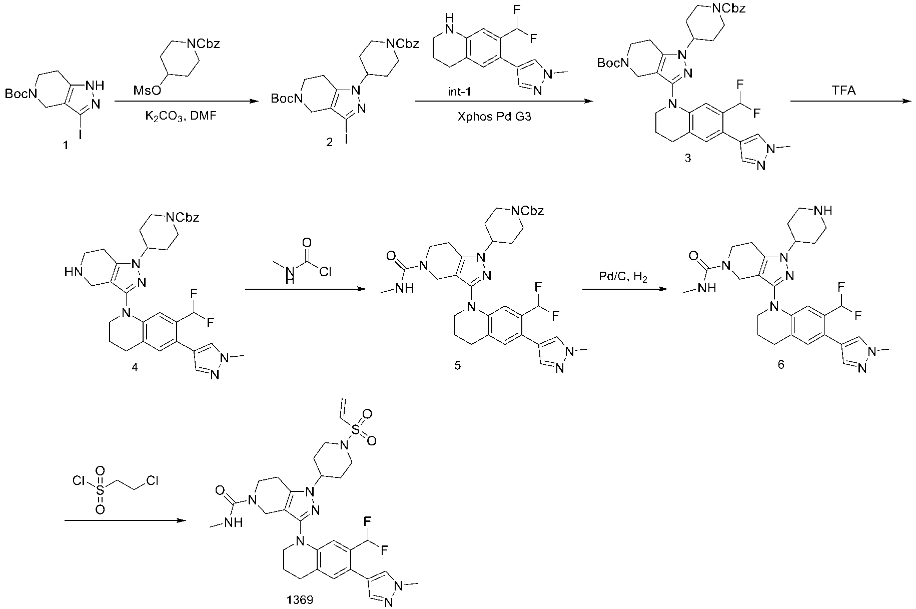

To a solution of tert-butyl 3-iodo-1H,4H,6H,7H-pyrazolo[4,3-c]pyridine-5-carboxylate (1000 mg, 2.86 mmol) in DMF (20 mL) were added benzyl 4-(methanesulfonyloxy)piperidine-1-carboxylate (1032 mg, 3.29 mmol) and K₂CO₃ (1187 mg, 8.59 mmol) at 25 °C. After addition, the mixture was stirred at 100 °C for 16 h. The reaction mixture was diluted with water (10 mL) and extracted with EtOAc (20 mL × 2). The combined organic phases were dried over Na₂SO₄ and filtered, and the filtrate was concentrated under reduced pressure. The residue was purified by silica gel chromatography eluting with 0% to 90% EtOAc in petroleum ether over 15 min to afford tert-butyl 1-(1-((benzyloxy)carbonyl)piperidin-4-yl)-3-iodo-1,4,6,7-tetrahydro-5H-pyrazolo[4,3-c]pyridine-5-carboxylate (700 mg, 40% yield) as a yellow solid. ¹H NMR (400 MHz, DMSO-*d₆*) δ 7.50 - 7.35 (m, 5H), 5.10 (s, 2H), 4.31 - 4.29 (m, 2H), 4.15 - 4.10 (m, 4H), 3.61 - 3.58 (m, 2H), 3.02 - 2.98 (m, 2H), 2.75 - 2.70 (m, 2H), 1.84 - 1.75 (m, 4H), 1.42 (s, 9H). LC-MS: (ESI) m/z [M+H]⁺ 567.1.

A solution of tert-butyl 1-(1-((benzyloxy)carbonyl)piperidin-4-yl)-3-iodo-1,4,6,7-tetrahydro-5H-pyrazolo[4,3-c]pyridine-5-carboxylate (1.5 g, 2.64 mmol), 7-(difluoromethyl)-6-(1-methyl-1H-pyrazol-4-yl)-1,2,3,4-tetrahydroquinoline (836 mg, 3.17 mmol), XPhos-Pd G3 (224 mg, 0.26 mmol) and t-BuONa (763 mg, 7.94 mmol) in 1,4-dioxane (20 mL) was heated at 110 °C for 16 h. LCMS showed consumption of starting material and formation of desired product. The mixture was diluted with H₂O (50 mL), extracted with EtOAc (50 mL × 3), washed with brine (50 mL), dried over anhydrous Na₂SO₄. The organic layer was concentrated under reduced pressure to afford crude product. The residue was purified by flash column chromatography (eluent: 0-10% MeOH in DCM) to afford tert-butyl 1-(1-((benzyloxy)carbonyl)piperidin-4-yl)-3-(7-(difluoromethyl)-6-(1-methyl-1H-pyrazol-4-yl)-3,4-dihydroquinolin-1(2H)-yl)-1,4,6,7-tetrahydro-5H-pyrazolo[4,3-c]pyridine-5-carboxylate (400 mg, 19% yield) as a yellow solid. LC-MS: (ESI) m/z [M+H]⁺ 702.2.

To a solution of tert-butyl 1-(1-((benzyloxy)carbonyl)piperidin-4-yl)-3-(7-(difluoromethyl)-6-(1-methyl-1H-pyrazol-4-yl)-3,4-dihydroquinolin-1(2H)-yl)-1,4,6,7-tetrahydro-5H-pyrazolo[4,3-c]pyridine-5-carboxylate (400 mg, 0.57 mmol) in DCM (4 mL) was added TFA (4 mL) at 25 °C. The mixture was stirred at 25 °C for 1 h, then concentrated under reduced pressure to afford benzyl 4-(3-(7-(difluoromethyl)-6-(1-methyl-1H-pyrazol-4-yl)-3,4-dihydroquinolin-1(2H)-yl)-4,5,6,7-tetrahydro-1H-pyrazolo[4,3-c]pyridin-1-yl)piperidine-1-carboxylate (300 mg, 78% yield) as a yellow solid. LC-MS: (ESI) m/z [M+H]⁺ 602.3.

To a solution of benzyl 4-(3-(7-(difluoromethyl)-6-(1-methyl-1H-pyrazol-4-yl)-3,4-dihydroquinolin-1(2H)-yl)-4,5,6,7-tetrahydro-1H-pyrazolo[4,3-c]pyridin-1-yl)piperidine-1-carboxylate (300 mg, 0.49 mmol) in DCM (5 mL) were added N-methylcarbamoyl chloride (69 mg, 0.74 mmol) and TEA (151 mg, 1.49 mmol). After addition, the mixture was stirred at 25 °C for 1 h. The reaction mixture was diluted with water (10 mL) and extracted with EtOAc (20 mL × 2). The combined organic phases were dried over Na₂SO₄ and filtered, and the filtrate was concentrated under reduced pressure. The residue was purified by silica gel chromatography eluting with 0% to 90% EtOAc in petroleum ether over 15 min to afford benzyl 4-(3-(7-(difluoromethyl)-6-(1-methyl-1H-pyrazol-4-yl)-3,4-dihydroquinolin-1(2H)-yl)-5-(methylcarbamoyl)-4,5,6,7-tetrahydro-1H-pyrazolo[4,3-c]pyridin-1-yl)piperidine-1-carboxylate (250 mg, 68% yield) as a yellow solid. LC-MS: (ESI) m/z [M+H]⁺ 659.3.

To a stirred solution of benzyl 4-(3-(7-(difluoromethyl)-6-(1-methyl-1H-pyrazol-4-yl)-3,4-dihydroquinolin-1(2H)-yl)-5-(methylcarbamoyl)-4,5,6,7-tetrahydro-1H-pyrazolo[4,3-c]pyridin-1-yl)piperidine-1-carboxylate (120 mg, 0.18 mmol) in EtOH (3 mL) were added Pd/C (19 mg, 10 wt%, 0.18 mmol) and 1,4-dioxane/HCl (0.3 mL) successively. The reaction mixture was stirred at 25 °C under H₂ atmosphere (balloon) for 5 h. The reaction mixture was filtered through a pad of Celite, then the filtrate was concentrated under reduced pressure to afford 3-(7-(difluoromethyl)-6-(1-methyl-1H-pyrazol-4-yl)-3,4-dihydroquinolin-1(2H)-yl)-N-methyl-1-(piperidin-4-yl)-1,4,6,7-tetrahydro-5H-pyrazolo[4,3-c]pyridine-5-carboxamide (80 mg, 75% yield) as a white solid. LC-MS: (ESI) m/z [M+H]⁺ 525.3.

To a solution of 3-(7-(difluoromethyl)-6-(1-methyl-1H-pyrazol-4-yl)-3,4-dihydroquinolin-1(2H)-yl)-N-methyl-1-(piperidin-4-yl)-1,4,6,7-tetrahydro-5H-pyrazolo[4,3-c]pyridine-5-carboxamide (50 mg, 0.09 mmol) in DCM (1 mL) were added 2-chloroethanesulfonyl chloride (23 mg, 0.14 mmol) and DIEA (12 mg, 0.09 mmol). After addition, the mixture was stirred at 25 °C for 1 h. The reaction mixture was concentrated under reduced pressure. The residue was purified by reverse-phase column (eluent: 22-60% ACN in H₂O, 0.1% FA) to afford 3-(7-(difluoromethyl)-6-(1-methyl-1H-pyrazol-4-yl)-3,4-dihydroquinolin-1(2H)-yl)-N-methyl-1-(1-(vinylsulfonyl)piperidin-4-yl)-1,4,6,7-tetrahydro-5H-pyrazolo[4,3-c]pyridine-5-carboxamide (Compound 1369, 7 mg, 11% yield) as a white solid.

¹H NMR (400 MHz, DMSO-*d₆*) δ 7.75 (s, 1H), 7.50 (s, 1H), 7.10 (s, 1H), 6.93 - 6.65 (m, 3H), 6.54 - 6.52 (m, 1H), 6.18 - 6.11 (m, 2H), 4.25 - 4.23 (m, 1H), 4.03(s, 2H), 3.85 (s, 3H), 3.61 - 3.55 (m, 6H), 2.95 - 2.75 (m, 4H), 2.75 - 2.33 (m, 2H), 2.51 - 2.50 (m, 3H), 2.01 - 1.95 (m, 6H). ¹⁹F NMR (400MHz, DMSO-d6) δ ppm -108.18. LC-MS: (ESI) m/z [M+H]⁺ 615.3.

### Example 1-10 (Synthesis of 1377)

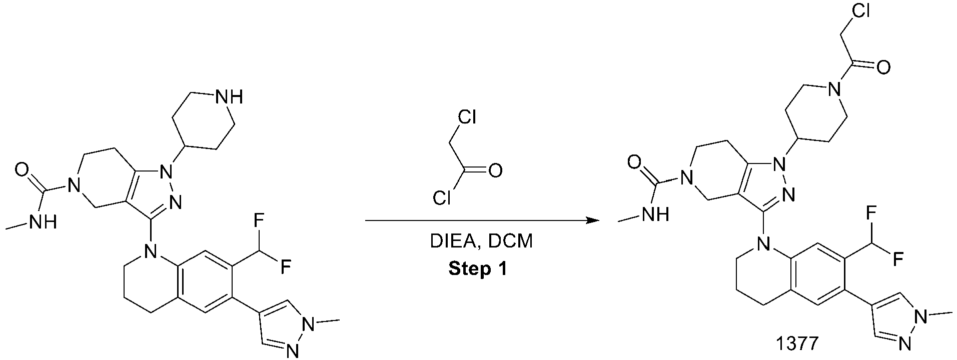

To a solution of 3-[7-(difluoromethyl)-6-(1-methylpyrazol-4-yl)-3,4-dihydro-2H-quinolin-1-yl]-N-methyl-1-(piperidin-4-yl)-4H,6H,7H-pyrazolo[4,3-c]pyridine-5-carboxamide (20 mg, 0.03 mmol) in DCM (1 mL) were added 2-chloroacetyl chloride (8 mg, 0.07 mmol) and DIEA (14 mg, 0.11 mmol) at 25 °C. After addition, the mixture was stirred at 25 °C for 1 h. The reaction mixture was concentrated under reduced pressure. The reaction mixture was purified by preparative HPLC (0% to 100% CH₃CN over 10 min) to afford 1-(1-(2-chloroacetyl)piperidin-4-yl)-3-(7-(difluoromethyl)-6-(1-methyl-1H-pyrazol-4-yl)-3,4-dihydroquinolin-1(2H)-yl)-N-methyl-1,4,6,7-tetrahydro-5H-pyrazolo[4,3-c]pyridine-5-carboxamide (4.2 mg, 16% yield) as a white solid.

¹H NMR (400 MHz, DMSO-*d₆*) δ 7.75 (s, 1H), 7.49 (s, 1H), 7.09 (s, 1H), 6.92 - 6.64 (m, 2H), 6.56 - 6.55 (m, 1H), 4.50 - 4.27 (m, 5H), 4.01 - 3.88 (m, 2H), 3.86 (s, 3H), 3.58 - 3.56 (m, 2H), 2.83 - 2.74 (m, 6H), 2.55 - 2.50 (m, 3H), 2.03 - 1.76 (m, 6H).

### Example 1-11 (Synthesis of 1378)

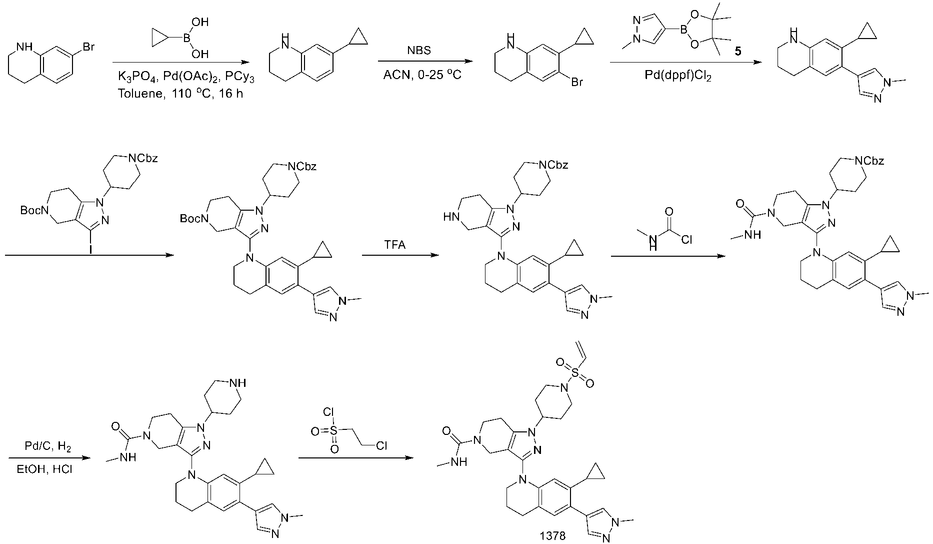

To a solution of 7-bromo-1,2,3,4-tetrahydroquinoline (2.0 g, 0.0094 mol), cyclopropylboranediol (1.2 g, 0.014 mol) and K₃PO₄ (6.0 g, 0.028 mol) in toluene/H₂O (55 mL) were added Pd(OAc)₂ (0.21 g, 0.0009 mol) and Pcy₃ (0.4 g, 0.0014 mol). The mixture was stirred at 110 °C for 16 h under N₂ protection. The mixture was concentrated under reduced pressure. The residue was purified by silica gel chromatography eluting with 0 to 10% EtOAc in petroleum ether over 15 min to afford 7-cyclopropyl-1,2,3,4-tetrahydroquinoline (1.05 g, 62% yield) as a yellow solid.

To a solution of 7-cyclopropyl-1,2,3,4-tetrahydroquinoline (1.09 g, 0.0063 mol) in CAN (40 mL) was added N-Bromosuccinimide (1.12 g, 0.0063 mol) at 0 °C. The mixture was stirred at 25 °C for 2 h. The mixture was concentrated under reduced pressure. The residue was purified by silica gel chromatography eluting with 0 to 10% EtOAc in petroleum ether over 20 min to afford 6-bromo-7-cyclopropyl-1,2,3,4-tetrahydroquinoline (1.1 g, 68% yield) as a yellow liquid.

To a solution of 6-bromo-7-cyclopropyl-1,2,3,4-tetrahydroquinoline (500 mg, 1.98 mmol) and 1-methyl-4-(4,4,5,5-tetramethyl-1,3,2-dioxaborolan-2-yl)-pyrazole (619 mg, 2.97 mmol) in dioxane/H₂O (11 mL) were added K₂CO₃ (822 mg, 5.95 mmol) and Pd(dppf)Cl₂ (145 mg, 0.20 mmol). The mixture was stirred at 90 °C for 3 h. The mixture was concentrated under reduced pressure. The residue was purified by silica gel chromatography eluting with 0 to 20% EtOAc in petroleum ether over 15 min to afford 7-cyclopropyl-6-(1-methyl-1H-pyrazol-4-yl)-1,2,3,4-tetrahydroquinoline (380 mg, 76% yield) as a yellow gum.

To a solution of benzyl 4-{5-[(tert-butoxy)carbonyl]-3-iodo-4H,6H,7H-pyrazolo[4,3-c]pyridin-1-yl}piperidine-1-carboxylate (1020 mg, 1.80 mmol) and 7-cyclopropyl-6-(1-methypyrazol-4-yl)-1,2,3,4-tetrahydroquinoline (380 mg, 1.50 mmol) in 1,4-dioxane (20 mL) were added RuPhos Pd G4 (255 mg, 0.30 mmol) and t-BuONa (432 mg, 4.50 mmol). The mixture was stirred at 80 °C for 4 h. The mixture was concentrated under reduced pressure. The residue was purified by silica gel chromatography eluting with 0 to 80% EtOAc in petroleum ether over 20 min to afford benzyl 4-{5-[(tert-butoxy)carbonyl]-3-[7-cyclopropyl-6-(1-methylpyrazol-4-yl)-3,4-dihydro-2H-quinolin-1-yl]-4H,6H,7H-pyrazolo[4,3-c]pyridin-1-yl)piperidine-1-carboxylate (580 mg, 53% yield) as a yellow solid.

A mixture of benzyl 4-{5-[(tert-butoxy)carbonyl]-3-[7-cyclopropyl-6-(1-methylpyrazol-4-yl)-3,4-dihydro-2H-quinolin-1-yl]-4H,6H,7H-pyrazolo[4,3-c]pyridin-1-yl)piperidine-1-carboxylate (100 mg, 0.14 mmol) in DCM/TFA (4 mL) was stirred at 25 °C for 1 h. The mixture was concentrated under reduced pressure to afford 4-{3-[7-cyclopropyl-6-(-1-methylpyrazol-4-yl)-3,4-dihydro-2H -quinolin-1-yl]-4H,5H,6H, 7H -pyridin-1-yl }piperidine-1-carboxylate (80 mg, 94% yield) as a yellow gum.

To a solution of benzyl 4-{3-[7-cyclopropyl-6-(1-methylpyrazol-4-yl)-3,4-dihydro-2H-quinolin-1-yl]-4H,5H,6H,7H-pyrazolo[4,3-c]pyridin-1-yl}piperidine-1-carboxylate (80 mg, 0.14 mmol) and TEA (68 mg, 0.68 mmol) in DCM (2 mL) was added N-methylcarbamoyl chloride (38 mg, 0.41 mmol). The mixture was stirred at 25 °C for 1 h. LCMS showed consumption of starting material and detection of desired mass. The mixture was concentrated under reduced pressure, and the residue was purified by silica gel chromatography eluting with 0 to 100% THF in petroleum ether over 15 min to afford benzyl 4-{3-[7-cyclopropyl-6-(1-methylpyrazol-4-yl)-3,4-dihydro-2H-quinolin-1-yl]-5-(methylcarbamoyl)-4H,6H,7H-pyrazolo[4,3-c]pyridin-1-yl}piperidine-1-carboxylate (80 mg, 87% yield) as a yellow solid.

To a solution of benzyl 4-{3-[7-cyclopropyl-6-(1-methylpyrazol-4-yl)-3,4-dihydro-2H-quinolin-1-yl]-5-(methylcarbamoyl)-4H,6H,7H-pyrazolo[4,3-c]pyridin-1-yl}piperidine-1-carboxylate (90 mg, 0.1387 mmol) and Pd/C (29.52 mg, 0.2774 mmol) in EtOH (5 mL) was added a solution of HCl in dioxane (0.1 mL). The mixture was stirred at 25 °C for 2 h. LCMS showed consumption of starting material and detection of desired mass. The mixture was filtered and concentrated under reduced pressure to afford 3-[7-cyclopropyl-6-(1-methylpyrazol-4-yl)-3,4-dihydro-2H-quinolin-1-yl]-N-methyl-1-(piperidin-4-yl)-4H,6H,7H-pyrazolo[4,3-c]pyridine-5-carboxamide (70 mg, 98% yield) as a yellow gum.

To a solution of 3-[7-cyclopropyl-6-(1-methylpyrazol-4-yl)-3,4-dihydro-2H-quinolin-1-yl]-N-methyl-1-(piperidin-4-yl)-4H,6H,7H-pyrazolo[4,3-c]pyridine-5-carboxamide (70 mg, 0.14 mmol) and TEA (83 mg, 0.82 mmol) in DCM (3 mL) was added 2-chloroethanesulfonyl chloride (44 mg, 0.27 mmol). The mixture was stirred at 25 °C for 0.5 h. The mixture was concentrated under reduced pressure. The residue was purified by pre-HPLC eluting with 43% to 53% CH₃CN in CH₃CN/H₂O over 10 min to afford 3-[7-cyclopropyl-6-(1-methylpyrazol-4-yl)-3,4-dihydro-2H-quinolin-1-yl]-1-(1-(vinylsulfonyl)piperidin-4-yl)-N-methyl-4H,6H,7H-pyrazolo[4,3-c]pyridine-5-carboxamide (12 mg, 12% yield) as a gray solid.

¹H NMR (400 MHz, dmso-*d₆*) δ 7.88 (s, 1H), 7.65 (s, 1H), 6.99 (s, 1H), 6.99-6.84 (m, 1H), 6.24 - 6.08 (m, 3H), 5.58-5.24 (m, 1H), 4.26 - 4.17 (m, 1H), 3.97 (s, 2H), 3.87 (s, 3H), 3.67 - 3.57 (m, 4H), 3.55 - 3.50 (m, 2H), 2.90 - 2.81 (m, 3H), 2.78 - 2.69 (m, 4H), 2.54 (s, 3H), 2.01 - 1.84 (m, 7H), 0.84 - 0.73 (m, 2H), 0.41 - 0.31 (m, 2H).

### Example 1-12 (Synthesis of 1379)

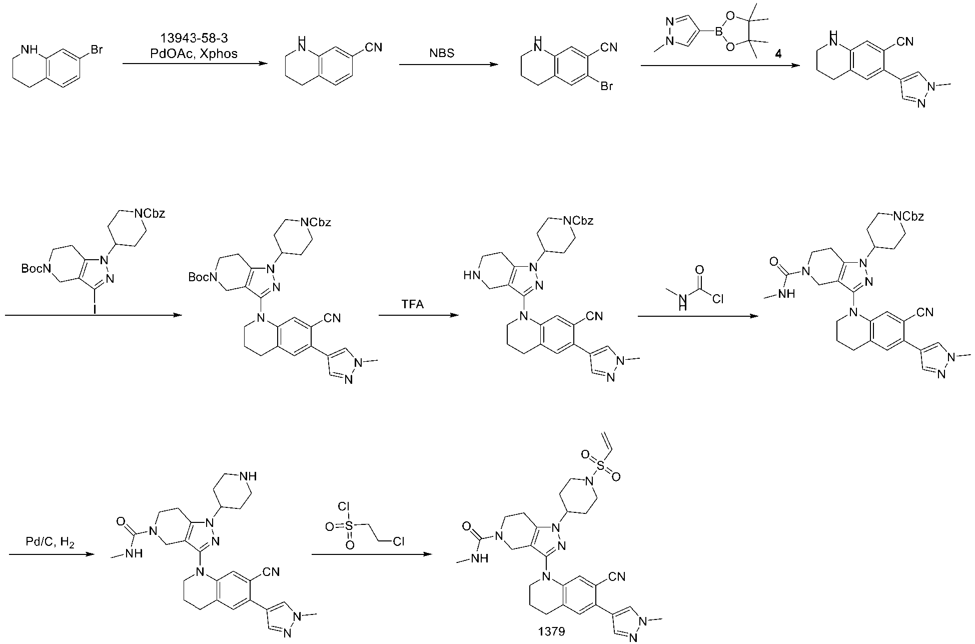

To a solution of 7-bromo-1,2,3,4-tetrahydroquinoline (2.3 g, 0.011 mol) and potassium ferrocyanide(II) (2.3 g, 0.0054 mol) in 1,4-dioxane/H₂O = 1:1 (8 mL) were added XPhos (0.51 g, 0.0010 mol), XPhos Pd G3 (0.9 g, 0.001 mol) and Pd(OAc)₂ (7.3 g, 0.032 mol). The mixture was stirred at 110 °C under N₂ for 12 h. The mixture was concentrated under reduced pressure. The residue was purified by silica gel chromatography eluting with 0 to 10% EtOAc in petroleum ether over 20 min to afford 1,2,3,4-tetrahydroquinoline-7-nitrile (1.3 g, 71% yield) as a yellow solid.

To a solution of 1,2,3,4-tetrahydroquinoline-7-nitrile (1.3 g, 0.0079 mol) in DCM (50 mL) was added N-bromosuccinimide (1.4 g, 0.0079 mol) at 0 °C. The mixture was stirred at 25 °C for 1 h. The mixture was concentrated under reduced pressure. The residue was purified by silica gel chromatography eluting with 0 to 10% EtOAc in petroleum ether over 20 min to afford 6-bromo-1,2,3,4-tetrahydroquinoline-7-nitrile (0.4 g, 22% yield) as a yellow solid.

To a solution of 6-bromo-1,2,3,4-tetrahydroquinoline-7-nitrile (340 mg, 1.43 mmol) and 1-methyl-4-(4,4,5,5-tetramethyl-1,3,2-dioxaborolan-2-yl)-1H-pyrazole (418 mg, 2.01 mmol) in 1,4-dioxane/H₂O (22 mL) were added K₂CO₃ (595 mg, 4.30 mmol) and Pd(dppf)Cl₂·DCM (234 mg, 0.29 mmol). The mixture was stirred at 90 °C for 3 h. The mixture was concentrated under reduced pressure. The residue was purified by silica gel chromatography eluting with 0 to 50% EtOAc in petroleum ether over 20 min to afford 6-(1-methylpyrazol-4-yl)-1,2,3,4-tetrahydroquinoline-7-nitrile (270 mg, 79% yield) as a white solid.

To a solution of benzyl 4-{5-[(tert-butoxy)carbonyl]-3-iodo-4H,6H,7H-pyrazolo[4,3-c]pyridin-1-yl}piperidine-1-carboxylate (742 mg, 1.31 mmol) and 6-(1-methylpyrazol-4-yl)-1,2,3,4-tetrahydroquinoline-7-nitrile (250 mg, 1.05 mmol) in 1,4-dioxane (25 mL) were added t-BuONa (377 mg, 3.93 mmol) and RuPhos Pd G4 (223 mg, 0.26 mmol). The mixture was stirred at 80 °C for 6 h. The mixture was concentrated under reduced pressure. The residue was purified by silica gel chromatography eluting with 0 to 80% EtOAc in petroleum ether over 20 min to afford benzyl 4-{5-[(tert-butoxy)carbonyl]-3-[7-cyano-6-(1-methylpyrazol-4-yl)-3,4-dihydro-2H-quinolin-1-yl]-4H,6H,7H-pyrazolo[4,3-c]pyridin-1-yl}piperidine-1-carboxylate (320 mg, 34% yield) as a yellow solid.

A mixture of benzyl 4-{5-[(tert-butoxy)carbonyl]-3-[7-cyano-6-(1-methylpyrazol-4-yl)-3,4-dihydro-2H-quinolin-1-yl]-4H,6H,7H-pyrazolo[4,3-c]pyridin-1-yl}piperidine-1-carboxylate (100 mg, 0.15 mmol) in DCM/TFA (2 mL) was stirred at 25 °C for 1 h. The mixture was concentrated under reduced pressure to afford 4-{3-[7-cyano-6-(1-ethylpyrazol-4-yl)-3,4-dihydro-2H-quinolin-1-yl)-4H,5H,6H,7H-pyrazolo[4,3-c]pyridin-1-yl}piperidine-1-carboxylate (80 mg, 89% yield) as a yellow gum.

To a solution of benzyl 4-{3-[7-cyano-6-(1-methylpyrazol-4-yl)-3,4-dihydro-2H-quinolin-1-yl]-4H,5H,6H,7H-pyrazolo[4,3-c]pyridin-1-yl)piperidine-1-carboxylate (80 mg, 0.14 mmol) and TEA (70 mg, 0.69 mmol) in DCM (2 mL) was added N-methylcarbamoyl chloride (39 mg, 0.42 mmol). The mixture was stirred at 25 °C for 1 h. The mixture was concentrated under reduced pressure. The residue was purified by silica gel chromatography eluting with 0 to 100% THF in petroleum ether over 20 min to afford benzyl 4-{3-[7-cyano-6-(1-methylpyrazol-4-yl)-3,4-dihydro-2H-quinolin-1-yl]-5-(methylcarbamoyl)-4H,6H,7H-pyrazolo[4,3-c]pyridin-1-yl}piperidine-1-carboxylate (70 mg, 76% yield) as a yellow solid.

To a solution of benzyl 4-{3-[7-cyano-6-(1-methylpyrazol-4-yl)-3,4-dihydro-2H-quinolin-1-yl]-5-(methylcarbamoyl)-4H,6H,7H-pyrazolo[4,3-c]pyridin-1-yl}piperidine-1-carboxylate (50 mg, 0.079 mmol) in EtOH (5 mL) was added Pd/C (25 mg, 0.24 mmol). The mixture was stirred at 25 °C for 3 h. The mixture was filtered and concentrated under reduced pressure to afford 3-[7-cyano-6-(1-methylpyrazol-4-yl)-3,4-dihydro-2H-quinolin-1-yl]-N-methyl-1-(piperidin-4-yl)-4H,6H,7H-pyrazolo[4,3-c]pyridine-5-carboxamide (40 mg, 96% yield) as a yellow solid.

To a solution of 3-[7-cyano-6-(1-methylpyrazol-4-yl)-3,4-dihydro-2H-quinolin-1-yl]-N-methyl-1-(piperidin-4-yl)-4H,6H,7H-pyrazolo[4,3-c]pyridine-5-carboxamide (40 mg, 0.080 mmol) and TEA (41 mg, 0.40 mmol) in DCM (5 mL) was added 2-chloroethanesulfonyl chloride (26 mg, 0.16 mmol). The mixture was stirred at 25 °C for 1 h. The mixture was concentrated under reduced pressure. The residue was purified by Pre-HPLC eluting with 40% to 50% CH₃CN inH₂O over 10 min to afford 3-[7-cyano-6-(1-methylpyrazol-4-yl)-3,4-dihydro-2H-quinolin-1-yl]-1-[1-(vinylsulfonyl)piperidin-4-yl]-N-methyl-4H,6H,7H-pyrazolo[4,3-c]pyridine-5-carboxamide (40 mg, 80% yield) as a yellow solid.

1H NMR (400 MHz, dmso-d6) δ 8.04 (s, 1H), 7.77 (s, 1H), 7.33 (s, 1H), 6.87-6.82 (m, 1H), 6.67 (s, 1H), 6.53 (d, J = 4.8 Hz, 1H), 6.16-6.09 (m, 2H), 4.26 - 4.16 (m, 1H), 4.01 (d, J = 9.6 Hz, 2H), 3.86 (s, 3H), 3.59-3.52 (m, 6H), 2.85 (d, J = 5.6 Hz, 4H), 2.70 (s, 2H), 2.53 (d, J = 4.8 Hz, 3H), 1.95 (s, 6H).

### Example 1-13 (Synthesis of 1374 and 1375)

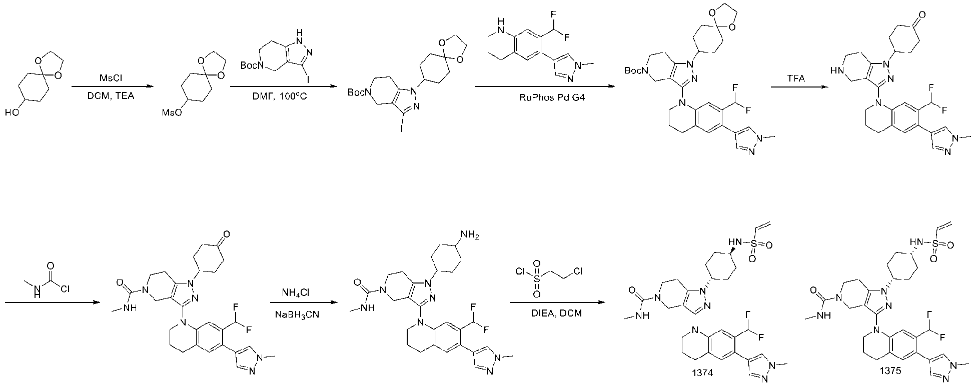

To a solution of 1,4-dioxaspiro[4.5]decan-8-ol (2 g, 12.64 mmol) in DCM (50 mL) were added TEA (6.38 g, 63.21 mmol) and MsCl (4.34 g, 37.92 mmol) at 25 °C. After addition, the mixture was stirred at 25 °C for 1 h. The reaction mixture was diluted with water (100 mL) and extracted with EtOAc (200 mL × 2). The combined organic phases were dried over Na₂SO₄ and filtered. The filtrate was concentrated under reduced pressure. The residue was purified by silica gel chromatography eluting with 0 to 90% EtOAc in petroleum ether over 15 min to afford 1,4-dioxaspiro[4.5]decan-8-yl methanesulfonate (2 g, 63% yield) as a yellow solid.

To a solution of tert-butyl 3-iodo-1H,4H,6H,7H-pyrazolo[4,3-c]pyridine-5-carboxylate (2.0 g, 5.72 mmol) in DMF (20 mL) were added Cs₂CO₃ (5.59 g, 17.18 mmol) and 1,4-dioxaspiro[4.5]decan-8-yl methanesulfonate (1.75 g, 7.44 mmol) at 25 °C. After addition, the mixture was stirred at 100 °C for 8 h. The reaction mixture was diluted with water (100 mL) and extracted with EtOAc (200 mL × 2). The combined organic phases were dried over Na₂SO₄ and filtered. The filtrate was concentrated under reduced pressure. The residue was purified by silica gel chromatography eluting with 0 to 90% EtOAc in petroleum ether over 15 min to afford tert-butyl 3-iodo-1-(1,4-dioxaspiro[4.5]decan-8-yl)-1,4,6,7-tetrahydro-5H-pyrazolo[4,3-c]pyridine-5-carboxylate (1.5 g, 50% yield) as a yellow solid.

A mixture of tert-butyl 3-iodo-1-(1,4-dioxaspiro[4.5]decan-8-yl)-1,4,6,7-tetrahydro-5H-pyrazolo[4,3-c]pyridine-5-carboxylate (1.2 g, 2.45 mmol), RuPhos Pd G4 (208 mg, 0.24 mmol), 7-(difluoromethyl)-6-(1-methylpyrazol-4-yl)-1,2,3,4-tetrahydroquinoline (645 mg, 2.45 mmol) and t-BuONa (706 mg, 7.35 mmol) in dioxane (20 mL) was stirred at 25 °C under N₂. The mixture was heated to 100 °C and stirred at this temperature for 16 h. The reaction mixture was concentrated under reduced pressure. The residue was purified by silica gel chromatography eluting with 0 to 80% EtOAc in petroleum ether over 15 min to afford tert-butyl 3-(7-(difluoromethyl)-6-(1-methyl-1H-pyrazol-4-yl)-3,4-dihydroquinolin-1(2H)-yl)-1-(1,4-dioxaspiro[4.5]decan-8-yl)-1,4,6,7-tetrahydro-5H-pyrazolo[4,3-c]pyridine-5-carboxylate (0.8 g, 45% yield) as a yellow solid.

To a solution of tert-butyl 3-(7-(difluoromethyl)-6-(1-methyl-1H-pyrazol-4-yl)-3,4-dihydroquinolin-1(2H)-yl)-1-(1,4-dioxaspiro[4.5]decan-8-yl)-1,4,6,7-tetrahydro-5H-pyrazolo[4,3-c]pyridine-5-carboxylate (100 mg, 0.16 mmol) in DCM (2 mL) was added TFA (1 mL) at 25 °C. After addition, the mixture was stirred at 25 °C for 1 h. The reaction mixture was concentrated under reduced pressure. The residue was purified by silica gel chromatography eluting with 0 to 90% EtOAc in petroleum ether over 15 min to afford 4-(3-(7-(difluoromethyl)-6-(1-methyl-1H-pyrazol-4-yl)-3,4-dihydroquinolin-1(2H)-yl)-4,5,6,7-tetrahydro-1H-pyrazolo[4,3-c]pyridin-1-yl)cyclohexan-1-one (70 mg, 81% yield) as a yellow solid.

To a solution of 4-(3-(7-(difluoromethyl)-6-(1-methyl-1H-pyrazol-4-yl)-3,4-dihydroquinolin-1(2H)-yl)-4,5,6,7-tetrahydro-1H-pyrazino[4,3-c]pyridin-1-yl)cyclohexan-1-one (270 mg, 0.56 mmol) in DCM (5 mL) were added DIEA (217 mg, 1.6857 mmol) and N-methylcarbamoyl chloride (78 mg, 0.84 mmol) at 25 °C. After addition, the mixture was stirred at 25 °C for 0.5 h. The reaction mixture was concentrated under reduced pressure. The residue was purified by silica gel chromatography eluting with 0 to 90% EtOAc in petroleum ether over 15 min to afford 3-(7-(difluoromethyl)-6-(1-methyl-1H-pyrazol-4-yl)-3,4-dihydroquinolin-1(2H)-yl)-N-methyl-1-(4-oxocyclohexyl)-1,4,6,7-tetrahydro-5H-pyrazolo[4,3-c]pyridine-5-carboxamide (230 mg, 68% yield) as a yellow solid.

To a solution of 3-(7-(difluoromethyl)-6-(1-methyl-1H-pyrazol-4-yl)-3,4-dihydroquinolin-1(2H)-yl)-N-methyl-1-(4-oxocyclohexyl)-1,4,6,7-tetrahydro-5H-pyrazolo[4,3-c]pyridine-5-carboxamide (300 mg, 0.55 mmol) in MeOH (5 mL) were added NH₄Cl (149 mg, 2.79 mmol) and NaCNBH₃ (175 mg, 2.79 mmol) at 25 °C. After addition, the mixture was stirred at 25 °C for 1 h. The reaction mixture was concentrated under reduced pressure. The residue was purified by silica gel chromatography eluting with 0 to 90% EtOAc in petroleum ether over 15 min to afford 1-(4-aminocyclohexyl)-3-(7-(difluoromethyl)-6-(1-methyl-1H-pyrazol-4-yl)-3,4-dihydroquinolin-1(2H)-yl)-N-methyl-1,4,6,7-tetrahydro-5H-pyrazolo[4,3-c]pyridine-5-carboxamide (200 mg, 59% yield) as a yellow solid.

To a solution of 1-(4-aminocyclohexyl)-3-(7-(difluoromethyl)-6-(1-methyl-1H-pyrazol-4-yl)-3,4-dihydroquinolin-1(2H)-yl)-N-methyl-1,4,6,7-tetrahydro-5H-pyrazolo[4,3-c]pyridine-5-carboxamide (60 mg, 0.11 mmol) in DCM (2 mL) were added DIEA (43 mg, 0.34 mmol) and 2-chloroethanesulfonyl chloride (36 mg, 0.22 mmol) at 25 °C. After addition, the mixture was stirred at 25 °C for 10 min. The reaction mixture was concentrated under reduced pressure. The reaction mixture was purified by preparative HPLC (0% to 80% CH₃CN over 10 min) to afford 3-(7-(difluoromethyl)-6-(1-methyl-1H-pyrazol-4-yl)-3,4-dihydroquinolin-1(2H)-yl)-N-methyl-1-((1r,4r)-4-(vinylsulfonamido)cyclohexyl)-1,4,6,7-tetrahydro-5H-pyrazolo[4,3-c]pyridine-5-carboxamide (3 mg, 4% yield) and 3-(7-(difluoromethyl)-6-(1-methyl-1H-pyrazol-4-yl)-3,4-dihydroquinolin-1(2H)-yl)-N-methyl-1-((1s,4s)-4-(vinylsulfonamido)cyclohexyl)-1,4,6,7-tetrahydro-5H-pyrazolo[4,3-c]pyridine-5-carboxamide (1.5 mg, 2% yield) as white solids.

1374: 1H NMR (400 MHz, DMSO-d6) δ 7.74 (s, 1H), 7.50 (s, 1H), 7.37 (s, 1H), 7.09 (s, 1H), 6.79 - 6.64 (m, 3H), 6.53 (s, 1H), 6.06 - 5.92 (m, 2H), 4.00 - 3.98 (m, 3H), 3.86 (s, 3H), 3.55 - 3.52 (m, 2H), 3.01 (s, 1H), 2.88 - 2.71 (m, 4H), 2.55 - 2.50 (m, 3H), 2.01 - 1.82 (m, 8H), 1.45 - 1.24 (m, 4H).

1375: 1H NMR (400 MHz, DMSO-d6) δ 7.75 (s, 1H), 7.50 (s, 1H), 7.33 (s, 1H), 7.09 (s, 1H), 6.72 - 6.65 (m, 3H), 6.53 (s, 1H), 6.05 - 5.94 (m, 2H), 4.06 - 4.00 (m, 3H), 3.86 (s, 3H), 3.58 - 3.55 (m, 2H), 3.33 (s, 1H), 2.71 - 2.67 (m, 4H), 2.55 - 2.50 (m, 3H), 2.12 - 1.85 (m, 6H), 1.70 - 1.65 (m, 4H), 1.24 - 1.23 (m, 2H).

### Example 1-14 (Synthesis of 1383)

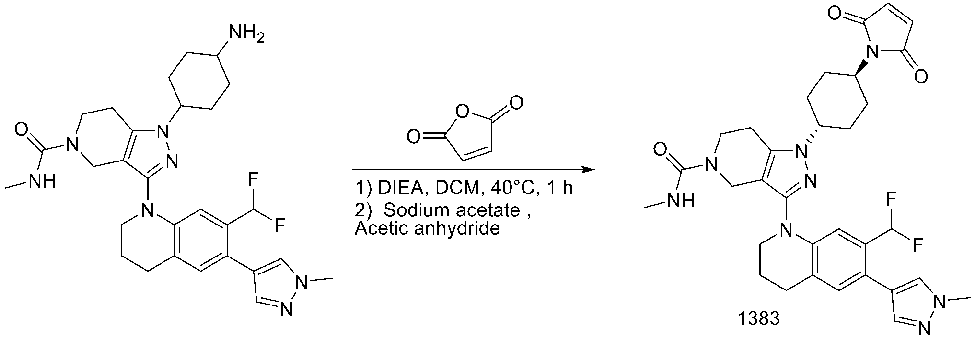

A solution of 1-(4-aminocyclohexyl)-3-[7-(difluoromethyl)-6-(1-methylpyrazol-4-yl)-3,4-dihydro-2H-quinolin-1-yl]-N-methyl-4H,6H,7H-pyrazolo[4,3-c]pyridine-5-carboxamide (40 mg, 0.074 mmol) and furan-2,5-dione (15 mg, 0.15 mmol) in DCM (2 mL) was stirred at 40 °C for 1 h. The mixture was concentrated under reduced pressure. The residue and NaOAc (6 mg, 0.074 mmol) were added to Ac₂O (2 mL). The mixture was stirred at 100 °C for 2 h. The mixture was purified by Pre-HPLC eluting with 50% to 80% CH₃CN in CH₃CN/H₂O over 10 min to afford 3-(7-(difluoromethyl)-6-(1-methyl-1H-pyrazol-4-yl)-3,4-dihydroquinolin-1(2H)-yl)-1-((1r,4r)-4-(2,5-dioxo-2,5-dihydro-1H-pyrrol-1-yl)cyclohexyl)-N-methyl-1,4,6,7-tetrahydro-5H-pyrazolo[4,3-c]pyridine-5-carboxamide (5 mg, 10% yield) as a yellow solid.

1H NMR (400 MHz, DMSO-d6) δ 7.75 (s, 1H), 7.50 (s, 1H), 7.09 (s, 1H), 6.99 (s, 2H), 6.92 (s, 0.25H), 6.80 (d, J = 13.6 Hz, 1.5H), 6.64 (s, 0.25H), 6.54 (d, J = 4.4 Hz, 1H), 4.01 (s, 2H), 3.86 (s, 3H), 3.59 (d, J = 5.2 Hz, 4H), 2.84 (s, 2H), 2.76 (s, 2H), 2.54 (d, J = 4.8Hz, 4H), 2.20 (d, J = 12.4 Hz, 1H), 1.96-1.86(m, 7H), 1.74 (d, J = 10.4 Hz, 2H), 1.23 (s, 2H).

### Example 1-15 (Synthesis of 1387 and 1388)

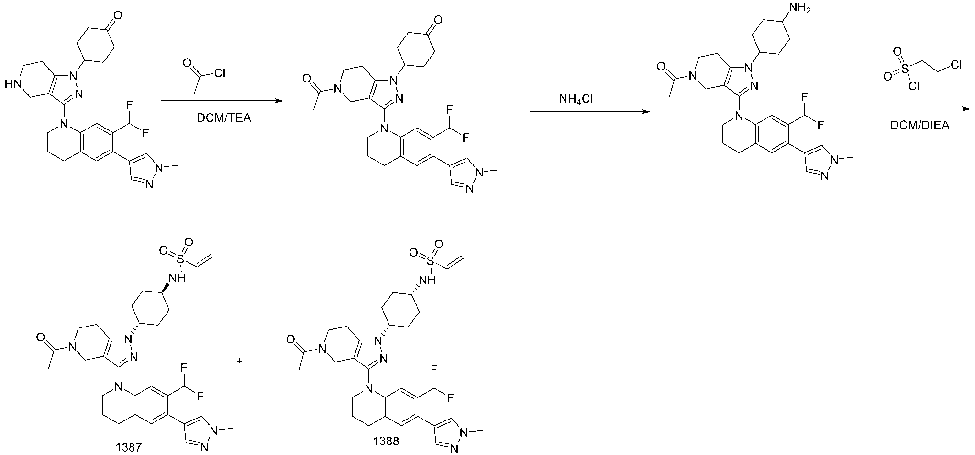

To a solution of 4-{3-[7-(difluoromethyl)-6-(1-methylpyrazol-4-yl)-3,4-dihydro-2H-quinolin-1-yl]-4H,5H,6H,7H-pyrazolo[4,3-c]pyridin-1-yl}cyclohexan-1-one (900 mg, 1.87 mmol) in DCM (20 mL) were added DIEA (726 mg, 5.61 mmol) and acetyl chloride (220 mg, 2.81 mmol) at 25 °C. After addition, the mixture was stirred at 25 °C for 1 h. The reaction mixture was concentrated under reduced pressure. The residue was purified by silica gel chromatography eluting with 0 to 90% EtOAc in petroleum ether over 15 min to afford 4-(5-acetyl-3-(7-(difluoromethyl)-6-(1-methyl-1H-pyrazol-4-yl)-3,4-dihydroquinolin-1(2H)-yl)-4,5,6,7-tetrahydro-1H-pyrazolo[4,3-c]pyridin-1-yl)cyclohexan-1-one (900 mg, 82% yield) as a yellow solid.

To a solution of 4-(5-acetyl-3-(7-(difluoromethyl)-6-(1-methyl-1H-pyrazol-4-yl)-3,4-dihydroquinolin-1(2H)-yl)-4,5,6,7-tetrahydro-1H- pyrazino[4,3-c]pyridin-1-yl)cyclohexan-1-one (400 mg, 0.76 mmol) in MeOH (10 mL) were added NH₄Cl (204 mg, 3.82 mmol) and sodium cyanoborohydride (240 mg, 3.82 mmol) at 25 °C. After addition, the mixture was stirred at 25 °C for 1 h. The reaction mixture was concentrated under reduced pressure. The residue was purified by silica gel chromatography eluting with 0 to 90% EtOAc in petroleum ether over 15 min to afford 1-(1-(4-aminocyclohexyl)-3-(7-(difluoromethyl)-6-(1-methyl-1H-pyrazol-4-yl)-3,4-dihydroquinolin-1(2H)-yl)-1,4,6,7-tetrahydro-5H-pyrazolo[4,3-c]pyridin-5-yl)ethan-1-one (200 mg, 47% yield) as a yellow solid.

To a solution of 1-(1-(4-aminocyclohexyl)-3-(7-(difluoromethyl)-6-(1-methyl-1H-pyrazol-4-yl)-3,4-dihydroquinolin-1(2H)-yl)-1,4,6,7-tetrahydro-5H-pyrazolo[4,3-c]pyridin-5-yl)ethan-1-one (200 mg, 0.38 mmol) in DCM (5 mL) were added DIEA (148 mg, 1.14 mmol) and 2-chloroethanesulfonyl chloride (80 mg, 0.49 mmol) at 25 °C. After addition, the mixture was stirred at 25 °C for 1 h. The reaction mixture was concentrated under reduced pressure. The residue was purified by preparative HPLC (10% to 70% CH₃CN over 10 min) to afford N-((1r,4r)-4-(5-acetyl-3-(7-(difluoromethyl)-6-(1-methyl-1H-pyrazol-4-yl)-3,4-dihydroquinolin-1(2H)-yl)-4,5,6,7-tetrahydro-1H-pyrazolo[4,3-c]pyridin-1-yl)cyclohexyl)ethenesulfonamide (24 mg, 9% yield) and N-((1s,4s)-4-(5-acetyl-3-(7-(difluoromethyl)-6-(1-methyl-1H-pyrazol-4-yl)-3,4-dihydroquinolin-1(2H)-yl)-4,5,6,7-tetrahydro-1H-pyrazolo[4,3-c]pyridin-1-yl)cyclohexyl)ethenesulfonamide (12 mg, 4% yield) as white solids.

1387: 1H NMR (400 MHz, DMSO-d6) δ 7.75 (s, 1H), 7.36 (s, 1H), 7.30 (s, 1H), 7.09 (s, 1H), 6.79 - 6.64 (m, 3H), 6.07 - 6.03 (m, 1H), 5.95 - 5.92 (m, 1H), 4.25 - 4.11 (m, 2H), 4.01 (s, 1H), 3.86 (s, 3H), 3.74 - 3.65 (m, 2H), 3.55 - 3.50 (m, 2H), 3.01 (s, 1H), 2.80 - 2.73 (m, 4H), 2.07 - 1.80 (m, 11H), 1.45 - 1.42 (m, 2H).

1388: 1H NMR (400 MHz, DMSO-d6) δ 7.75 (s, 1H), 7.50 (s, 1H), 7.33 (s, 1H), 7.10 (s, 1H), 6.73 - 6.66 (m, 3H), 6.05 - 5.94 (m, 2H), 4.15 - 4.10 (m, 3H), 3.86 (s, 3H), 3.75 - 3.62 (m, 4H), 3.33 (s, 1H), 2.84 - 2.72 (m, 4H), 2.10 - 2.07 (m, 4H), 1.97 - 1.86 (m, 5H), 1.70 - 1.65 (m, 4H).

### Example 1-16 (Synthesis of 1384)

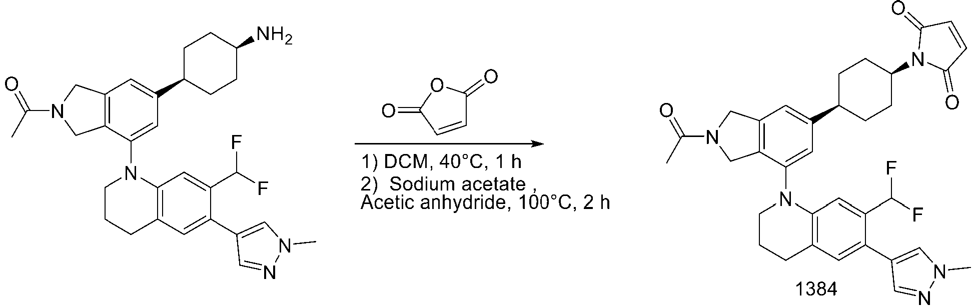

To a solution of 1-[6-(4-aminocyclohexyl)-4-[7-(difluoromethyl)-6-(1-methylpyrazol-4-yl)-3,4-dihydro-2H-quinolin-1-yl]-1,3-dihydroisoindol-2-yl]ethenone (40 mg, 0.077 mmol) and furan-2,5-dione (15 mg, 0.15 mmol) in DCM (2 mL) was added. The mixture was stirred at 40 °C for 1 h. The mixture was concentrated under reduced pressure. The residue and NaOAc (19 mg, 0.23 mmol) were added to Ac₂O (2 mL). The mixture was stirred at 100 °C for 2 h. The mixture was concentrated under reduced pressure. The residue was purified by Prep-HPLC eluting with 57% to 70% CH₃CN in CH₃CN/H₂O over 9 min to afford 1-((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)-1H-pyrrole-2,5-dione (3.8 mg, 8% yield) as a yellow solid.

1H NMR (400 MHz, DMSO-d6) δ 7.74 (d, J = 3.6 Hz, 1H), 7.49 (s, 1H), 7.30 (d, J = 10.4 Hz, 1H), 7.24 (d, J = 9.6 Hz, 1H), 7.13 (s, 1H), 6.92 (d, J = 3.6 Hz, 2H), 6.86 (s, 0.25H), 6.72 (s, 0.5H), 6.58 (s, 0.25H), 6.45 (d, J = 26.4 Hz, 1H), 4.89 (s, 1H), 4.66 (d, J = 18.4 Hz, 2H), 4.31 (s, 1H), 3.96-9-3.92 (m, 1H), 3.86 (s, 3H), 3.60 (d, J = 5.6 Hz, 2H), 3.03 (s, 1H), 2.89 (s, 2H), 2.22 (d, J = 10.4 Hz, 2H), 2.13 - 1.98 (m, 7H), 1.87 - 1.76 (m, 2H), 1.51 (s, 2H).

### Example 1-17 (Synthesis of 1385)

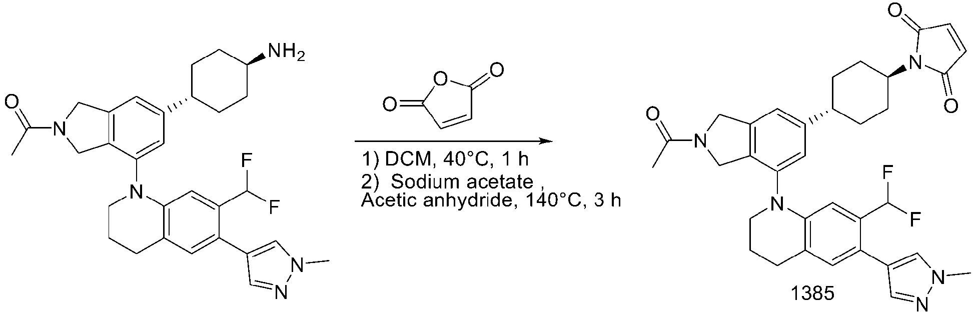

A solution of 1-[6-(4-aminocyclohexyl)-4-[7-(difluoromethyl)-6-(1-methylpyrazol-4-yl)-3,4-dihydro-2H-quinolin-1-yl]-1,3-dihydroisoindol-2-yl]ethenone (50 mg, 0.096 mmol) and furan-2,5-dione (9 mg, 0.096 mol) in DCM (2 mL) was stirred at 40 °C for 1 h. The mixture was concentrated under reduced pressure. The residue and NaOAc (6 mg, 0.067 mmol) were added to Ac₂O (2 mL). The mixture was stirred at 140 °C for 2 h. The mixture was purified by pre-HPLC, eluting with 50% to 80% aqueous CH₃CN over 10 min to afford 1-(4-{2-acetyl-7-[7-(difluoromethyl)-6-(1-methylpyrazol-4-yl)-3,4-dihydro-2H-quinolin-1-yl]-1,3-dihydroisoindol-5-yl}cyclohexyl)pyrrole-2,5-dione (12 mg, 21% yield) as a white solid.

1H NMR (400 MHz, DMSO-d6) δ 7.75 (s, 1H), 7.49 (s, 1H), 7.20-7.15 (m, 2H), 7.12 (s, 1H), 6.98 (d, J = 2.8 Hz, 2H), 6.87-6.60 (m, 1H), 6.41 (d, J = 27.6 Hz, 1H), 4.85 (s, 1H), 4.64-4.60 (m, 2H), 4.27 (s, 1H), 3.98 - 3.90 (m, 1H), 3.87 (d, J = 10.4 Hz, 3H), 3.61 - 3.50 (m, 2H), 2.88 (s, 2H), 2.20 - 2.09 (m, 2H), 2.05 - 1.95 (m, 5H), 1.90 (d, J = 8.4 Hz, 2H), 1.77 - 1.66 (m, 2H), 1.65 - 1.55 (m, 2H), 1.25-1.20 (m, 1H).

### Example 1-18 (Synthesis of 1370)

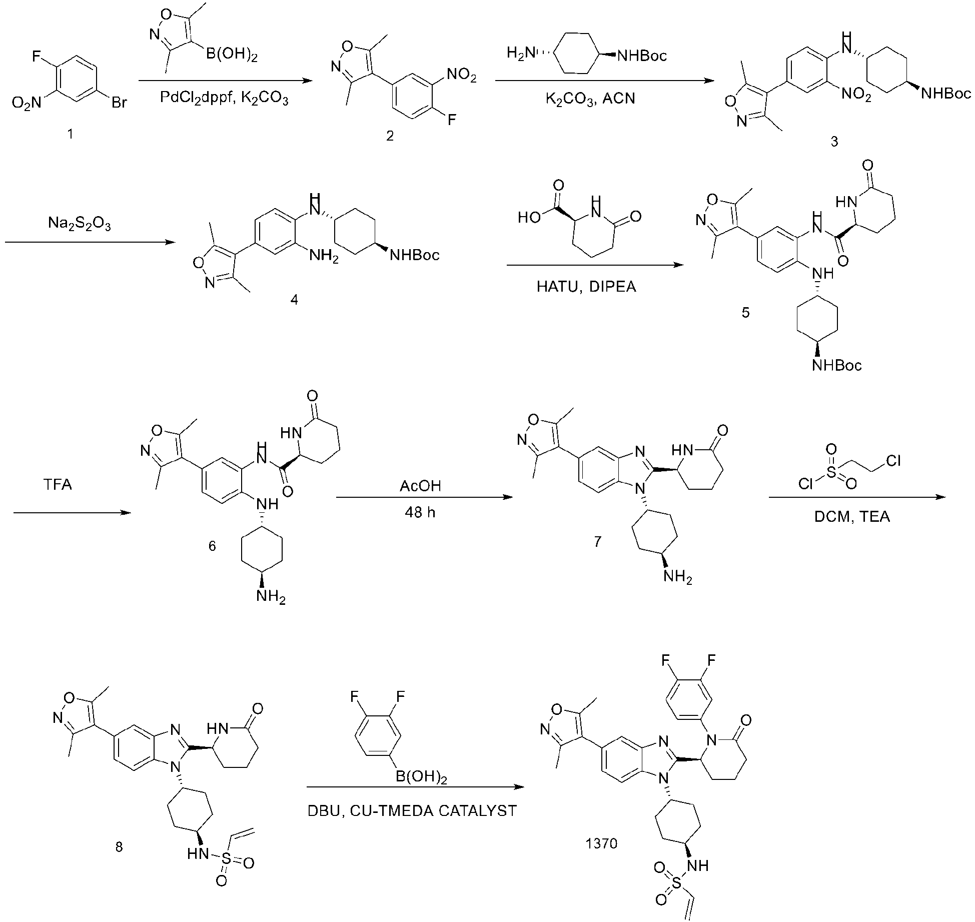

To a solution of 4-bromo-1-fluoro-2-nitrobenzene (3.00 g, 0.0136 mol) and (3,5-dimethyl-1,2-oxazol-4-yl)boranediol (2.30 g, 0.0163 mol) in 1,4-dioxane (30 mL) and H₂O (6.0 mL) were added K₂CO₃ (5.64 g, 0.0408 mol) and Pd(dppf)Cl₂·DCM (1.11 g, 0.00136 mol). The reaction mixture was stirred at 90 °C under N₂ for 16 h. The mixture was quenched with water (50 mL) and then extracted with EtOAc (80 mL × 2). The combined organic phases were dried over Na₂SO₄ and filtered. The filtrate was concentrated under reduced pressure. The residue was purified by silica gel chromatography, eluting with 0% to 30% THF in petroleum ether to afford 4-(4-fluoro-3-nitrophenyl)-3,5-dimethylisoxazole (2.70 g, 81% yield) as a white solid. LC-MS (ESI): m/z [M+H]⁺ 273.1.

To a solution of 4-(4-fluoro-3-nitrophenyl)-3,5-dimethylisoxazole (2.50 g, 0.0106 mol) and tert-butyl N-(4-aminocyclohexyl)carbamate (2.50 g, 0.0116 mol) in ACN (50 mL) was added K₂CO₃ (3.66 g, 0.0265 mol) at 25 °C. The reaction mixture was stirred at 80 °C for 3 h. The mixture was quenched with water (50 mL) and then extracted with EtOAc (80 mL × 2). The combined organic phases were dried over Na₂SO₄ and filtered. The filtrate was concentrated under reduced pressure. The residue was purified by silica gel chromatography, eluting with 0% to 25% EtOAc in petroleum ether to afford tert-butyl ((1r,4r)-4-((4-(3,5-dimethylisoxazol-4-yl)-2-nitrophenyl)amino)cyclohexyl)carbamate (3.90 g, 81% yield) as a red solid. LC-MS (ESI): m/z [M+H]⁺ 431.2.

To a solution of tert-butyl ((1r,4r)-4-((4-(3,5-dimethylisoxazol-4-yl)-2-nitrophenyl)amino)cyclohexyl)carbamate (1.80 g, 0.00420 mol) in THF (36 mL) and H₂O (36 mL) were added Na₂S₂O₃ (9.13 g, 0.0462 mol) and NH₄OH (10.8 g, 0.0924 mol). The reaction mixture was stirred at 25 °C under N₂ for 3 h. The mixture was quenched with water (50 mL) and then extracted with EtOAc (60 mL × 2). The combined organic phases were dried over Na₂SO₄ and filtered. The filtrate was concentrated under reduced pressure. The residue was purified by silica gel chromatography, eluting with 0% to 50% EtOAc in petroleum ether to afford tert-butyl ((1r,4r)-4-((2-amino-4-(3,5-dimethylisoxazol-4-yl)phenyl)amino)cyclohexyl)carbamate (1.40 g, 78% yield) as a red solid. LC-MS (ESI): m/z [M+H]⁺ 401.2.

To a solution of (2S)-6-oxopiperidine-2-carboxylic acid (0.550 g, 3.85 mmol) in DMF (14 mL) were added HATU (1.46 g, 3.85 mmol), tert-butyl ((1r,4r)-4-((2-amino-4-(3,5-dimethylisoxazol-4-yl)phenyl)amino)cyclohexyl)carbamate (1.40 g, 3.50 mmol) and TEA (0.530 g, 5.25 mmol). The reaction mixture was stirred at 25 °C for 2 h. The reaction mixture was poured into water (50 mL), and the aqueous phase was extracted with EtOAc (60 mL × 2). The combined organic phases were washed with aqueous NaCl solution (20 mL × 3), dried over Na₂SO₄, filtered and concentrated under reduced pressure. The residue was purified by silica gel chromatography, eluting with 0% to 6% methanol in DCM to afford tert-butyl ((1S,4r)-4-((4-(3,5-dimethylisoxazol-4-yl)-2-((S)-6-oxopiperidine-2-carboxamido)phenyl)amino)cyclohexyl)carbamate (1.80 g, 94% yield) as a white solid. LC-MS (ESI): m/z [M+H]⁺ 526.3.

To a solution of tert-butyl ((1S,4r)-4-((4-(3,5-dimethylisoxazol-4-yl)-2-((S)-6-oxopiperidine-2-carboxamido)phenyl)amino)cyclohexyl)carbamate (1.60 g, 0.00300 mol) in DCM (8.0 mL) was added TFA (8.0 mL) at 25 °C. The reaction mixture was stirred at 25 °C for 30 min. The filtrate was concentrated under reduced pressure to afford (S)-N-(2-((((1r,4S)-4-aminocyclohexyl)amino)-5-(3,5-dimethylisoxazol-4-yl)phenyl)-6-oxopiperidine-2-carboxamide (1.20 g, crude) as a yellow oil, which was used directly in the next step without further purification. LC-MS (ESI): m/z [M+H]⁺ 426.2.

A solution of (S)-N-(2-((((1r,4S)-4-aminocyclohexyl)amino)-5-(3,5-dimethylisoxazol-4-yl)phenyl)-6-oxopiperidine-2-carboxamide (1.20 g, 0.00280 mol) in AcOH (12 mL) at 25 °C. The reaction mixture was stirred at 65 °C for 72 h, then concentrated under reduced pressure and purified by reverse-phase chromatography (ACN-water (0.1% NH₄HCO₃); 5% to 25%) to afford (S)-6-(1-((1r,4S)-4-aminocyclohexyl)-5-(3,5-dimethylisoxazol-4-yl)-1H-benzo[d]imidazol-2-yl)piperidin-2-one (0.860 g, 71% yield) as a white solid. LC-MS (ESI): m/z [M+H]⁺ 408.2.

To a solution of (S)-6-(1-((1r,4S)-4-aminocyclohexyl)-5-(3,5-dimethylisoxazol-4-yl)-1H-benzo[d]imidazol-2-yl)piperidin-2-one (131 mg, 0.322 mmol) in DCM (6.0 mL) were added 2-chloroethanesulfonyl chloride (78.6 mg, 0.482 mmol) and TEA (97.6 mg, 0.965 mmol). The reaction mixture was stirred at 25 °C for 30 min. LCMS showed the starting material was consumed and the desired compound was detected. The mixture was quenched with water (20 mL) and then extracted with EtOAc (30 mL × 2). The combined organic phases were dried over Na₂SO₄ and filtered. The filtrate was concentrated under reduced pressure. The residue was purified by silica gel chromatography, eluting with 0% to 25% methanol in DCM to afford N-((1S,4r)-4-(5-(3,5-dimethylisoxazol-4-yl)-2-((S)-6-oxopiperidin-2-yl)-1H-benzoimidazol-1-yl)cyclohexyl)ethanesulfonamide (80.0 mg, 45% yield) as a white solid. LC-MS (ESI): m/z [M+H]⁺ 498.2.

To a solution of N-((1 S,4r)-4-(5-(3,5-dimethylisoxazol-4-yl)-2-((S)-6-oxopiperidin-2-yl)-1H-benzoimidazol-1-yl)cyclohexyl)ethanesulfonamide (80.0 mg, 0.161 mmol) in DCM (14 mL) was added DBU (25.4 mg, 0.161 mmol), and the mixture was stirred for 10 min. Cu-TMEDA catalyst (14.9 mg, 0.0322 mmol) was added, and the mixture was sonicated and stirred for 10 min. (3,4-Difluorophenyl)boronyne diol (25.4 mg, 0.161 mmol) was added, and the reaction was stirred at 25 °C for 16 h. The mixture was quenched with water (20 mL) and then extracted with EtOAc (30 mL × 2). The combined organic phases were dried over Na₂SO₄ and filtered. The filtrate was concentrated under reduced pressure. The residue was purified by Prep HPLC (column: Xbridge-C18 150 × 19 mm, 5 µm; mobile phase: ACN-H₂O, 45-75% MeCN in 0.1% FA/water, 30 mL/min) to afford N-((1S,4r)-4-(2-((S)-1-(3,4-difluorophenyl)-6-oxopiperidin-2-yl)-5-(3,5-dimethylisoxazol-4-yl)-1H-benzo[d]imidazol-1-yl)cyclohexyl)ethanesulfonamide (Compound 1370, 10.0 mg, 10% yield) as a white solid.

¹H NMR (400 MHz, DMSO-*d₆*) δ 7.89 (d, *J* = 8.4 Hz, 1H), 7.69 (d, *J* = 1.4 Hz, 1H), 7.46 (d, *J* = 6.8 Hz, 1H), 7.42 - 7.29 (m, 2H), 7.16 - 7.10 (m, 1H), 7.10 - 7.01 (m, 1H), 6.83 - 6.74 (m, 1H), 6.09 (d, *J* = 16.4 Hz, 1H), 5.97 (d, *J* = 10.0 Hz, 1H), 5.83 - 5.73 (m, 1H), 4.40 - 4.25 (m, 1H), 3.36 - 3.33 (m, 1H), 2.64 - 2.52 (m, 2H), 2.45 - 2.12 (m, 10H), 2.04 - 1.89 (m, 4H), 1.84 - 1.74 (m, 2H), 1.58 - 1.46 (m, 2H). ¹⁹F NMR (400 MHz, DMSO-*d₆*) δ -137.907, -140.684. LC-MS: (ESI) m/z [M+H] ⁺ 610.2.

### Example 1-19 (Synthesis of 1371)

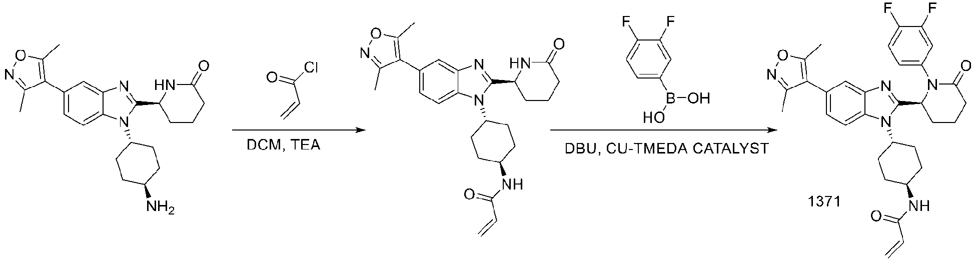

To a solution of (S)-6-(1-((1r,4S)-4-aminocyclohexyl)-5-(3,5-dimethylisoxazol-4-yl)-1H-benzo[d]imidazol-2-yl)piperidin-2-one (220 mg, 0.540 mmol) in DCM (8.0 mL) were added prop-2-enoyl chloride (73.3 mg, 0.810 mmol) and TEA (164 mg, 1.62 mmol) at 25 °C. The reaction mixture was stirred at 25 °C for 10 min. The mixture was quenched with water (20 mL) and then extracted with EtOAc (30 mL × 2). The combined organic phases were dried over Na₂SO₄ and filtered. The filtrate was concentrated under reduced pressure. The residue was purified by silica gel chromatography, eluting with 0% to 6% MeOH in DCM to afford N-((1S,4r)-4-(5-(3,5-dimethylisoxazol-4-yl)-2-((S)-6-oxopiperidin-2-yl)-1H-benzo[d]imidazol-1-yl)cyclohexyl)acrylamide (180 mg, 71% yield) as a white solid.

To a solution of N-{4-[5-(3,5-dimethyl-1,2-oxazol-4-yl)-2-[(2S)-6-oxopiperidin-2-yl]-1,3-benzodiazol-1-yl]cyclohexyl}prop-2-enamide (80.0 mg, 0.173 mmol) in DCM (6.0 mL) was added DBU (58.0 mg, 0.381 mmol), and the mixture was stirred for 10 min. Cu-TMEDA catalyst (16.1 mg, 0.0346 mmol) was added, and the mixture was sonicated and stirred for 10 min. (3,4-difluorophenyl)boronic acid (30.1 mg, 0.191 mmol) was added, and the reaction was stirred at 25 °C for 16 h. LCMS showed the starting material was consumed and the desired compound was detected. The mixture was quenched with water (20 mL) and then extracted with EtOAc (30 mL × 2). The combined organic phases were dried over Na₂SO₄ and filtered. The filtrate was concentrated under reduced pressure. The residue was purified by Prep HPLC (column: Xbridge-C18 150 × 19 mm, 5 µm; mobile phase: ACN-H₂O, 45-75% MeCN in 0.1% FA/water, 30 mL/min) to afford N-(4-{2-[(2S)-1-(3,4-difluorophenyl)-6-oxopiperidin-2-yl]-5-(3,5-dimethyl-1,2-oxazol-4-yl)-1,3-benzodiazol-1-yl}]cyclohexyl)prop-2-enamide (13.2 mg, 13% yield) as a white solid.

¹H NMR (400 MHz, DMSO-*d₆*) δ 8.05 (d, *J* = 7.6 Hz, 1H), 7.89 (d, *J* = 8.5 Hz, 1H), 7.70 (d, *J* = 1.4 Hz, 1H), 7.44 - 7.28 (m, 2H), 7.15 (dd, *J* = 8.5, 1.6 Hz, 1H), 7.07 - 6.99 (m, 1H), 6.32 - 6.21 (m, 1H), 6.16 - 6.06 (m, 1H), 5.82 - 5.71 (m, 1H), 5.64 - 5.56 (m, 1H), 4.42 - 4.26 (m, 1H), 3.96 - 3.80 (m, 1H), 2.61 - 2.55 (m, 2H), 2.46 - 2.15 (m, 10H), 2.09 - 1.88 (m, 4H), 1.85 - 1.72 (m, 2H), 1.52 - 1.39 (m, 2H).

### Example 1-20 (Synthesis of 1373)

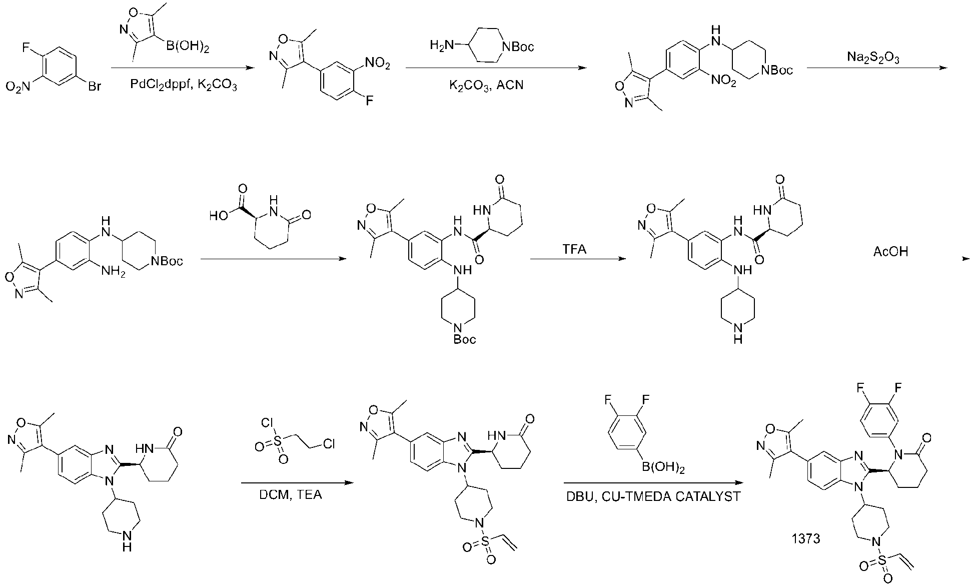

To a solution of 4-bromo-1-fluoro-2-nitrobenzene (3.00 g, 0.0136 mol) and (3,5-dimethyl-1,2-oxazol-4-yl)borane (2.30 g, 0.01 mol) in 1,4-dioxane (30 mL) and H₂O (6.0 mL) were added K₂CO₃ (5.64 g, 0.0408 mol) and Pd(dppf)Cl₂·DCM (1.11 g, 0.00136 mol). The reaction mixture was stirred at 90 °C under N₂ for 16 h. The mixture was quenched with water (50 mL) and then extracted with EtOAc (80 mL × 2). The combined organic phases were dried over Na₂SO₄ and filtered. The filtrate was concentrated under reduced pressure. The residue was purified by silica gel chromatography, eluting with 0% to 30% THF in PE to afford 4-(4-fluoro-3-nitrophenyl)-3,5-dimethylisoxazole (2.70 g, 81% yield) as a white solid.

To a solution of 4-(4-fluoro-3-nitrophenyl)-3,5-dimethyl-1,2-oxazole (1.32 g, 0.00560 mol) and tert-butyl 4-aminopiperidine-1-carboxylate (1.35 g, 0.00672 mol) in ACN (12 mL) was added K₂CO₃ (1.93 g, 0.0140 mol) at 25 °C. The reaction mixture was stirred at 80 °C for 16 h. The mixture was quenched with water (50 mL) and then extracted with EtOAc (80 mL × 2). The combined organic phases were dried over Na₂SO₄ and filtered. The filtrate was concentrated under reduced pressure. The residue was purified by silica gel chromatography, eluting with 0% to 25% EtOAc in petroleum to afford tert-butyl 4-((4-(3,5-dimethylisoxazol-4-yl)-2-nitrophenyl)amino)piperidine-1-carboxylate (2.00 g, 82% yield) as a red solid.

To a solution of tert-butyl 4-{[4-(3,5-dimethyl-1,2-oxazol-4-yl)-2-nitrophenyl]amino}piperidine-1-carboxylate (1.90 g, 0.00460 mol) in THF (38 mL) and H₂O (38 mL) were added Na₂S₂O₃ (10.0 g, 0.0506 mol) and NH₄OH (11.8 g, 0.101 mol). The reaction mixture was stirred at 25 °C under N₂ for 3 h. The mixture was quenched with water (50 mL) and then extracted with EtOAc (60 mL × 2). The combined organic phases were dried over Na₂SO₄ and filtered. The filtrate was concentrated under reduced pressure. The residue was purified by silica gel chromatography, eluting with 0% to 50% EtOAc in petroleum to afford tert-butyl 4-((2-amino-4-(3,5-dimethylisoxazol-4-yl)phenyl)amino)piperidine-1-carboxylate (1.30 g, 72% yield) as a red solid.

To a solution of (2S)-6-oxopiperidine-2-carboxylic acid (530 mg, 3.70 mmol) in DMF (13 mL) were added HATU (1407 mg, 3.70 mmol), tert-butyl 4-((2-amino-4-(3,5-dimethylisoxazol-4-yl)phenyl)amino)piperidine-1-carboxylate (1300 mg, 3.36 mmol) and TEA (511 mg, 5.05 mmol) at 25 °C. The reaction mixture was stirred at 25 °C for 2 h. The reaction mixture was added into water (50 mL), and the aqueous phase was extracted with EtOAc (60 mL × 2). The combined organic phases were washed with aqueous NaCl solution (20 mL × 3), dried over Na₂SO₄, filtered and concentrated under reduced pressure. The residue was purified by silica gel chromatography, eluting with 0% to 6% MeOH in DCM to afford tert-butyl (S)-4-(((4-(3,5-dimethylisoxazol-4-yl)-2-(6-oxopiperidine-2-carboxamido)phenyl)amino)piperidine-1-carboxylate (1300 mg, 94% yield) as a white solid.

To a solution of tert-butyl (S)-4-((4-(3,5-dimethylisoxazol-4-yl)-2-(6-oxopiperidine-2-carboxamido)phenyl)amino)piperidine-1-carboxylate (1.30 g, 0.00250 mol) in DCM (8 mL) was added TFA (8 mL) at 25 °C. The reaction mixture was stirred at 25 °C for 30 min. The filtrate was concentrated under reduced pressure to afford (S)-N-(5-(3,5-dimethylisoxazol-4-yl)-2-(piperidin-4-ylamino)phenyl)-6-oxopiperidine-2-carboxamide (1.00 g, crude) as a yellow oil, which was used directly in the next step without further purification.

To a solution of (S)-N-(5-(3,5-dimethylisoxazol-4-yl)-2-(piperidin-4-ylamino)phenyl)-6-oxopiperidine-2-carboxamide (1.00 g, 0.00240 mol) in AcOH (8 mL) was added at 25 °C. The reaction mixture was stirred at 65 °C for 72 h. The reaction was concentrated under reduced pressure and purified by reverse-phase chromatography (ACN-water (0.1% NH₄HCO₃); 5% to 25%) to afford (S)-6-(5-(3,5-dimethylisoxazol-4-yl)-1-(piperidin-4-yl)-1H-benzo[d]imidazol-2-yl)piperidin-2-one (0.580 g, 54% yield) as a white solid.

To a solution of (S)-6-(5-(3,5-dimethylisoxazol-4-yl)-1-(piperidin-4-yl)-1H-benzo[d]imidazol-2-yl)piperidin-2-one (300 mg, 0.762 mmol) in DCM (6.0 mL) were added 2-chloroethanesulfonyl chloride (186 mg, 1.14 mmol) and TEA (231 mg, 2.29 mmol) at 25 °C. The reaction mixture was stirred at 25 °C for 30 min. The mixture was quenched with water (20 mL) and then extracted with EtOAc (30 mL × 2). The combined organic phases were dried over Na₂SO₄ and filtered. The filtrate was concentrated under reduced pressure. The residue was purified by silica gel chromatography, eluting with 0% to 25% MeOH in DCM to afford (S)-6-(5-(3,5-dimethylisoxazol-4-yl)-1-(1-(vinylsulfonyl)piperidin-4-yl)-1H-benzo[d]imidazol-2-yl)piperidin-2-one (90.0 mg, 23% yield) as a white solid.

To a solution of (S)-6-(5-(3,5-dimethylisoxazol-4-yl)-1-(1-(vinylsulfonyl)piperidin-4-yl)-1H-benzo[d]imidazol-2-yl)piperidin-2-one (90.0 mg, 0.186 mmol) in DCM (6.0 mL) was added DBU (62.3 mg, 0.409 mmol), and the mixture was stirred for 10 min. Cu-TMEDA catalyst (34.6 mg, 0.0744 mmol) was added, and the mixture was sonicated and stirred for 10 min. (3,4-Difluorophenyl)borane diol (58.8 mg, 0.372 mmol) was added, and the reaction was stirred at 25 °C for 16 h. The mixture was quenched with water (20 mL) and then extracted with EtOAc (30 mL × 2). The combined organic phases were dried over Na₂SO₄ and filtered. The filtrate was concentrated under reduced pressure. The residue was purified by Prep HPLC (column: Xbridge-C18 150 × 19 mm, 5 µm; mobile phase: ACN-H₂O, 45-75% MeCN in 0.1% FA/water, 30 mL/min) to afford (S)-1-(3,4-difluorophenyl)-6-(5-(3,5-dimethylisoxazol-4-yl)-1-(1-(vinylsulfonyl)piperidin-4-yl)-1H-benzo[d]imidazol-2-yl)piperidin-2-one (9.20 mg, 10% yield) as a white solid.

¹H NMR (400 MHz, DMSO-d6) δ 7.72 (s, 1H), 7.68 (d, J = 8.4 Hz, 1H), 7.41 - 7.28 (m, 2H), 7.25 - 7.16 (m, 1H), 7.07 - 7.01 (m, 1H), 7.01 - 6.88 (m 1H), 6.31 - 6.10 (m, 2H), 5.58 - 5.69 (m, 1H), 4.64 - 4.51 (m, 1H), 3.77 - 3.64 (m, 2H), 2.94 - 2.83 (m, 2H), 2.58 - 2.54 (m, 2H), 2.46 - 2.14 (m, 10H), 2.09 - 1.76 (m, 4H).

The compounds shown below were prepared according to the procedures described in Examples 1-1 to 1-20:

| Compound | Characterization Data |
|---|---|
| 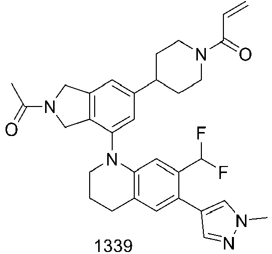 | ¹H NMR (400 MHz, DMSO-*d₆*) δ 7.75 (s, 1H), 7.49 (s, 1H), 7.15-7.12 (m, 3H), 6.84-6.78 (m, 1H), 6.74-6.60(m, 1H), 6.43-6.36 (m, 1H), 6.13 - 6.05 (m, 1H), 5.70 - 5.63 (m, 1H), 4.85 (s, 1H), 4.67 - 4.51 (m, 3H), 4.33 - 4.10 (m, 2H), 3.85 (s, 3H), 3.55 (s, 2H), 3.17-3.11 (m, 1H), 2.84 (s, 3H), 2.73 - 2.64 (m, 1H), 2.05 - 1.96 (m, 5H), 1.86 -1.83(m, 2H), 1.60 - 1.44 (m, 2H). |
| 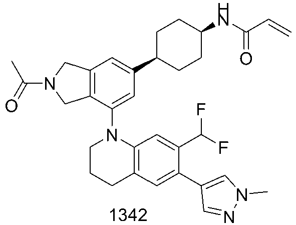 | ¹H NMR (400 MHz, DMSO-*d₆*) δ 8.03 (d, *J* = 7.6 Hz, 1H), 7.74 (s, 1H), 7.49 (s, 1H), 7.25 - 7.09 (m, 3H), 6.74 (t, *J* = 55.2 Hz, 1H), 6.50 - 6.35 (m, 2H), 6.15 - 6.05 (m, 1H), 5.62 - 5.54 (m, 1H), 4.95 - 4.80 (m, 1H), 4.75 - 4.50 (m, 2H), 4.40 - 4.20 (m, 1H), 4.12 - 4.02 (m, 1H), 3.86 (s, 3H), 3.62 - 3.51 (m, 2H), 2.95 - 2.85 (m, 2H), 2.63 - 2.56 (m, 1H), 2.06 - 1.97 (m, 5H), 1.83 - 1.70 (m, 4H), 1.68 - 1.58 (m, 4H). |
| 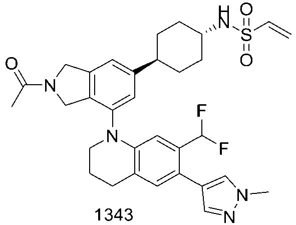 | ¹H NMR (400 MHz, DMSO-*d₆*) δ 7.74 (s, 1H), 7.49 (s, 1H), 7.33 (t, *J* = 7.2 Hz, 1H), 7.18 - 7.07 (m, 3H), 6.90 - 6.58 (m, 2H), 6.45 - 6.34 (m, 1H), 6.08 - 5.99 (m, 1H), 5.96 - 5.88 (m, 1H), 4.92 - 4.80 (m, 1H), 4.75 - 4.45 (m, 2H), 4.36 - 4.15 (m, 1H), 3.86 (s, 3H), 3.62 - 3.49 (m, 2H), 3.10 - 2.96 (m, 1H), 2.93 - 2.83 (m, 2H), 2.50 - 2.43 (m, 1H), 2.06 - 1.89 (m, 7H), 1.85 - 1.73 (m, 2H), 1.60 - 1.30 (m 4H). |
| 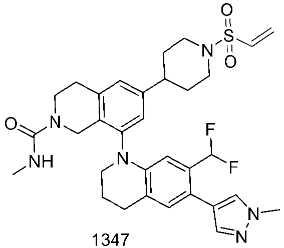 | ¹H NMR (400 MHz, DMSO-*d₆*) δ 7.72 (s, 1H), 7.47 (s, 1H), 7.16 - 7.05 (m, 2H), 7.05 - 6.98 (m, 1H), 6.87 - 6.78 (m, 1H), 6.73 - 6.53 (m, 1H), 6.52 - 6.34 (m, 1H), 6.20 - 6.08 (m, 3H), 4.43 (d, *J* = 16.8 Hz, 1H), 4.10 (d, *J* = 16.8 Hz, 1H), 3.86 (s, 3H), 3.74 - 3.70 (m, 2H), 3.64 - 3.61 (m, 2H), 3.00 - 2.75 (m, 6H), 2.72 - 2.60 (m 3H), 2.54 (s, 3H), 2.14 - 1.97 (m, 2H), 1.96 - 1.78 (m, 2H), 1.74 - 1.54 (m, 2H). |
| 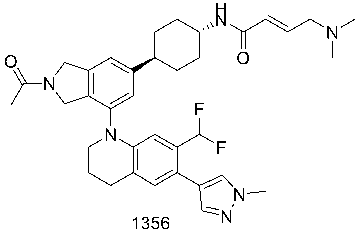 | ¹H NMR (400 MHz, DMSO-*d₆*) δ 7.98 - 7.87 (m, 1H), 7.75 (s, 1H), 7.49 (s, 1H), 7.22 - 7.10 (m, 3H), 6.74 (t, *J* = 55.2 Hz, 1H), 6.58 - 6.48 (m, 1H), 6.46 - 6.34 (m, 1H), 6.06 - 5.98 (m, 1H), 4.95 - 4.77 (m, 1H), 4.75 - 4.50 (m, 2H), 4.28 (s, 1H), 3.86 (s, 3H), 3.70 - 3.62 (m, 1H), 3.58 - 3.52 (m, 2H), 2.98 - 2.93 (m, 2H), 2.91 - 2.84 (m, 2H), 2.58 - 2.55 (m, 1H), 2.13 (d, *J* = 1.3 Hz, 6H), 2.05 - 1.96 (m, 5H), 1.93 - 1.79 (m, 4H), 1.64 - 1.50 (m, 2H), 1.38 - 1.24 (m 2H). |
| 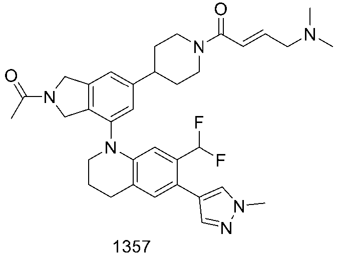 | ¹H NMR (400 MHz, DMSO-*d₆*) δ 7.74 (s, 1H), 7.49 (s, 1H), 7.25 - 7.09 (m, 3H), 6.76 (t, *J* = 55.2 Hz, 1H), 6.65 - 6.52 (m, 2H), 6.46 - 6.30 (m, 1H), 4.89 - 4.80 (m, 1H), 4.68 - 4.51 (m, 2H), 4.36 - 4.23 (m, 1H), 4.22 - 4.08 (m, 1H), 3.85 (s, 3H), 3.63 - 3.49 (m, 2H), 3.20 - 3.04 (m, 3H), 2.92 - 2.80 (m, 3H), 2.72 - 2.64 (m, 1H), 2.53 - 2.52 (m, 1H), 2.17 (s, 6H), 2.06 - 1.95 (m, 5H), 1.90 - 1.76 (m, 2H), 1.63 - 1.43 (m, 2H). |
| 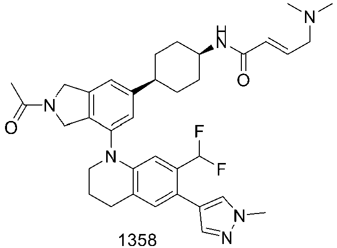 | ¹H NMR (400 MHz, DMSO-*d₆*) δ 7.96 (d, *J* = 7.2 Hz, 1H), 7.74 (s, 1H), 7.49 (s, 1H), 7.24 - 7.08 (m, 3H), 6.74 (t, *J* = 55.2 Hz, 1H), 6.59 - 6.48 (m, 1H), 6.47 - 6.34 (m, 1H), 6.29 - 6.17 (m, 1H), 4.92 - 4.82 (m, 1H), 4.75 - 4.50 (m, 2H), 4.37 - 4.20 (m, 1H), 4.14 - 4.02 (m, 1H), 3.86 (s, 3H), 3.59 - 3.52 (m, 2H), 2.97 (d, *J* = 5.6 Hz, 2H), 2.91 - 2.84 (m, 2H), 2.61 - 2.55 (m, 1H), 2.13 (s, 6H), 2.06 - 1.97 (m, 5H), 1.85 - 1.68 (m, 4H), 1.66 - 1.55 (m, 4H). |
| 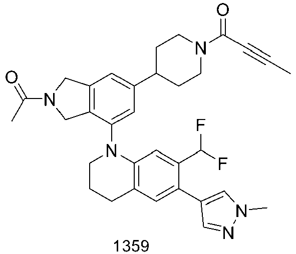 | ¹H NMR (400 MHz, DMSO-*d₆*) δ 7.73 (s, 1H), 7.48 (s, 1H), 7.20 - 7.05 (m, 3H), 6.85 - 6.59 (m, 1H), 6.42 - 6.35 (m, 1H), 4.84 (s, 1H), 4.63 (s, 1H), 4.42 - 4.27 (m, 2H), 3.85 (s, 3H), 3.37 - 3.19 (m, 2H), 2.80 - 2.65 (m, 6H), 2.55 - 2.50 (m, 1H), 2.05 - 1.90 (m, 8H), 1.86 - 1.80 (m, 2H), 1.60 - 1.43 (m, 2H), |
| 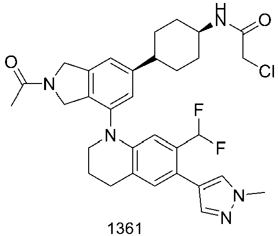 | ¹H NMR (400 MHz, DMSO-*d₆*) δ 8.25 - 8.15 (m, 1H), 7.74 (s, 1H), 7.49 (s, 1H), 7.24 - 7.10 (m, 3H), 6.74 (t, *J* = 55.2 Hz, 1H), 6.48 - 6.32 (m, 1H), 4.95 - 4.80 (m, 1H), 4.73 - 4.54 (m, 2H), 4.36 - 4.23 (m, 1H), 4.11 - 4.06 (m, 2H), 4.02 - 3.95 (m, 1H), 3.86 (s, 3H), 3.58 - 3.53 (m, 2H), 2.93 - 2.84 (m, 2H), 2.65 - 2.57 (m, 1H), 2.07 - 1.96 (m, 5H), 1.80 - 1.70 (m, 4H), 1.68 - 1.56 (m, 4H). |
| 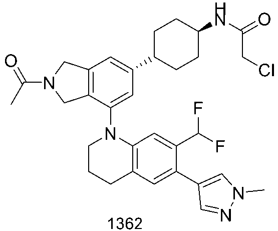 | ¹H NMR (400 MHz, DMSO-*d₆*) δ 8.19 - 8.11 (m, 1H), 7.74 (s, 1H), 7.49 (s, 1H), 7.21 - 7.09 (m, 3H), 6.74 (t, *J* = 55.2 Hz, 1H), 6.47 - 6.34 (m, 1H), 4.93 - 4.80 (m, 1H), 4.72 - 4.43 (m, 2H), 4.38 - 4.20 (m, 1H), 4.06 - 3.98 (m, 2H), 3.86 (s, 3H), 3.68 - 3.61(m, 1H), 3.58 - 3.52 (m, 2H), 2.91 - 2.83 (m, 2H), 2.61 - 2.53 (m, 1H), 2.08 - 1.95 (m, 5H), 1.94 - 1.74 (m, 4H), 1.66 - 1.46 (m, 2H), 1.44 - 1.24 (m, 2H). |
| 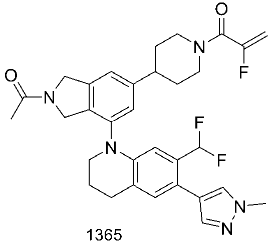 | ¹H NMR (400 MHz, DMSO-*d₆*) δ 7.74 (s, 1H), 7.49 (s, 1H), 7.26 - 7.12 (m, 3H), 6.82 - 6.60 (m, 1H), 6.44 - 6.37 (m, 1H), 5.27 - 5.09 (m, 2H), 4.84(s, 1H), 4.65 (s, 1H), 4.02 - 3.99 (m, 2H), 3.86 (s, 3H), 3.60 - 3.55 (m, 2H), 2.95 - 2.75 (m, 4H), 2.55 - 2.50 (m, 1H), 2.03 - 1.84 (m, 7H), 1.60 - 1.43 (m, 2H). |
| 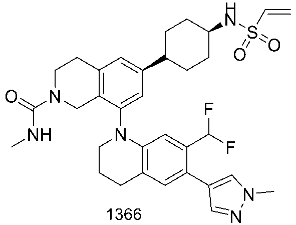 | ¹H NMR (400 MHz, DMSO-*d₆*) δ 7.71 (s, 1H), 7.54 - 7.34 (m, 2H), 7.14 - 6.98 (m, 3H), 6.85 - 6.55 (m, 2H), 6.51 - 6.45 (m, 1H), 6.19 (s, 1H), 6.02 (d, *J* = 16.4 Hz, 1H), 5.92 (d, *J* = 10.0 Hz, 1H), 4.42 (d, *J* = 16.8 Hz, 1H), 4.12 (d, *J* = 16.8 Hz, 1H), 3.86 (s, 3H), 3.53 - 3.49 (m, 2H), 3.04 - 2.72 (m, 5H), 2.59 - 2.53 (m, 3H), 2.49 - 2.41 (m, 1H), 2.16 - 1.98 (m, 2H), 1.89 - 1.69 (m, 4H), 1.65 - 1.40 (m, 4H). |
| 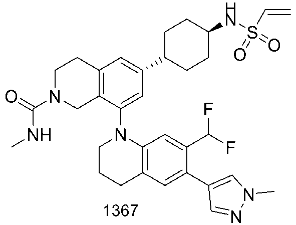 | ¹H NMR (400 MHz, DMSO-*d₆*) δ 7.71 (s, 1H), 7.50 - 7.44 (m, 1H), 7.38 - 7.26 (m, 1H), 7.12 - 6.95 (m, 3H), 6.84 - 6.52 (m, 2H), 6.50 - 6.42 (m, 1H), 6.17 (s, 1H), 6.03 (d, *J* = 16.4 Hz, 1H), 5.92 (d, *J* = 10.0 Hz, 1H), 4.41 (d, *J* = 16.8 Hz, 1H), 4.10 (d, *J* = 16.8 Hz, 1H), 3.86 (s, 3H), 3.56 - 3.47 (m, 2H), 3.08 - 2.96 (m, 1H), 2.93 - 2.75 (m, 4H), 2.55 (d, *J* = 4.4 Hz, 3H), 2.47 - 2.34 (m, 1H), 2.16 - 1.98 (m, 2H), 1.95 - 1.86 (m, 2H), 1.82 - 1.70 (m, 2H), 1.57 - 1.42 (m, 2H), 1.41 - 1.28 (m, 2H). |
| 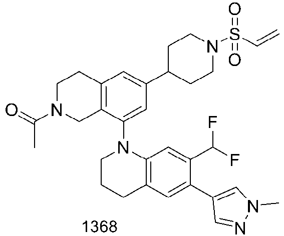 | ¹H NMR (400 MHz, DMSO-*d₆*) δ 7.72 (s, 1H), 7.47 (s, 1H), 7.10 -7.06 (m, 3H), 6.88 - 6.79 (m, 1H), 6.63-6.55 (m, 1H), 6.20 - 6.08 (m, 3H), 4.52-4.45 (m, 1H), 4.30-4.23 (m, 1H), 3.86 (s, 3H), 3.67 - 3.58 (m, 4H), 3.58 - 3.50 (m, 1H), 3.00 - 2.82 (m, 4H), 2.67-2.50 (m, 4H), 2.06 (s, 4H), 1.86 (d, *J* = 13.6 Hz, 3H), 1.71-1.62 (m, 2H). |
| 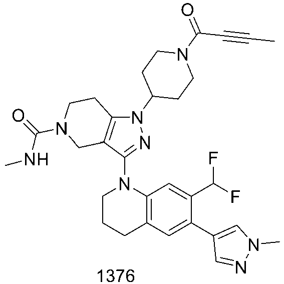 | ¹H NMR (400 MHz, DMSO-d6) δ 7.76 (s, 1H), 7.50 (s, 1H), 7.09 (s, 1H), 6.92 - 6.78 (m, 2H), 6.55 - 6.54 (m, 1H), 4.36 - 4.34 (m, 3H), 4.02 (s, 2H), 3.86 (s, 3H), 3.57 - 3.54 (m, 4H), 2.84 - 2.74 (m, 6H), 2.55 - 2.50 (m, 3H), 2.02 - 1.72 (m, 9H). |
| 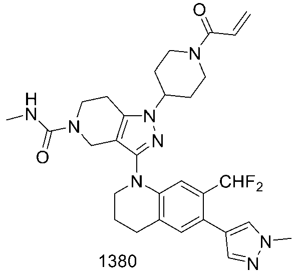 | m/z [M+H] ⁺ =579.4 |
| 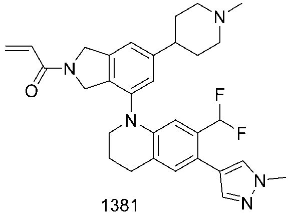 | m/z [M+H] ⁺ =531.5 |
| 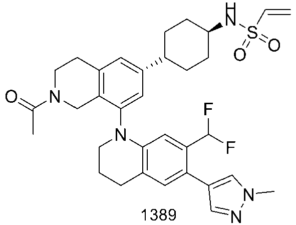 | m/z [M+H] ⁺ =624.5 |
| 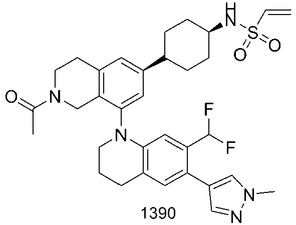 | m/z [M+H] ⁺ =624.5 |
| 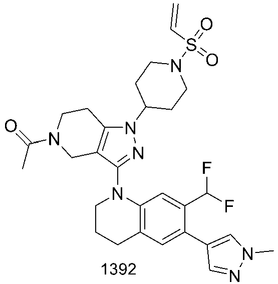 | m/z [M+H] ⁺ =600.5 |
| 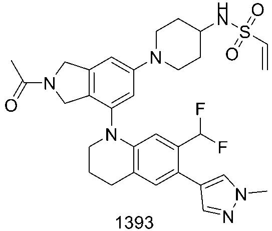 | m/z [M+H] ⁺ =611.4 |
| 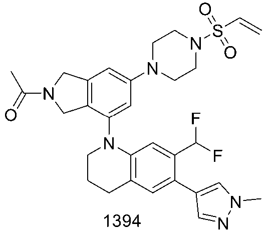 | m/z [M+H] ⁺ =597.6 |
| 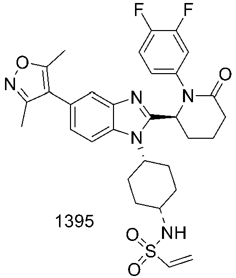 | m/z [M+H] ⁺ =610.8 |
| 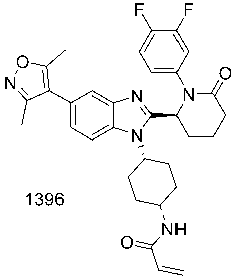 | m/z [M+H] ⁺ =574.5 |
| 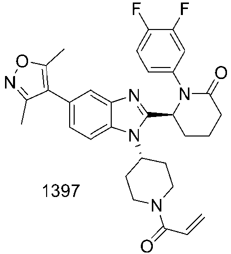 | m/z [M+H] ⁺ =560.3 |
| 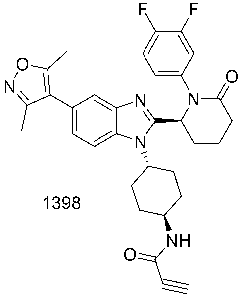 | m/z [M+H] ⁺ =572.5 |
| 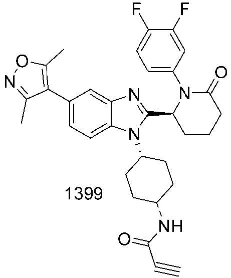 | m/z [M+H] ⁺ =572.5 |
| 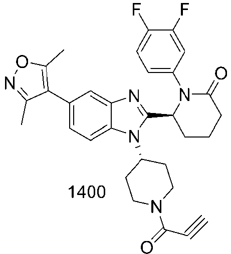 | m/z [M+H] ⁺ =558.4 |

### Example 2-1

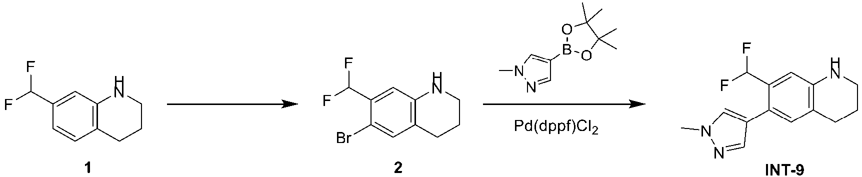

To a solution of 7-(difluoromethyl)-1,2,3,4-tetrahydroquinoline (18 g, 98.2 mmol, 1 eq) in DCM (180 mL) was added NBS (17.5 g, 98.2 mmol, 1 eq) at 0 °C. The mixture was stirred at 25 °C for 16 h, then quenched with H₂O (200 mL), extracted with DCM (150 mL×2), dried over Na₂SO₄, filtered and concentrated under reduced pressure. The residue was purified by column chromatography (petroleum ether/ethyl acetate = 0 to 10%) to afford 6-bromo-7-(difluoromethyl)-1,2,3,4-tetrahydroquinoline (16 g, 61.1 mmol, 62.1% yield) as a yellow solid.

MS (ESI): 262.6 (M+H)⁺.

¹H NMR (400MHz, CDCl₃) δ = 7.18 (s, 1H), 7.04 (s, 1H), 6.63 (s, 1H), 4.09 - 3.82 (m, 1H), 3.28 - 3.18 (m, 2H), 2.66 (br t, J=6.3 Hz, 2H), 1.88 - 1.79 (m, 2H)

A mixture of 6-bromo-7-(difluoromethyl)-1,2,3,4-tetrahydroquinoline (11 g, 41.9 mmol, 1 eq), 1-methyl-4-(4,4,5,5-tetramethyl-1,3,2-dioxaborolan-2-yl)-1H-pyrazole (8.7 g, 41.9 mmol, 1 eq), Pd(dppf)Cl₂ (3.0 g, 4.2 mmol, 0.1 eq) and K₂CO₃ (11.6 g, 83.9 mmol, 2 eq) in 1,4-dioxane (80 mL) and H₂O (20 mL) was stirred at 110 °C under N₂ for 12 h. After concentration under reduced pressure, the residue was purified by column chromatography (petroleum ether/ethyl acetate = 0 to 40%) to afford 7-(difluoromethyl)-6-(1-methyl-1H-pyrazol-4-yl)-1,2,3,4-tetrahydroquinoline (INT-9, 10 g, 37.9 mmol, 90.5% yield) as a brown solid.

MS (ESI): 263.9 (M+H)⁺.

¹H NMR (400MHz, DMSO-d6) δ = 7.68 (s, 1H), 7.43 (s, 1H), 6.90 (s, 1H), 6.73 (s, 1H), 6.01 (s, 1H), 3.84 (s, 3H), 3.24 - 3.14 (m, 2H), 2.68 (br t, J=6.0 Hz, 2H), 1.79 (quin, J=5.9 Hz, 2H).

### Example 2-2 (Synthesis of 2001)

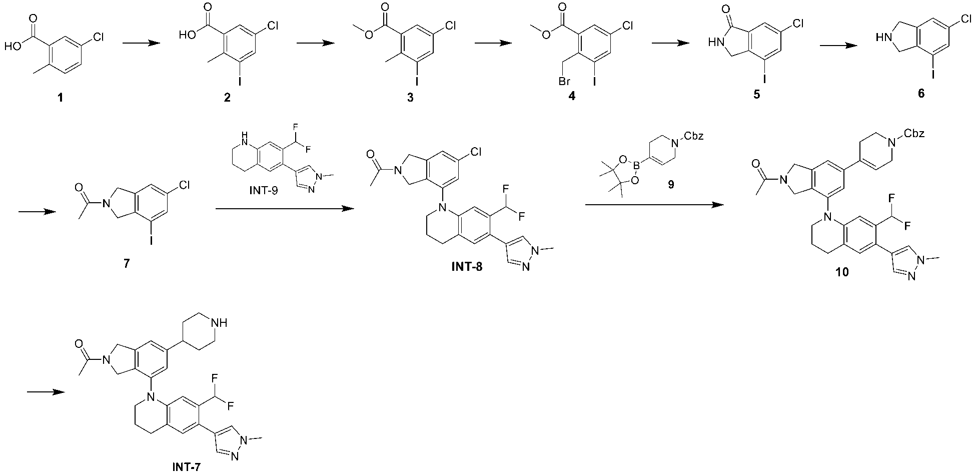

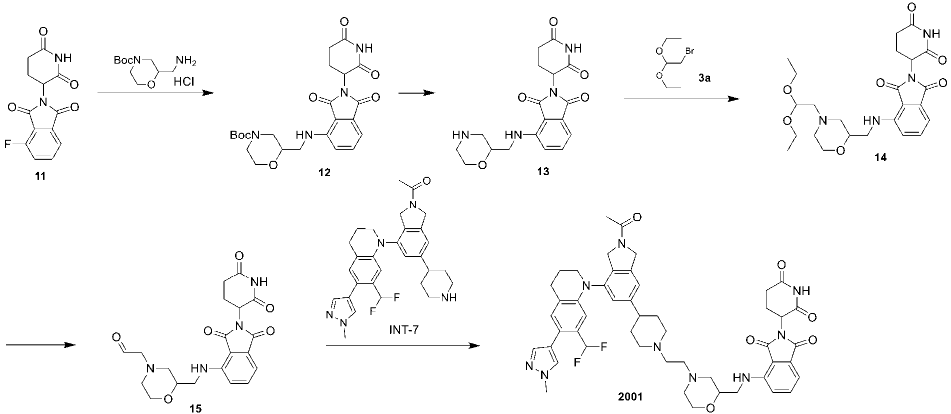

To a solution of 5-chloro-2-methylbenzoic acid (50 g, 293.1 mmol, 1 eq) in concentrated H₂SO₄ (350 mL) was added dropwise a solution of NIS (76 g, 331 mmol, 1.13 eq) in H₂SO₄ (30 mL) at 0 °C. The mixture was stirred at 25 °C for 16 h. The mixture was poured into ice water (500 mL), stirred for 30 min and filtered to afford 5-chloro-3-iodo-2-methylbenzoic acid (68.5 g, 231 mmol, 78.8% yield) as a brown solid.

MS (ESI): 294.8 (M-H)⁺.

To a solution of 5-chloro-3-iodo-2-methylbenzoic acid (68.5 g, 231 mmol, 1 eq) in MeOH (350 mL) was added concentrated H₂SO₄ (12 mL). The mixture was stirred at 60 °C for 16 h. The reaction mixture was concentrated under reduced pressure. The residue was diluted with DCM (300 mL), and the combined organic phase was washed with aqueous NaHCO₃ (200 mL) and brine (200 mL), dried over anhydrous Na₂SO₄, filtered and concentrated in vacuo to afford methyl 5-chloro-3-iodo-2-methylbenzoate (58.5 g, 188.4 mmol, 81.5% yield) as a yellow oil.

¹H NMR (400MHz, CHLOROFORM-d) δ = 7.98 (d, J=2.0 Hz, 1H), 7.76 (d, J=1.8 Hz, 1H), 3.92 (s, 4H), 2.64 (s, 3H).

To a solution of methyl 5-chloro-3-iodo-2-methylbenzoate (55 g, 177.12 mmol, 1 eq) in CCl₄ (350 mL) were added NBS (33 g, 185.41 mmol, 1.05 eq) and BPO (4.5 g, 18.58 mmol, 1e-1 eq). The mixture was stirred at 80 °C for 17 h. The reaction mixture was filtered and concentrated under reduced pressure. The residue was purified by column chromatography (petroleum ether/ethyl acetate = 100/0 to 10/1) to afford methyl 2-(bromomethyl)-5-chloro-3-iodobenzoate (40 g, 103.1 mmol, 57% yield) as a colorless oil.

¹H NMR (400MHz, CHLOROFORM-d) δ = 7.97 (d, J=2.2 Hz, 1H), 7.84 (d, J=2.2 Hz, 1H), 5.01 (s, 2H), 3.89 (s, 3H).

Methyl 2-(bromomethyl)-5-chloro-3-iodobenzoate (40 g, 102.5 mmol, 1 eq) was added to NH₃/MeOH solution (150 mL). The mixture was stirred at 60 °C for 2.5 h. The reaction mixture was concentrated under reduced pressure to remove MeOH. The residue was washed with EA (150 mL×3) to afford methyl 2-(bromomethyl)-5-chloro-3-iodobenzoate (20.6 g, 70.4 mmol, 68.5% yield) as a white solid. MS (ESI): 295.8 (M+H)⁺.

¹H NMR (400MHz, DMSO-d6) δ = 8.96 (br s, 1H), 8.09 (s, 1H), 7.71 (s, 1H), 4.18 (s, 2H)

To a solution of methyl 2-(bromomethyl)-5-chloro-3-iodobenzoate (17 g, 57.92 mmol, 1 eq) in DCM (150 mL) at 0 °C under N₂ was added dropwise DIBAL-H (1 M, 202.73 mL, 3.5 eq). The mixture was stirred at 45 °C for 54 h. The reaction was quenched with 15% NaOH solution (100 mL) at 0 °C. The reaction mixture was filtered and washed with EtOAc. The filtrate was washed with brine (200 mL), dried over Na₂SO₄, filtered and concentrated under reduced pressure. The residue was purified by flash silica gel chromatography (flash silica gel column, 0 to10% MeOH/DCM eluent) to afford 6-chloro-4-iodoisoindole (5.5 g, 19.6 mmol, 33.9% yield) as a brown solid.

¹H NMR (400MHz, DMSO-d6) δ = 7.64 (s, 1H), 7.37 (s, 1H), 4.20 (s, 2H), 3.93 (s, 2H).

To a solution of 6-chloro-4-iodoisoindole (4.5 g, 16.1 mmol, 1 eq) and TEA (4.1 g, 40.3 mmol, 5.6 mL, 2.5 eq) in DCM (80 mL) was added dropwise acetic anhydride (2.46 g, 24.1 mmol, 2.26 mL, 1.5 eq) at 0 °C under N₂. The mixture was stirred at 25 °C for 16 h. The residue was diluted with H₂O (100 mL) and extracted with DCM (40 mL×2). The combined organic layers were washed with saturated brine (40 mL×2), dried over Na₂SO₄, filtered and concentrated under reduced pressure. The residue was purified by flash silica gel chromatography (0 to 100% ethyl acetate/petroleum ether gradient eluent) to afford 1-(6-chloro-4-iodoisoindol-2-yl)ethan-1-one (3.25 g, 8.2 mmol, 51% yield, 70% purity) as a brown solid. MS (ESI): 322.3 (M+H)⁺.

1-(6-chloro-4-iodoisoindol-2-yl)ethan-1-one (1.15 g, 3.58 mmol, 1 eq), 7-(difluoromethyl)-6-(1-methyl-1H-pyrazol-4-yl)-1,2,3,4-tetrahydroquinoline (941.6 mg, 3.58 mmol, 1 eq), NaOBu-t (859.2 mg, 8.94 mmol, 2.5 eq) and CPhos Pd G3 (288.4 mg, 357.6 µmol, 0.1 eq) were dissolved in 1,4-dioxane (10 mL). The mixture was heated at 110°C under microwave for 50 min. The reaction mixture was diluted with H₂O (20 mL) and extracted with EtOAc (20 mL×3). The combined organic layers were washed with brine (50 mL), dried over Na₂SO₄, filtered and concentrated under reduced pressure. The residue was purified by flash silica gel chromatography to afford 1-(6-chloro-4-(7-(difluoromethyl)-6-(1-methyl-1H-pyrazol-4-yl)-3,4-dihydroquinolin-1(2H)-yl)isoindol-2-yl)ethan-1-one (INT-8, 700 mg, 1.53 mmol, 42.8% yield) as a brown solid.

1-(6-chloro-4-(7-(difluoromethyl)-6-(1-methyl-1H-pyrazol-4-yl)-3,4-dihydroquinolin-1(2H)-yl)isoindol-2-yl)ethan-1-one (600 mg, 1.31 mmol), benzyl 4-(4,4,5,5-tetramethyl-1,3,2-dioxaborolan-2-yl)-3,6-dihydropyridine-1(2H)-carboxylate (450.71 mg, 1.31 mmol), XPhos Pd G3 (111.15 mg, 131.32 µmol) and K₃PO₄ (836.23 mg, 3.94 mmol) were dissolved in THF (5 mL) and H₂O (1 mL). The mixture was heated at 60 °C under microwave for 30 min. The mixture was extracted with EtOAc (20 mL), washed with brine (10 mL), dried over Na₂SO₄, filtered and concentrated to afford a crude product, which was purified by silica gel column chromatography (DCM/MeOH from 100% to 80%) to afford benzyl 4-(2-acetyl-7-(7-(difluoromethyl)-6-(1-methyl-1H-pyrazol-4-yl)-3,4-dihydroquinolin-1(2H)-yl)isoindol-5-yl)-3,6-dihydropyridine-1(2H)-carboxylate (714 mg, 1.12 mmol, 85.3% yield) as a brown oil. LCMS (ESI⁺): m/z 638.4 (M+H)⁺, Rt: 1.91 min.

To a solution of benzyl 4-(2-acetyl-7-(7-(difluoromethyl)-6-(1-methyl-1H-pyrazol-4-yl)-3,4-dihydroquinolin-1(2H)-yl)isoindol-5-yl)-3,6-dihydropyridine-1(2H)-carboxylate (714 mg, 1.12 mmol) in THF (10 mL) was added Pd(OH)₂ (393.08 mg, 559.81 µmol, 20% purity). The mixture was stirred at 15 °C under H₂ (15 psi) for 16 h. The mixture was filtered and concentrated to afford a crude product, which was purified by prep-HPLC (FA) to afford INT-7 (30 mg, 59.34 µmol, 30.0% yield) as a white solid. LCMS (ESI+): 506.4 (M+H)+, Rt:1.88 min.

¹H NMR (400MHz, DMSO-*d₆*) δ 8.30 (br s, 1H), 7.71 (br s, 1H), 7.47 (s, 1H), 7.35 -6.98 (m, 3H), 6.90 - 6.53 (m, 1H), 6.43 - 6.28 (m, 1H), 4.85 (br s, 1H), 4.72 - 4.38(m, 1H), 4.26 (br s, 1H), 3.83 (s, 3H), 3.70 - 3.16 (m, 6H), 3.06 - 2.61 (m, 5H), 2.12 -1.59 (m, 8H).

To a solution of 2-(2,6-dioxopiperidin-3-yl)-4-fluoroisoindole-1,3-dione (874 mg, 3.17 mmol) in DMSO (20.0 mL) were added tert-butyl 2-(aminomethyl)morpholine-4-carboxylate hydrochloride (800 mg, 3.17 mmol) and DIPEA (1227 mg, 9.50 mmol). The mixture was stirred at 130 °C for 6 h. LCMS showed consumption of starting material and formation of desired product. The reaction was quenched with water (50 mL) and extracted with EtOAc (3×50 mL). The combined organic layers were washed with brine (3×100 mL), dried over anhydrous Na₂SO₄ and purified by silica gel chromatography to afford tert-butyl 2-((2-(2,6-dioxopiperidin-3-yl)-1,3-dioxoisoindolin-4-yl)amino)methyl)morpholine-4-carboxylate (1.3 g, 60% yield) as a yellow solid. LC-MS: m/z [M-100+H]⁺ 373.2.

To a solution of tert-butyl 2-((2-(2,6-dioxopiperidin-3-yl)-1,3-dioxoisoindolin-4-yl)amino)methyl)morpholine-4-carboxylate (1.2 g, 2.5 mmol) in DCM (10.0 mL) was added HCl/1,4-dioxane (4 M, 5.0 mL). The mixture was stirred at 20 °C for 1 h. LCMS showed consumption of starting material and formation of desired product. The resulting mixture was concentrated under reduced pressure to afford 2-(2,6-dioxopiperidin-3-yl)-4-((morpholin-2-ylmethyl)amino)isoindoline-1,3-dione (0.95 g, 100% yield) as a yellow solid. LC-MS: m/z [M+H]⁺ 373.1.

To a solution of 2-(2,6-dioxopiperidin-3-yl)-4-((morpholin-2-ylmethyl)amino)isoindoline-1,3-dione (1.22 g, 3.3 mmol) in ACN (20.0 mL) were added 2-bromo-1,1-diethoxyethane (0.72 g, 3.6 mmol) and DIPEA (1.28 g, 9.9 mmol). The mixture was stirred at 100 °C for 48 h. LCMS showed consumption of starting material and formation of desired product. The resulting mixture was concentrated under reduced pressure. The residue was purified by flash column chromatography eluting with 0-50% EtOAc in PE, and then purified by flash column chromatography eluting with 0-5% MeOH in DCM to afford 4-((4-((2,2-diethoxyethyl)morpholin-2-yl)methyl)amino)-2-(2,6-dioxopiperidin-3-yl)isoindoline-1,3-dione (1.6 g, 100% yield) as a yellow solid. LC-MS: m/z [M+H]⁺ 489.3.

To a solution of 4-((4-((2,2-diethoxyethyl)morpholin-2-yl)methyl)amino)-2-(2,6-dioxopiperidin-3-yl)isoindoline-1,3-dione (500 mg, 1.02 mmol) in DCM (1.0 mL) was added HCl/1,4-dioxane (4 M, 10 mL). The mixture was stirred at 25 °C for 10 h. LCMS showed consumption of starting material and formation of desired product. The resulting mixture was concentrated under reduced pressure to afford 2-(2-((2-(2,6-dioxopiperidin-3-yl)-1,3-dioxoisoindolin-4-yl)amino)methyl)morpholino)acetaldehyde (400 mg, 94% yield) as a yellow solid. LC-MS: m/z [M+H₂O+H]⁺ 433.1.

To a solution of 2-(2-((2-(2,6-dioxopiperidin-3-yl)-1,3-dioxoisoindolin-4-yl)amino)methyl)morpholino)acetaldehyde (300 mg, 0.72 mmol) in DMSO (2.0 mL) and THF (2.0 mL) were added 1-{4-[7-(difluoromethyl)-6-(1-methylpyrazol-4-yl)-3,4-dihydro-2H-quinolin-1-yl]-6-(piperidin-4-yl)isoindol-2-yl}ethan-1-one (366.0 mg, 0.72 mmol) and trimethyl orthoacetate (87.0 mg, 0.72 mmol). The mixture was stirred at 25 °C for 1 h, then sodium borohydride acetate (767.1 mg, 3.62 mmol) was added. The mixture was stirred at 25 °C for 12 h. LCMS showed consumption of starting material and formation of desired product. The crude product was purified by prep-HPLC, eluting with 26 to 36% ACN in 0.1% FA/water over 8 min to afford 4-((4-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)morpholin-2-yl)methyl)amino)-2-(2,6-dioxopiperidin-3-yl)isoindoline-1,3-dione (Compound 2001, 81.3 mg, 12% yield) as a white solid.

¹H NMR (400 MHz, DMSO-*d₆*) δ 11.09 (s, 1H), 7.74 (s, 1H), 7.60 - 7.56 (m, 1H), 7.49 (s, 1H), 7.16 - 7.10 (m, 4H), 7.07 (d, *J* = 15.2 Hz, 1H), 7.03 - 6.73 (m, 1H), 6.62 - 6.59 (m, 1H), 6.41 (d, *J* = 28.8 Hz, 1H), 5.07 - 5.03 (m, 1H), 4.85 (s, 1H), 4.64 (s, 1H), 4.28 (s, 1H), 3.86 (s, 3H), 3.81 (d, *J* = 10.8 Hz, 1H), 3.66 - 3.60 (m, 2H), 3.61 - 3.44 (m, 5H), 3.00 - 2.98 (m, 2H), 2.95 - 2.81 (m, 4H), 2.79 - 2.65 (m, 2H), 2.65 - 2.51 (m, 4H), 2.51 - 2.29 (m, 4H), 2.18 - 1.87 (m, 10H), 1.83 - 1.53 (m, 4H). ¹⁹F NMR (377 MHz, DMSO-*d₆*) δ -108.12. LC-MS: m/z [M+H]⁺ 904.4.

### Example 2-3 (Synthesis of 2003)

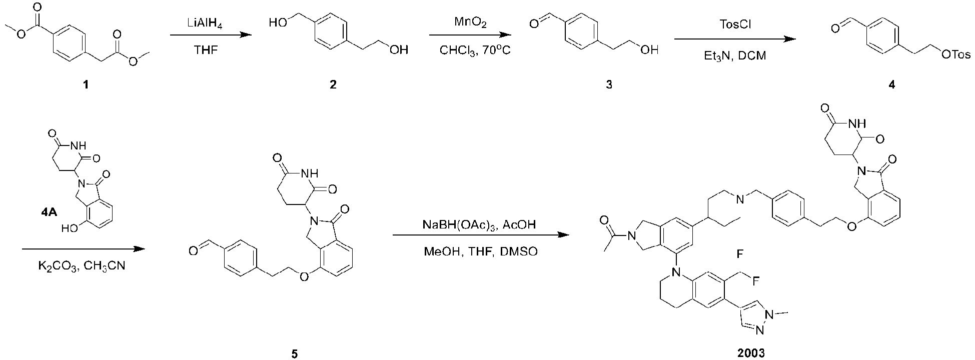

To a solution of methyl 4-(2-methoxy-2-oxoethyl)benzoate (5.0 g, 24.0 mmol) in THF (100 mL) was added dropwise LiAlH₄ (2.7 g, 72.0 mmol) at 0 °C over 20 min. After addition, the mixture was stirred at 20 °C for 16 h. The mixture was quenched with H₂O (1.6 mL), 10% NaOH (1.6 mL) and H₂O (4.8 mL). The organic layer was dried over Na₂SO₄, filtered and concentrated to afford a crude product, which was purified by silica gel column chromatography (100-40% petroleum ether/ethyl acetate) to afford 2-(4-(hydroxymethyl)phenyl)ethan-1-ol (1.7 g, 11.2 mmol, 46.5% yield) as a white solid.

¹H NMR (400 MHz, DMSO-*d₆*) δ 7.23 - 7.18 (m, 2H), 7.18 - 7.12 (m, 2H), 5.09 (t, *J* = 5.6 Hz, 1H), 4.62 (t, *J=* 5.0 Hz, 1H), 4.44 (d, *J=* 5.6 Hz, 2H), 3.57 (q, *J=* 6.3 Hz, 2H), 2.69 (t, *J=* 7.2 Hz, 2H).

To a solution of 2-(4-(hydroxymethyl)phenyl)ethan-1-ol (4.4 g, 28.9 mmol) in CHCl₃ (100 mL) was added MnO₂ (25.1 g, 289.1 mmol). The mixture was stirred at 70 °C for 16 h. The mixture was filtered through filter paper and the filtrate was concentrated to give a crude product, which was purified by silica gel column chromatography (100-60% petroleum ether/ethyl acetate) to afford 4-(2-hydroxyethyl)benzaldehyde (2.6 g, 17.6 mmol, 60.8% yield) as a yellow oil.

¹H NMR (400 MHz, DMSO-*d₆*) δ 9.96 (s, 1H), 7.82 (d, *J* = 7.8 Hz, 2H), 7.45 (d, *J* = 7.8 Hz, 2H), 4.71 (t, *J* = 5.0 Hz, 1H), 3.65 (q, *J* = 6.4 Hz, 2H), 2.82 (t, *J* = 6.7 Hz, 2H)

To a solution of 4-(2-hydroxyethyl)benzaldehyde (4.3 g, 28.6 mmol) in DCM (50 mL) were added Et₃N (8.7 g, 85.7 mmol, 11.9 mL) and TosCl (8.2 g, 42.9 mmol). The mixture was stirred at 20 °C for 16 h. The mixture was quenched with aqueous NaHCO₃ (20 mL). The organic layer was dried over Na₂SO₄, filtered and concentrated to give a crude product, which was purified by silica gel column chromatography (100-65% petroleum ether/ethyl acetate) to afford 4-formylphenethyl 4-methylbenzenesulfonate (7.6 g, 25.0 mmol, 87.4% yield) as a yellow solid.

¹H NMR (400MHz, DMSO-*d₆*) δ 9.97 (s, 1H), 7.77 (d, *J* = 7.7 Hz, 2H), 7.64 (d, *J* = 7.9 Hz, 2H), 7.37 (t, *J* = 6.8 Hz, 4H), 4.30 (t, *J* = 6.1 Hz, 2H), 2.99 (t, *J* = 6.2 Hz, 2H), 2.39 (s, 3H)

To a solution of 4-formylphenethyl 4-methylbenzenesulfonate (1.5 g, 4.9 mmol) and 3-(4-hydroxy-1-oxoisoindolin-2-yl)piperidine-2,6-dione (1.1 g, 4.2 mmol) in ACN (150 mL) was added K₂CO₃ (1.7 g, 12.3 mmol). The mixture was stirred at 80 °C for 16 h. The mixture was filtered through filter paper and the filtrate was concentrated to afford a crude product, which was purified by silica gel column chromatography (100 to 20% petroleum ether/ethyl acetate) to afford 4-(2-((2,6-dioxopiperidin-3-yl)-1-oxoisoindolin-4-yl)oxy)ethyl)benzaldehyde (220 mg, 560.7 µmol, 11.4% yield) as a white solid.

¹H NMR (400MHz, DMSO-*d₆*) δ 11.00 (s, 1H), 9.98 (s, 1H), 7.86 (d, *J* = 7.9 Hz, 2H), 7.57 (d, *J* = 7.9 Hz, 2H), 7.51 - 7.44 (m, 1H), 7.33 - 7.26 (m, 2H), 5.14 - 5.06 (m, 1H), 4.42 - 4.35 (m, 2H), 4.32 - 4.24 (m, 1H), 4.19 - 4.11 (m, 1H), 3.17 (t, *J* = 6.4 Hz, 2H), 2.96 - 2.85 (m, 1H), 2.63 - 2.54 (m, 2H), 2.47 - 2.40 (m, 1H).

To a solution of 4-(2-((2-(2,6-dioxopiperidin-3-yl)-1-oxoisoindolin-4-yl)oxy)ethyl)benzaldehyde (40 mg, 101.9 µmol) in MeOH (2 mL) were added THF (2 mL), DMSO (2 mL), 1-(4-(7-(difluoromethyl)-6-(1-methyl-1H-pyrazol-4-yl)-3,4-dihydroquinolin-1(2H)-yl]-6-(piperidin-4-yl)isoindolin-2-yl}ethan-1-one (51.5 mg, 101.9 µmol), AcOH (6.1 mg, 101.9 µmol) and NaBH₃CN (32.0 mg, 509.7 µmol). The mixture was stirred at 30 °C for 16 h. H₂O (20 mL) was added to the mixture and the mixture was filtered to obtain a filter cake. The cake was purified by prep-HPLC (FA) to afford 3-(4-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)phenethoxy)-1-oxoisoindolin-2-yl)piperidine-2,6-dione (33.1 mg, 37.5 µmol, 36.8% yield) as a pale yellow solid. MS ES⁺: 882.5.

¹H NMR (400MHz, DMSO-*d*₆) δ 11.01 (s, 1H), 7.75 (s, 1H), 7.53 - 7.40 (m, 2H),7.32 - 7.23 (m, 1H), 7.18 - 7.07 (m, 3H), 6.90 - 6.58 (m, 1H), 6.46 - 6.32 (m, 1H),5.16 - 5.06 (m, 1H), 4.84 (s, 1H), 4.63 (s, 1H), 4.37 - 4.25 (m, 4H), 4.23 - 4.10 (m,2H), 3.85 (s, 3H), 3.60 - 3.52 (m, 3H), 3.06 - 2.99 (m, 2H), 2.95 - 2.83 (m, 5H), 2.60- 2.53 (m, 2H), 2.46 - 2.39 (m, 1H), 2.16 - 1.87 (m, 9H), 1.79 - 1.60 (m, 4H).

### Example 2-4 (Synthesis of 2004)

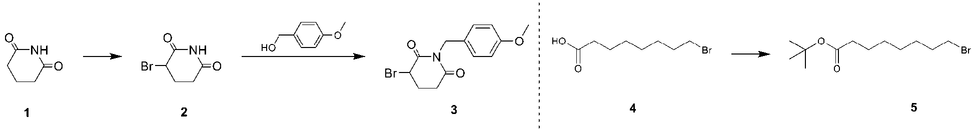

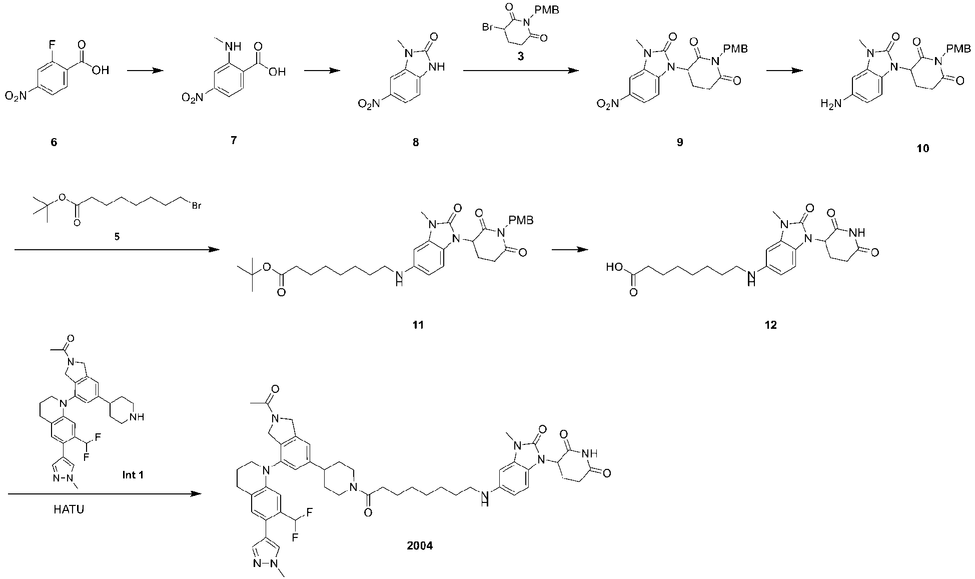

To a stirred solution of piperidine-2,6-dione (10.0 g, 88.4 mmol) in CHCl₃ (100 mL) was added Br₂ (13.9 g, 87.2 mmol) at 20 °C under N₂. The reaction mixture was stirred at 110 °C for 4 h. The mixture was concentrated and the crude product was purified by flash silica gel chromatography (ISCO^{®}; 40g SepaFlash^{®} flash silica gel column, 0 to 55% ethyl acetate/petroleum ether) to afford 3-bromopiperidine-2,6-dione (8.1 g, 42.1 mmol, 47.7% yield) as a white solid.

¹H NMR (400 MHz, DMSO- *d6*) δ 10.96 - 11.19 (m, 1 H) 4.89 (t, *J* = 4.52 Hz, 1 H) 2.53 - 2.68 (m, 2 H) 2.43 - 2.50 (m, 1 H) 2.15 (m, 1 H).

A solution of 3-bromopiperidine-2,6-dione (12.5 g, 65.1 mmol), (4-methoxyphenyl)methanol (9.89 g, 71.6 mmol) and PPh₃ (18.7 g, 71.6 mmol) in anhydrous THF (240 mL) was stirred at 0 °C for 1 h, then DEAD (17.0 g, 97.6 mmol, 17.75 mL) was added dropwise under N₂ at 0 °C over 30 min. The mixture was stirred at 20 °C under N₂ for another 16 h. The mixture was quenched with H₂O (100 mL) at 0 °C and extracted with ethyl acetate (150 mL×3). The organic layer was dried over anhydrous Na₂SO₄ and concentrated. The residue was purified by column chromatography (SiO₂, petroleum ether/ethyl acetate = 1/0 to 2/1, then petroleum ether/DCM = 1/0 to 1/1) to afford 3-bromo-1-(4-methoxybenzyl)piperidine-2,6-dione (12.7 g, 40.6 mmol, 62.4% yield) as a pale yellow solid.

¹H NMR (400 MHz, DMSO- *d₆*) δ 7.32 (d, *J=* 8.68 Hz, 2 H) 6.78 - 6.87 (m, 2 H) 4.82 - 5.06 (m, 2 H) 4.67 - 4.76 (m, 1 H) 3.04 (m, 1 H) 2.70 - 2.81 (m, 1 H) 2.30 - 2.43 (m, 1 H) 2.19 - 2.28 (m, 1 H)

To a solution of 8-bromooctanoic acid (1.00 g, 4.48 mmol) in DCM (20.0 mL) was added trifluoroacetic anhydride (1.88 g, 8.96 mmol) at 0 °C. The solution was stirred at 15 °C for 2 h, then tert-butanol (996 mg, 13.4 mmol) was added and the solution was stirred at 15 °C for 16 h. Saturated aqueous NaHCO₃ (10 mL) was added, and the organic layer was separated, dried over anhydrous Na₂SO₄, filtered and concentrated. The crude product was purified by flash silica gel chromatography (ISCO^{®}; 20g SepaFlash^{®} flash silica gel column, 0 to 50% ethyl acetate/petroleum ether, 40mL/min gradient) to afford tert-butyl 8-bromooctanoate (0.9 g, 3.2 mmol, 71.9% yield) as a pale yellow oil.

¹H NMR (400 MHz, CHLOROFORM-d) δ 3.33 (t, *J=* 6.85 Hz, 2 H) 2.14 *(t, J=* 7.46 Hz, 2 H) 1.78 (m, 2 H) 1.51 (m, 2 H) 1.32 - 1.42 (m, 11 H) 1.20 - 1.30 (m, 4 H)

A reaction solution of 2-fluoro-4-nitrobenzoic acid (15 g, 81.0 mmol) and CH₃NH₂ (41.9 g, 405 mmol, 45 mL, 30% purity) was stirred in a sealed tube at 80 °C for 40 h. The mixture was cooled to room temperature, diluted with water (500 mL), and acidified to pH = 6 with 2 M HCl. After filtration, the filter cake was washed with water (50 mL). The filter cake was dried under reduced pressure to afford 2-(methylamino)-4-nitrobenzoic acid (15 g, crude) as a yellow solid.

¹H NMR (400 MHz, DMSO- *d₆*) δ 8.07 (d, *J=* 8.68 Hz, 1 H) 7.45 (d, *J=* 2.20 Hz, 1 H) 7.38 (dd, *J=* 8.68, 2.32 Hz, 1 H) 2.99 (s, 3 H).

To a solution of 2-(methylamino)-4-nitrobenzoic acid (10.0 g, 50.9 mmol) and DIPEA (19.7 g, 152.9 mmol) in t-BuOH (60 mL) was added DPPA (16.8 g, 61.1 mmol, 13.26 mL) at 20 °C under N₂. The resulting mixture was stirred at 90 °C for 16 h. The mixture was cooled to room temperature and concentrated under reduced pressure. The crude product was purified by flash silica gel chromatography (ISCO^{®}; 120 g SepaFlash^{®} flash silica gel column, 0 to 100% ethyl acetate/petroleum ether, 40mL/min gradient eluent) to afford 3-methyl-5-nitro-1H-benzoimidazol-2-one (7 g, 36.2 mmol, 71.0% yield) as a pale yellow solid.

¹H NMR (400 MHz, DMSO- *d6)* δ 11.65 (s, 1 H) 7.94 - 8.05 (m, 2 H) 7.13 - 7.17 (m, 1 H) 3.37 (s, 3 H).

To a solution of 3-methyl-5-nitro-1H-benzoimidazol-2-one (6.0 g, 31.0 mmol) in DMF (80 mL) was added dropwise NaH (2.5 g, 62.1 mmol, 60% purity) at 0 °C. After addition, the mixture was stirred at this temperature for 1 h, then a solution of 3-bromo-1-[(4-methoxyphenyl)methyl]piperidine-2,6-dione (14.5 g, 46.5 mmol) in DMF (30 mL) was added dropwise at 0 °C. The resulting mixture was stirred at 20 °C for 16 h. After completion, the mixture was acidified to pH = 3 to 5 with FA, diluted with water (200 mL) and extracted with ethyl acetate (2×100 mL). The organic layer was washed with brine (100 mL), dried over anhydrous Na₂SO₄, filtered and concentrated. The crude product was purified by flash silica gel chromatography (ISCO^{®}; 80 g SepaFlash^{®} flash silica gel column, 0 to 50% ethyl acetate/petroleum ether, 40mL/min gradient eluent) to afford 1-[(4-methoxyphenyl)methyl]-3-(3-methyl-5-nitro-2-oxobenzoimidazol-1-yl)piperidine-2,6-dione (3.5 g, 6.6 mmol, 21.5% yield, 81% purity) as a pale yellow solid. MS ES⁺: 425.3.

¹H NMR (400 MHz, CHLOROFORM-d) δ 7.81 - 7.90 (m, 2 H) 7.28 (d, *J =* 8.68 Hz, 2 H) 6.72 - 6.82 (m, 2 H) 6.44 (d, *J=* 8.56 Hz, 1 H) 5.17 (dd, *J=* 13.33, 5.50 Hz, 1 H) 4.90 (d, *J =* 1.47 Hz, 2 H) 3.74 (s, 3 H) 3.44 (s, 3 H) 2.93 - 3.05 (m, 1 H) 2.71 - 2.81 (m, 1 H) 2.52 (m, 1 H) 2.16 (m, 1 H).

1-[(4-methoxyphenyl)methyl]-3-(3-methyl-5-nitro-2-oxobenzoimidazol-1-yl)piperidine-2,6-dione (3.5 g, 8.3 mmol), Fe (4.6 g, 82.4 mmol) and NH₄Cl (4.4 g, 82.4 mmol) were dissolved in THF (100 mL) and H₂O (40 mL). The mixture was stirred at 60 °C under N₂ for 16 h. The reaction mixture was filtered and concentrated under reduced pressure. The crude product was purified by flash silica gel chromatography (ISCO^{®}; 40 g SepaFlash^{®} flash silica gel column, 0 to 100% ethyl acetate/petroleum ether, 40mL/min gradient eluent) to afford 3-(5-amino-3-methyl-2-oxobenzoimidazol-1-yl)-1-[(4-methoxyphenyl)methyl]piperidine-2,6-dione (2.7 g, 6.8 mmol, 83.0% yield) as a pale yellow solid.

¹H NMR (400 MHz, DMSO- *d₆*) δ 7.20 (d, *J=* 8.68 Hz, 2 H) 6.81 - 6.88 (m, 2 H) 6.63 (d, *J* =8.31 Hz, 1 H) 6.38 (d, *J=* 1.96 Hz, 1 H) 6.22 (dd, *J=* 8.31, 1.96 Hz, 1 H) 5.37 (dd, *J=* 13.08, 5.26 Hz, 1 H) 4.70 - 4.92 (m, 4 H) 3.70 - 3.76 (m, 3 H) 3.20 - 3.30 (m, 3 H) 2.96 - 3.12 (m, 1 H) 2.75 - 2.83 (m, 1 H) 2.66 (m, 1 H) 1.98 - 2.07 (m, 1 H).

A mixture of 3-(5-amino-3-methyl-2-oxobenzoimidazol-1-yl)-1-[(4-methoxyphenyl)methyl]piperidine-2,6-dione (700 mg, 1.8 mmol), tert-butyl 8-bromooctanoate (594.6 mg, 2.1 mmol), K₂CO₃ (735.8 mg, 5.3 mmol), NaI (266.0 mg, 1.8 mmol) and DMF (10 mL) was stirred at 110 °C for 16 h. The reaction mixture was filtered and concentrated in vacuo. The crude product was purified by flash silica gel chromatography (ISCO^{®}; 20 g SepaFlash^{®} flash silica gel column, 0 to 100% ethyl acetate/petroleum ether, 40mL/min gradient eluent) to afford tert-butyl 8-[[1-[1-[(4-methoxyphenyl)methyl]-2,6-dioxo-3-piperidinyl)-3-methyl-2-oxobenzoimidazol-5-yl]amino]octanoate (700 mg, 1.2 mmol, 66.5% yield) as a pale yellow solid.

¹H NMR (400 MHz, CHLOROFORM-d) δ 7.30 (d, J= 8.68 Hz, 2 H) 6.75 *(d, J=* 8.68 Hz, 2 H) 6.10 - 6.35 (m, 3 H) 5.08 (dd, *J =* 13.08, 5.38 Hz, 1 H) 4.83 - 4.93 (m, 2 H) 3.72 (s, 3 H) 3.51 (m, 1 H) 3.26 - 3.34 (m, 3 H) 2.92 - 3.17 (m, 2 H) 2.89 - 2.91 (m, 1 H) 2.68 - 2.79 (m, 1 H) 2.44 - 2.56 (m, 1 H) 2.03 - 2.18 (m, 3 H) 1.53 - 1.59 (m, 3 H) 1.42 - 1.51 (m, 2 H) 1.31 - 1.41 (m, 9H) 1.21 - 1.30 (m, 4 H)

Tert-butyl 8-[[1-[1-[(4-methoxyphenyl)methyl]-2,6-dioxo-3-piperidinyl)-3-methyl-2-oxobenzoimidazol-5-yl]amino]octanoate (160 mg, 269 µmol) and MsOH (1.3 g, 13.5 mmol) were dissolved in toluene (1 mL) and the mixture was stirred at 110 °C under N₂ for 4 h. After cooling to room temperature, the resulting mixture was added dropwise to ice water (2 mL) at 0 °C. Then the reaction mixture was adjusted to pH = 7 with saturated NaHCO₃ solution. The aqueous layer was extracted with ethyl acetate (4×10 mL), dried over Na₂SO₄, filtered and concentrated under reduced pressure to afford 8-[[1-(2,6-dioxo-3-piperidinyl)-3-methyl-2-oxobenzoimidazol-5-yl)amino)octanoic acid (80 mg, crude) as a pale yellow solid. MS ES⁺: 417.4.

8-[[1-[2,6-dioxo-3-piperidinyl)-3-methyl-2-oxobenzoimidazol-5-yl)amino)octanoic acid (50.0 mg, 120 µmol), 1-[4-(7-(difluoromethyl)-6-(1-methylpyrazol-4-yl)-3,4-dihydro-2H-quinolin-1-yl]-6-(4-piperidinyl)isoindol-2-yl]ethanone (60.7 mg, 120 µmol) and DIPEA (46.5 mg, 360 µmol) were dissolved in DMF (5.00 mL), then HATU (68.4 mg, 180 µmol) was added. The resulting solution was stirred at 15 °C for 16 h. The reaction was quenched by dropwise addition of H₂O (5 mL), and the solid was filtered. The crude product was further purified by prep-HPLC (FA). The pure fractions were collected and volatiles were removed in vacuo. The residue was dissolved in ACN (2 mL) and water (10 mL), and the solution was lyophilized to afford 3-[5-[[8-[4-[2-acetyl-7-[7-(difluoromethyl)-6-(1-methylpyrazol-4-yl)-3,4-dihydro-2H-quinolin-1-yl]isoindol-5-yl]piperidin-1-yl]-8-oxooctyl]amino]-3-methyl-2-oxobenzoimidazol-1-yl]piperidine-2,6-dione (17 mg, 18.1 µmol, 15.1% yield, 96.6% purity) as a pale yellow solid. MS ES⁺: 904.5

¹H NMR (400 MHz, METHANOL-*d*₄) δ 11.05 (s, 1 H), 7.74 (s, 1 H), 7.49 (s, 1 H), 7.18 - 7.10 (m, 3 H), 6.88 -6.60 (m, 2 H), 6.45 - 6.35 (m, 2 H), 6.27 (d, *J =* 8.2 Hz, 1 H), 5.23 (dd, *J =* 5.1, 12.9 Hz, 2 H), 4.84 (s,1 H), 4.65 - 4.52 (m, 2 H), 4.27 (m, 1 H), 3.96 (m, 1 H), 3.86 (s, 3 H), 3.54 (s, 2 H), 3.24 (s, 3 H), 3.09 - 2.97 (m, 2 H), 2.87 (s, 3 H), 2.66 - 2.55 (m, 3 H), 2.31 (m, 2 H), 2.08 (s, 5 H), 2.03 - 1.97 (m, 5 H), 1.79 (m, 2 H), 1.53 (m, 4 H), 1.39 - 1.27 (m, 6 H)

### Example 2-5 (Synthesis of 2005)

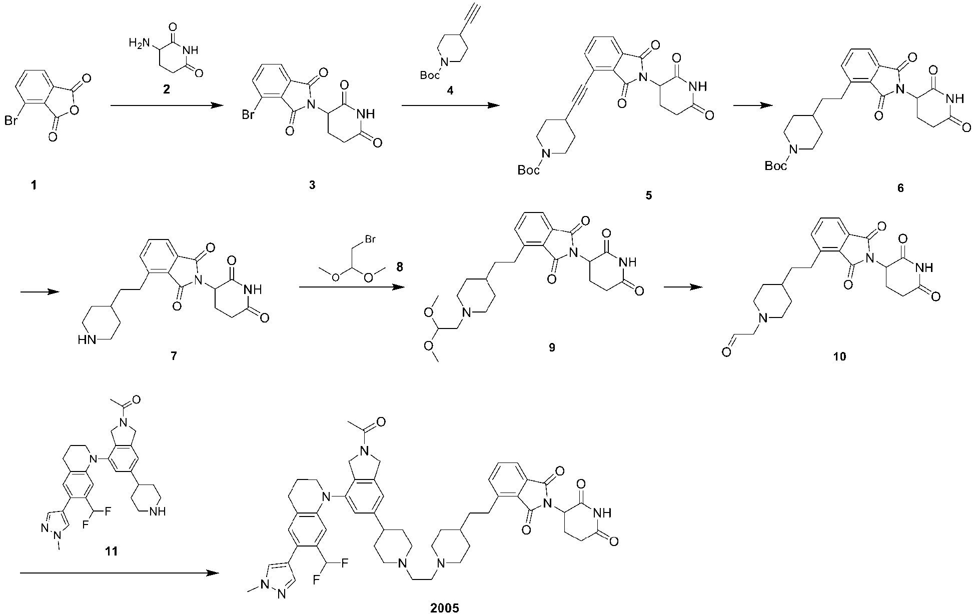

To a solution of 4-bromoisobenzofuran-1,3-dione (5.0 g, 22.0 mmol) and 3-aminopiperidine-2,6-dione (3.1 g, 24.2 mmol) in HOAc (50 mL) was added KOAc (6.5 g, 66.1 mmol). The reaction mixture was stirred at 90 °C for 16 h. The reaction mixture was cooled to 25 °C, diluted with ice water (100 mL) and stirred at 0 °C for 0.5 h. The mixture was filtered and the filter cake was dried in vacuo to afford 4-bromo-2-(2,6-dioxo-3-piperidinyl)isoindoline-1,3-dione (5.7 g, 16.9 mmol, 76.7% yield) as a pale yellow solid.

¹H NMR (400 MHz, DMSO- *d*₆) δ 11.18 (s, 1 H) 8.07 *(d, J=* 7.95 Hz, 1 H) 7.94 *(d, J=* 7.21 Hz, 1 H) 7.74 -7.82 (m, 1 H) 5.18 (dd, *J=* 12.84, 5.38 Hz, 1 H) 2.82 - 2.97 (m, 1 H) 2.52 - 2.66 (m, 2 H) 2.01 - 2.14 (m, 1 H)

To a solution of 4-bromo-2-(2,6-dioxo-3-piperidinyl)isoindoline-1,3-dione (2.0 g, 5.9 mmol) in DMF (15 mL) were added tert-butyl 4-ethynylpiperidine-1-carboxylate (1.4 g, 6.5 mmol), Pd(dppf)Cl₂ (217 mg, 296 µmol), CuI (112 mg, 593 µmol) and TEA (6.0 g, 59.3 mmol). The resulting mixture was stirred at 80 °C under N₂ for 16 h. The reaction mixture was concentrated under reduced pressure to remove solvent. The crude product was purified by flash silica gel chromatography (ISCO^{®}; 40g SepaFlash^{®} flash silica gel column, 0 to 50% ethyl acetate/petroleum ether, 40mL/min gradient eluent) to afford tert-butyl 4-[2-[2-(2,6-dioxo-3-piperidinyl)-1,3-dioxoisoindolin-4-yl]ethynyl]piperidine-1-carboxylate (1.8 g, 3.3 mmol, 56.0% yield, 86% purity) as a pale yellow solid. MS ES⁺: 488.4 (M+23)

¹H NMR (400 MHz, DMSO- *d₆*) δ 11.15 (s, 1 H) 8.03 - 8.10 (m, 1 H) 7.83 - 7.86 (m, 1 H) 7.54 - 7.62 (m, 1 H) 5.15 - 5.20 (m, 1 H) 3.62 (d, *J= 6.24* Hz, 2 H) 3.02 (m, 1 H) 2.84 - 2.93 (m, 1 H) 2.57 - 2.70 (m, 2 H) 2.01 - 2.14 (m, 2 H) 1.82 (m, 2 H) 1.50 - 1.67 (m, 2 H) 1.36 - 1.47 (s, 9 H)

To a solution of tert-butyl 4-[2-[2-(2,6-dioxo-3-piperidinyl)-1,3-dioxoisoindol-4-yl]ethynyl]piperidine-1-carboxylate (900 mg, 1.9 mmol) in ethyl acetate (20 mL) was added PtO₂ (219 mg, 96.6 µmol, 10% purity). The reaction mixture was stirred at 20 °C under H₂ (15 Psi) balloon for 16 h. The reaction mixture was filtered through a pad of Celite and the filtrate was concentrated under reduced pressure. The residue was purified by prep-HPLC (FA) to afford tert-butyl 4-[2-[2-(2,6-dioxo-3-piperidinyl)-1,3-dioxoisoindol-4-yl]ethyl]piperidine-1-carboxylate (600 mg, 1.3 mmol, 66.1% yield) as a pale yellow solid.

¹H NMR (400 MHz, CHLOROFORM-d) δ 8.01 (s, 1 H) 7.66 (d, *J =* 6.72 Hz, 1 H) 7.57 (m, 1 H) 7.45 (d, *J=* 7.70 Hz, 1 H) 4.90 (dd, *J =* 12.29, 5.32 Hz, 1 H) 2.96 - 3.09 (m, 2 H) 2.49 - 2.91 (m, 6 H) 2.05 - 2.14 (m, 1 H) 1.69 (m, 3 H) 1.48 - 1.58 (m, 3 H) 1.32 - 1.47 (m, 11 H) 1.04 - 1.16 (m, 2 H)

Tert-butyl 4-[2-[2-(2,6-dioxo-3-piperidinyl)-1,3-dioxoisoindol-4-yl]ethyl]piperidine-1-carboxylate (600 mg, 1.3 mmol) and HCl/1,4-dioxane (4 M, 9.6 mL) were dissolved in 1,4-dioxane (10 mL), and the reaction mixture was stirred at 25 °C under N₂ for 3 h. The mixture was concentrated under reduced pressure to afford 2-(2,6-dioxo-3-piperidinyl)-4-[2-(4-piperidinyl)ethyl]isoindoline-1,3-dione (700 mg, crude, 2HCl) as a pale yellow solid.

¹H NMR (400 MHz, DMSO- *d₆*) δ 11.13 (s, 1 H) 8.68 (s, 1 H) 8.40 (s, 1 H) 7.63 - 7.87 (m, 3 H) 5.13 (dd, *J=* 12.72, 5.38 Hz, 1 H) 3.20 - 3.30 (m, 2 H) 2.99 - 3.11 (m, 2 H) 2.75 - 2.96 (m, 3 H) 2.52 - 2.66 (m, 2 H) 2.02 - 2.10 (m, 1 H) 1.82 - 1.94 (m, 2 H) 1.48 - 1.63 (m, 3 H) 1.29 - 1.42 (m, 2 H).

2-(2,6-dioxo-3-piperidinyl)-4-[2-(4-piperidinyl)ethyl]isoindoline-1,3-dione (300 mg, 739 µmol, HCl salt), 2-bromo-1,1-dimethoxyethane (374 mg, 2.22 mmol) and DIPEA (382 mg, 3.0 mmol) were dissolved in ACN (10 mL). The reaction mixture was stirred at 80 °C under N₂ for 16 h. The reaction mixture was concentrated in vacuo. The residue was purified by column chromatography (SiO₂, petroleum ether/n-butyl acetate = 1/0 to 0/1, then MeOH: DCM = 0/1 to 9/1) to afford 4-[2-[1-(2,2-dimethoxyethyl)-4-piperidinyl]ethyl]-2-(2,6-dioxo-3-piperidinyl)isoindoline-1,3-dione (160 mg, 349.7 µmol, 47.3% yield) as a pale yellow solid. MS ES⁺: 458.4.

¹H NMR (400 MHz, DMSO- *d₆*) δ 11.13 (s, 1 H) 7.64 - 7.83 (m, 3 H) 5.13 (dd, *J =* 12.90, 5.32 Hz, 1 H) 4.45 (s, 1 H) 3.24 (s, 6 H) 2.99 - 3.09 (m, 2 H) 2.83 - 2.89 (m, 2 H) 2.56 - 2.66 (m, 1 H) 2.38 (m, 2 H) 1.86 - 2.12 (m, 3 H) 1.67 (m, 2 H) 1.47 - 1.57 (m, 2 H) 1.10 - 1.32 (m, 4 H) 0.83 - 0.90 (m, 1 H).

To a solution of 4-[2-[1-(2,2-dimethoxyethyl)-4-piperidinyl]ethyl]-2-(2,6-dioxo-3-piperidinyl)isoindoline-1,3-dione (100 mg, 218 µmol) in ACN (3 mL) was added 3 M HCl (1 mL, 218 µmol). The reaction mixture was stirred at 55 °C under N₂ for 16 h. The reaction mixture was cooled to 0 °C, adjusted to pH = 7 with saturated NaHCO₃ solution, diluted with 5 mL ethyl acetate and extracted with 20 mL ethyl acetate (10 mL×2). The combined organic layers were washed with 5 mL brine (5 mL×1), dried over Na₂SO₄, filtered and concentrated under reduced pressure to afford 2-[4-[2-[2-(2,6-dioxo-3-piperidinyl)-1,3-dioxoisoindol-4-yl]ethyl]-1-piperidinyl)acetaldehyde (80 mg, crude) as a pale yellow solid. MS ES⁺: 412.4.

2-[4-[2-[2-(2,6-dioxo-3-piperidinyl)-1,3-dioxoisoindol-4-yl]ethyl]-1-piperidinyl)acetaldehyde (80 mg, 194 µmol) and 1-[4-[7-(difluoromethyl)-6-(1-methylpyrazol-4-yl)-3,4-dihydro-2H-quinolin-1-yl]-6-(4-piperidinyl)isoindol-2-yl]ethanone (88.4 mg, 174 µmol) was dissolved in THF (1 mL), MeOH (1 mL) and DMSO (1 mL), then NaBH₃CN (36.6 mg, 583 µmol) was added. The mixture was stirred at 30 °C under N₂ for 16 h. The reaction was quenched by addition of water (5 mL) at 20 °C. The resulting mixture was extracted with ethyl acetate (3×10 mL). The combined organic layers were washed with brine (20 mL), dried over anhydrous Na₂SO₄, filtered and concentrated under reduced pressure. The residue was purified by prep-TLC and prep-HPLC (TFA) to afford a desired compound. Then the compound was added 1 M HCl (2 mL) and lyophilized to afford 4-(2-(2-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)piperidin-4-yl)ethyl)-2-(2,6-dioxopiperidin-3-yl)isoindoline-1,3-dione (4.86 mg, 4.5 µmol, 2.3% yield, 83.9%) as a yellow solid. MS ES⁺: 901.6.

¹H NMR (400MHz, DMSO- *d*₆) 11.14 (s, 1 H), 10.76 (s, 1 H), 10.46 (s, 1 H), 7.87 - 7.65 (m, 4 H), 7.50 (s, 1 H), 7.30 - 7.10 (m, 3 H), 6.93 - 6.59 (m, 1 H), 6.50 - 6.32 (m, 1 H), 5.14 (dd, *J=* 5.4, 12.7 Hz, 1 H), 4.89 (m, 1 H), 4.68 (m, 1 H), 4.29 (m, 1 H), 3.86 (s, 3 H), 3.76 (m, 3 H), 3.58 - 3.42 (m, 5 H), 3.17 - 2.82 (m, 10 H), 2.61 (m, 5 H), 2.16 - 1.93 (m, 12 H), 1.57 (m, 4 H), 1.28 - 1.21 (m, 1 H)

### Example 2-6 (Synthesis of 2006)

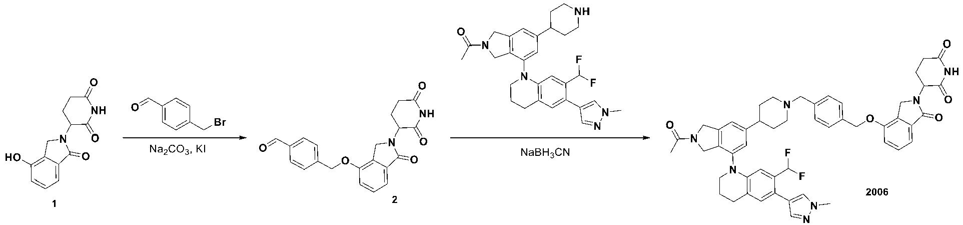

To a solution of 3-(4-hydroxy-1-oxo-3*H*-isoindol-2-yl)piperidine-2,6-dione (2000 mg, 7.7 mmol) and Na₂CO₃ (815 mg, 7.7 mmol) in DMF (20 mL) was added 4-(bromomethyl)benzaldehyde (1071 mg, 5.4 mmol) and KI (191 mg, 1.2 mmol). The mixture was stirred at 25 °C for 16 h. The mixture was concentrated under reduced pressure. The residue was added to H₂O/EtOAc (20 mL) and filtered to afford 4-({[2-(2,6-dioxopiperidin-3-yl)-1-oxo-3*H-*isoindol-4-yl]oxy}methyl)benzaldehyde (960 mg, yield 30%) as a brown solid. LC-MS: m/z [M+H]⁺ 379.1.

To a solution of 4-({[2-(2,6-dioxopiperidin-3-yl)-1-oxo-3H-isoindol-4-yl]oxy}methyl)benzaldehyde (0.67 g, 0.0017 mol), 1-{4-[7-(difluoromethyl)-6-(1-methylpyrazol-4-yl)-3,4-dihydro-2H-quinolin-1-yl)-6-(piperidin-4-yl)-1,3-dihydroisoindol-2-yl)ethenone (0.8 g, 0.0016 mol) and AcOH (0.1 g, 0.0016 mol) in DMSO/MeOH (24 mL) was added sodium borohydride triacetate (1.4 g, 0.0064 mol). The mixture was stirred at 45 °C for 4 h. The mixture was eluted by prep-HPLC to afford 3-[4-({4-[(4-{2-acetyl-7-[7-(difluoromethyl)-6-(1-methylpyrazol-4-yl)-3,4-dihydro-2H-quinolin-1-yl]-1 ,3-dihydroisoindol-5-yl }piperidin-1-yl)methyl]phenyl}methoxy)-1-oxo-3*H*-isoindol-2-yl]piperidine-2,6-dione (0.5 g, 38% yield) as a yellow solid.

¹H NMR (400 MHz, DMSO-*d*₆) δ 10.97 (s, 1H), 7.74 (s, 1H), 7.51 - 7.42 (m, 4H), 7.38 - 7.30 (m, 4H), 7.09-7.17 (m, 3H), 6.87 -6.59(m, 1H), 6.43-6.36 (m, 1H), 5.23 (s, 2H), 5.11-5.08 (m, 1H), 4.84 (s, 1H), 4.64 (s, 2H), 4.42 (d, *J =* 16.2 Hz, 1H), 4.25 (d, *J =* 16.8Hz, 2H), 3.86 (s, 3H), 3.60 - 3.47 (m, 4H), 2.96 - 2.83 (m, 5H), 2.56 (d, *J =* 18.6 Hz, 2H), 2.43 (d, *J =* 8.4 Hz, 1H), 2.09 - 1.94 (m, 8H), 1.75-1.63 (m, 4H). LC-MS: m/z [M+Na]+ 868.6.

### Example 2-7 (Synthesis of 2007)

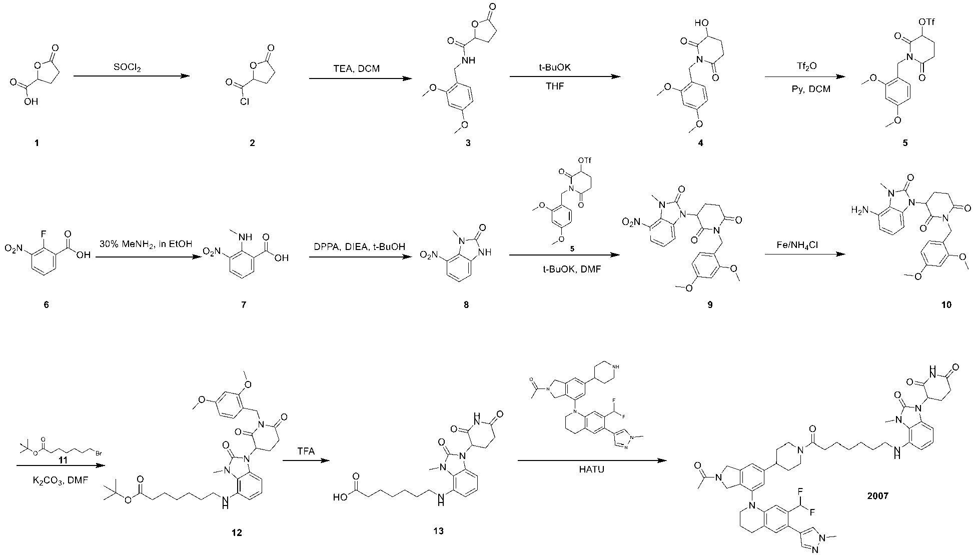

To 5-oxotetrahydrofuran-2-carboxylic acid (3.5 g, 26.9 mmol) was slowly added SOCl₂ (6.40 g, 53.8 mmol) at 0 °C. The mixture was stirred at 80 °C for 3 h. The mixture was concentrated in vacuo to afford 5-oxotetrahydrofuran-2-carbonyl chloride (4 g, crude) as a pale yellow oil.

(2,4-Dimethoxyphenyl)methanamine (4.5 g, 26.9 mmol) and TEA (5.5 g, 53.8 mmol) were dissolved in dry DCM (40 mL) at 0 °C under nitrogen protection. Then a solution of 5-oxotetrahydrofuran-2-carbonyl chloride (4 g, 26.9 mmol) in DCM (10 mL) was added, and the mixture was stirred at 15 °C for 1 h. Water (60 mL) was added, and the mixture was extracted with DCM (3×50 mL). The combined organic phases were washed with brine (50 mL), dried over anhydrous Na₂SO₄ and filtered. The filtrate was concentrated in vacuo. The crude product was purified by flash silica gel chromatography (ISCO^{®}; 40 g SepaFlash^{®} flash silica gel column, 0 to 35% EtOAc/petroleum ether, 40 mL/min gradient eluent) to afford N-[(2,4-dimethoxyphenyl)methyl]-5-oxotetrahydrofuran-2-carboxamide (2 g, 7.2 mmol, 26.59% yield) as a pale yellow solid.

¹H NMR (400 MHz, DMSO-*d*₆) δ 8.45 (t, *J =* 5.44 Hz, 1 H) 7.02 - 7.12 (m, 1 H) 6.55 (m, 1 H) 6.49 (m, 1 H) 4.91 (m, 1 H) 4.14 - 4.26 (m, 2 H) 3.77 (m, 6 H) 2.51 - 2.59 (m, 2 H) 2.37 - 2.48 (m, 1 H) 2.04 - 2.19 (m, 1 H).

N-[(2,4-dimethoxyphenyl)methyl]-5-oxotetrahydrofuran-2-carboxamide (4.6 g, 16.4 mmol) was dissolved in anhydrous THF (50 mL) and cooled to -70 °C. Then a solution of t-BuOK (2.0 g, 18.1 mmol) in anhydrous THF (30 mL) was slowly added dropwise at -70 °C under nitrogen atmosphere. The resulting reaction mixture was stirred at -50 °C for 1 h. The reaction mixture was quenched with saturated NH₄Cl solution (100 mL). The mixture was extracted with EtOAc (3×100 mL). The combined organic layers were washed with brine (100 mL), dried over anhydrous Na₂SO₄, filtered, and the filtrate was concentrated in vacuo to afford 1-[(2,4-dimethoxyphenyl)methyl]-3-hydroxypiperidine-2,6-dione (5 g, crude) as a pale yellow solid.

¹H NMR (400 MHz, CHLOROFORM-d) δ 6.96 (d, *J=* 8.19 Hz, 1 H) 6.37 - 6.45 (m, 2 H) 4.85 - 5.05 (m, 2 H) 4.25 (dd, *J=* 12.59, 5.62 Hz, 1 H) 3.79 (d, *J= 9.54* Hz, 6 H) 2.85 - 2.99 (m, 1 H) 2.61 - 2.75 (m, 1 H) 2.31 - 2.45 (m, 1 H) 1.93 (qd, *J=* 13.08, 4.77 Hz, 1 H).

To a solution of 1-[(2,4-dimethoxyphenyl)methyl]-3-hydroxypiperidine-2,6-dione (7.4 g, 26.5 mmol) and pyridine (4.2 g, 52.9 mmol) in DCM (140 mL) was added dropwise Tf₂O (11.2 g, 39.7 mmol) at -10 °C. The mixture was stirred at -10 °C under nitrogen for 1.5 h. After completion, the mixture was concentrated in vacuo. The crude product was purified by flash silica gel chromatography (ISCO^{®}; 40 g SepaFlash^{®} flash silica gel column, 0 to 15% EtOAc/petroleum ether, 40 mL/min gradient eluent) to afford [1-[(2,4-dimethoxyphenyl)methyl)-2,6-dioxo-3-piperidinyl] trifluoromethanesulfonate (6.2 g, 15.1 mmol, yield 56.9%) as a pale yellow solid.

¹H NMR (400 MHz, CHLOROFORM-d) δ 7.06 (d, *J=* 9.05 Hz, 1 H) 6.37 - 6.44 (m, 2 H) 5.30 - 5.33 (m, 1 H) 4.98 - 5.06 (m, 1 H) 4.78 - 4.90 (m, 1 H) 3.78 (s, 6 H) 2.93 - 3.04 (m, 1 H) 2.74 (m, 1 H) 2.29 - 2.46 (m, 2 H).

2-Fluoro-3-nitrobenzoic acid (15.0 g, 81.0 mmol) was dissolved in CH₃NH₂ (41.9 g, 405.2 mmol, 30% purity, in ethanol) and stirred in a sealed tube at 80 °C for 40 h. The mixture was cooled to room temperature and diluted with water (500 mL). The mixture was acidified to pH = 6 with 2 M HCl solution. The resulting mixture was filtered. The filter cake was washed with water (50 mL). The filter cake was dried under reduced pressure to afford 2-(methylamino)-3-nitrobenzoic acid (19 g, crude) as a pale yellow solid.

¹H NMR (400 MHz, DMSO-*d*₆) δ 13.46 (s, 1 H) 8.62 (s, 1 H) 7.93 - 8.23 (m, 2 H) 6.73 (t, *J* = 7.89 Hz, 1 H) 2.70 (s, 3 H).

To a solution of 2-(methylamino)-3-nitrobenzoic acid (15 g, 76.4 mmol) and DIPEA (29.6 g, 229.4 mmol) in t-BuOH (100 mL) was added DPPA (25.2 g, 91.7 mmol) at 20 °C under nitrogen atmosphere. The resulting mixture was stirred at 90 °C for 16 h. The mixture was cooled to room temperature and concentrated under reduced pressure. The crude product was purified by flash silica gel chromatography (ISCO^{®}; 40 g SepaFlash^{®} flash silica gel column, 0-100% EtOAc/petroleum ether, 40 mL/min gradient eluent) to afford 3-methyl-4-nitro-1H-benzoimidazol-2-one (11.5 g, 59.5 mmol, 77.9% yield) as a pale yellow solid.

¹H NMR (400 MHz, DMSO-*d*₆) δ 11.64 (s, 1 H) 7.61 (dd, J= 8.44, 0.73 Hz, 1 H) 7.33 (dd, *J=* 7.70, 0.73 Hz, 1 H) 7.10 - 7.21 (m, 1 H) 3.36 (s, 3 H).

To a solution of 3-methyl-4-nitro-1H-benzoimidazol-2-one (1.6 g, 8.28 mmol) in THF (50 mL) at -10 °C was added t-BuOK (1.9 g, 16.5 mmol) at 0 °C. After one hour, a solution of [1-[(2,4-dimethoxyphenyl)methyl)-2,6-dioxo-3-piperidinyl] trifluoromethanesulfonate (5.1 g, 12.4 mmol) in THF (50 mL) was added to the above mixture, and the reaction mixture was stirred at 0 to 20 °C for 15 h. The mixture was acidified to pH = 3 to 5 with acetic acid, diluted with water (100 mL), and extracted with EtOAc (2×100 mL). The organic layer was washed with brine (100 mL) and concentrated in vacuo. The crude product was purified by flash silica gel chromatography (ISCO^{®}; 40 g SepaFlash^{®} flash silica gel column, 0 to 50% EtOAc/petroleum ether, 40 mL/min gradient eluent) to afford 1-[(2,4-dimethoxyphenyl)methyl)-3-(3-methyl-4-nitro-2-oxobenzoimidazol-1-yl)piperidine-2,6-dione (2.5 g, 3.3 mmol, 39.2% yield, purity 59%) as a pale yellow oil. MS ES⁺: 455.3.

1-[(2,4-Dimethoxyphenyl)methyl)-3-(3-methyl-4-nitro-2-oxobenzoimidazol-1-yl)piperidine-2,6-dione (2.5 g, 2.5 mmol), NH₄Cl (1.35 g, 25.3 mmol) and Fe (1.4 g, 25.3 mmol) were dissolved in THF (50 mL) and H₂O (20 mL), and the mixture was stirred at 60 °C under N₂ atmosphere for 16 h. The reaction mixture was filtered and concentrated under reduced pressure. The residue was purified by column chromatography (SiO₂, petroleum ether/EtOAc = 1:0 to 0:1, then DCM/MeOH = 1:0 to 85:15) to afford 3-(4-amino-3-methyl-2-oxobenzoimidazol-1-yl)-1-[(2,4-dimethoxyphenyl)methyl]piperidine-2,6-dione (1.4 g, crude) as a pale yellow solid. MS ES⁺: 425.4.

A mixture of 3-(4-amino-3-methyl-2-oxobenzoimidazol-1-yl)-1-[(2,4-dimethoxyphenyl)methyl)piperidine-2,6-dione (0.8 g, 1.9 mmol), tert-butyl 7-bromoheptanoate (624 mg, 2.4 mmol), K₂CO₃ (781 mg, 5.7 mmol), NaI (282 mg, 1.9 mmol) and DMF (10 mL) was stirred in an oil bath at 100 °C for 16 h. The reaction mixture was filtered and concentrated in vacuo. The crude product was purified by flash silica gel chromatography (ISCO^{®}; 12 g SepaFlash^{®} flash silica gel column, 0 to 100% EtOAc/petroleum ether, 40 mL/min gradient eluent) to afford tert-butyl 7-[[1-[1-[(2,4-dimethoxyphenyl)methyl]-2,6-dioxo-3-piperidinyl]-3-methyl-2-oxobenzoimidazol-4-yl)amino)heptanoate (260 mg, 380.1 µmol, 20.2% yield, purity 89%) as a dark brown oil. MS ES⁺: 609.7.

A solution of tert-butyl 7-[[1-[1-[(2,4-dimethoxyphenyl)methyl]-2,6-dioxo-3-piperidinyl]-3-methyl-2-oxobenzoimidazol-4-yl]amino]heptanoate (200 mg, 328 µmol) in TFA (3 mL) was degassed and purged with N₂ three times, then the mixture was stirred in a sealed tube at 100 °C for 96 h. The reaction mixture was concentrated under reduced pressure. The residue was purified by column chromatography (SiO₂, DCM/MeOH = 1:0 to 85:15) to afford 7-[[1-(2,6-dioxo-3-piperidinyl)-3-methyl-2-oxobenzoimidazol-4-yl]amino]heptanoic acid (150 mg, 283.3 µmol, 86.2% yield, purity 76%) as a dark brown solid. MS ES⁺: 403.5.

7-[[1-(2,6-dioxo-3-piperidinyl)-3-methyl-2-oxobenzoimidazol-4-yl]amino]heptanoic acid (50 mg, 124 µmol) was dissolved in DMF (2 mL), DIPEA (48.1 mg, 372 µmol), HATU (70.8 mg, 186 µmol), and 1-[4-[7-(difluoromethyl)-6-(1-methylpyrazol-4-yl)-3,4-dihydro-2*H*-quinolin-1-yl]-6-(4-piperidinyl)isoindolin-2-yl]ethanone (50.2 mg, 99.3 µmol). The resulting solution was stirred at 20 °C for 16 h. The reaction mixture was filtered and concentrated under reduced pressure. The residue was purified by prep-HPLC (FA) to afford 3-[4-[7-[4-[2-acetyl-7-[7-(difluoromethyl)-6-(1-methylpyrazol-4-yl)-3,4-dihydro-2*H*-quinolin-1-yl]isoindol-5-yl]-1-piperidinyl]-7-oxoheptyl]amino]-3-methyl-2-oxobenzoimidazol-1-yl]piperidine-2,6-dione as a white solid (26.19 mg, 29.4 µmol, 23.7% yield, 100%).

MS ES⁺: 890.4

¹H NMR (400 MHz, DMSO- *d₆*) δ 11.06 (s, 1 H) 7.75 (s, 1 H) 7.49 (s, 1 H) 7.08 - 7.23 (m, 3 H) 6.58 - 6.94 (m, 2 H) 6.33 - 6.54 (m, 3 H) 5.28 (d, *J =* 7.51 Hz, 1 H) 4.81 - 5.03 (m, 2 H) 4.42 - 4.75 (m, 3 H) 4.28 (s, 1 H) 3.97 (s, 1 H) 3.86 (s, 3 H) 3.61 (s, 3 H) 3.55 (s, 2 H) 2.75 - 3.15 (m, 8 H) 2.68 (s, 2 H) 2.34 (s, 2 H) 1.73 - 2.11 (m, 8 H) 1.29 - 1.66 (m, 10 H)

### Example 2-8 (Synthesis of 2009)

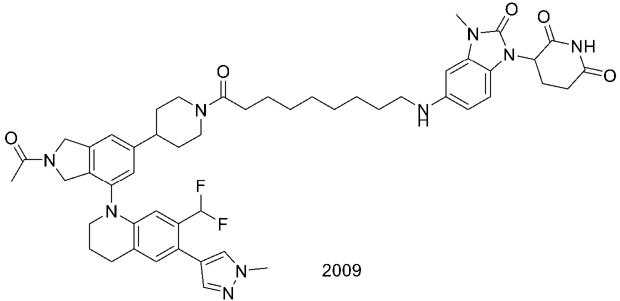

With reference to Synthesis of 004.

### Example 2-9 (Synthesis of INT-6)

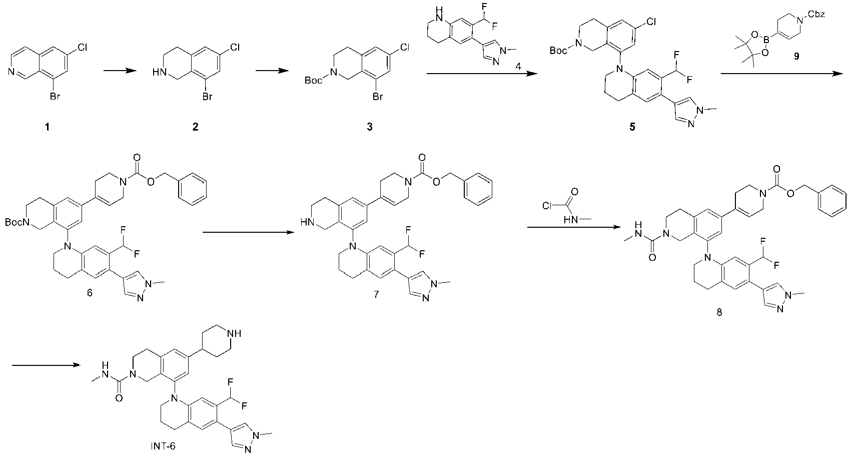

A solution of 8-bromo-6-chloroisoquinoline (8.6 g, 35.46 mmol) in AcOH (80 mL) was cooled to 0 °C, then NaBH₄ (2.68 g, 70.93 mmol) was added in portions. The mixture was stirred at 25 °C for 0.5 h. The mixture was poured into H₂O to quench the reaction, adjusted to pH = 8 with NH₄Cl, extracted with EtOAc (200 mL), and concentrated to afford 8-bromo-6-chloro-1,2,3,4-tetrahydroisoquinoline (8.48 g, 34.40 mmol, 97.0% yield, crude) as a yellow solid.

¹H NMR (400 MHz, CD₃Cl) δ 7.42 (s, 1 H), 7.09 (s, 1 H), 4.00 (s, 2H), 3.15-3.18(m, 2H), 2.85-2.88 (m, 2H).

To a solution of 8-bromo-6-chloro-1,2,3,4-tetrahydroisoquinoline (8.48 g, 34.40 mmol) in DCM (100 mL) were added TEA (6.96 g, 68.79 mmol) and Boc₂O (11.26 g, 51.60 mmol). The mixture was stirred at 25 °C for 12 h. The mixture was washed with saturated brine (50 mL × 2) to afford a crude product, which was purified by silica gel column chromatography (petroleum ether/ethyl acetate from 100% to 70%) to afford tert-butyl 8-bromo-6-chloro-3,4-dihydroisoquinoline-2(1H)-carboxylate (6.7 g, 19.33 mmol, 56.2% yield) as a yellow oil.

¹H NMR (400 MHz, CD₃Cl) δ 7.42 (s, 1 H), 7.10 (s, 1 H), 4.49 (s, 2H), 3.61-3.63(m, 2H), 2.80-2.82 (m, 2H), 1.50 (s, 9H).

Tert-butyl 8-bromo-6-chloro-3,4-dihydroisoquinoline-2(1H)-carboxylate (29.3 g, 84.52 mmol), 7-(difluoromethyl)-6-(1-methyl-1H-pyrazol-4-yl)-1,2,3,4-tetrahydroquinoline (13.35 g, 50.71 mmol), tBuONa (24.37 g, 253.57 mmol), and CPhos Pd G3 (6.82 g, 8.45 mmol) was dissolved in 1,4-dioxane (300 mL). The mixture was degassed and purged with N₂ three times, then the mixture was stirred at 100 °C under N₂ for 2 h. The mixture was extracted with EtOAc (200 mL) to afford a crude product, which was purified by silica gel column chromatography (petroleum ether/ethyl acetate from 100% to 70%) to afford tert-butyl 6-chloro-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (22.6 g, 42.72 mmol, 50.5% yield) as a yellow oil. LCMS (ESI+) : m/z 529.3 (M+H)⁺, Rt: 2.38 min.

To a solution of tert-butyl 6-chloro-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (300 mg, 0.567 mmol) and benzyl 4-(4,4,5,5-tetramethyl-1,3,2-dioxaborolan-2-yl)-3,6-dihydro-2H-pyridine-1-carboxylate (292 mg, 0.851 mmol) in THF (10 mL) and H₂O (1.0 mL) were added K₃PO₄ (360 mg, 1.70 mmol) and XPhos-Pd G3 (47.9 mg, 0.0567 mmol) at 25 °C. The reaction mixture was stirred at 60 °C under N₂ for 2 h. The mixture was quenched with water (100 mL) and extracted with ethyl acetate (100 mL × 2). The combined organic phases were dried over Na₂SO₄ and filtered. The filtrate was concentrated under reduced pressure. The residue was purified by silica gel chromatography, eluting with 0 to 5% methanol in DCM, to afford tert-butyl 6-(1-((benzyloxy)carbonyl)-1,2,3,6-tetrahydropyridin-4-yl)-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (308 mg, 77% yield) as a yellow solid. LC-MS: (ESI) m/z [M+H] ⁺ 710.3.

To a solution of tert-butyl 6-(1-((benzyloxy)carbonyl)-1,2,3,6-tetrahydropyridin-4-yl)-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (308 mg, 0.434 mmol) in DCM (3.0 mL) was added TFA (3.0 mL) at 25 °C. The reaction mixture was stirred at 25 °C for 1 h and concentrated under reduced pressure to afford benzyl 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-3,6-dihydropyridine-1(2H)-carboxylate (270 mg, crude) as a brown oil, which was used directly in the next step without further purification. LC-MS: (ESI) m/z [M+H] ⁺ 610.4.

To a solution of benzyl 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-3,6-dihydropyridine-1(2H)-carboxylate (265 mg, 0.435 mmol) in DCM (8.0 mL) was added TEA (132 mg, 0.869 mmol) at 25 °C. The reaction mixture was stirred at 25 °C for 10 min and concentrated under reduced pressure to afford benzyl 4-(8-(7-(difluoromethyl)-6-(1-methyl-1H-pyrazol-4-yl)-3,4-dihydroquinolin-1(2H)-yl)-2-(methylcarbamoyl)-1,2,3,4-tetrahydroisoquinolin-6-yl)-3,6-dihydropyridine-1(2H)-carboxylate (260 mg, 85% yield) as a white solid. LC-MS: (ESI) m/z [M+H] ⁺ 627.3.

To a solution of benzyl 4-(8-(7-(difluoromethyl)-6-(1-methyl-1H-pyrazol-4-yl)-3,4-dihydroquinolin-1(2H)-yl)-2-(methylcarbamoyl)-1,2,3,4-tetrahydroisoquinolin-6-yl)-3,6-dihydropyridine-1(2H)-carboxylate (260 mg, 0.390 mmol) in MeOH (10 mL) was added Pd/C (41.5 mg, 0.390 mmol). The mixture was stirred at 25 °C under H₂ balloon pressure for 16 h. The mixture was filtered to remove solids and concentrated to afford 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (160 mg, 73% yield) as a yellow solid. LC-MS: (ESI) m/z [M+H] ⁺ 535.3.

### Example 2-10 (Synthesis of 2010)

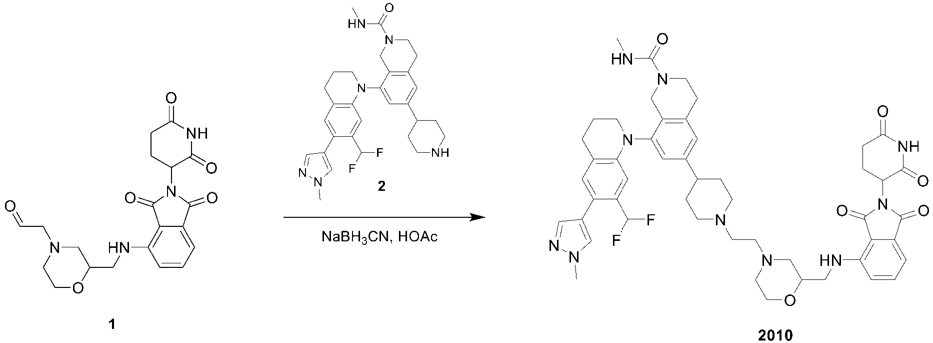

To a solution of 2-(2-((2-(2,6-dioxopiperidin-3-yl)-1,3-dioxoisoindolin-4-yl)amino)methyl)morpholino)acetaldehyde (200 mg, 443 µmol, HCl) in MeOH (2 mL), THF (2 mL) and DMSO (2 mL) were added 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (237 mg, 443 µmol), AcOH (26.6 mg, 443 µmol) and NaBH₃CN (139 mg, 2.2 mmol). The reaction mixture was stirred at 30 °C for 16 h. H₂O (20 mL) was added to the mixture, which was filtered to afford a filter cake. Purification by prep-HPLC (FA) afforded 8-(7-(difluoromethyl)-6-(1-methyl-1H-pyrazol-4-yl)-3,4-dihydroquinolin-1(2H)-yl)-6-(1-(2-((2-(2,6-dioxopiperidin-3-yl)-1,3-dioxoisoindolin-4-yl)amino)methyl)morpholino)ethyl)piperidin-4-yl)-N-methyl-3,4-dihydroisoquinoline-2(1H)-carboxamide (20.7 mg, 22.2 µmol, 5.0% yield) as a yellow solid. MS ES⁺: 933.5.

¹H NMR (400 MHz, DMSO- *d*₆) 11.12 (s, 1H), 8.24 (s, 1H), 7.72 (s, 1H), 7.57 (t, J = 7.8 Hz, 1H), 7.47 (s, 1H), 7.14 (d, J = 8.7 Hz, 1H), 7.09 - 6.98 (m, 4H), 6.72 - 6.54 (m, 2H), 6.53 - 6.45 (m, 1H), 6.18 (s, 1H), 5.05 (dd, J = 5.4, 12.9 Hz, 1H), 4.41 (d, J = 16.8 Hz, 1H), 4.10 (d, J = 16.8 Hz, 1H), 3.85 (s, 3H), 3.83 - 3.77 (m, 2H), 3.65 - 3.59 (m, 4H), 3.01 - 2.93 (m, 3H), 2.89 - 2.79 (m, 6H), 2.73 - 2.66 (m, 2H), 2.55 - 2.53 (m, J = 4.3 Hz, 4H), 2.44 (s, 4H), 2.11 - 1.97 (m, 6H), 1.88 (t, J = 10.5 Hz, 1H), 1.77 - 1.52 (m, 6H), 1.23 (s, 1H)

### Example 2-11 (Synthesis of 2012)

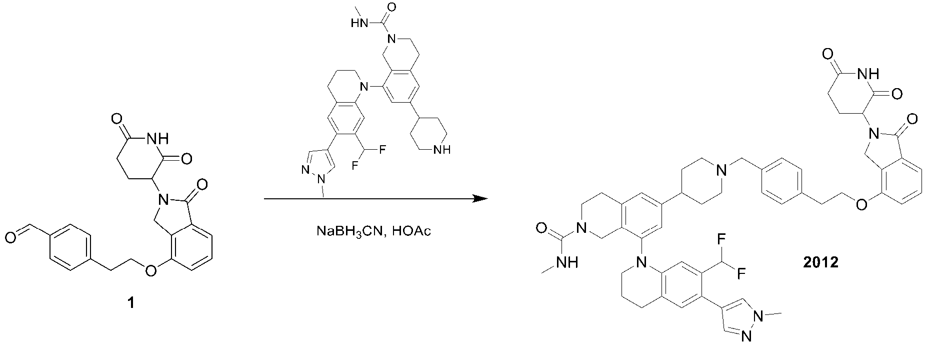

To a solution of 4-(2-((2-(2,6-dioxopiperidin-3-yl)-1-oxoisoindolin-4-yl)oxy)ethyl)benzaldehyde (40 mg, 101.9 µmol) in MeOH (2 mL), THF (2 mL) and DMSO (2 mL) were added 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (54.5 mg, 101.9 µmol), AcOH (6.1 mg, 101.9 µmol, 5.8 µL) and NaBH₃CN (32.0 mg, 509.7 µmol). The mixture was stirred at 30 °C for 12 h. H₂O (20 mL) was added to the mixture, which was filtered to afford a filter cake. The filter cake was purified by prep-HPLC (FA) to afford 8-(7-(difluoromethyl)-6-(1-methyl-1H-pyrazol-4-yl)-3,4-dihydroquinolin-1(2H)-yl)-6-(1-(4-(2-(2,6-dioxopiperidin-3-yl)-1-oxoisoindolin-4-yl)oxy)ethyl)benzyl)piperidin-4-yl)-N-methyl-3,4-dihydroisoquinoline-2(1H)-carboxamide (24.4 mg, 26.7 µmol, 26.2% yield) as a pink solid. MS ES⁺: 911.4.

¹H NMR (400 MHz, DMSO- *d*₆) 11.00 (s, 1H), 8.30 (s, 1H), 7.71 (s, 1H), 7.49 - 7.43 (m, 2H), 7.31 - 7.23 (m, 6H), 7.11 - 6.96 (m, 3H), 6.83 - 6.54 (m, 1H), 6.53 - 6.43 (m, 1H), 6.17 (s, 1H), 5.10 (dd, J = 5.0, 13.3 Hz, 1H), 4.46 - 4.38 (m, 1H), 4.34 - 4.27 (m, 3H), 4.22 - 4.04 (m, 4H), 3.85 (s, 4H), 3.45 (s, 3H), 3.03 (t, J = 6.4 Hz, 2H), 2.95 - 2.78 (m, 7H), 2.59 (s, 1H), 2.54 (d, J = 4.0 Hz, 3H), 2.47 - 2.37 (m, 2H), 2.09 - 1.94 (m, 5H), 1.74 - 1.55 (m, 4H)

### Example 2-12 (Synthesis of 2013)

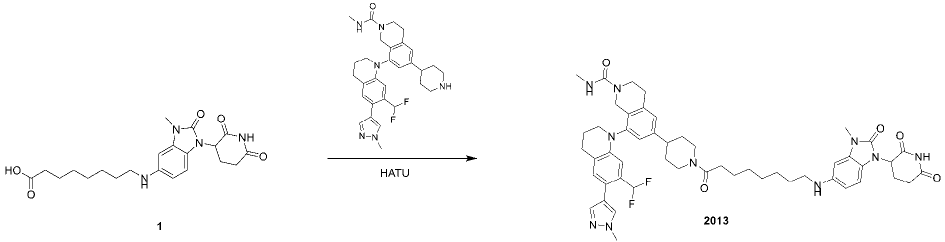

To a solution of 8-[[1-(2,6-dioxo-3-piperidinyl)-3-methyl-2-oxobenzoimidazol-5-yl)amino)octanoic acid (220 mg, 528 µmol) and 8-(7-(difluoromethyl)-6-(1-methylpyrazol-4-yl)-3,4-dihydro-2H-quinolin-1-yl)-N-methyl-6-(4-piperidinyl)-3,4-dihydro-1H-isoquinoline-2-carboxamide (254 mg, 475 µmol) in DMF (4 mL) were added DIPEA (204 mg, 1.6 mmol) and HATU (301 mg, 792 µmol). The resulting solution was stirred at 15 °C for 16 h. H₂O (5 mL) was added dropwise, and the solid was filtered. The crude product was further purified by prep-HPLC (column: Welch Xtimate C18 150×30 mm×5 µm; mobile phase A: water (FA), mobile phase B: acetonitrile; flow rate: 25 mL/min; gradient from 0% B to 40% B). Pure fractions were collected and volatiles were removed in vacuo. The residue was dissolved in acetonitrile (2 mL) and water (10 mL), and lyophilized to afford 8-(7-(difluoromethyl)-6-(1-methyl-1H-pyrazol-4-yl)-3,4-dihydroquinolin-1(2H)-yl)-6-(8-((1-(2,6-dioxopiperidin-3-yl)-3-methyl-2-oxo-2,3-dihydro-1H-benzo[d]imidazol-5-yl)amino)octanoyl)piperidin-4-yl)-N-methyl-3,4-dihydroisoquinoline-2(1H)-carboxamide (50 mg, 50.5 µmol, 9.6% yield, 94.2% purity) as a pale yellow solid. MS ES⁺: 933.5.

¹H NMR (400 MHz, DMSO- *d*₆) 11.06 (s, 1 H) 7.72 (s, 1 H) 7.47 (s, 1 H) 6.96 - 7.12 (m, 3 H) 6.55 - 6.85 (m, 2 H) 6.49 (s, 1 H) 6.38 (s, 1 H) 6.27 (d, *J=* 8.56 Hz, 1 H) 6.18 (s, 1 H) 5.19 - 5.35 (m, 2 H) 4.34 - 4.60 (m, 2 H) 4.11 (dd, J = 16.81, 5.93 Hz, 1 H) 3.80 - 4.00 (m, 4 H) 3.44 (m, 4 H) 3.25 (s, 2 H) 2.96 - 3.10 (m, 2 H) 2.80 - 2.93(m, 5 H) 2.62 - 2.75 (m, 3 H) 2.53 - 2.59 (m, 5 H) 2.31 (m, 2 H) 1.90 - 2.13 (m, 4 H) 1.76 (m, 2 H) 1.52 (m, 6 H) 1.31 (m, 6 H)

### Example 2-13 (Synthesis of 2014)

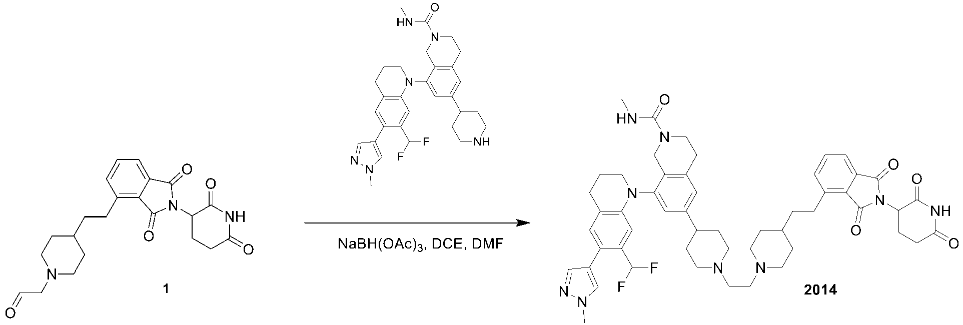

A mixture of 2-[4-[2-[2-(2,6-dioxo-3-piperidinyl)-1,3-dioxoisoindol-4-yl]ethyl]-1-piperidinyl]acetaldehyde (40 mg, 97.2 µmol) and 8-[7-(difluoromethyl)-6-(1-methylpyrazol-4-yl)-3,4-dihydro-2H-quinolin-1-yl]-N-methyl-6-(4-piperidinyl)-3,4-dihydro-1H-isoquinoline-2-carboxamide (52.0 mg, 97.2 µmol) was dissolved in DCE (2 mL) and DMF (1 mL). The mixture was degassed and purged with N₂ three times. NaBH(OAc)₃ (123 mg, 583 µmol) was added, and the mixture was stirred at 30 °C for 16 h. The reaction was quenched by addition of water (5 mL) at 20 °C. The resulting mixture was extracted with ethyl acetate (3×10 mL). The combined organic layers were washed with brine (20 mL), dried over anhydrous Na₂SO₄, filtered, and concentrated under reduced pressure. The residue was purified by prep-TLC and prep-HPLC (TFA conditions) to afford the desired compound. 1 M HCl (2 mL) was added, and the mixture was lyophilized to afford 8-(7-(difluoromethyl)-6-(1-methylpyrazol-4-yl)-3,4-dihydro-2H-quinolin-1-yl)-6-[1-[2-[4-[2-[2-(2,6-dioxo-3-piperidinyl)-1,3-dioxoisoindolin-4-yl]ethyl]-1-piperidinyl]ethyl]-4-piperidinyl]-N-methyl-3,4-dihydro-1H-isoquinoline-2-carboxamide (2 mg, 2.1 µmol, 2.1% yield, HCl salt) as a pale yellow solid..MS ES⁺: 930.5.

¹H NMR (400MHz, DMSO- *d*₆)11.13 (s, 1 H), 10.63 (s, 1 H), 10.42 (s, 1 H), 7.75 (d, J= 11.2 Hz, 5 H), 7.47 (s, 1 H), 7.16 - 6.95 (m, 3 H), 6.85 - 6.54 (m, 1 H), 6.17 (s, 1 H), 5.13 (d, *J=* 11.5 Hz, 1 H), 4.43 (d, J = 17.9 Hz, 1 H), 4.11 (d, *J=* 16.4 Hz, 2 H), 3.84 (s, 3 H), 3.56 (s, 9 H), 3.49 -3.30 (m, 2 H), 3.05 (s, 3 H), 2.87 (d, J = 13.7 Hz, 9 H), 2.62 (s, 3 H), 2.53 (s, 3 H), 2.03 (m, 8 H), 1.70 (m, 1 H), 1.55 (m, 4 H)

### Example 2-14 (Synthesis of 2015)

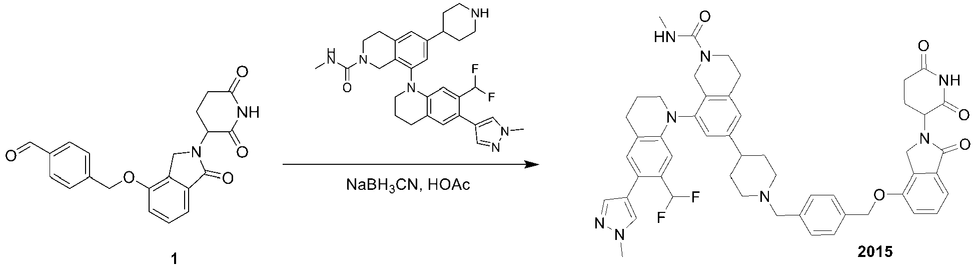

To a solution of 4-((2-(2,6-dioxopiperidin-3-yl)-1-oxoisoindolin-4-yl)oxy)methyl)benzaldehyde (50 mg, 132.1 µmol) in THF (1 mL), MeOH (1 mL) and DMSO (1 mL) were added 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (91.8 mg, 171.8 µmol), AcOH (7.9 mg, 132.1 µmol, 7.6 µL) and NaBH₃CN (41.5 mg, 660.7 µmol). The mixture was stirred at 20 °C for 16 h. H₂O (30 mL) was added to the mixture, which was filtered to afford a filter cake. The filter cake was purified by prep-HPLC (FA) to afford 8-(7-(difluoromethyl)-6-(1-methyl-1H-pyrazol-4-yl)-3,4-dihydroquinolin-1(2H)-yl)-6-(1-(4-(((2-(2,6-dioxopiperidin-3-yl)-1-oxoisoindolin-4-yl)oxy)methyl)benzyl)piperidin-4-yl)-N-methyl-3,4-dihydroisoquinoline-2(1H)-carboxamide (11.6 mg, 12.9 µmol, 9.8% yield) as a white solid. MS ES⁺: 897.5.

¹H NMR (400 MHz, DMSO- *d*₆) 10.98 (s, 1H), 8.26 - 8.07 (m, 1H), 7.72 (s, 1H), 7.51 - 7.41 (m, 4H), 7.39 - 7.29 (m, 4H), 7.13 - 6.94 (m, 3H), 6.86 - 6.43 (m, 2H), 6.21 - 6.12 (m, 1H), 5.22 (s, 2H), 5.16 - 5.07 (m, 1H), 4.50 - 4.37 (m, 2H), 4.29 - 4.20 (m, 1H), 4.14 - 4.04 (m, 1H), 3.85 (s, 3H), 3.59 (s, 1H), 3.49 - 3.35 (m, 5H), 2.98 - 2.74 (m, 7H), 2.68 - 2.60 (m, 1H), 2.57 (s, 1H), 2.54 (d, J = 3.9 Hz, 4H), 2.43 - 2.38 (m, 1H), 2.14 - 1.91 (m, 5H), 1.76 - 1.56 (m, 3H)

### Example 2-15 (Synthesis of 2016)

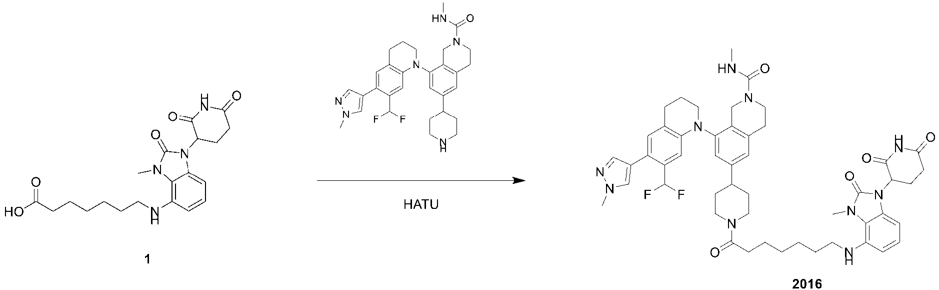

After dissolving 7-[[1-(2,6-dioxo-3-piperidinyl)-3-methyl-2-oxobenzoimidazol-4-yl)amino)heptanoic acid (50 mg, 124 µmol) in DMF (2 mL), DIPEA (48.1 mg, 372 µmol), HATU (70.8 mg, 186 µmol) and 8-[7-(difluoromethyl)-6-(1-methylpyrazol-4-yl)-3,4-dihydro-2H-quinolin-1-yl]-N-methyl-6-(4-piperidinyl)-3,4-dihydro-1H-isoquinoline-2-carboxamide (53.1 mg, 99.3 µmol) were added. The resulting solution was stirred at 15 °C for 16 h. The reaction mixture was filtered and concentrated under reduced pressure. The residue was purified by prep-HPLC (FA) to afford 8-[7-(difluoromethyl)-6-(1-methylpyrazol-4-yl)-3,4-dihydro-2H-quinolin-1-yl)-6-[1-[7-[[1-(2,6-dioxo-3-piperidinyl)-3-methyl-2-oxobenzoimidazol-4-yl]amino]heptanoyl]-4-piperidinyl]-N-methyl-3,4-dihydro-1H-isoquinoline-2-carboxamide (22.44 mg, 24.4 µmol, 19.6% yield, 100% purity) as a white solid. MS ES⁺: 941.3 (M+23).

¹H NMR (400 MHz, DMSO-*d*₆) δ 11.07 (s, 1 H) 7.72 (s, 1 H) 7.47 (s, 1 H) 6.99 - 7.13 (m, 3 H) 6.53 - 6.93 (m, 2 H) 6.37 - 6.51 (m, 3 H) 6.18 (s, 1 H) 5.28 (dd, *J =* 12.46, 5.30 Hz, 1 H) 5.01 (s, 1 H) 4.36 - 4.58 (m, 2 H) 3.91 - 4.17 (m, 2 H) 3.84 - 3.89 (m, 3 H) 3.61 (s, 3 H) 3.47 - 3.53 (m, 3 H) 2.97 - 3.11 (m, 3 H) 2.78 - 2.96 (m, 6 H) 2.61 - 2.77 (m, 6 H) 2.28 - 2.36 (m, 3 H) 1.89 - 2.15 (m, 4 H) 1.77 (s, 2 H) 1.31 - 1.67 (m, 10 H).

### Example 2-16 (Synthesis of 2018)

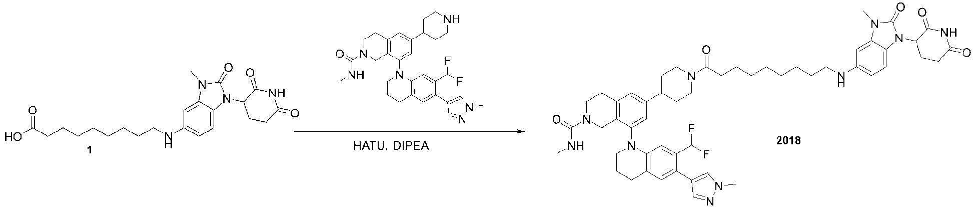

To a solution of 9-((1-(2,6-dioxopiperidin-3-yl)-3-methyl-2-oxo-2,3-dihydro-1H-benzo[d]imidazol-5-yl)amino)nonanoic acid (100 mg, 0.23 mmol) and 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisoquinoline-2(1H)-carboxamide (124.2 mg, 0.23 mmol) in DMF (2.0 mL) were added DIPEA (90.1 mg, 0.70 mmol) and HATU (106.0 mg, 0.28 mmol). The mixture was stirred at 25 °C for 1 h. The reaction was quenched by addition of water (20 mL), extracted with EtOAc (20 mL×3), evaporated to dryness, and purified by prep-HPLC (eluting from 30% to 60% ACN in 0.1% FA/water over 8 min) to afford 8-(7-(difluoromethyl)-6-(1-methyl-1H-pyrazol-4-yl)-3,4-dihydroquinolin-1(2H)-yl)-6-(1-((2,6-dioxopiperidin-3-yl)-3-methyl-2-oxo-2,3-dihydro-1H-benzo[d]imidazol-5-yl)amino)nonanoyl)piperidin-4-yl)-N-methyl-3,4-dihydroisoquinoline-2(1H)-carboxamide (38 mg, 17% yield) as a white solid.

¹H NMR (400 MHz, DMSO-*d*₆) δ 11.02 (s, 1H), 7.71 (s, 1H), 7.46 (s, 1H), 7.08 (s, 1H), 7.04 - 7.00 (m, 2H), 6.83 (s, 0.24 H), 6.79 (d, *J =* 8.4 Hz, 1H), 6.69 (s, 0.50H), 6.55 (s, 0.26H), 6.47 - 6.44 (m, 1H), 6.37 (d, *J= 2.0* Hz, 1H), 6.31 - 6.24 (m, 1H), 6.17 (s, 1H), 5.26-5.20 (m, 2H), 4.52 - 4.39 (m, 3H), 4.13 - 4.08 (m, 1H), 3.96 - 3.91 (m, 1H), 3.85 (s, 3H), 3.62 - 3.35 (m, 4H), 3.24 (s, 3H), 3.09 - 3.03 (m, 1H), 2.98 -2.96 (m, 2H), 2.93 - 2.80 (m, 4H), 2.75 - 2.61 (m, 5H), 2.54 (d, *J=* 4.4 Hz, 3H), 2.38 - 2.24 (m, 2H), 2.08 - 1.94 (m, 4H), 1.79 - 1.72 (m, 2H), 1.56 - 1.48 (m, 4H), 1.40 - 1.21 (m, 8H). ¹⁹F NMR (400 MHz, DMSO-*d*₆) δ 108.04. LC-MS: m/z 947.4 [M+H]⁺.

### Example 2-17 (Synthesis of 2019)

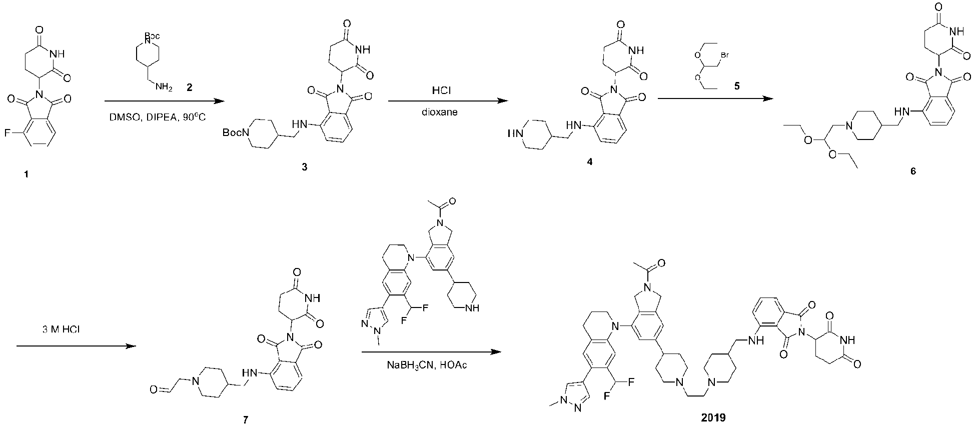

To a solution of 2-(2,6-dioxopiperidin-3-yl)-4-fluoroisoindoline-1,3-dione (1 g, 3.6 mmol) in DMSO (15 mL) were added DIPEA (2.3 g, 18.1 mmol, 3.2 mL) and tert-butyl 4-(aminomethyl)piperidine-1-carboxylate (775.9 mg, 3.6 mmol). The mixture was stirred at 90 °C for 12 h. The reaction mixture was quenched with H₂O (10 mL), diluted with ethyl acetate (20 mL) and extracted with ethyl acetate (20 mL×2). The combined organic layers were washed with 10 mL brine (10 mL×1), dried over Na₂SO₄, filtered, and concentrated under reduced pressure to give a residue. The residue was purified by silica gel column chromatography to afford tert-butyl 4-((2-(2,6-dioxopiperidin-3-yl)-1,3-dioxoisoindolin-4-yl)amino)methyl)piperidine-1-carboxylate (850 mg, 1.8 mmol, 49.9% yield). MS ES⁺: 471.3.

To a solution of tert-butyl 4-((2-(2,6-dioxopiperidin-3-yl)-1,3-dioxoisoindolin-4-yl)amino)methyl)piperidine-1-carboxylate (2.4 g, 5.1 mmol) in 1,4-dioxane (10 mL) was added HCl/1,4-dioxane (4 M, 15 mL). The mixture was stirred at 20 °C for 2 h. The mixture was concentrated to afford 2-(2,6-dioxopiperidin-3-yl)-4-((piperidin-4-ylmethyl)amino)isoindoline-1,3-dione (2.6 g, crude, HCl) as a yellow solid, which was used directly in the next step. MS ES⁺: 371.2.

To a solution of 2-(2,6-dioxopiperidin-3-yl)-4-((piperidin-4-ylmethyl)amino)isoindoline-1,3-dione (2.57 g, 6.9 mmol) in ACN (30 mL) were added DIPEA (9.0 g, 69.4 mmol, 12.1 mL) and 2-bromo-1,1-diethoxyethane (5.5 g, 27.8 mmol, 4.2 mL). The mixture was stirred at 80 °C for 12 h. The mixture was concentrated to afford a crude product. Purification by silica gel column chromatography followed by prep-HPLC (FA) afforded 4-((1-(2,2-diethoxyethyl)piperidin-4-yl)methyl)amino)-2-(2,6-dioxopiperidin-3-yl)isoindoline-1,3-dione (1.5 g, 3.0 mmol, 43.5% yield) as a yellow solid. MS ES⁺: 487.4.

To a solution of 4-((1-(2,2-diethoxyethyl)piperidin-4-yl)methyl)amino)-2-(2,6-dioxopiperidin-3-yl)isoindoline-1,3-dione (470 mg, 966.0 µmol) in ACN (3 mL) was added HCl (3 M, 8 mL). The mixture was stirred at 55 °C for 16 h. The mixture was neutralized with aqueous NaHCO₃ and extracted with DCM (20 mL). The organic layer was dried over Na₂SO₄, filtered, and concentrated to afford 2-(4-((2-(2,6-dioxopiperidin-3-yl)-1,3-dioxoisoindolin-4-yl)amino)methyl)piperidin-1-yl)acetaldehyde (230 mg, crude, HCl) as a yellow solid, which was used directly in the next step.

¹H NMR (400MHz, DMSO-*d*₆) δ 11.11 (s, 1H), 9.57 (s, 1H), 7.60 - 7.53 (m, 1H), 7.14 (d, *J* = 8.7 Hz, 1H), 7.02 (d, *J =* 7.0 Hz, 1H), 6.65 - 6.54 (m, 1H), 5.05 (dd, *J =* 5.2, 12.8 Hz, 1H), 3.23 (t, *J=* 6.3 Hz, 2H), 3.11 (s, 2H), 2.94 - 2.86 (m, 1H), 2.82 (d, *J =* 11.0 Hz, 2H), 2.62 - 2.52 (m, 2H), 2.09 - 1.97 (m, 3H), 1.71 - 1.50 (m, 3H), 1.35 - 1.22 (m, 2H).

To a solution of 2-(4-(((2-(2,6-dioxopiperidin-3-yl)-1,3-dioxoisoindolin-4-yl)amino)methyl)piperidin-1-yl)acetaldehyde (80 mg, 194.0 µmol) in MeOH (2 mL), THF (2 mL) and DMSO (2 mL) were added 1-(4-(7-(difluoromethyl)-6-(1-methyl-1H-pyrazol-4-yl)-3,4-dihydroquinolin-1(2H)-yl)-6-(piperidin-4-yl)isoindolin-2-yl)ethan-1-one (107.9 mg, 213.4 µmol), AcOH (11.7 mg, 194.0 µmol, 11.1 µL) and NaBH₃CN (61.0 mg, 969.8 µmol). The mixture was stirred at 30 °C for 16 h. H₂O (20 mL) was added to the mixture, which was filtered to afford a filter cake. The filter cake was purified by prep-HPLC (TFA) to afford 4-((1-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)piperidin-4-yl)methyl)amino)-2-(2,6-dioxopiperidin-3-yl)isoindoline-1,3-dione (1.9 mg, 2.1 µmol, 1% yield) as a yellow solid. MS ES⁺: 902.5.

¹H NMR (400 MHz, DMSO- *d*₆) 11.11 (s, 1H), 10.75 (s, 1H), 10.39 (s, 1H), 7.75 (s, 1H), 7.59 (t, J = 7.6 Hz, 1H), 7.49 (s, 1H), 7.24 - 7.09 (m, 4H), 7.07 - 7.01 (m, 1H), 6.91 - 6.59 (m, 2H), 6.46 - 6.31 (m, 1H), 5.11 - 5.00 (m, 1H), 4.88 (s, 1H), 4.67 (s, 1H), 4.29 (s, 1H), 3.86 (s, 3H), 3.79 - 3.66 (m, 4H), 3.56 (s, 2H), 2.88 (s, 6H), 2.67 (s, 2H), 2.60 (s, 3H), 2.42 (s, 1H), 2.33 (s, 1H), 2.06 - 1.97 (m, 9H), 1.61 - 1.42 (m, 3H), 1.23 (s, 6H), 0.88 - 0.80 (m, 1H)

### Example 2-18 (Synthesis of 2020)

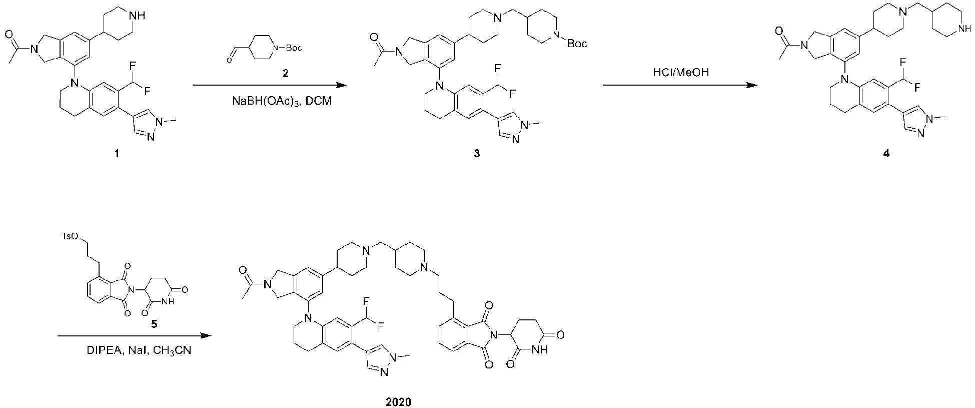

1-[4-[7-(difluoromethyl)-6-(1-methylpyrazol-4-yl)-3,4-dihydro-2H-quinolin-1-yl]-6-(4-piperidinyl)isoindol-2-yl]ethanone (150 mg, 296 µmol), tert-butyl 4-formylpiperidine-1-carboxylate (63.2 mg, 296 µmol) and NaBH(OAc)₃ (66.0 mg, 311.5 µmol) were dissolved in DCM (4 mL). The mixture was stirred at 15 °C for 48 h. The mixture was concentrated in vacuo. The residue was purified by column chromatography (SiO₂, DCM:MeOH = 1:0 to 9:1) to afford tert-butyl 4-[[4-[2-acetyl-7-[7-(difluoromethyl)-6-(1-methylpyrazol-4-yl)-3,4-dihydro-2H-quinolin-1-yl]isoindol-5-yl]-1-piperidinyl]methyl]piperidine-1-carboxylate (100 mg, 139 µmol, 47.0% yield, 98% purity) as a pale yellow solid. MS ES⁺: 703.7.

To a solution of tert-butyl 4-[[4-[2-acetyl-7-[7-(difluoromethyl)-6-(1-methylpyrazol-4-yl)-3,4-dihydro-2H-quinolin-1-yl]isoindol-5-yl]-1-piperidinyl]methyl]piperidine-1-carboxylate (100 mg, 142 µmol) in MeOH (1 mL) was added HCl/1,4-dioxane (5 mL). The mixture was stirred at 15 °C for 2 h. The mixture was concentrated under reduced pressure to afford 1-[4-[7-(difluoromethyl)-6-(1-methylpyrazol-4-yl)-3,4-dihydro-2H-quinolin-1-yl]-6-[1-(4-piperidinylmethyl)-4-piperidinyl]isoindol-2-yl]ethanone (200 mg, crude, 2HCl) as a pale yellow solid. MS ES⁺: 603.6.

To a solution of 1-[4-[7-(difluoromethyl)-6-(1-methylpyrazol-4-yl)-3,4-dihydro-2H-quinolin-1-yl]-6-[1-(4-piperidinylmethyl)-4-piperidinyl]isoindol-2-yl]ethanone (80.0 mg, 125.1 µmol, HCl salt) and 3-[2-(2,6-dioxo-3-piperidinyl)-1,3-dioxoisoindol-4-yl]propyl 4-methylbenzenesulfonate (58.8 mg, 125.1 µmol) in ACN was added DIPEA (80.8 mg, 625 µmol, 4 mL). The mixture was stirred at 80 °C for 16 h. The reaction mixture was filtered and concentrated under reduced pressure. The residue was purified by prep-HPLC (FA) to afford 4-[3-[4-[4-[2-acetyl-7-[7-(difluoromethyl)-6-(1-methylpyrazol-4-yl)-3,4-dihydro-2H-quinolin-1-yl]isoindol-5-yl]-1-piperidinyl]methyl]-1-piperidinyl]propyl]-2-(2,6-dioxo-3-piperidinyl)isoindol-1,3-dione (28.32 mg, 28.4 µmol, 22.6% yield, 90.2% purity) as a white solid. MS ES⁺: 901.4.

¹H NMR (400 MHz, DMSO-*d*₆) δ 11.13 (s, 1 H) 8.15 (s, 1 H) 7.68 - 7.85 (m, 4 H) 7.46 - 7.51 (m, 2 H) 7.08 - 7.18 (m, 4 H) 6.59 - 6.91 (m, 1 H) 6.35 - 6.48 (m, 1 H) 5.14 (dd, J=12.70, 5.42 Hz, 1 H) 4.86 (s, 1 H) 4.65 (s, 1 H) 4.30 (s, 1 H) 3.87 (s, 3 H) 3.55 (d, J=4.77 Hz, 1 H) 3.03 - 3.17 (m, 6 H) 2.84 - 3.02 (m, 6 H) 2.55 - 2.65 (m, 5 H) 2.22 - 2.31 (m, 4 H) 1.97 - 2.16 (m, 8 H) 1.89 (br s, 2 H) 1.59 - 1.83 (m, 6 H) 1.14 - 1.26 (m, 2 H)

### Example 2-19 (Synthesis of 2021)

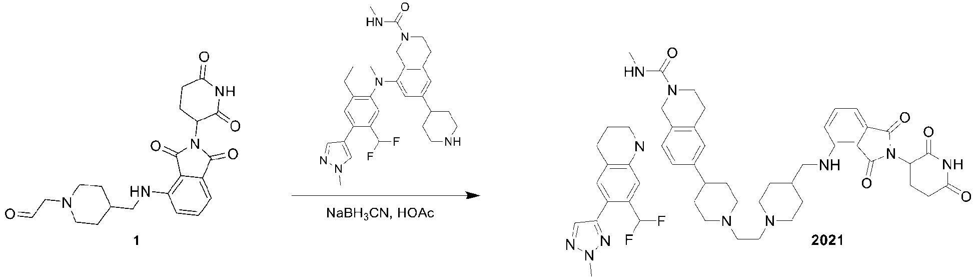

To a solution of 2-(4-(((2-(2,6-dioxopiperidin-3-yl)-1,3-dioxoisoindolin-4-yl)amino)methyl)piperidin-1-yl)acetaldehyde (91 mg, 202.7 µmol, HCl) in MeOH (2 mL), DMSO (2 mL) and THF (2 mL) were added 8-(7-(difluoromethyl)-6-(1-methyl-1H-pyrazol-4-yl)-3,4-dihydroquinolin-1(2H)-yl)-N-methyl-6-(piperidin-4-yl)-3,4-dihydroisopropanolquinoline-2(1H)-carboxamide (108.4 mg, 202.7 µmol), AcOH (12.2 mg, 202.7 µmol, 11.6 µL) and NaBH₃CH (63.7 mg, 1.0 mmol). The mixture was stirred at 30 °C for 2 h. Water (20 mL) was added and the mixture was filtered to afford a filter cake. The filter cake was purified by prep-HPLC (FA) to afford 8-(7-(difluoromethyl)-6-(1-methyl-1H-pyrazol-4-yl)-3,4-dihydroquinolin-1(2H)-yl)-6-(1-(2-(2,6-dioxopiperidin-3-yl)-1,3-dioxoisoindolin-4-yl)amino)methyl)piperidin-1-yl)ethyl)piperidin-4-yl)-N-methyl-3,4-dihydroisoquinoline-2(1H)-carboxamide (18.6 mg, 19.9 µmol, 9.8% yield) as a yellow solid. MS ES⁺: 931.5.

¹H NMR (400 MHz, DMSO- *d*₆) 11.11 (s, 1H), 8.27 (s, 1H), 7.71 (s, 1H), 7.57 (t, J = 7.8 Hz, 1H), 7.46 (s, 1H), 7.15 - 7.07 (m, 2H), 7.05 - 6.94 (m, 3H), 6.85 - 6.54 (m, 2H), 6.52 - 6.43 (m, 1H), 6.17 (s, 1H), 5.09 - 5.00 (m, 1H), 4.41 (d, J = 16.6 Hz, 1H), 4.10 (d, J = 16.8 Hz, 1H), 3.85 (s, 3H), 3.49 - 3.45 (m, 3H), 3.44 - 3.40 (m, 2H), 3.20 (t, J = 5.6 Hz, 2H), 3.00 - 2.85 (m, 7H), 2.85 - 2.76 (m, 3H), 2.62 - 2.58 (m, 1H), 2.54 (d, J = 4.0 Hz, 3H), 2.44 (s, 4H), 2.10 - 1.98 (m, 5H), 1.93 (t, J = 11.1 Hz, 2H), 1.74 - 1.53 (m, 7H), 1.28 - 1.16 (m, 2H)

### Example 2-20 (Synthesis of 2022)

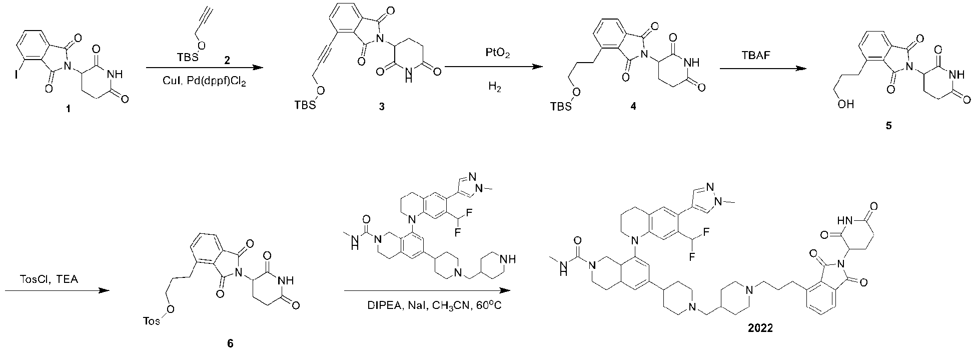

2-(2,6-Dioxopiperidin-3-yl)-4-iodoisoindoline-1,3-dione (1 g, 2.6 mmol), tert-butyldimethyl(prop-2-yn-1-yloxy)silane (532.1 mg, 3.1 mmol, 633.4 µL), Pd(PPh₃)₂Cl₂ (182.7 mg, 260.3 µmol), CuI (49.6 mg, 260.3 µmol) and DIPEA (3.4 g, 26.0 mmol, 4.5 mL) were dissolved in ACN (15 mL). The mixture was degassed and purged with N₂ three times, then stirred at 100 °C under N₂ for 16 h. The mixture was concentrated to afford a crude product. The crude product was purified by silica gel column chromatography to afford 4-(3-((tertbutyldimethylsilyl)oxy)prop-1-yn-1-yl)-2-(2,6-dioxopiperidin-3-yl)isoindoline-1,3-dione (680 mg, 1.6 mmol, 61.2% yield) as a yellow solid.

¹H NMR (400MHz, DMSO-*d*₆) δ 11.16 (s, 1H), 7.95 - 7.90 (m, 1H), 7.89 - 7.83 (m, 2H), 5.15 (dd, *J= 5.3,* 12.8 Hz, 1H), 4.64 (s, 2H), 2.95 - 2.83 (m, 1H), 2.64 - 2.53 (m, 2H), 2.10 - 2.01 (m, 1H), 0.90 (s, 9H), 0.15 (s, 6H).

To a solution of 4-(3-((tert-butyldimethylsilyl)oxy)prop-1-yn-1-yl)-2-(2,6-dioxopiperidin-3-yl)isoindoline-1,3-dione (788 mg, 1.9 mmol) in EtOAc (50 mL) was added PtO₂ (419.5 mg, 1.9 mmol) under N₂. The mixture was stirred at 20 °C under H₂ (15 psi) for 16 h, filtered and concentrated to afford 4-(3-((tert-butyldimethylsilyl)oxy)propyl)-2-(2,6-dioxopiperidin-3-yl)isoindoline-1,3-dione (700 mg, crude) as a yellow solid, which was used directly in the next step.

¹H NMR (400MHz, DMSO-d6) δ 11.13 (s, 1H), 7.80 - 7.73 (m, 2H), 7.71 - 7.65 (m, 1H), 5.13 (dd, *J=* 5.3, 12.9 Hz, 1H), 3.62 (t, *J =* 6.2 Hz, 2H), 3.11 - 3.04 (m, 2H), 2.95 - 2.84 (m, 1H), 2.64 - 2.52 (m, 2H), 2.09 - 2.00 (m, 1H), 1.85 - 1.77 (m, 2H), 0.85 (s, 9H), 0.02 (s, 6H)

To a solution of 4-(3-((tert-butyldimethylsilyl)oxy)propyl)-2-(2,6-dioxopiperidin-3-yl)isoindoline-1,3-dione (700 mg, 1.6 mmol) in THF (5 mL) was added TBAF (1 M, 3.3 mL). The mixture was stirred at 20 °C for 16 h. The mixture was extracted with EA (20 mL), washed with NaCl (10 mL × 2), dried, filtered and concentrated to afford a crude product. The crude product was purified by silica gel column chromatography to afford 2-(2,6-dioxopiperidin-3-yl)-4-(3-hydroxypropyl)isoindoline-1,3-dione (250 mg, 790.4 µmol, 48.6% yield) as a pale yellow solid.

¹H NMR (400MHz, DMSO-d6) δ 11.13 (s, 1H), 7.79 - 7.72 (m, 2H), 7.72 - 7.67 (m, 1H), 5.13 (dd, *J=* 5.1, 12.7 Hz, 1H), 4.54 (t, *J =* 5.0 Hz, 1H), 3.47 - 3.40 (m, 2H), 3.05 (t, *J= 7.6* Hz, 2H), 2.95 - 2.83 (m, 1H), 2.64 - 2.53 (m, 2H), 2.09 - 2.01 (m, 1H), 1.80 - 1.71 (m, 2H)

To a solution of 2-(2,6-dioxopiperidin-3-yl)-4-(3-hydroxypropyl)isoindoline-1,3-dione (130 mg, 411.0 µmol) in DCM (4 mL) were added TEA (124.8 mg, 1.2 mmol, 171.6 µL) and TosCl (117.5 mg, 616.5 µmol). The mixture was stirred at 20 °C for 16 h. The mixture was concentrated to give a crude product. The crude product was purified by silica gel column chromatography to afford 3-(2-(2,6-dioxopiperidin-3-yl)-1,3-dioxoisoindolin-4-yl)propyl 4-methylbenzenesulfonate (170 mg, 361.3 µmol, 87.9% yield) as a colorless oil.

¹H NMR (400MHz, DMSO-*d*₆) δ 11.14 (s, 1H), 7.79 - 7.68 (m, 4H), 7.56 (d, *J* = 7.1 Hz, 1H), 7.46 (d, *J=* 8.1 Hz, 2H), 5.13 (dd, *J=* 5.4, 12.7 Hz, 1H), 4.03 (t, *J=* 6.1 Hz, 2H), 3.01 (t, *J* = 7.5 Hz, 2H), 2.94 - 2.84 (m, 1H), 2.65 - 2.52 (m, 2H), 2.41 (s, 3H), 2.05 - 1.98 (m, 1H), 1.97 - 1.89 (m, 2H)

To a solution of 8-(7-(difluoromethyl)-6-(1-methyl-1H-pyrazol-4-yl)-3,4-dihydroquinolin-1(2H)-yl)-N-methyl-6-(l-(piperidin-4-ylmethyl)piperidin-4-yl)-3,4-dihydroisoquinoline-2(1H)-carboxamide (120 mg, 189.9 µmol) in ACN (5 mL) were added DIPEA (122.7 mg, 949.7 µmol, 165.4 µL), NaI (28.5 mg, 189.9 µmol) and 3-(2,6-dioxopiperidin-3-yl)-1,3-dioxoisoindolin-4-yl)propyl 4-methylbenzenesulfonate (80.4 mg, 170.9 µmol). The mixture was stirred at 80 °C for 16 h. The mixture was extracted with EA (20 mL), washed with NaCl (10 mL × 2), dried, filtered and concentrated to afford a crude product. The crude product was purified by prep-HPLC (FA) to afford 8-(7-(difluoromethyl)-6-(1-methyl-1H-pyrazol-4-yl)-3,4-dihydroquinolin-1(2H)-yl)-6-((1-(3-(2-(2,6-dioxopiperidin-3-yl)-1,3-dioxoisoindolin-4-yl)propyl)piperidin-4-yl)methyl)piperidin-4-yl)-N-methyl-3,4-dihydroisoquinoline-2(1H)-carboxamide (69.8 mg, 75.1 µmol, 39.5% yield) as a white solid. MS ES⁺: 930.5.

¹H NMR (400 MHz, DMSO- *d*₆) 11.12 (s, 1H), 8.22 (s, 1H), 7.79 - 7.69 (m, 4H), 7.46 (s, 1H), 7.09 - 6.97 (m, 3H), 6.84 - 6.54 (m, 1H), 6.51 - 6.44 (m, 1H), 6.18 (s, 1H), 5.16 - 5.09 (m, 1H), 4.41 (d, J = 16.9 Hz, 1H), 4.11 (d, J = 16.8 Hz, 1H), 3.85 (s, 3H), 3.53 - 3.50 (m, 1H), 3.44 - 3.41 (m, 1H), 3.03 (t, J = 7.5 Hz, 2H), 2.94 - 2.78 (m, 10H), 2.69 - 2.56 (m, 2H), 2.54 (d, J = 4.3 Hz, 3H), 2.46 - 2.39 (m, 2H), 2.37 - 2.31 (m, 2H), 2.11 (d, J = 6.8 Hz, 2H), 2.08 - 2.01 (m, 3H), 1.96 - 1.86 (m, 4H), 1.81 - 1.75 (m, 2H), 1.73 - 1.56 (m, 7H), 1.11 - 1.01 (m, 2H)

### Example 2-21 (Synthesis of 2023)

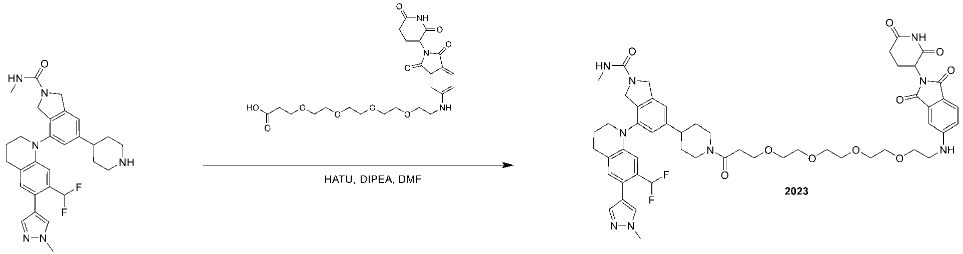

In a 25mL round-bottom flask, 3-[2-[2-[2-[2-[[2-(2,6-dioxo-3-piperidinyl)-1,3-dioxoisoindolin-5-yl]amino]ethoxy]ethoxy]ethoxy]propanoic acid (15.0 mg, 28.8 µmol) was dissolved in DMF (4 mL), and DIPEA (11.1 mg, 86.4 µmol), HATU (16.4 mg, 43.2 µmol) and 4-[7-(difluoromethyl)-6-(1-methylpyrazol-4-yl)-3,4-dihydro-2H-quinolin-1-yl]-N-methyl-6-(4-piperidinyl)isoindoline-2-carboxamide (15.0 mg, 28.8 µmol) were added. The mixture was stirred at 15 °C for 16 h. The reaction was quenched with H₂O (5 mL), extracted with 10 mL EtOAc (10 mL × 2). The combined organic layers were washed with 5 mL brine (5 mL × 1), dried over Na₂SO₄, filtered and concentrated under reduced pressure. The residue was purified by prep-HPLC (FA) to afford 4-[7-(difluoromethyl)-6-(1-methylpyrazol-4-yl)-3,4-dihydro-2H-quinolin-1-yl]-6-[1-[3-[2-[2-[2-[2-[[2-(2,6-dioxo-3-piperidinyl)-1,3-dioxoisoindolin-5-yl]amino]ethoxy]ethoxy]ethoxy]propanoyl]-4-piperidinyl]-N-methylisoindoline-2-carboxamide (6 mg, 5.9 µmol, 20.3% yield, 100% purity) as a pale yellow solid. MS ES⁺: 1024.5.

¹H NMR (400 MHz, DMSO- d₆) 11.08 (s, 1 H) 7.74 (s, 1 H) 7.46 - 7.62 (m, 2 H) 7.06 - 7.28 (m, 4 H) 7.00 (s, 1 H) 6.89 (m, 2 H) 6.24 - 6.39 (m, 2 H) 5.03 (dd, *J =* 12.78, 5.20 Hz, 1 H) 4.45 - 4.63 (m, 2 H) 4.28 (s, 1 H) 3.98 (m, 1 H) 3.86 (s, 3 H) 3.43 - 3.67 (m, 22 H) 3.06 (m, 1 H) 2.73 - 2.93 (m, 4 H) 2.58 (m, 6 H) 2.51 - 2.54 (m, 2 H) 1.93 - 2.06 (m, 3 H) 1.79 (m, 2 H) 1.36 - 1.68 (m, 2 H)

### Example 2-22 (Synthesis of 2024)

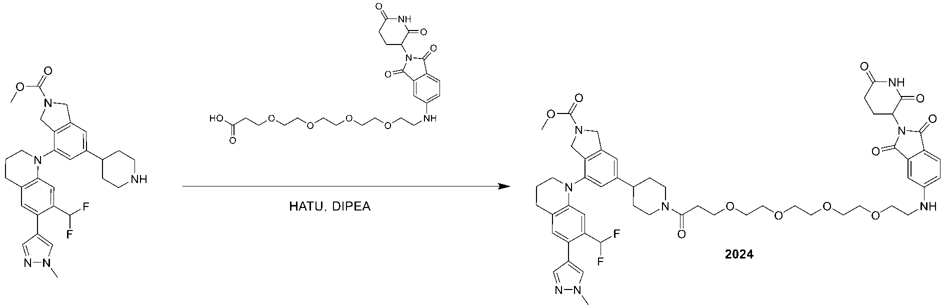

Methyl 4-[7-(difluoromethyl)-6-(1-methylpyrazol-4-yl)-3,4-dihydro-2H-quinolin-1-yl]-6-(4-piperidinyl)isoindoline-2-carboxylate (20 mg, 38.3 µmol, 1eq) was dissolved in DMF (1 mL), and DIPEA (4.96 mg, 38.3 µmol), HATU (14.6 mg, 38.3 µmol) and 3-[2-[2-[2-[2-[[2-(2,6-dioxo-3-piperidinyl)-1,3-dioxoisoindolin-5-yl]amino]ethoxy]ethoxy]ethoxy]propanoic acid (20 mg, 38.3 µmol) were added. The mixture was stirred at 20 °C for 16 h. The reaction mixture was filtered and concentrated under reduced pressure. The residue was purified by prep-HPLC (FA) to afford methyl 4-[7-(difluoromethyl)-6-(1-methylpyrazol-4-yl)-3,4-dihydro-2H-quinolin-1-yl]-6-[1-[3-[2-[2-[2-[2-[[2-(2,6-dioxo-3-piperidinyl)-1,3-dioxoisoindolin-5-yl]amino]ethoxy]ethoxy]ethoxy]propanoyl]-4-piperidinyl]isoindoline-2-carboxylate (8.68 mg, 8.47 µmol, 22.1% yield) as a pale yellow solid. MS ES⁺: 1025.3

¹H NMR (400 MHz, DMSO- d₆) 11.06 (s, 1 H) 8.42 (s, 1 H) 7.75 (s, 1 H) 7.45 - 7.60 (m, 2 H) 7.09 - 7.21 (m, 4 H) 7.00 (s, 1 H) 6.58 - 6.93 (m, 2 H) 6.38 (d, J=4.29 Hz, 1 H) 5.03 (m, 1 H) 4.66 (s, 2 H) 4.53 (m, 1 H) 4.32 (m, 2 H) 3.99 (m, 1 H) 3.86 (s, 3 H) 3.44 - 3.71 (m, 21 H) 3.07 (m, 2 H) 2.80 - 2.94 (m, 4 H) 2.52 - 2.63 (m, 5 H) 2.01 (m, 3 H) 1.79 (m, 2 H) 1.33 - 1.68 (m, 3 H)

### Example 2-23 (Synthesis of 2025)

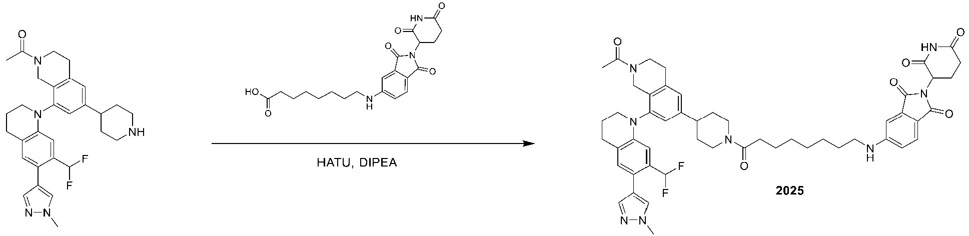

1-[8-[7-(Difluoromethyl)-6-(1-methylpyrazol-4-yl)-3,4-dihydro-2H-quinolin-1-yl]-6-(4-piperidinyl)-3,4-dihydro-1H-isoquinolin-2-yl]ethanone (20 mg, 30.4 µmol, 79% purity) was dissolved in DMF (1 mL), and DIPEA (3.93 mg, 30.41 µmol), HATU (11.6 mg, 30.4 µmol) and 8-[[2-(2,6-dioxo-3-piperidinyl)-1,3-dioxoisoindolin-5-yl]amino]octanoic acid (12.6 mg, 30.4 µmol) were added. The mixture was stirred at 20 °C for 16 h. The reaction mixture was filtered and concentrated under reduced pressure. The residue was purified by prep-HPLC (FA) to afford 5-[[8-[4-[2-acetyl-8-[7-(difluoromethyl)-6-(1-methylpyrazol-4-yl)-3,4-dihydro-2H-quinolin-1-yl]-3,4-dihydro-1H-isoquinolin-6-yl]-1-piperidinyl]-8-oxooctyl]amino]-2-(2,6-dioxo-3-piperidinyl)isoindoline-1,3-dione (8 mg, 8.72 µmol, 28.7% yield, 100% purity) as a yellow solid. MS ES⁺: 939.3 (M+23).

¹H NMR (400 MHz, DMSO- *d*₆) 11.06 (s, 1 H) 7.71 (s, 1 H) 7.55 (d, J=8.34 Hz, 1 H) 7.47 (s, 1 H) 7.07 - 7.14 (m, 3 H) 7.04 (br s, 1 H) 6.93 (s, 1 H) 6.67 - 6.87 (m, 2 H) 6.12 - 6.23 (m, 1 H) 5.02 (dd, J=12.87, 5.36 Hz, 1 H) 4.43 - 4.60 (m, 2 H) 4.21 - 4.34 (m, 1 H) 3.91 - 4.03 (m, 1 H) 3.85 (s, 3 H) 3.51 - 3.73 (m, 3 H) 3.14 (m, 3 H) 2.71 - 2.95 (m, 7 H) 2.54 - 2.61 (m, 2 H) 2.31 (m, 2 H) 1.94 - 2.08 (m, 6 H) 1.77 (m, 2 H) 1.46 - 1.62 (m, 5 H) 1.18 - 1.41 (m, 8 H)

### Example 2-24 (Synthesis of 2026)

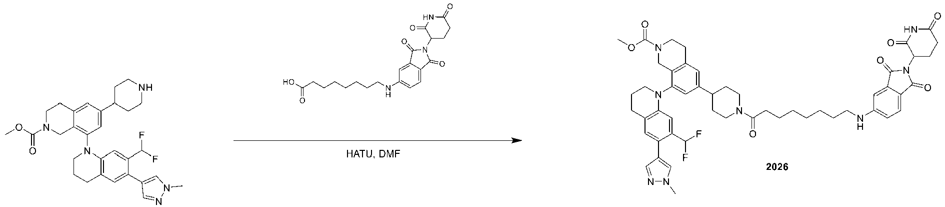

8-[[2-(2,6-Dioxo-3-piperidinyl)-1,3-dioxoisoindolin-5-yl]amino]octanoic acid (5.4 mg, 13.0 µmol) was dissolved in DMF (2 mL), and DIPEA (5.1 mg, 39.2 µmol), methyl 8-[7-(difluoromethyl)-6-(1-methylpyrazol-4-yl)-3,4-dihydro-2H-quinolin-1-yl]-6-(4-piperidinyl)-3,4-dihydro-1H-isoquinoline-2-carboxylate (7.0 mg, 13.0 µmol) and HATU (7.5 mg, 19.6 µmol) were added. The mixture was stirred at 15 °C for 16 h. The reaction was quenched with water (5 mL) at 20°C, and the resulting mixture was extracted with EtOAc (3×10 mL). The combined organic layers were washed with brine (20 mL), dried over anhydrous Na₂SO₄, filtered and concentrated under reduced pressure. The reaction mixture was purified by prep-HPLC (FA) to afford methyl 8-[7-(difluoromethyl)-6-(1-methylpyrazol-4-yl)-3,4-dihydro-2H-quinolin-1-yl]-6-[1-[8-[[2-(2,6-dioxo-3-piperidinyl)-1,3-dioxoisoindolin-5-yl]amino]octanoyl]-4-piperidinyl]-3,4-dihydro-1H-isoquinoline-2-carboxylate (9 mg, 9.6 µmol, 73.8% yield, 100% purity) as a pale yellow solid. MS ES⁺: 933.4.

¹H NMR (400 MHz, DMSO- *d*₆) 11.08 (s, 1 H) 8.41 (s, 1 H) 7.73 (s, 1 H) 7.56 (d, *J=* 8.31 Hz, 1 H) 7.48 (s, 1 H) 7.00 - 7.17 (m, 4 H) 6.55 - 6.96 (m, 3 H) 6.17 (s, 1 H) 5.03 (dd, J = 12.90, 5.44 Hz, 1 H) 4.39 - 4.64 (m, 1 H) 4.38 - 4.64 (m, 1 H) 4.25 (s, 1 H) 3.95 (m, 1 H) 3.86 (m, 3 H) 3.46 - 3.64 (m, 2 H) 3.00 - 3.17 (m, 4 H) 2.66 - 2.97 (m, 8 H) 2.54 - 2.61 (m, 2 H) 2.31 (m, 2 H) 1.93 - 2.09 (m, 4 H) 1.76 (m, 2 H) 1.42 - 1.61 (m, 5 H) 1.19 - 1.41 (m, 9H)

### Example 2-25 (Synthesis of 2027)

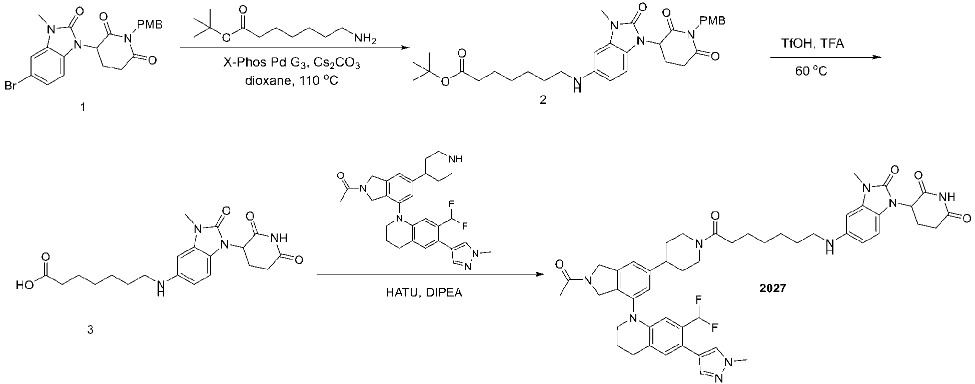

To a solution of 3-(5-bromo-3-methyl-2-oxo-1,3-benzodiazol-1-yl)-1-(2-methoxy-5-methylphenyl)piperidine-2,6-dione (1.03 g, 2.2 mmol) and tert-butyl 7-aminoheptanoate (0.53 g, 2.6 mmol) in 1,4-dioxane (70 mL) were added X-Phos Pd G3 (0.19 g, 0.2 mmol) and Cs₂CO₃ (2.15 g, 6.6 mmol). The mixture was then stirred at 110 °C for 16 h under N₂ atmosphere. After filtration, the filtrate was concentrated under reduced pressure and purified by silica gel chromatography (EtOAc/DCM from 0% to 20%) to afford tert-butyl 7-({1-[1-(2-methoxy-5-methylphenyl)-2,6-dioxopiperidin-3-yl]-3-methyl-2-oxo-1,3-benzodiazol-5-yl}amino)heptanoate (0.41 g, yield 32%) as a yellow oil. LC-MS: m/z 579.3 [M+H]⁺.

tert-Butyl 7-({1-[1-(2-methoxy-5-methylphenyl)-2,6-dioxopiperidin-3-yl]-3-methyl-2-oxo-1,3-benzodiazol-5-yl}amino)heptanoate (410 mg, 0.71 mmol) was dissolved in TFA (2 mL) and TfOH (0.2 mL), and stirred at 60 °C for 6 h. The reaction mixture was concentrated under reduced pressure and purified by reverse-phase chromatography (H₂O (0.1% FA)-ACN, 20%) to afford 7-{[1-(2,6-dioxopiperidin-3-yl)-3-methyl-2-oxo-1,3-benzodiazol-5-yl]amino}heptanoic acid (120 mg, yield 42%) as a white solid. LC-MS: m/z 403.2 [M+H]⁺.

7-{[1-(2,6-Dioxopiperidin-3-yl)-3-methyl-2-oxo-1,3-benzodiazol-5-yl]amino}heptanoic acid (120 mg, 0.30 mmol) and 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethanone (151 mg, 0.30 mmol) were dissolved in DMF (5 mL), and DIEA (116 mg, 0.89 mmol) and HATU (136 mg, 0.36 mmol) were added. The mixture was then stirred at 25 °C for 1 h. Water (50 mL) was added to quench the reaction, and the mixture was extracted with EtOAc (50 mL×3). After evaporation to dryness, the residue was purified by Prep-HPLC (column: Xbridge-C18 150×19 mm, 5 µm; mobile phase: ACN-H₂O (0.1% FA)) to afford a white solid (62.8 mg, 24% yield).

¹H NMR (400 MHz, DMSO-*d₆*) δ 11.03 (s, 1H), 8.39 (s, 0.22H), 7.74 (s, 1H), 7.49 (s, 1H), 7.18-7.09 (m, 3H), 6.88 (s, 0.31 H), 6.79 (d, J = 8.4 Hz, 1H), 6.74 (s, 0.52H), 6.60 (s, 0.30H), 6.44-6.36 (m, 2H), 6.27 (d, J = 8.4 Hz, 1H), 5.31-5.20 (m, 2H), 4.84 (s, 1H), 4.68-4.50 (m, 3H), 4.28 (s, 3H), 4.01-3.92 (m, 1H), 3.86 (s, 3H), 3.58-3.50 (m, 2H), 3.24 (s, 3H), 3.12-2.95 (m, 3H), 2.94-2.76 (m, 4H), 2.70-2.55 (m, 3H), 2.36-2.29 (m, 2H), 2.05-1.93 (m, 6H), 1.86-1.74 (m, 2H), 1.62-1.28 (m, 10H). ¹⁹F NMR (400 MHz, DMSO-*d*₆) δ 108.09 LC-MS: m/z 890.4 [M+H]⁺.

### Example 2-26 (Synthesis of 2028)

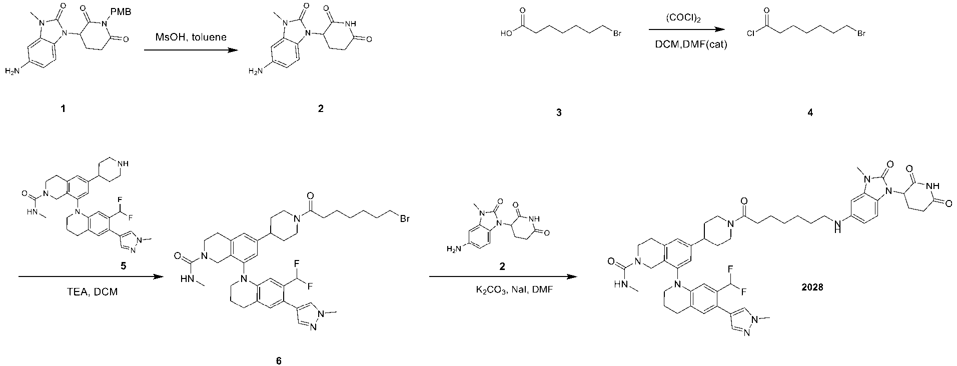

3-(5-Amino-3-methyl-2-oxo-benzimidazol-1-yl)-1-[(4-methoxyphenyl)methyl]piperidine-2,6-dione (750 mg, 1.9 mmol) and MsOH (6.4 g, 66.5 mmol) were dissolved in toluene (5 mL), and the mixture was stirred at 100 °C for 5 h under N₂ atmosphere. After cooling to room temperature, the resulting mixture was added dropwise to ice water (10 mL) at 0 °C. Saturated NaHCO₃ solution was then added to adjust the pH of the reaction mixture to 7. The aqueous layer was extracted with ethyl acetate (5×10 mL), dried over Na₂SO₄, filtered and concentrated under reduced pressure. The residue was purified by column chromatography (SiO₂, DCM/MeOH = 1/0 to 85/15) to afford 3-(5-amino-3-methyl-2-oxo-benzimidazol-1-yl)piperidine-2,6-dione (260 mg, 947 µmol, yield 49.9%) as a pale yellow solid.

¹H NMR (400 MHz, DMSO- *d₆*) δ 11.06 (s, 1 H) 6.79 (d, *J=* 8.31 Hz, 1 H) 6.43 (s, 1 H) 6.33 (br d, *J=* 8.31 Hz, 1 H) 5.37 (br s, 2 H) 5.24 (dd, *J=* 12.90, 5.07 Hz, 1 H) 3.24 (s, 3 H) 2.82 - 2.96 (m, 1 H) 2.65 - 2.72 (m, 1 H) 2.58 (s, 1 H) 2.56 - 2.56 (m, 1 H) 1.91 - 2.02 (m, 1 H).

To a mixture of 7-bromoheptanoic acid (0.5 g, 2.4 mmol) in DCM (20 mL) were added oxalyl dichloride (607 mg, 4.8 mmol) and DMF (17.4 mg, 239 µmol), and the reaction mixture was stirred at 15 °C for 16 h under N₂ atmosphere. The reaction mixture was concentrated under reduced pressure to afford 7-bromoheptanoyl chloride (0.5 g, 2.2 mmol, yield 91.8%) as a pale yellow oil.

¹H NMR (400 MHz, DMSO- *d₆*) δ 3.55 (t, *J =* 6.66 Hz, 2 H) 2.23 *(t, J=* 7.27 Hz, 2 H) 1.82 (m, 2 H) 1.53 (m, 2 H) 1.38 - 1.46 (m, 2 H) 1.28 - 1.36 (m, 2 H).

8-[7-(Difluoromethyl)-6-(1-methylpyrazol-4-yl)-3,4-dihydro-2H-quinolin-1-yl]-N-methyl-6-(4-piperidinyl)-3,4-dihydro-1H-isoquinoline-2-carboxamide (50 mg, 93.5 µmol)was dissolved in DCM (5 mL), and TEA (36.2 mg, 358 µmol)and 7-bromoheptanoyl chloride (21.2 mg, 93.5 µmol)were added. The resulting solution was stirred at 15 °C for 4 h. The reaction mixture was concentrated under reduced pressure. The residue was purified by column chromatography (SiO₂, DCM:MeOH = 1/0 to 75/25) to afford 6-[1-(7-bromoheptanoyl)-4-piperidinyl]-8-[7-(difluoromethyl)-6-(1-methylpyrazol-4-yl)-3,4-dihydro-2H-quinolin-1-yl]-N-methyl-3,4-dihydro-1H-isoquinoline-2-carboxamide (76 mg, 92.1 µmol, 98.5% yield, 88% purity) as a pale yellow oil. MS ES⁺: 749.0 (M+23).

6-[1-(7-Bromoheptanoyl)-4-piperidinyl]-8-[7-(difluoromethyl)-6-(1-methylpyrazol-4-yl)-3,4-dihydro-2H-quinolin-1-yl]-N-methyl-3,4-dihydro-1H-isoquinoline-2-carboxamide (76.0 mg, 104 µmol), 3-(5-amino-3-methyl-2-oxo-benzimidazol-1-yl)piperidine-2,6-dione (28.7 mg, 104 µmol), K₂CO₃ (43.4 mg, 313 µmol)and NaI (15.7 mg, 104 µmol)were dissolved in DMF (4 mL), and the mixture was stirred at 90 °C for 16 h under N₂ atmosphere. The reaction mixture was quenched by addition of 5 mL H₂O at 15 °C, then diluted with 10 mL ethyl acetate and extracted with 20 mL solvent (10 mL×2). The combined organic layers were washed with 5 mL brine (5 mL×1), dried over Na₂SO₄, filtered and concentrated under reduced pressure. The residue was purified by prep-HPLC (FA) to afford 8-[7-(difluoromethyl)-6-(1-methylpyrazol-4-yl)-3,4-dihydro-2H-quinolin-1-yl]-6-[1-[7-[[1-(2,6-dioxo-3-piperidinyl)-3-methyl-2-oxo-benzimidazol-5-yl]amino]heptanoyl]-4-piperidinyl]-N-methyl-3,4-dihydro-1H-isoquinoline-2-carboxamide (7 mg, 6.34 µmol, 6.1% yield, 83.2% purity) as a pale yellow solid. MS ES⁺: 919.5.

¹H NMR (400 MHz, DMSO-*d*₆) 11.05 (s, 1 H) 8.46 (s, 1 H) 7.72 (s, 1 H) 7.47 (s, 1 H) 6.96 - 7.13 (m, 3 H) 6.64 - 6.75 (m, 1 H) 6.23 - 6.59 (m, 3 H) 6.17 (s, 1 H) 5.29 (d, *J* = 7.09 Hz, 1 H) 4.84 (s, 1 H) 4.34 - 4.59 (m, 2 H) 4.10 (d, *J=* 16.87 Hz, 1 H) 3.80 - 3.99 (m, 4 H) 3.42 - 3.69 (m, 3 H) 3.21 - 3.29 (m, 8 H) 2.70 - 3.10 (m, 10 H) 2.44 (m, 3 H) 2.01 (m, 4 H) 1.75 (m, 2 H) 1.37 - 1.59 (m, 5 H) 1.26 (m, 4 H)

### Example 2-27 (Synthesis of 2029)

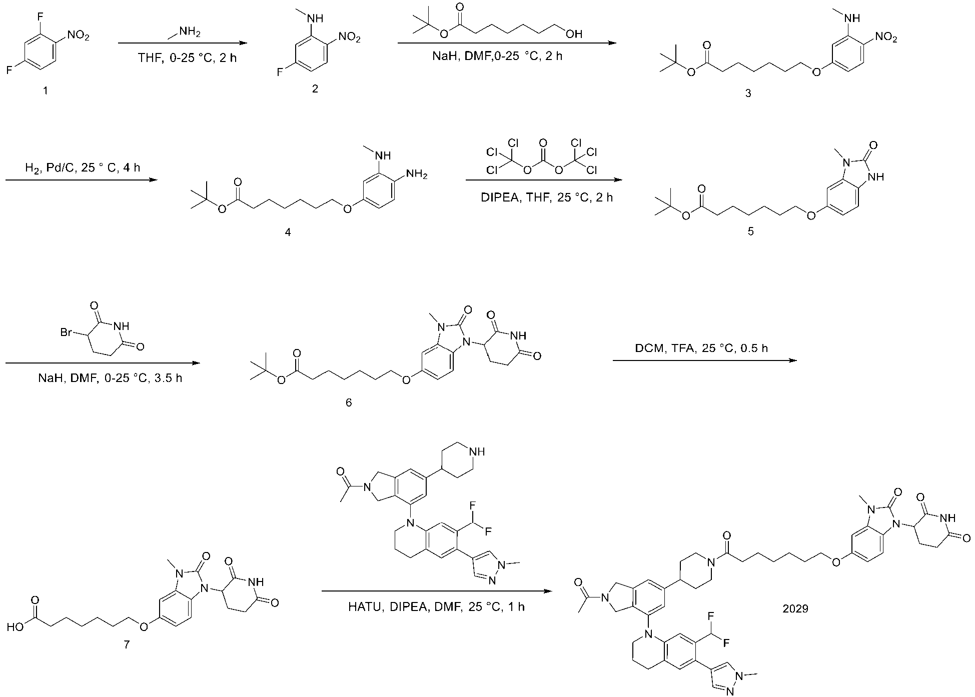

To a solution of 2,4-difluoro-1-nitrobenzene (10 g, 0.0629 mol) in THF (150 mL) was added 40% aqueous methylamine solution (3.91 g, 0.126 mol) at 0 °C. The mixture was stirred at 25 °C for 2 h. Petroleum ether (150 mL) and ethyl acetate (150 mL) were added to the reaction, which was washed with water, 2 mol/L hydrochloric acid, aqueous sodium bicarbonate solution and saturated brine, dried over sodium sulfate and rotary evaporated to afford 5-fluoro-N-methyl-2-nitroaniline (10.3 g, 91%) as a yellow solid. LC-MS: m/z 171.1 [M+H]⁺.

To a solution of tert-butyl 7-hydroxyheptanoate (500 mg, 2.4717 mmol) in DMF (10.0 mL) under nitrogen was added NaH (71.2 mg, 2.97 mmol) at 0 °C. The reaction was stirred at 0 °C for 30 min, then 5-fluoro-N-methyl-2-nitroaniline (504 mg, 2.97 mmol) was added. The reaction mixture was stirred at 25 °C for 1.5 h. The mixture was quenched with saturated NH₄Cl solution (30 mL) and extracted with EtOAc (10 mL×3). The combined organic layers were washed with brine (30 mL), dried over Na₂SO₄, filtered and concentrated. The residue was purified by silica gel chromatography, eluting with 0% to 10% MeOH/DCM over 15 min to afford tert-butyl 7-(3-(methylamino)-4-nitrophenoxy)heptanoate (610 mg, 63% yield) as a yellow solid. LC-MS: m/z 375.2 [M+Na]⁺.

A mixture of tert-butyl 7-(3-(methylamino)-4-nitrophenoxy)heptanoate (500 mg, 1.42 mmol) and Pd/C (30.2 mg, 0.284 mmol) in MeOH (20 mL) was stirred at 25 °C for 4 h under H₂ atmosphere. The mixture was filtered and concentrated to afford tert-butyl 7-(4-amino-3-(methylamino)phenoxy)heptanoate (430 mg, 84%) as a yellow oil, which was used directly in the next step. LC-MS: m/z 323.3 [M+H]⁺.

To a solution of tert-butyl 7-(4-amino-3-(methylamino)phenoxy)heptanoate (420 mg, 1.30 mmol) in THF (20.0 mL) were added DIPEA (336 mg, 2.61 mmol) and triphosgene (193 mg, 0.651 mmol) at 0 °C. The reaction mixture was stirred at 25 °C for 2 h. The reaction mixture was poured into aqueous NaHCO₃ solution (30 mL), and the aqueous layer was extracted with DCM (10 mL×3). The combined organic layers were dried over Na₂SO₄ and concentrated, and the residue was purified by silica gel chromatography, eluting with methanol in DCM from 0% to 7% over 10 min to afford tert-butyl 7-((3-methyl-2-oxo-2,3-dihydro-1H-benzoimidazol-5-yl)oxy)heptanoate (450 mg, 89%) as a yellow solid. LC-MS: m/z [M+Na]⁺ 371.2.

To a solution of tert-butyl 7-((3-methyl-2-oxo-2,3-dihydro-1H-benzo[d]imidazol-5-yl)oxy)heptanoate (350 mg, 1.00 mmol) in DMF (5.0 mL) was added NaH (241 mg, 10.0 mmol) at 0 °C under nitrogen. The reaction was stirred at 0 °C for 30 min, then 3-bromopiperidine-2,6-dione (386 mg, 2.00 mmol) was added. The reaction mixture was stirred at 25 °C for 3 h. The mixture was quenched with saturated NH₄Cl solution (30 mL) and extracted with EtOAc (10 mL×3). The combined organic layers were washed with brine (30 mL), dried over Na₂SO₄, filtered and concentrated. The residue was purified by silica gel chromatography, eluting with EtOAc/petroleum ether (EtOAc from 0% to 50% over 15 min) to afford tert-butyl 7-((1-(2,6-dioxopiperidin-3-yl)-3-methyl-2-oxo-2,3-dihydro-1H-benzo[d]imidazol-5-yl)oxy)heptanoate (400 mg, yield 47%) as a colorless oil. LC-MS: m/z 482.2 [M+Na]⁺.

tert-Butyl 7-((1-(2,6-dioxopiperidin-3-yl)-3-methyl-2-oxo-2,3-dihydro-1H-benzo[d]imidazol-5-yl)oxy)heptanoate (400 mg, 0.870 mmol) was dissolved in DCM (6.0 mL) and TFA (2.0 mL), and stirred at 25 °C for 0.5 h. The reaction was concentrated under reduced pressure and purified by reverse-phase chromatography (H₂O (0.1% FA)-ACN, 20%) to afford 7-((1-(2,6-dioxopiperidin-3-yl)-3-methyl-2-oxo-2,3-dihydro-1H-benzo[d]imidazol-5-yl)oxy)heptanoic acid (253 mg, yield 64%) as a yellow solid. LC-MS: m/z 404.2 [M+H]⁺.

To a solution of 7-((1-(2,6-dioxopiperidin-3-yl)-3-methyl-2-oxo-2,3-dihydro-1H-benzo[d]imidazol-5-yl)oxy)heptanoic acid (100 mg, 0.248 mmol) in DMF (5.0 mL) were added 1-(4-(7-(difluoromethyl)-6-(1-methyl-1H-pyrazol-4-yl)-3,4-dihydroquinolin-1(2H)-yl)-6-(piperidin-4-yl)isoindolin-2-yl)ethan-1-one (163 mg, 0.322 mmol), HATU (189 mg, 0.496 mmol) and DIPEA (96.1 mg, 0.744 mmol) at 25 °C. The reactant was stirred at 25 °C for 1 h. The reactant was diluted with water (30 mL) and extracted with EtOAc (10 mL×3). The combined organic layers were dried over Na₂SO₄ and concentrated, and the residue was purified by prep-HPLC, eluting with aqueous acetonitrile (0.1% TFA) from 32% to 42% over 9.0 min (instrument: GILSON Prep LC with UV detector; column: Xbridge 5u C18 150×30 mm; flow rate: 50 mL/min) to afford 3-(5-((7-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)-7-oxoheptyl)oxy)-3-methyl-2-oxo-2,3-dihydro-1H-benzo[d]imidazol-1-yl)piperidine-2,6-dione (1.4 mg, 0.61%) as a white solid.

¹H NMR (400 MHz, DMSO-*d*₆) δ 11.08 (s, 1H), 7.74 (s, 1H), 7.49 (s, 1H), 7.17 - 7.10 (m, 3H), 6.97 (d, *J =* 8.4 Hz, 1H), 6.85 (s, 1H), 6.74 (t, *J =* 55.2, 1H), 6.62 - 6.58 (m, 1H), 6.40 (d, *J* = 28.4 Hz, 1H), 5.35 - 5.28 (m, 1H), 4.84 (s, 1H), 4.64 (s, 1H), 4.57 - 4.50 (m, 1H), 4.27 (s, 1H), 3.97 - 3.93(m, 2H), 3.85 (s, 3H), 3.57 - 3.50 (m, 2H), 3.30 (s, 3H), 3.15 - 3.0 (m, 2H), 2.91 - 2.83 (m, 3H), 2.72 - 2.57 (m, 5H), 2.35 - 2.31 (m, 2H), 2.04 - 1.96 (m, 6H), 1.83 - 1.67 (m, 4H), 1.55 - 1.50 (m, 2H), 1.45 - 1.40 (m, 2H), 1.38 - 1.33 (m, 2H), 1.25 - 1.21 (m, 2H). ¹⁹F NMR (400MHz, DMSO-*d*₆) δ -108.13. LC-MS: m/z 891.4 [M+H]⁺.

### Example 2-28 (Synthesis of 2030)

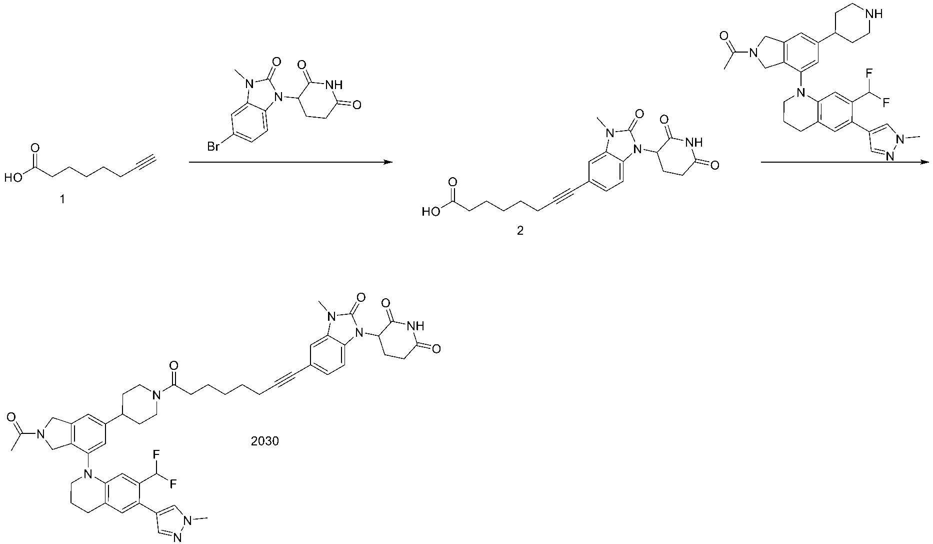

Oct-7-ynoic acid (165 mg, 1.20 mmol), 3-(5-bromo-3-methyl-2-oxo-1,3-benzodiazol-1-yl)piperidine-2,6-dione (100 mg, 0.29 mmol), CuI (5 mg, 0.03 mmol), TEA (89 mg, 0.88 mmol) and Pd(PPh₃)₄ (34 mg, 0.03 mmol) were dissolved in DMSO (2 mL) at 25 °C under N₂. The mixture was heated to 90 °C and stirred at this temperature for 16 h. The reaction mixture was cooled to 25 °C, diluted with water (10 mL) and extracted with EtOAc (20 mL×2). The combined organic phases were dried over Na₂SO₄ and filtered. The filtrate was concentrated under reduced pressure. The residue was purified by silica gel chromatography, eluting with 0% to 80% EtOAc/petroleum ether over 15 min to afford 8-[1-(2,6-dioxopiperidin-3-yl)-3-methyl-2-oxo-1,3-benzodiazol-5-yl]oct-7-ynoic acid (80 mg, yield 57%) as a yellow solid. LC-MS: (ESI) m/z [M+H]⁺ 398.1.

To a solution of 8-[1-(2,6-dioxopiperidin-3-yl)-3-methyl-2-oxo-1,3-benzodiazol-5-yl]oct-7-ynoic acid (50 mg, 0.12 mmol), HATU (57 mg, 0.15 mmol) and TEA (38 mg, 0.37 mmol) in DMF (2 mL) was added 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethenone (50 mg, 0.11 mmol) at 0 °C. After addition, the mixture was stirred at 25 °C for 1 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by prep-HPLC (ACN from 10% to 80% over 10 min) to afford 3-{5-[8-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)-8-oxo-1-yn-1-yl]-3-methyl-2-oxo-1,3-benzodiazol-1-yl}piperidine-2,6-dione (4 mg, yield 4%) as a white solid.

¹H NMR (400 MHz, DMSO-*d*₆) δ 11.11 (s, 1H), 7.74 (s, 1H), 7.50 (s, 1H), 7.25 - 7.05 (m, 6H), 6.88 - 6.60 (m, 1H), 6.48 - 6.37 (m, 1H), 5.40 - 5.35 (m, 1H), 4.80 (s, 1H), 4.70 - 4.50 (m, 3H), 4.28 (s, 1H), 4.02 - 3.98 (m, 1H), 3.86 (s, 3H), 3.60 - 3.50 (m, 2H), 3.30 (s, 3H), 3.15 - 3.01 (m, 1H), 2.98 - 2.75 (m, 4H), 2.74 - 2.33 (m, 8H), 2.10 - 1.98 (m, 5H), 1.81 - 1.44 (m, 10H). ¹⁹F NMR (400MHz, DMSO-*d*₆) δ ppm -108.08. LC-MS: (ESI) m/z [M+H]⁺ 858.4.

### Example 2-29 (Synthesis of 2031)

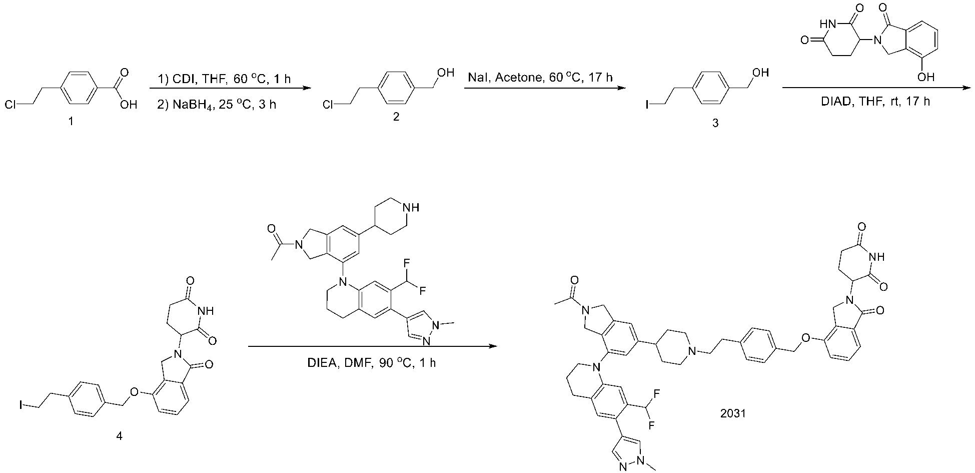

To a stirred mixture of 4-(2-chloroethyl)benzoic acid (3.0 g, 16.2 mmol) in THF (40 mL) was added CDI (5.25 g, 32.4 mmol). The mixture was stirred at 60 °C until gas evolution ceased. After cooling to room temperature, sodium borohydride (1.23 g, 32.4 mmol) was slowly added to the mixture, and the mixture was stirred at 25 °C for 3 h. The solution was acidified with 1 N HCl and extracted twice with EtOAc (15 mL×2). The organic phase was separated and dried over MgSO₄. After evaporation of the solvent, the residue was purified by silica gel column chromatography using PE/EtOAc (7:3) as eluent to afford (4-(2-chloroethyl)phenyl)methanol (1.5 g, yield 49%) as a yellow oil.

¹H NMR (400 MHz, DMSO-*d*₆) δ 7.26-7.21 (m, 4H), 5.12 (t, J= 5.6 Hz, 1H), 4.46 *(d, J=* 5.6 Hz, 2H), 3.82 (t, *J=* 7.2 Hz, 2H), 3.00 (t, *J=* 7.2 Hz, 2H).

A mixture of (4-(2-chloroethyl)phenyl)methanol (1.0 g, 5.90 mmol) and NaI (1.77 g, 11.8 mmol) in acetone (20 mL) was heated at 60 °C and stirred for 17 h. The mixture was concentrated under reduced pressure, and the residue was purified by silica gel chromatography (PE/EtOAc = 6:1) to afford (4-(2-iodoethyl)phenyl)methanol (0.8 g, yield 36%) as a white solid.

¹H NMR (400 MHz, CDCl₃) δ 7.33 (d, *J=* 8.4 Hz, 2H), 7.19 (d, *J =* 8.0 Hz, 2H), 4.68 (s, 2H), 3.35 (t, *J=* 7.2 Hz, 2H), 3.18 (t, *J =* 7.2 Hz, 2H).

To a solution of (4-(2-iodoethyl)phenyl)methanol (150 mg, 0.572 mmol), 3-(4-hydroxy-1-oxoisoindolin-2-yl)piperidine-2,6-dione (149 mg, 0.572 mmol) and PPh₃ (195 mg, 0.744 mmol) in THF (8 mL) was added DIAD (139 mg, 0.687 mmol) at 25 °C. After addition, the mixture was stirred at this temperature for 17 h. The mixture was concentrated under reduced pressure, and the residue was purified by prep-HPLC to afford 3-(4-((4-(2-iodoethyl)benzyl)oxy)-1-oxoisoindolin-2-yl)piperidine-2,6-dione (23 mg, yield 8%) as a yellow oil. LC-MS: m/z 505.0 [M+H]⁺.

1-(4-(7-(Difluoromethyl)-6-(1-methyl-1H-pyrazol-4-yl)-3,4-dihydroquinolin-1(2H)-yl)-6-(piperidin-4-yl)isoindol-2-yl)ethan-1-one (23.0 mg, 0.0456 mmol), 3-(4-(4-(2-iodoethyl)benzyl)oxy)-1-oxoisoindol-2-yl)piperidine-2,6-dione (23 mg, 0.0456 mmol) and DIEA (29.5 mg, 0.228 mmol) were dissolved in DMF (0.5 mL) and reacted at 100 °C under microwave for 1 h. After cooling to 25 °C, the mixture was purified by prep-HPLC to afford 3-(4-((4-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)benzyl)oxy)-1-oxoisoindolin-2-yl)piperidine-2,6-dione (1.5 mg, yield 4%) as a white solid.

¹H NMR (400 MHz, DMSO-*d*₆) δ 10.98 (s, 1H), 7.75 (s, 1H), 7.49-7.40 (m, 4H), 7.33-7.27 (m, 4H), 7.18-7.10 (m, 3H), 6.88-6.61 (m, 1H), 6.45-6.38 (m, 1H), 5.21 (s, 2H), 5.11 (dd, J = 12.8, 4.8 Hz, 1H), 4.86 (s, 1H), 4.65 (s, 1H), 4.43-4.38 (m, 1H), 4.26-4.22 (m, 1H), 3.86 (s, 3H), 3.56-3.53 (m, 2H), 3.16 (m, 2H), 2.94-2.81 (m, 5H), 2.67-2.50 (m, 6H), 2.43-2.33 (m, 3H), 2.04-1.98 (m, 6H), 1.83-1.72 (m, 4H).¹⁹F NMR (400 MHz, DMSO-*d*₆) δ 108.11. LC-MS: m/z 882.4 [M+H]⁺.

### Example 2-30 (Synthesis of 2032)

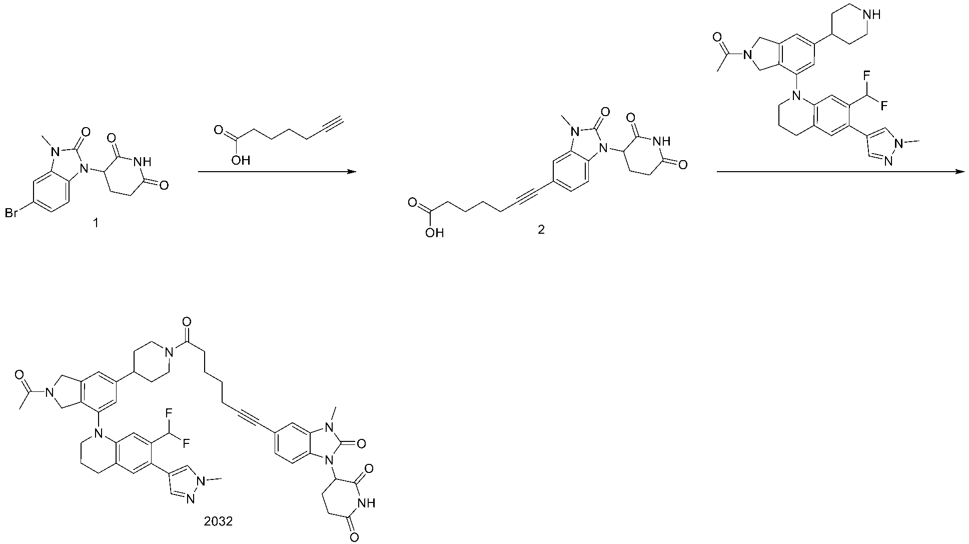

Hept-6-ynoic acid (111 mg, 0.88 mmol), 3-(5-bromo-3-methyl-2-oxo-1,3-benzodiazol-1-yl)piperidine-2,6-dione (100 mg, 0.29 mmol), Pd(PPh₃)₂Cl₂ (20 mg, 0.03 mmol), CuI (5 mg, 0.03 mmol) and TEA (89 mg, 0.88 mmol) were dissolved in DMSO (3 mL) at 25 °C under N₂. The mixture was heated to 90 °C and stirred at this temperature for 16 h. The reaction mixture was cooled to 25 °C, diluted with water (10 mL) and extracted with EtOAc (20 mL×2). The combined organic phases were dried over Na₂SO₄ and filtered. The filtrate was concentrated under reduced pressure. The residue was purified by silica gel chromatography, eluting with 0% to 100% EtOAc in petroleum ether over 15 min to afford 7-[1-(2,6-dioxopiperidin-3-yl)-3-methyl-2-oxo-1,3-benzodiazol-5-yl]hept-6-ynoic acid (80 mg, 70% yield) as a yellow solid. LC-MS: (ESI) m/z [M+H]⁺ 384.1.

To a solution of 7-[1-(2,6-dioxopiperidin-3-yl)-3-methyl-2-oxo-1,3-benzodiazol-5-yl]hept-6-ynoic acid (50 mg, 0.13 mmol), TEA (39 mg, 0.39 mmol) and HATU (59 mg, 0.15 mmol) in DMF (1 mL) was added 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethanone (52 mg, 0.11 mmol) at 0 °C. After addition, the mixture was stirred at 25 °C for 1 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by prep-HPLC (ACN from 10% to 70% over 10 min) to afford 3-{5-[7-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)-7-oxohept-1-yn-1-yl]-3-methyl-2-oxo-1,3-benzodiazol-1-yl}piperidine-2,6-dione (4 mg, yield 4%) as a white solid.

¹H NMR (400 MHz, DMSO-*d*₆) δ 11.11 (s, 1H), 7.74 (s, 1H), 7.50 (s, 1H), 7.13- 7.03 (m, 6H), 6.88 - 6.60 (m, 1H), 6.48 - 6.37 (m, 1H), 5.40 - 5.35 (m, 1H), 4.83 (s, 1H), 4.63 - 4.50 (m, 3H), 4.28 (s, 1H), 4.02 - 3.98 (m, 1H), 3.86 (s, 3H), 3.60 - 3.50 (m, 2H), 3.30 (s, 3H), 3.10 - 3.01 (m, 1H), 2.98 - 2.75 (m, 4H), 2.74 - 2.33 (m, 8H), 2.10 - 1.98 (m, 5H), 1.81 - 1.47 (m, 8H). ¹⁹F NMR (400MHz, DMSO-*d*₆) δ ppm -108.09. LC-MS: (ESI) m/z [M+H]⁺ 871.4.

### Example 2-31 (Synthesis of 2035)

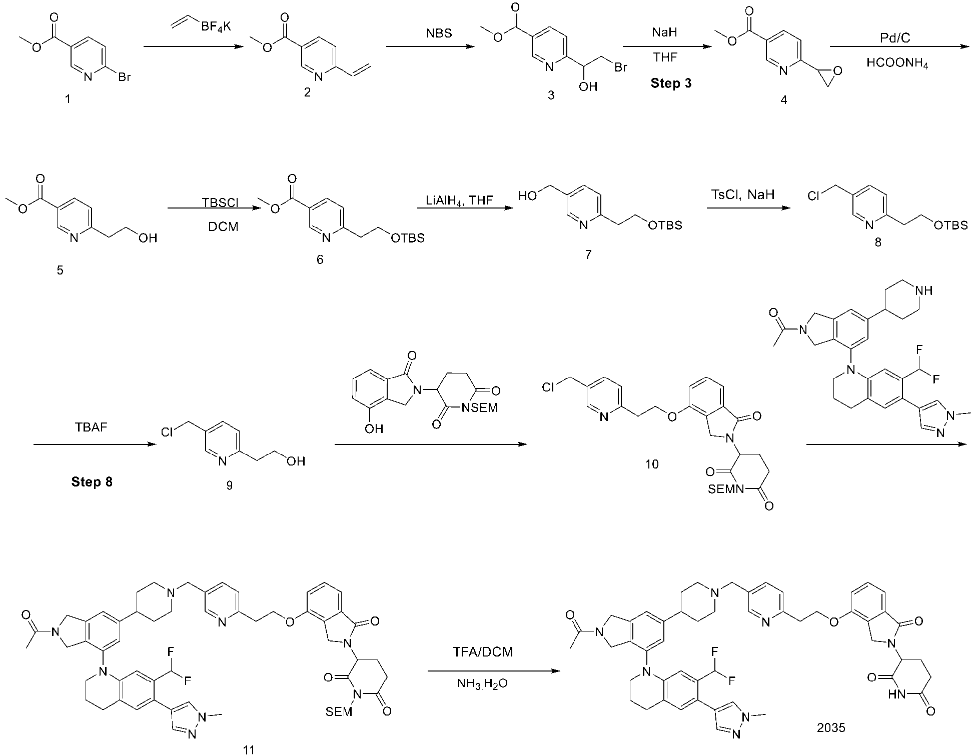

Methyl 6-bromopyridine-3-carboxylate (2.5 g, 0.012 mol), Pd(dppf)Cl₂ (0.85 g, 0.0012 mol), potassium vinyltrifluoroborate (2.6 g, 0.019 mol) and TEA (3.5 g, 0.035 mol) were dissolved in isopropanol (50 mL) at 25 °C under N₂. The mixture was heated to 100 °C and stirred at this temperature for 2 h. The reaction mixture was cooled to 25 °C, diluted with water (100 mL) and extracted with EtOAc (200 mL×2). The combined organic phases were dried over Na₂SO₄ and filtered. The filtrate was concentrated under reduced pressure. The residue was purified by silica gel chromatography, eluting with 0% to 80% EtOAc in petroleum ether over 15 min to afford methyl 6-vinylpyridine-3-carboxylate (1.8 g, yield 85%) as a yellow solid. LC-MS: (ESI) m/z [M+H]⁺ 164.1.

To a solution of methyl 6-vinylpyridine-3-carboxylate (3.7 g, 0.023 mol) in H₂O (50 mL) and t-BuOH (25 mL) was added N-bromosuccinimide (4 g, 0.023 mol) at 25 °C. After addition, the mixture was stirred at 40 °C for 1 h. The reaction mixture was diluted with water (100 mL) and extracted with EtOAc (200 mL×2). The combined organic phases were dried over Na₂SO₄ and filtered. The filtrate was concentrated under reduced pressure. The residue was purified by silica gel chromatography, eluting with 0% to 75% EtOAc in petroleum ether over 15 min to afford methyl 6-(2-bromo-1-hydroxyethyl)pyridine-3-carboxylate (2 g, yield 32%) as a yellow solid. LC-MS: (ESI) m/z [M+H]⁺ 262.0.

To a solution of methyl 6-(1-bromo-2-hydroxyethyl)pyridine-3-carboxylate (2.2 g, 85 mmol) in THF (150 mL) was added NaH (0.61 g, 250 mmol) at 25 °C. After addition, the mixture was stirred at 25 °C for 1 h. The reaction mixture was diluted with water (100 mL) and extracted with EtOAc (200 mL×2). The combined organic phases were dried over Na₂SO₄ and filtered. The filtrate was concentrated under reduced pressure. The residue was purified by silica gel chromatography, eluting with 0% to 75% EtOAc in petroleum ether over 15 min to afford methyl 6-(oxiran-2-yl)pyridine-3-carboxylate (1.3 g, yield 81%) as a yellow solid. LC-MS: (ESI) m/z [M+H]⁺ 180.1.

To a solution of methyl 6-(oxiran-2-yl)pyridine-3-carboxylate (1.2 g, 0.0067 mol) in ethanol (30 mL) were added ammonium formate (1.69 g, 0.027 mol) and Pd/C (0.71 g, 0.0067 mol) at 25 °C. After addition, the mixture was stirred at 25 °C for 1 h. The reaction mixture was diluted with water (100 mL) and extracted with EtOAc (200 mL×2). The combined organic phases were dried over Na₂SO₄ and filtered. The filtrate was concentrated under reduced pressure. The residue was purified by silica gel chromatography, eluting with 0% to 85% EtOAc in petroleum ether over 15 min to afford methyl 6-(2-hydroxyethyl)pyridine-3-carboxylate (1 g, yield 77%) as a yellow solid. LC-MS: (ESI) m/z [M+H]⁺ 182.1.

To a solution of methyl 6-(2-hydroxyethyl)pyridine-3-carboxylate (1.2 g, 0.0066 mol) in DCM (20 mL) were added imidazole (0.9 g, 0.013 mol) and TBSCl (1.49 g, 0.001 mol) at 25 °C. After addition, the mixture was stirred at 25 °C for 1 h. The reaction mixture was diluted with water (30 mL) and extracted with EtOAc (50 mL×2). The combined organic phases were dried over Na₂SO₄ and filtered. The filtrate was concentrated under reduced pressure. The residue was purified by silica gel chromatography, eluting with 0% to 55% EtOAc in petroleum ether over 15 min to afford methyl 6-(2-{[tert-butyl(methyl)silyl]methoxy}ethyl)pyridine-3-carboxylate (1.7 g, yield 83%) as a yellow solid. LC-MS: (ESI) m/z [M+H]⁺ 296.2.

To a solution of methyl 6-{2-[(tert-butyldimethylsilyl)oxy]ethyl}pyridine-3-carboxylate (300 mg, 1.01 mmol) in THF (10 mL) was added LiAlH₄ (38 mg, 1.01 mmol) at 0 °C. After addition, the mixture was stirred at 25 °C for 1 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by silica gel chromatography, eluting with 0% to 85% EtOAc in petroleum ether over 15 min to give (6-{2-[(tert-butyldimethylsilyl)oxy]ethyl}pyridin-3-yl)methanol (180 mg, yield 62%) as a yellow solid. LC-MS: (ESI) m/z [M+H]⁺ 268.2.

To a solution of (6-{2-[(tert-butyldimethylsilyl)oxy]ethyl}pyridin-3-yl)methanol (620 mg, 2.31 mmol) in DMF (10 mL) were added NaH (111 mg, 4.63 mmol) and TsCl (441 mg, 2.31 mmol) at 25 °C. After addition, the mixture was stirred at 25 °C for 1 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by silica gel chromatography, eluting with 0% to 90% EtOAc in petroleum ether over 15 min to afford 2-{2-[(tert-butyldimethylsilyl)oxy]ethyl}-5-(chloromethyl)pyridine (400 mg, yield 54%) as a yellow solid. LC-MS: (ESI) m/z [M+H]⁺ 286.2.

To a solution of 2-{2-[(tert-butyldimethylsilyl)oxy]ethyl}-5-(chloromethyl)pyridine (150 mg, 0.52 mmol) in THF (3 mL) was added TBAF (205 mg, 0.78 mmol) at 25 °C. After addition, the mixture was stirred at 25 °C for 1 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by silica gel chromatography, eluting with 0% to 90% EtOAc in petroleum ether over 15 min to afford 2-[5-(chloromethyl)pyridin-2-yl]ethanol (50 mg, yield 49%) as a yellow solid. LC-MS: (ESI) m/z [M+H]⁺ 172.1.

To a solution of 2-[5-(chloromethyl)pyridin-2-yl)ethanol (50 mg, 0.29 mmol), 3-(4-hydroxy-1-oxo-3H-isoindol-2-yl)-1-{[2-(trimethylsilyl)ethoxy]methyl}piperidine-2,6-dione (113 mg, 0.29 mmol) and PPh₃ (152 mg, 0.58 mmol) in THF (3 mL) was added DIAD (176 mg, 0.87 mmol) at 0 °C. After addition, the mixture was stirred at 25 °C for 5 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by silica gel chromatography, eluting with 0% to 90% EtOAc/petroleum ether over 15 min to afford 3-(4-{2-[5-(chloromethyl)pyridin-2-yl]ethoxy}-1-oxo-3H-isoindol-2-yl)-1-{(2-(trimethylsilyl)ethoxy)methyl)piperidine-2,6-dione (20 mg, yield 11%) as a yellow solid. LC-MS: (ESI) m/z [M+H]⁺ 544.1.

To a solution of 3-(4- {2-[5-(chloromethyl)pyridin-2-yl]ethoxy}-1-oxo-3H-isoindol-2-yl)-1-{(2-(trimethylsilyl)ethoxy)methyl)piperidine-2,6-dione (20 mg, 0.036 mmol) in ACN (5 mL) at were added 1-{4-[7-(difluoromethyl)-6-(1-methylpyrazol-4-yl)-3,4-dihydro-2H-quinolin-l-yl]-6-(piperidin-4-yl)-1,3-dihydroisoindol-2-yl}ethanone (27 mg, 0.055 mmol), K₂CO₃ (15 mg, 0.11 mmol) and KI (6 mg, 0.036 mmol) 25 °C. After addition, the mixture was stirred at 50 °C for 2 h. The reaction mixture was diluted with water (10 mL) and extracted with DCM (20 mL×2). The combined organic phases were dried over Na₂SO₄ and filtered. The filtrate was concentrated under reduced pressure. The residue was purified by silica gel chromatography, eluting with 0% to 90% EtOAc in petroleum ether over 15 min to afford 3-[4-(2-{5-[(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)methyl]pyridin-2-yl}ethoxy)-1-oxo-3H-isoindol-2-yl]-1-{[2-(trimethylsilyl)ethoxy]methyl}piperidine-2,6-dione (20 mg, yield 48%) as a yellow solid. LC-MS: (ESI) m/z [1/2M+H]⁺507.3.

To a solution of 3-[4-(2- {5-[(4-{2-acetyl-7-[7-(difluoromethyl)-6-(1-methylpyrazol-4-yl)-3,4-dihydro-2H-quinolin--yl]-1,3-dihydroisoindol-5-yl}piperidin-1-yl)methyl]pyridin-2-yl}ethoxy)-1-oxo-3H-isoindol-2-yl]-1-{[2-(trimethylsilyl)ethoxy]methyl}piperidine-2,6-dione in DCM (3 mL) was added TFA (1 mL). After addition, the mixture was stirred at 25 °C for 1 h, then aqueous ammonia (2 mL) was added. The mixture was stirred at 25 °C for 1 h. The reaction mixture was diluted with water (10 mL) and extracted with DCM (20 mL×2). The combined organic phases were dried over Na₂SO₄ and filtered. The filtrate was concentrated under reduced pressure. The residue was purified by prep-HPLC (ACN from 10% to 70% over 10 min) to afford 3-[4-(2-{5-[(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)methyl]pyridin-2-yl}ethoxy)-1-oxo-3H-isoindol-2-yl]piperidine-2,6-dione (1.5 mg, yield 8%) as a white solid.

¹H NMR (400 MHz, DMSO-*d₆*) δ 11.00 (s, 1H), 8.50 (s, 1H), 7.75 (s, 1H), 7.62 - 7.60 (m, 1H), 7.52 - 7.49 (m, 2H), 7.40 - 7.30 (m, 3H), 7.20 - 7.02 (m, 3H), 6.80 - 6.53 (m, 1H), 6.45 - 6.36 (m, 1H), 5.12 - 5.08 (m, 1H), 4.90 (s, 1H), 4.70 - 4.45 (m, 3H), 4.30 - 4.05 (m, 2H), 3.86 (s, 3H), 3.55 - 3.45 (m, 4H), 3.25 - 3.18 (m, 2H), 2.98 - 2.80 (m, 6H), 2.55 - 2.50 (m, 1H), 2.48 - 2.33 (m, 2H), 2.08 - 1.95 (m, 7H), 1.75 - 1.50 (m, 6H),. ¹⁹F NMR (400MHz, DMSO-d6) δ ppm - 108.10. LC-MS: (ESI) m/z [M+H]⁺ 883.1.

### Example 2-32 (Synthesis of 2046)

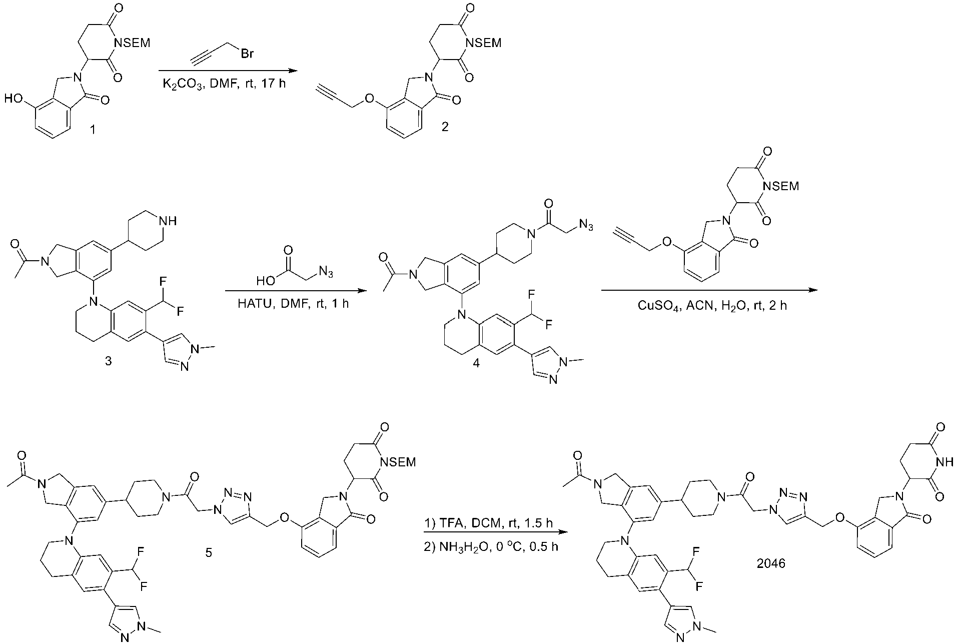

To a solution of 3-(4-hydroxy-1-oxoisoindolin-2-yl)-1-((2-(trimethylsilyl)ethoxy)methyl)piperidine-2,6-dione (200 mg, 0.512 mmol) and K₂CO₃ (106 mg, 0.768 mmol) in DMF (5.0 mL) was added 3-bromo-1-propyne (91.4 mg, 0.768 mmol). The mixture was stirred at 25 °C for 17 h. The mixture was quenched with NH₄Cl (25 mL), the aqueous layer was extracted with EtOAc (15 mL×3), and the combined organic layers were dried and concentrated. The residue was purified by silica gel chromatography (THF) to afford 3-(1-oxo-4-(prop-2-yn-1-yloxy)isoindol-2-yl)-1-((2-(trimethylsilyl)ethoxy)methyl)piperidine-2,6-dione (180 mg, yield 74%) as a yellow solid.

¹H NMR (400 MHz, DMSO-d₆) δ 7.53 (t, *J=* 8.0 Hz, 1H), 7.37 (d, *J=* 7.6 Hz, 1H), 7.32 (d, *J=* 8.0 Hz, 1H), 5.24 (dd, *J=* 13.6, 5.2 Hz, 1H), 5.05 (t, *J=* 9.6 Hz, 2H), 4.95 (d, *J=* 2.4 Hz, 2H), 4.44-4.39 (m, 1H), 4.22-4.18 (m, 1H), 3.63 (t, *J=* 2.4 Hz, 1H), 3.56-3.48 (m, 2H), 3.11-3.02 (m, 1H), 2.80-2.76 (m, 1H), 2.47-2.37 (m, 1H), 2.05-2.03 (m, 1H), 0.90-0.78 (m, 2H), 0.00 (s, 9H).

To a solution of 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (200 mg, 0.396 mmol), 2-azidoacetic acid (48.0 mg, 0.475 mmol) and DIEA (76.7 mg, 0.593 mmol) in DMF (5.0 mL) was added HATU (181 mg, 0.475 mmol). The mixture was stirred at this temperature for 1 h. The mixture was concentrated under reduced pressure, and the residue was purified by prep-TLC (THF) to afford 1-(4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)-2-azidocycloheptan-1-one (110 mg, yield 43%) as a colorless oil.

¹H NMR (400 MHz, DMSO-*d*₆) δ 7.74 (s, 1H), 7.49 (s, 1H), 7.17-7.12 (m, 3H), 6.88-6.60 (m, 1H), 6.43-6.36 (m, 1H), 4.85 (s, 1H), 4.65 (s, 1H), 4.51-4.49 (m, 1H), 4.28 (m, 1H), 4.17-4.12 (m, 2H), 3.86 (s, 3H), 3.75-3.73 (m, 1H), 3.55-3.54 (m, 2H), 3.10-3.03 (m, 1H), 2.87-2.82 (m, 3H), 2.71-2.67 (m, 1H), 2.03-1.98 (m, 5H), 1.84-1.76 (m, 2H), 1.68-1.62 (m, 1H), 1.52-1.47 (m, 1H), 1.25-1.22 (m, 1H).

To a solution of 1-(4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)-2-azidocyclobutanone (100 mg, 0.170 mmol) and 3-(1-oxo-4-(prop-2-yn-1-yloxy)isoindol-2-yl)-1-((2-(trimethylsilyl)ethoxy)methyl)piperidine-2,6-dione (80.1 mg, 0.187 mmol) in ACN (10.0 mL) at 25 °C was added a solution of sodium ascorbate (42.1 mg, 0.213 mmol) and CuSO₄·5H₂O (21.2 mg, 0.0849 mmol) in H₂O (4 mL). The mixture was stirred at 25 °C for 2 h. The aqueous layer was extracted with EtOAc (5 mL×2), and the combined organic layers were dried and concentrated. The residue was purified by prep-TLC (THF) to afford 3-(4-((2-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)-2-oxoethyl)-1H-1,2,3-triazol-4-yl)methoxy)-1-oxoisoindolin-2-yl)-1-((2-(trimethylsilyl)ethoxy)methyl)piperidine-2,6-dione (90 mg, yield 47%) as a yellow oil. LC-MS: m/z 1017.0 [M+H]⁺.

To a solution of 3-(4-((1-(2-(2-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)-2-oxoethyl)-1H-1,2,3-triazol-4-yl)methoxy)-1-oxoisoindolin-2-yl)-1-((2-(trimethylsilyl)ethoxy)methyl)piperidine-2,6-dione (90 mg, 0.0885 mmol) in DCM (2.0 mL) was added TFA (1.01 g, 8.85 mmol) at 25 °C. The mixture was stirred at this temperature for 1.5 h. The mixture was cooled to 0 °C, NH₃·H₂O (775 mg, 22.1 mmol) was added to the mixture, and the mixture was stirred at this temperature for 0.5 h. The aqueous layer was extracted with DCM (5 mL×2), and the combined organic layers were dried and concentrated. The residue was purified by prep-HPLC to afford 3-(4-((2-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)-2-oxoethyl)-1H-1,2,3-triazol-4-yl)methoxy)-1-oxoindol-2-yl)piperidine-2,6-dione (20.5 mg, yield 25%) as a white solid.

¹H NMR (400 MHz, DMSO-*d*₆) δ 10.96 (s, 1H), 8.16 (d, *J=* 2.8 Hz, 1H), 7.75 (s, 1H), 7.54 - 7.47 (m, 3H), 7.34 (d, *J =* 7.2 Hz, 1H), 7.19-7.13 (m, 3H), 6.89-6.61 (m, 1H), 6.45-6.38 (m, 1H), 5.51 (s, 2H), 5.33 (s, 2H), 5.10 (dd, *J =* 13.2, 5.2 Hz, 1H), 4.86 (s, 1H), 4.66 (s, 1H), 4.45 (s, 1H), 4.38-4.29 (m, 2H), 4.22-4.17 (m, 1H), 4.03-4.01 (m, 1H), 3.86 (s, 3H), 3.55 (s, 2H), 3.23-3.16 (m, 1H), 2.94 - 2.83 (m, 4H), 2.75-2.67 (m, 1H), 2.54-2.50 (m, 2H), 2.45-2.41 (m, 1H), 2.04-1.95 (m, 6H), 1.86 - 1.74 (m, 3H), 1.54 - 1.48 (m, 1H).¹⁹F NMR (400 MHz, DMSO-*d*₆) *δ* 108.14, 108.08. LC-MS: m/z 887.3 [M+H]⁺.

### Example 2-33 (Synthesis of 2047)

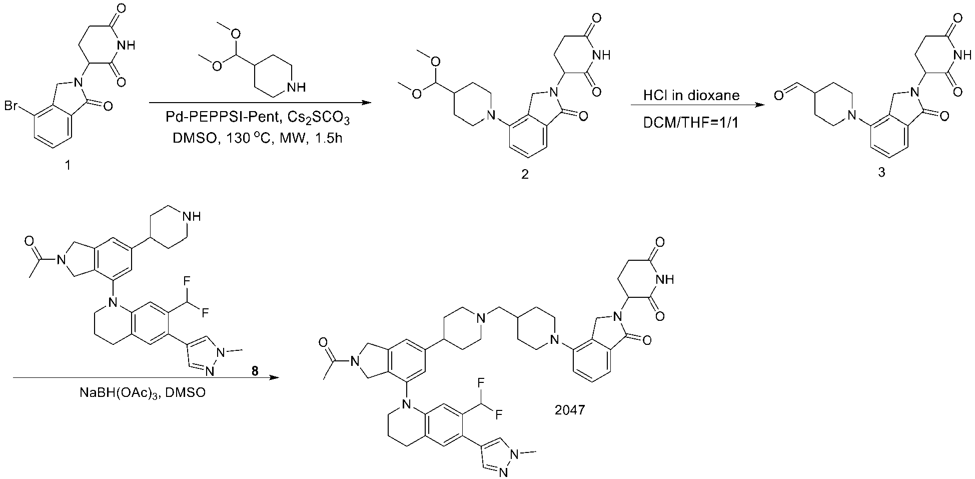

To a solution of 3-(4-bromo-1-oxoisoindolin-2-yl)piperidine-2,6-dione (20 mg, 0.062 mmol) and 4-(dimethoxymethyl)piperidine (30 mg, 0.19 mmol) in anhydrous DMSO (1 mL) were added Pd-PEPPSI-IPent (5 mg, 0.0061 mmol) and Cs₂CO₃ (61 mg, 0.19 mmol). The reaction mixture was reacted under microwave at 130 °C for 1 h and 20 min under nitrogen atmosphere. The reaction was quenched by addition of NH₄Cl (6 mL) and extracted with EtOAc (20 mL×3). The combined organic layers were washed with brine (50 mL×3), evaporated to dryness and purified by silica gel chromatography (THF/PE from 0% to 60%) to afford 3-(4-(4-(dimethoxymethyl)piperidin-1-yl)-1-oxoisoindolin-2-yl)piperidine-2,6-dione (90 mg, yield 65%) as a yellow solid. LC-MS: m/z 402.2 [M+H]⁺.

A solution of 3-(4-(4-(dimethoxymethyl)piperidin-1-yl)-1-oxoisoindolin-2-yl)piperidine-2,6-dione (90 mg, 0.22 mmol) in DMF (1 mL) and HCl/1,4-dioxane (4 N, 1 mL) was stirred at 25 °C for 12 h. The reaction was concentrated under reduced pressure, adjusted to pH=8 with aqueous NaHCO₃ solution and extracted with EtOAc (50 mL×3). The combined organic layers were dried over anhydrous Na₂SO₄. After filtration, the filtrate was concentrated under reduced pressure to give 1-(2-(2,6-dioxopiperidin-3-yl)-1-oxoisoindolin-4-yl)piperidine-4-formaldehyde (70 mg, yield 88%) as a yellow solid. LC-MS: m/z 356.1 [M+H]⁺.

To a solution of 1-(2-(2,6-dioxopiperidin-3-yl)-1-oxoisoindolin-4-yl)piperidine-4-formaldehyde (70 mg, 0.20 mmol) in DMSO (2 mL) was added 1-(4-(7-(difluoromethyl)-6-(1-methyl-1H-pyrazol-4-yl)-3,4-dihydroquinolin-1(2H)-yl)-6-(piperidin-4-yl)isoindolin-2-yl)ethan-1-one (100 mg, 0.20 mmol). After stirring at 25 °C for 0.5 h, NaBH(OAc)₃ (63 mg, 0.30 mmol) was added. The mixture was then stirred at 25 °C for 1 h. The solution was purified by Prep-HPLC (column: Xbridge 5u C18 150×19 mm; mobile phase: ACN-H₂O (0.1% FA); from 25% to 35% in 9 min; flow rate: 25 mL/min) to afford 3-(4-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidin-1-yl)-1-oxoisoindolin-2-yl)piperidine-2,6-dione (25.6 mg, yield 14%) as a yellow solid.

¹H NMR (400 MHz, DMSO-*d*₆) δ 10.98 (s, 1H), 7.75 (s, 1H), 7.49 (s, 1H), 7.46 - 7.40 (m, 1H), 7.30 (d, *J=* 7.4 Hz, 1H), 7.21 - 7.06 (m, 4H), 6.88 (s, 0.22H), 6.74 (s, 0.48H), 6.61 (s, 0.25H), 6.47 - 6.37 (m, 1H), 5.12 (dd, *J=* 13.2, 5.2 Hz, 1H), 4.87 (s, 1H), 4.76 - 4.56 (m, 2H), 4.47 - 4.39 (m, 1H), 4.38 - 4.25 (m, 2H), 3.86 (s, 3H), 3.59 - 3.54 (m, 2H), 3.45 - 3.33 (m, 4H), 3.28 - 3.17 (m, 2H), 2.98 - 2.85 (m, 3H), 2.82 - 2.69 (m, 3H), 2.64 - 2.56 (m, 2H), 2.55 - 2.52 (m, 1H), 2.47 - 2.41 (m, 1H), 2.07 - 1.95 (m, 6H), 1.86 (d, *J= 9.2* Hz, 7H), 1.32 (d, *J=* 10.8 Hz, 2H). LC-MS: m/z 845.5 [M+H]⁺.

### Example 2-34 (Synthesis of 2048)

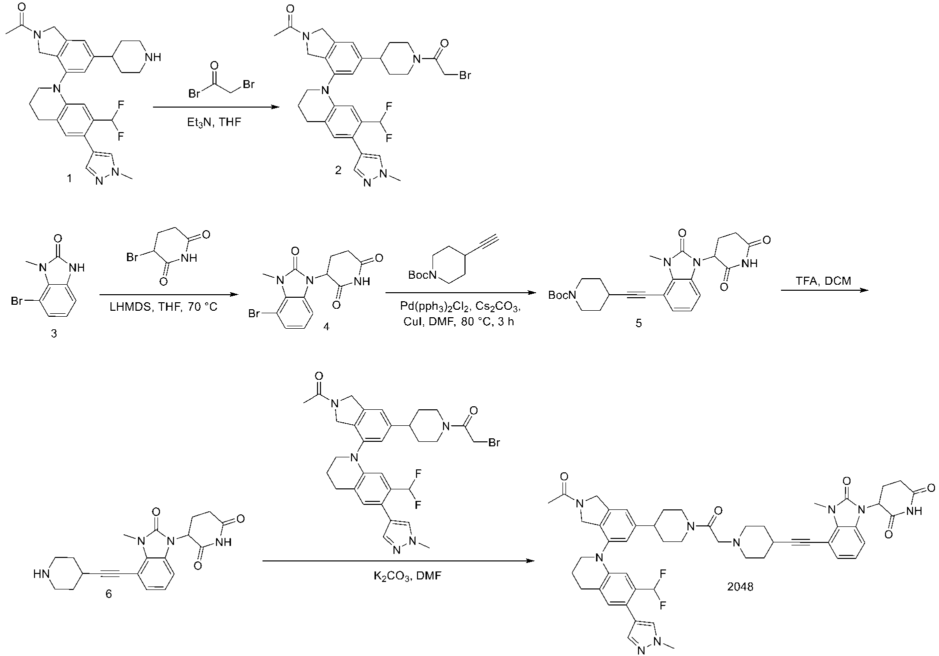

To a solution of 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (50.0 mg, 0.098 mmol) and Et₃N (12.0 mg, 0.296 mmol) in THF (5.0 mL) was added 2-bromoacetyl bromide (22.0 mg, 0.108 mmol). The reaction solution was stirred at 25 °C for 1 h. The mixture was concentrated under reduced pressure. The residue was purified by silica gel chromatography, eluting with MeOH/DCM (MeOH from 0% to 10%) over 10 min to afford 1-(4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)-2-bromoethan-1-one (50.0 mg, yield 73%) as a yellow solid. LC-MS: m/z [M+H]⁺ 588.1.

To a solution of 7-bromo-1-methyl-3H-1,3-benzodiazol-2-one (500 mg, 2.20 mmol) in THF (10.0 mL) was added LHMDS (1105 mg, 6.60 mmol) at 0 °C. The mixture was stirred at 0 °C for 0.5 h. Then 3-bromopiperidine-2,6-dione (634 mg, 3.30 mmol) was added and the mixture was stirred at 70 °C for 14.5 h. The mixture was cooled to 25 °C, quenched with NH₄Cl (50 mL) and extracted with DCM (50 mL×2). The combined organic phases were dried over Na₂SO₄, filtered and concentrated under reduced pressure. The residue was purified by silica gel chromatography, eluting with EA/PE (EA from 0% to 50%) over 15 min to afford 3-(4-bromo-3-methyl-2-oxo-2,3-dihydro-1H-benzo[d]imidazol-1-yl)piperidine-2,6-dione (140 mg, yield 18%) as a white solid. LC-MS: m/z [M+H]⁺ 339.8.

To a solution of 3-(4-bromo-3-methyl-2-oxo-2,3-dihydro-1H-benzo[d]imidazol-1-yl)piperidine-2,6-dione (140 mg, 0.414 mmol) in DMF (6.0 mL) were added tert-butyl 4-ethynylpiperidine-1-carboxylate (130 mg, 0.621 mmol), Cs₂CO₃ (270 mg, 0.828 mmol), CuI (16.0 mg, 0.082 mmol) and Pd(PPh₃)₂Cl₂ (58.0 mg, 0.082 mmol). The reaction solution was heated to 80 °C and stirred at this temperature under N₂ for 5 h. The reaction mixture was cooled to 25 °C, diluted with water (25 mL) and extracted with EtOAc (20 mL×2). The combined organic layers were washed with brine (50 mL), dried over Na₂SO₄, filtered and concentrated under reduced pressure. The residue was purified by silica gel chromatography, eluting with MeOH/DCM (MeOH from 0% to 5%) over 10 min to afford tert-butyl 4-((1-(2,6-dioxopiperidin-3-yl)-3-methyl-2-oxo-2,3-dihydro-1H-benzoimidazol-4-yl)ethynyl)piperidine-1-carboxylate (140 mg, yield 65%) as a yellow solid. LC-MS: m/z [M+Na]⁺ 489.1.

To a solution of tert-butyl 4-((1-(2,6-dioxopiperidin-3-yl)-3-methyl-2-oxo-2,3-dihydro-1H-benzoimidazol-4-yl)ethynyl)piperidine-1-carboxylate (25.0 mg, 0.053 mmol) in DCM (4.0 mL) was added TFA (1.0 mL). The mixture was stirred at 25 °C for 1 h. The mixture was concentrated under reduced pressure to afford 3-(3-methyl-2-oxo-4-(piperidin-4-ylethynyl)-2,3-dihydro-1H-benzo[d]imidazol-1-yl)piperidine-2,6-dione (20.0 mg, yield 96%) as a yellow oil. LC-MS: m/z [M+H]⁺ 367.0.

To a solution of 1-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)-2-bromoethan-1-one (25.0 mg, 0.039 mmol) in DMF (4.0 mL) were added K₂CO₃ (17.0 mg, 0.119 mmol) and 3-(3-methyl-2-oxo-4-(piperidin-4-ylethynyl)-2,3-dihydro-1H-benzo[d]imidazol-1-yl)piperidine-2,6-dione (18.0 mg, 0.047 mmol). The solution was stirred at 25 °C for 12 h. The mixture was concentrated, and the residue was purified by Prep-HPLC (ACN from 25% to 55% over 8 min) to afford 3-(4-((1-(2-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)-2-oxoethyl)piperidin-4-yl)ethynyl)-3-methyl-2-oxo-2,3-dihydro-1H-benzo[d]imidazol-1-yl)piperidine-2,6-dione (1.8 mg, yield 5%) as a white solid.

¹H NMR (400 MHz, DMSO-*d₆*) δ 10.83 (s, 1H), 7.69 (s, 1H), 7.46 (s, 1H), 7.15 (d, *J* = 12.0 Hz, 1H), 7.09 (d, *J =* 8.0 Hz, 3H), 7.04 (d, *J =* 8.0 Hz, 1H), 6.98 (t, *J* = 12.0 Hz, 1H), 6.82 - 6.55 (m, 1H), 6.44 (d, *J* = 24.0 Hz, 1H), 5.34 - 5.30 (m, 1H), 4.85 (s, 1H), 4.63 (d, *J* = 24.0 Hz, 2H), 4.33 (s, 1H), 3.86 (s, 3H), 3.64 (s, 3H), 3.57 - 3.53 (m, 2H), 2.86 (s, 5H), 2.77 - 2.63 (m, 6H), 2.55 - 2.45 (s, 1H), 2.32 (d, *J* = 12.0 Hz, 2H), 2.03 - 1.97 (m, 7H), 1.89 (s, 5H), 1.69 (s, 5H).¹⁹F NMR (400MHz, DMSO-*d₆*) δ -108.07. LC-MS: m/z [M+H]⁺ 912.4.

### Example 2-35 (Synthesis of 2050)

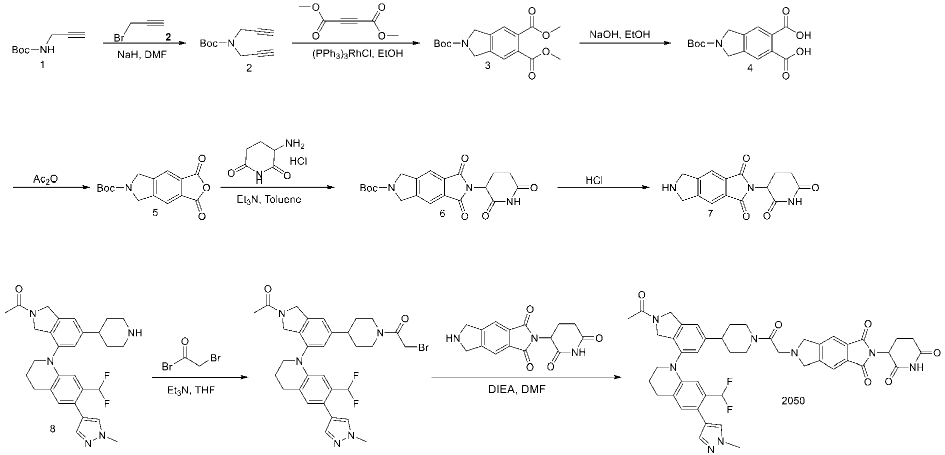

To a solution of tert-butyl prop-2-yn-1-ylcarbamate (10 g, 64 mmol) in DMF (50 mL) was added sodium hydride (2.82 g, 70.4 mmol, 60% in oil) at 0 °C . After stirring at 0 °C for 30 min, 3-bromo-1-propyne (8.37 g, 70.4 mmol) was added to the above solution. The reaction mixture was stirred at 25 °C for 4 h, then quenched with ice water. LCMS showed consumption of starting material and detection of the desired product. The mixture was extracted with ethyl acetate (50 mL×2), and the combined organic phases were dried over sodium sulfate and filtered. The filtrate was concentrated under reduced pressure. The residue was purified by silica gel flash chromatography (PE:EA=10:1) to afford tert-butyl bis(prop-2-yn-1-yl)carbamate (5 g, yield 38%) as a yellow oil. LC-MS: (ESI) m/z [M+Na] ⁺ 216.1.

¹H NMR (400 MHz, DMSO) δ 4.04 (d, *J* = 2.4 Hz, 4H), 3.23 (t, *J* = 2.4 Hz, 2H), 1.41 (s, 9H).

To a solution of tert-butyl bis(prop-2-yn-1-yl)carbamate (2 g, 10.3 mmol) and 1,4-dimethyl but-2-ynedioate (5.85 g, 41.2 mmol) in ethanol (20 mL) was added tris(triphenylphosphine)rhodium chloride (190 mg, 0.2 mmol) at 25 °C under N₂ protection. The reaction mixture was stirred at 80 °C for 12 h. LCMS showed detection of the desired product. The reaction mixture was cooled to 25 °C and concentrated in vacuo. The resulting brown residue was diluted in ethyl acetate (100 mL) and the mixture was concentrated under reduced pressure. The residue was purified by silica gel column chromatography (PE:EA=5:1) to afford 2-(tert-butyl)5,6-dimethyl-isoindoline-2,5,6-tricarboxylate (750 mg, yield 19%) as a white solid. LC-MS: (ESI) m/z [M+H] ⁺ 336.1.

To a solution of 2-(tert-butyl)5,6-dimethyl-isoindoline-2,5,6-tricarboxylate (700 mg, 2 mmol) in ethanol (10 mL) was added NaOH (3 mL, 3 M aqueous solution) at 25 °C. The reaction mixture was stirred at 80 °C for 3 h. LCMS showed consumption of starting material and detection of the desired product. The mixture was quenched with water (10 mL), acidified to pH=4 with HCl (1 M aqueous solution) and extracted with ethyl acetate (20 mL×2). The combined organic phases were dried over sodium sulfate and filtered. The filtrate was concentrated under reduced pressure to afford 2-(tert-butoxycarbonyl)isoindoline-5,6-dicarboxylic acid (530 mg, crude), which was used in the next step without further purification. LC-MS: (ESI) m/z [M+H] + 308.1.

2-(tert-Butoxycarbonyl)isoindoline-5,6-dicarboxylic acid (300 mg, 0.97 mmol) was dissolved in Ac₂O (4 mL), and the reaction mixture was stirred at 100 °C for 3 h. The reaction mixture was cooled to room temperature, and ethyl acetate was added. The combined organic phases were dried over sodium sulfate and filtered. The filtrate was concentrated under reduced pressure, and the residue was purified by silica gel flash chromatography (PE:EA=1:9) to afford tert-butyl 1,3-dioxo-5,7-dihydro-1H-furo[3,4-f]isoindole-6(3H)-carboxylate (200 mg, yield 71%) as a yellow solid. LC-MS: (ESI) m/z [M+H] ⁺ 290.1.

¹H NMR (400 MHz, DMSO) δ 8.04 (d, *J =* 2.4 Hz, 2H), 4.74 (d, *J* = 8.8 Hz, 4H), 1.47 (s, 9H).

To a solution of tert-butyl 1,3-dioxo-5,7-dihydro-1H-furo[3,4-f]isoindole-6(3H)-carboxylate (230 mg, 0.79 mmol) in toluene (5 mL) were added 3-aminopiperidine-2,6-dione (196 mg, 1.2 mmol) and Et₃N (241 mg, 2.38 mmol). The reaction mixture was stirred at 80 °C for 3 h, then cooled to room temperature. The mixture was quenched with water (10 mL) and extracted with ethyl acetate (20 mL×2). The combined organic phases were dried over sodium sulfate and filtered. The filtrate was concentrated under reduced pressure. The residue was purified by flash chromatography (PE:EA=1:9) to afford tert-butyl 6-(2,6-dioxopiperidin-3-yl)-5,7-dioxo-3,5,6,7-tetrahydropyrrolo[3,4-f]isoindole-2(1H)-carboxylate (195 mg, yield 64%) as a yellow solid.

¹H NMR (400 MHz, DMSO) δ 11.12 (s, 1H), 7.89 (d, *J =* 2.4 Hz, 2H), 5.15 (dd, *J =* 12.8, 5.2 Hz, 1H), 4.72 (d, *J =* 9.6 Hz, 4H), 2.97 - 2.81 (m, 1H), 2.68 - 2.52 (m, 2H), 2.15 - 2.03 (m, 1H), 1.47 (s, 9H). LC-MS: (ESI) m/z[M+H] ⁺ 400.1.

To a solution of tert-butyl 6-(2,6-dioxopiperidin-3-yl)-5,7-dioxo-1H,3H-pyrrolo[3,4-f]isoindol-2yl]carboxylate (280 mg, 0.7 mmol) in 1,4-dioxane (1 mL) was added HCl (3.0 mL, 4 M in 1,4-dioxane). The reaction mixture was stirred at 25 °C for 1 h. LCMS showed consumption of starting material and detection of the desired compound. The mixture was concentrated under reduced pressure to afford 2-(2,6-dioxopiperidin-3-yl)-6,7-dihydropyrrolo[3,4-f]isoindole-1,3(2H,5H)-dione (180 mg, yield 86%) as a yellow solid, which was used directly in the next step without further purification.

¹H NMR (400 MHz, DMSO) δ 11.14 (s, 1H), 9.96 (s, 2H), 7.96 (s, 2H), 5.16 (dd, *J* = 12.8, 5.2 Hz, 1H), 4.65 (s, 4H), 2.97 - 2.82 (m, 1H), 2.70 - 2.52 (m, 2H), 2.17 - 2.00 (m, 1H). LC-MS: (ESI) m/z [M+H] + 300.1.

To a solution of 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethene (200 mg, 0.4 mmol) and Et₃N (120 mg, 1.2 mmol) in THF (5 mL) was added 2-bromoacetyl bromide (88 mg, 0.44 mmol). The reaction solution was stirred at 25 °C for 1 h. LCMS showed consumption of starting material and formation of the desired product. The mixture was concentrated under reduced pressure. The residue was purified by silica gel chromatography, eluting with MeOH/DCM (MeOH from 0% to 10%) over 10 min to afford 1-(4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)-2-bromoethan-1-one (100 mg, yield 36%) as a yellow solid. LC-MS: (ESI) m/z [M+Na] + 648.2.

To a solution of 1-(4-{2-acetyl-7-[7-(difluoromethyl)-6-(1-methylpyrazol-4-yl)-3,4-dihydro-2H-quinolin-1-yl)-1,3-dihydroisoindolin-5-yl}piperidin-1-yl)-2-bromoethanone (100 mg, 0.16 mmol) in DMF (3 mL) were added DIEA (62 mg, 0.48 mmol) and 3-{1,3-dioxo-5H, 6H, 7H-pyrrolo[3,4-f]isoindol-2-yl}piperidine-2,6-dione (72 mg, 0.24 mmol). The mixture was stirred at 25 °C for 40 min. LCMS showed consumption of starting material and formation of the desired product. The mixture was concentrated. The residue was purified by Prep HPLC (column: Xbridge-C18 150×19 mm, 5 µm; mobile phase: ACN-H₂O, 45-55% ACN in 0.1% FA/water, 25 mL/min) to afford 6-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)-2-oxoethyl)-2-(2,6-dioxopiperidin-3-yl)-6,7-dihydropyrrolo[3,4-f]isoindole-1,3(2H,5H)-dione (24 mg, yield 17%) as a white solid.

¹H NMR (400 MHz, DMSO) δ 11.12 (s, 1H), 8.24 (s, 0.17H), 7.81 (d, *J =* 5.2 Hz, 2H), 7.75 - 7.72 (m, 1H), 7.49 (s, 1H), 7.19 - 7.09 (m, 1H), 6.87 (s, 0.26H), 6.74 (s, 0.52H), 6.60 (s, 0.26H), 6.45 - 6.33 (m, 1H), 5.13 (dd, *J =* 13.2, 5.2 Hz, 1H), 4.84 (s, 1H), 4.72 - 4.45 (m, 3H), 4.27 (s, 1H), 4.18 - 4.01 (m, 5H), 3.86 (s, 3H), 3.75 - 3.62 (m, 2H), 3.57 - 3.49 (m, 2H), 3.18 - 3.03 (m, 1H), 2.96 - 2.77 (m, 4H), 2.65 - 2.51 (m, 3H), 2.13 - 1.95 (m, 6H), 1.90 - 1.74 (m, 2H), 1.71 - 1.40 (m, 2H). ¹⁹F NMR (400 MHz, DMSO) δ -228.81. LC-MS: (ESI) m/z [M+H] ⁺ 845.3.

### Example 2-36 (Synthesis of 2053)

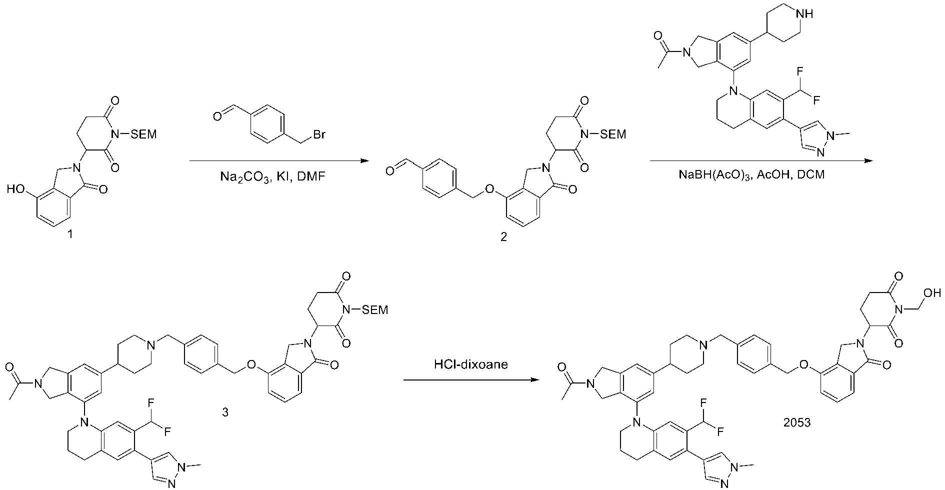

To a solution of 3-(4-hydroxy-1-oxoisoindolin-2-yl)-1-((2-(trimethylsilyl)ethoxy)methyl)piperidine-2,6-dione (200 mg, 0.511 mmol) in DMF (8.0 mL) were added 4-(bromomethyl)benzaldehyde (102 mg, 0.511 mmol), KI (17.0 mg, 0.102 mmol) and Na₂CO₃ (54.1 mg, 0.511 mmol). The mixture was stirred at 25 °C for 12 h. The mixture was concentrated under reduced pressure. The residue was purified by silica gel chromatography, eluting with 0% to 5% methanol in DCM over 10 min to afford 4-((2-(2,6-dioxo-1-((2-(trimethylsilyl)ethoxy)methyl)piperidin-3-yl)-1-oxoisoindol-4-yl)oxy)methyl)benzaldehyde (220 mg, yield 76%) as a yellow solid.

¹H NMR (400 MHz, DMSO-*d₆*) δ 10.02 (s, 1H), 7.94 (d, *J* = 8.0 Hz, 2H), 7.71 (d, *J =* 8.0 Hz, 2H), 7.50 (t, *J* = 7.6 Hz, 1H), 7.34 (dd, *J =* 13.6, 6.8 Hz, 2H), 5.38 (s, 2H), 5.26 (dd, *J =* 13.6, 5.2 Hz, 1H), 5.05 (dd, *J =* 19.2, 9.6 Hz, 2H), 4.48 (d, *J* = 17.2 Hz, 1H), 4.26 (d, *J =* 17.2 Hz, 1H), 3.57 - 3.45 (m, 2H), 3.14 - 2.99 (m, 1H), 2.84 - 2.72 (m, 1H), 2.46 - 2.38 (m, 1H), 2.08 - 2.01 (m, 1H), 0.89 - 0.78 (m, 2H), -0.00 - -0.05 (m, 9H).

To a solution of 4-((2-(2,6-dioxo-1-((2-(trimethylsilyl)ethoxy)methyl)piperidin-3-yl)-1-oxoisoindolin-4-yl)oxy)methyl)benzaldehyde (180 mg, 0.353 mmol), 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (179 mg, 0.353 mmol) and AcOH (2.12 mg, 0.0353 mmol) in DCM (10.0 mL) was added NaBH(OAc)₃ (225 mg, 1.060 mmol). The mixture was stirred at 25 °C for 12 h. The mixture was concentrated under reduced pressure. The residue was purified by silica gel chromatography, eluting with 0% to 8% methanol in DCM over 10 min to afford 3-(4-((4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)benzyl)oxy)-1-((2-(trimethylsilyl)ethoxy)methyl)piperidine-2,6-dione (240 mg, yield 64%) as a yellow solid. LC-MS: m/z [M+H]⁺ 998.4.

A solution of 3-(4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)benzyl)oxy)-1-oxoisoindol-2-yl)-1-((2-(trimethylsilyl)ethoxy)methyl)piperidine-2,6-dione (100 mg, 0.100 mmol) in HCl/1,4-dioxane (4 N, 10 mL) was stirred at 25 °C for 12 h. The mixture was concentrated, and eluted with aqueous acetonitrile (0.1% FA) from 16% to 46% over 9.0 min (instrument: Waters MS triggered Prep LC with QDA detector; column: Xbridge 5u C18 150×19 mm; flow rate: 20 mL/min) to afford 3-(4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)benzyl)oxy)-1-oxoisoindol-2-yl)-1-(hydroxymethyl)piperidine-2,6-dione (23.5 mg, yield 24.88%) as a pale yellow solid.

¹H NMR (400 MHz, DMSO-*d₆*) 7.74 (s, 1H), 7.52 - 7.42 (m, 4H), 7.37 - 7.32 (m, 4H), 7.17 - 7.09 (m, 3H), 6.74 (t, *J* = 55.2 Hz, 1H), 6.40 (d, *J* = 28.8 Hz, 1H), 6.20 - 5.95 (s, 1H), 5.26 - 5.17 (m, 3H), 5.10 - 4.97 (m, 2H), 4.84 (s, 1H), 4.64 (s, 1H), 4.45 - 4.40 (m, 1H), 4.30 - 4.22 (m, 2H), 3.86 (s, 3H), 3.57 - 3.49 (m, 5H), 3.09 - 2.99 (m, 1H), 2.93 - 2.85 (m, 4H), 2.77 - 2.70 (m, 1H), 2.48 - 2.32 (m, 2H), 2.08 - 1.97 (m, 8H), 1.78 - 1.63 (m, 4H). LC-MS: m/z [M+H]⁺ 899.3.

### Example 2-37 (Synthesis of 2054)

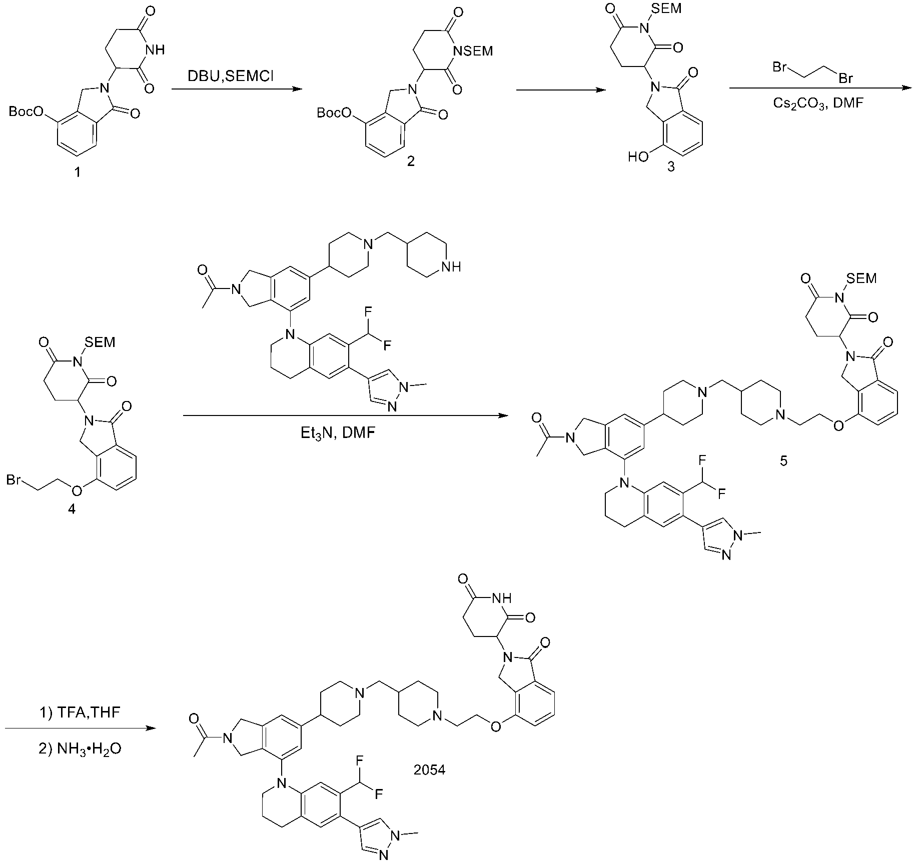

To a solution of tert-butyl (2-(2,6-dioxopiperidin-3-yl)-1-oxoisoindolin-4-yl) carbonate (2 g, 5.5 mmol) in DMF (20 mL) were added DBU (1.67 g, 11 mmol) and 2-(trimethylsilyl)ethoxymethyl chloride (1.38 g, 8.2 mmol). The reaction mixture was stirred at 25 °C under N₂ for 1 h. LCMS showed consumption of starting material and detection of the desired compound. The mixture was concentrated under reduced pressure. The residue was purified by silica gel chromatography, eluting with 0% to 80% EA in PE to afford tert-butyl (2-(2,6-dioxo-1-((2-(trimethylsilyl)ethoxy)methyl)piperidin-3-yl)-1-oxoisoindolin-4-yl) carbonate (2 g, yield 71%) as a white solid. LC-MS: (ESI) m/z [M+Na] ⁺ 513.5.

To a solution of tert-butyl (2-(2,6-dioxo-1-((2-(trimethylsilyl)ethoxy)methyl)piperidin-3-yl)-1-oxoisoindolin-4-yl) carbonate (2 g, 4.1 mmol) in DCM (20 mL) was added piperidine (6 mL) at 25 °C. The reaction mixture was stirred at 25 °C for 1 h. LCMS showed consumption of starting material and detection of the desired product. The mixture was quenched with water (30 mL), acidified to pH=4 with HCl (1 M aqueous solution), and extracted with DCM (30 mL×2). The combined organic phases were dried over Na₂SO₄, filtered and concentrated under reduced pressure. The residue was purified by silica gel chromatography, eluting with EA/PE (EA from 0% to 100%) over 25 min to afford 3-(4-hydroxy-1-oxoisoindolin-2-yl)-1-((2-(trimethylsilyl)ethoxy)methyl)piperidine-2,6-dione (1.3 g, yield 80%) as a white solid. LC-MS: (ESI) m/z [M+ Na] ⁺ 413.3.

To a solution of 3-(4-hydroxy-1-oxoisoindolin-2-yl)-1-((2-(trimethylsilyl)ethoxy)methyl)piperidine-2,6-dione (1300 mg, 3.3 mmol) in DMF (13 mL) were added cesium carbonate (1830 mg, 5.6 mmol) and 1,2-dibromoethane (2480 mg, 13.2 mmol). The solution was stirred at 60 °C under N₂ for 1 h. LCMS showed consumption of starting material and formation of the desired product. The mixture was quenched with H₂O (20 mL) and extracted with EA (50 mL×2). The combined organic layers were washed with brine (50 mL×3), dried over anhydrous Na₂SO₄, filtered and concentrated under reduced pressure. The residue was purified by silica gel chromatography, eluting with EA/PE (EA from 0% to 100%) over 25 min to afford 3-(4-(2-bromoethoxy)-1-oxoisoindolin-2-yl)-1-((2-(trimethylsilyl)ethoxy)methyl)piperidine-2,6-dione (0.56 g, yield 33%) as a white oil. LC-MS: (ESI) m/z [M+ Na] ⁺ 518.9.

To a solution of 1-{4-[7-(difluoromethyl)-6-(1-methylpyrazol-4-yl)-3,4-dihydro-2H-quinolin-1-yl]-6-[1-(piperidin-4-ylmethyl)piperidin-4-yl]-1,3-dihydroisoindol-2-yl}ethene (245 mg, 0.406 mmol) in DMF (7 mL) were added Et₃N (164 mg, 1.62 mmol) and 3-(4-(2-bromoethoxy)-1-oxoisoindolin-2-yl)-1-((2-(trimethylsilyl)ethoxy)methyl)piperidine-2,6-dione (304 mg, 0.609 mmol). The solution was stirred at 100 °C under microwave for 1 h. LCMS showed consumption of starting material and formation of the desired product. The mixture was diluted with water (30 mL) and extracted with EA (30 mL×3). The combined organic layers were dried over Na₂SO₄ and concentrated. The crude product was purified by prep-TLC (DCM/MeOH=10:1) to afford 3-(4-(2-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidin-1-yl)ethoxy)-1-(2-(trimethylsilyl)ethoxy)methyl)piperidine-2,6-dione (240 mg, yield 58%) as a white solid. LC-MS: (ESI) m/z [M+ H] ⁺ 1019.3.

To a solution of 3-(4-(2-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidin-1-yl)ethoxy)-1-(2-(trimethylsilyl)ethoxy)methyl)piperidine-2,6-dione (240 mg, 0.235 mmol) in THF (2.0 mL) was added TFA (4 mL) at 0 °C. The mixture was stirred at 25 °C for 2 h. Then aqueous ammonia (5 mL) was added and the mixture was stirred at 0 °C for 0.5 h. LCMS showed consumption of starting material and detection of the desired product. The mixture was quenched with H₂O (20 mL) and extracted with EA (30 mL×2). The combined organic layers were washed with brine (50 mL×3), dried over anhydrous Na₂SO₄, filtered and concentrated under reduced pressure. The crude product was purified by prep-HPLC, eluting with 48% to 58% ACN in 0.1% NH₄HCO₃/water over 20 min to afford 3-(4-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)methyl)piperidin-1-yl)ethoxy)-1-oxoisoindolin-2-yl)piperidine-2,6-dione (65 mg, yield 30%) as a yellow solid.

¹H NMR (400 MHz, DMSO-*d₆*) δ 10.97 (s, 1H), 7.74 (s, 1H), 7.55 - 7.43 (m, 2H), 7.34 - 7.23 (m, 2H), 7.19 - 7.06 (m, 3H), 6.88 (s, 0.25H), 6.74 (s, 0.50H), 6.60 (s, 0.26H), 6.46 - 6.35 (m, 1H), 5.18 - 5.04 (m, 1H), 4.84 (s, 1H), 4.69 - 4.55 (m, 1.5H), 4.43 - 4.30 (m, 1.5H), 4.29 - 4.15 (m, 3H), 3.86 (s, 3H), 3.63 - 3.49 (m, 2H), 3.01 - 2.82 (m, 7H), 2.76 - 2.65 (m, 2H), 2.63 - 2.53 (m, 2H), 2.48 - 2.37 (m, 2H), 2.18 - 1.90 (m, 12H), 1.81 - 1.59 (m, 6H), 1.49 (s, 1H), 1.17 - 1.02 (m, 2H). ¹⁹F NMR (400 MHz, DMSO) δ -108.10.LC-MS: (ESI) m/z [M+ H] ⁺ 889.1.

### Example 2-38 (Synthesis of 2055)

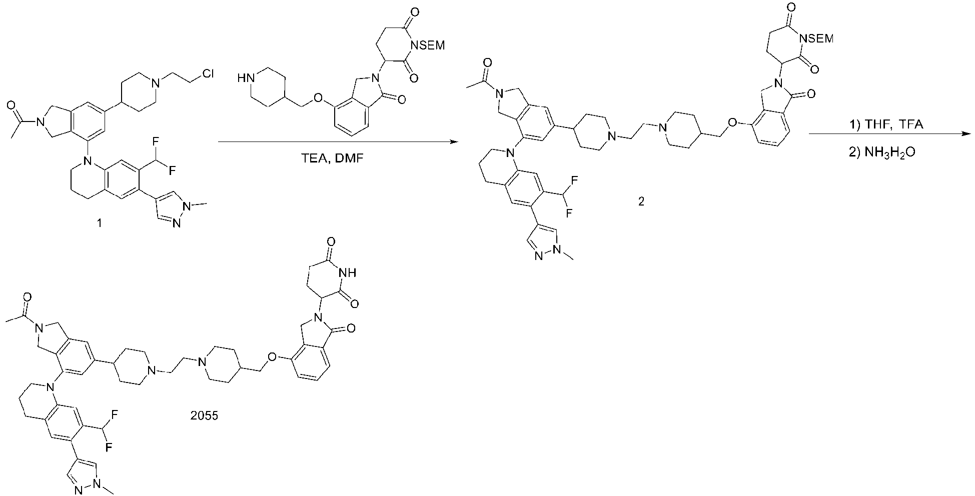

To a solution of 1-{6-[1-(2-chloroethyl)piperidin-4-yl]-4-[7-(difluoromethyl)-6-(1-methylpyrazol-4-yl)-3,4-dihydro-2H-quinolin-1-yl]-1,3-dihydroisoindol-2-yl}ethanone (30 mg, 0.05 mmol) in DMF (2 mL) were added 3-[1-oxo-4-(piperidin-4-ylmethoxy)-3H-isoindol-2-yl]-1-{[2-(trimethylsilyl)ethoxy]methyl}piperidine-2,6-dione (20 mg, 0.04 mmol), TEA (16 mg, 0.15 mmol) and KI (8 mg, 0.05 mmol) at 25 °C. After addition, the mixture was stirred at 50 °C for 1 h. The reaction mixture was diluted with water (10 mL) and extracted with EtOAc (20 mL×2). The combined organic phases were dried over Na₂SO₄ and filtered. The filtrate was concentrated under reduced pressure. The residue was purified by silica gel chromatography to afford 3-[4-({1-[2-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)ethyl]piperidin-4-yl}methoxy)-1-oxo-3H-isoindol-2-yl]-1-{[2-(trimethylsilyl)ethoxy]methyl}piperidine-2,6-dione (20 mg, yield 33%) as a yellow solid. LC-MS: (ESI) m/z [M+H]⁺ 1019.5.

To a solution of 3-[4-({1-[2-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)ethyl]piperidin-4-yl}methoxy)-1-oxo-3H-isoindol-2-yl]-1-{[2-(trimethylsilyl)ethoxy]methyl}piperidine-2,6-dione (20 mg, 0.02 mmol) in THF (2 mL) was added TFA (2 mL) at 25 °C. After addition, the mixture was stirred at 25 °C for 1 h, then NH₃·H₂O (2 mL) was added. The mixture was stirred at 25 °C for 1 h. The reaction was purified by prep-HPLC (ACN from 10% to 70% over 10 min) to afford 4-{1-[2-acetyl-6-(2-oxopiperidin-4-yl)-1,3-dihydroisoindol-4-yl]-7-(difluoromethyl)-3,4-dihydro-2H-quinolin-6-yl}-N,N,1-trimethylpyrazole-3-carboxamide (2.1 mg, yield 12%) as a white solid.

¹H NMR (400 MHz, DMSO-*d₆*) δ 10.99 (s, 1H), 7.75 (s, 1H), 7.60 - 7.48 (m, 2H), 7.40 - 7.25 (m, 2H), 6.85 - 6.61 (m, 1H), 6.50 - 6.38 (m, 1H), 5.15 - 5.05 (m, 1H), 4.88 (s, 1H), 4.70 - 4.60 (m, 2H), 4.40 - 4.21 (m, 3H), 4.05 - 4.01 (m, 2H), 3.86 (s, 3H), 3.60 - 3.50 (m, 2H), 3.40 - 3.30 (m, 12H), 3.00 - 2.88 (m, 4H), 2.60 - 2.40 (m, 3H), 2.10 - 1.90 (m, 12H), 1.70 - 1.60 (m, 2H). ¹⁹F NMR (400MHz, DMSO-d6) δ ppm -108.10. LC-MS: (ESI) m/z [M+H]⁺ 890.3.

### Example 2-39 (Synthesis of 2058)

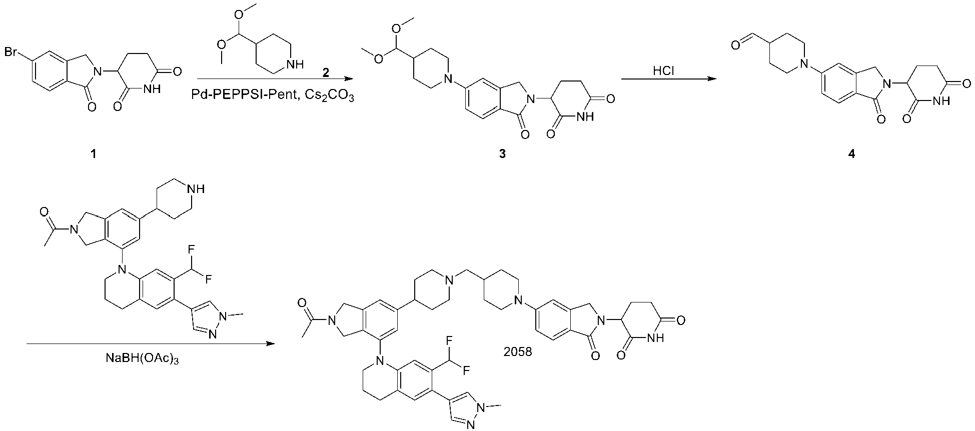

A solution of 3-(5-bromo-1-oxo-3H-isoindol-2-yl)piperidine-2,6-dione (400 mg, 1.23 mmol), 4-(dimethoxymethyl)piperidine (591 mg, 3.71 mmol), Pd-PEPPSI-IPent (104 mg, 0.12 mmol) and cesium carbonate (1209.9 mg, 3.71 mmol) in DMSO (6 mL) was stirred at 100 °C under nitrogen for 2 h. LCMS showed detection of the desired product. Water (20 mL) was added to quench the reaction, which was extracted with EtOAc (50 mL×3). The combined organic layers were washed with brine (30 mL×3) and dried over anhydrous Na₂SO₄. The crude product was purified by silica gel flash chromatography (PE:EA=1:10) to afford 3-(5-(4-(dimethoxymethyl)piperidin-1-yl)-1-oxoisoindolin-2-yl)piperidine-2,6-dione (300 mg, yield 60%) as a white solid. LC-MS: (ESI) m/z [M+H] + 402.2.

To a solution of 3-(5-(4-(dimethoxymethyl)piperidin-1-yl)-1-oxoisoindolin-2-yl)piperidine-2,6-dione (150 mg, 0.37 mmol) in DCM (1 mL) was added HCl/1,4-dioxane (3.0 mL, 4 N) at 25 °C. The reaction mixture was stirred at 25 °C for 2 h. LCMS showed consumption of starting material and detection of the desired compound. The mixture was quenched with water (30 mL), adjusted to pH=9 with NaHCO₃ (2 M aqueous solution), and extracted with ethyl acetate (30 mL×2). The combined organic phases were dried over sodium sulfate and filtered. The filtrate was concentrated under reduced pressure to afford 1-(2-(2,6-dioxopiperidin-3-yl)-1-oxoisoindolin-5-yl)piperidine-4-formaldehyde (100 mg, crude) as a white solid, which was used in the next step without further purification. LC-MS: (ESI) m/z [M+H] + 356.2.

To a solution of 1-(2-(2,6-dioxopiperidin-3-yl)-1-oxoisoindolin-5-yl)piperidine-4-formaldehyde (80 mg, 0.22 mmol) in DMSO (3.0 mL) was added 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethenone (113 mg, 0.22 mmol). The mixture was then stirred at 0 °C for 10 min. NaBH(OAc)₃ (71 mg, 0.33 mmol) was added to the mixture. The mixture was stirred at 25 °C for 2 h. LCMS showed consumption of starting material and detection of the desired product. The crude product was purified by prep-HPLC, eluting with 25% to 35% ACN in 0.1% FA/water over 10 min to afford 3-(5-((4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)methyl)piperidin-1-yl)-1-oxoisoindolin-2-yl)piperidine-2,6-dione (17.7 mg, yield 8%) as a yellow solid.

¹H NMR (400 MHz, DMSO) δ 10.94 (s, 1H), 8.21 (s, 0.55H), 7.75 (s, 1H), 7.49 (t, *J* = 4.4 Hz, 2H), 7.22 - 7.07 (m, 3H), 7.06 - 7.00 (m, 2H), 6.88 (s, 0.25H), 6.74 (s, 0.5H), 6.61 (s, 0.25H), 6.48 - 6.33 (m, 1H), 5.04 (dd, *J =* 13.6, 4.8 Hz, 1H), 4.85 (s, 1H), 4.74 - 4.54 (m, 2H), 4.42 - 4.13 (m, 3H), 3.86 (s, 3H), 3.74 - 3.48 (m, 2H), 3.65 - 3.48 (m, 8H), 3.02 - 2.75 (m, 2H), 2.64 - 2.52 (m, 1H), 2.44 - 2.29 (m, 1H), 2.18 (s, 2H), 2.08 - 1.91 (m, 9H), 1.84 - 1.60 (m, 7H), 1.30 - 1.10 (m, 2H). ¹⁹F NMR (400 MHz, DMSO) δ -73.41, -108.11. LC-MS: (ESI) m/z [M+H] + 845.6.

### Example 2-40 (Synthesis of 2059)

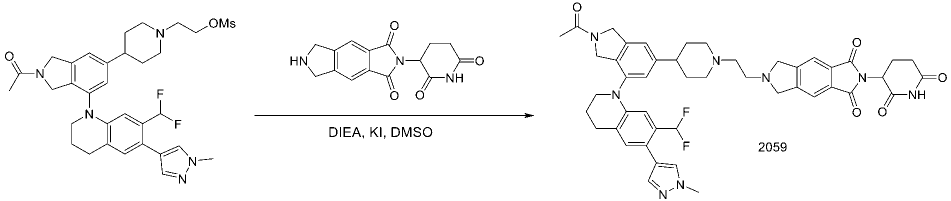

To a solution of ethyl 2-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)methanesulfonate (50 mg, 0.079 mmol) in DMSO (3 mL) were added DIEA (31 mg, 0.239 mmol), potassium iodide (26 mg, 0.159 mmol) and 3-{1,3-dioxo-5H,6H,7H-pyrrolo[3,4-f]isoindol-2-yl} piperidine-2,6-dione (40 mg, 0.119 mmol). The solution was stirred at 50 °C for 6 h. LCMS showed consumption of starting material and formation of the desired product. The mixture was concentrated, and the residue was purified by Prep HPLC (column: Xbridge-C18 150×19 mm, 5 µm; mobile phase: ACN-H₂O, 35-65% ACN in 0.1% FA/water, 25 mL/min) to afford 6-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)-2-(2,6-dioxopiperidin-3-yl)-6,7-dihydropyrrolo[3,4-f]isoindole-1,3(2H,5H)-dione (3.2 mg, yield 5%) as a yellow solid.

¹H NMR (400 MHz, DMSO) δ 11.12 (s, 1H), 7.83 - 7.77 (m, 2H), 7.74 (s, 1H), 7.49 (s, 1H), 7.23 - 7.08 (m, 3H), 6.88 (s, 0.24H), 6.74 (s, 0.5H), 6.60 (s, 0.27H), 6.47 - 6.34 (m, 1H), 5.13 (dd, *J =* 12.4, 5.2 Hz, 1H), 4.85 (s, 1H), 4.72 - 4.44 (m, 2H), 4.28 (s, 1H), 4.01 (s, 3H), 3.86 (s, 4H), 3.55 (s, 2H), 3.11 - 2.98 (m, 2H), 2.95 - 2.79 (m, 6H), 2.64 - 2.54 (m, 4H), 2.19 - 1.95 (m, 8H), 1.84 - 1.59 (m, 4H). ¹⁹F NMR (400 MHz, DMSO) δ -108.10. LC-MS: (ESI) m/z [M+H] ⁺ 831.5.

### Example 2-41 (Synthesis of 2060)

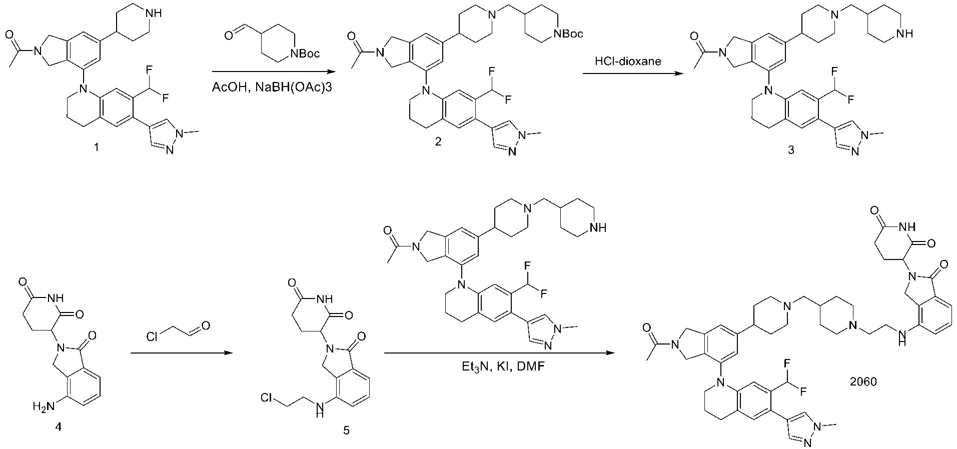

To a solution of 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (300 mg, 0.59 mmol) in DCM (4.0 mL) were added tert-butyl 4-formylpiperidine-1-carboxylate (379.7 mg, 1.8 mmol) and AcOH (3.56 mg, 0.06 mmol) at 25 °C, and the mixture was stirred at 25 °C for 0.5 h. Then NaBH(OAc)₃ (251.5 mg, 1.2 mmol) was added to the mixture, which was stirred at 25 °C for 2.5 h. The reaction was quenched with water (20 mL), extracted with DCM (3×20 mL), evaporated to dryness and purified by silica gel chromatography, eluting with 0% to 50% EtOAc in PE, then by flash column chromatography, eluting with 0% to 5% methanol in dichloromethane to afford tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidine-1-carboxylate (340 mg, yield 82%) as a yellow solid. LC-MS: m/z 703.4 [M+H]⁺.

To a solution of tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidine-1-carboxylate (300 mg, 0.43 mmol) in 1,4-dioxane (1.0 mL) was added HCl/1,4-dioxane (3.0 mL) at 25 °C. The mixture was then stirred at 25 °C for 30 min. The mixture was concentrated under reduced pressure to afford 1-(4-(7-(difluoromethyl)-6-(1-methyl-1H-pyrazol-4-yl)-3,4-dihydroquinolin-1(2H)-yl)-6-(1-(piperidin-4-ylmethyl)piperidin-4-yl)isoindol-2-yl)ethan-1-one (280 mg, yield 98%) as a yellow solid. LC-MS: m/z 603.4 [M+H]⁺.

To a solution of 3-(4-amino-1-oxo-3H-isoindol-2-yl)piperidine-2,6-dione (200 mg, 0.77 mmol) in MeOH (1.0 mL) and THF (1.0 mL) were added 2-chloroacetaldehyde (181.7 mg, 2.3 mmol) and AcOH (46.3 mg, 0.77 mmol), and the mixture was stirred at 25 °C for 0.5 h. Then borane-2-pyridine complex (165.0 mg, 1.5 mmol) was added to the mixture at 25 °C, which was stirred at 25 °C for 2.5 h. The reaction mixture was quenched with water (20 mL), extracted with DCM (3×20 mL), evaporated to dryness and purified by silica gel chromatography, eluting with 0% to 50% EtOAc in PE, then by flash column chromatography, eluting with 0% to 5% methanol in dichloromethane to afford 3-(4-((2-chloroethyl)amino)-1-oxoisoindolin-2-yl)piperidine-2,6-dione (200 mg, yield 81%) as a white solid. LC-MS: m/z 322.2 [M+H]⁺.

To a solution of 3-(4-((2-chloroethyl)amino)-1-oxoisoindolin-2-yl)piperidine-2,6-dione (150 mg, 0.47 mmol) in DMF (2.0 mL) were added 1-(4-(7-(difluoromethyl)-6-(1-methyl-1H-pyrazol-4-yl)-3,4-dihydroquinolin-1(2H)-yl)-6-(1-(piperidin-4-ylmethyl)piperidin-4-yl)isoindolin-2-yl)ethan-1-one (281.0 mg, 0.47 mmol), KI (77.3 mg, 0.47 mmol) and Et₃N (141.5 mg, 1.40 mmol) at 25 °C. The mixture was stirred at 100 °C for 9 h. The reaction mixture was purified by prep-HPLC, eluting with 48% to 58% ACN in H₂O (0.1% NH₄HCO₃) over 9 min to afford 3-(4-((2-((4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)methyl)piperidin-1-yl)ethyl)amino)-1-oxoisoindolin-2-yl)piperidine-2,6-dione (12.6 mg, yield 3%) as a yellow solid.

¹H NMR (400 MHz, DMSO-*d₆*) δ 11.00 (s, 1H), 8.23 (s, 1H), 7.74 (s, 1H), 7.49 (s, 1H), 7.29 (t, *J* = 8.0 Hz, 1H), 7.17 - 7.09 (m, 3H), 6.94 (d, *J* = 7.6 Hz, 1H), 6.78 (d, *J =* 8.0 Hz, 1H), 6.75 (t, *J* = 55.2 Hz, 1H), 6.40 (d, *J* = 28.8 Hz, 1H), 5.45 - 5.42 (m, 1H), 5.12 - 5.08 (m, 1H), 4.85 (s, 1H), 4.64 (s, 1H), 4.60 (s, 1H), 4.29 (s, 1H), 4.25 - 4.10 (m, 3H), 3.86 (s, 3H), 3.59 - 3.47 (m, 2H), 3.34 - 3.18 (m, 7H), 2.97 - 2.79 (m, 4H), 2.70 - 2.56 (m, 2H), 2.44 - 2.25 (m, 1H), 2.15 - 2.11 (m, 2H), 2.10 - 1.88 (m, 8H), 1.78 - 1.61 (m, 5H), 1.57 - 1.34 (m, 2H), 1.16 - 1.07 (m, 2H). ¹⁹F NMR (376 MHz, DMSO-*d₆*) δ -108.09. LC-MS: m/z 888.7 [M+H]⁺.

### Example 2-42 (Synthesis of 2061)

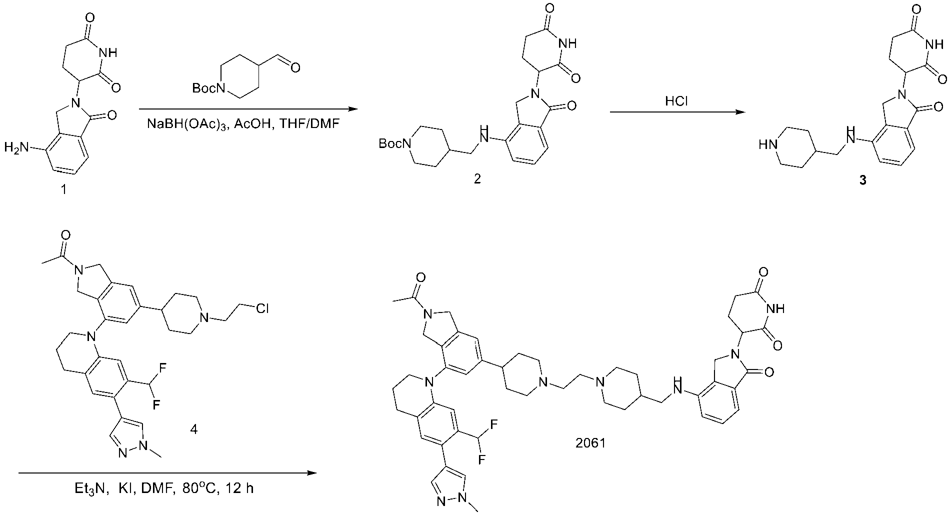

To a solution of 3-(4-amino-1-oxo-3H-isoindol-2-yl)piperidine-2,6-dione (500 mg, 1.928 mmol) in THF (10.0 mL) were added tert-butyl 4-formylpiperidine-1-carboxylate (411 mg, 1.928 mmol) and acetic acid (232 mg, 3.857 mmol), and the mixture was stirred at 25 °C for 0.5 h. Then NaBH(OAc)₃ (613 mg, 2.892 mmol) was added, and the mixture was stirred at 25 °C for 11.5 h. The mixture was concentrated under reduced pressure. The residue was purified by silica gel chromatography, eluting with EA/PE (EA from 0% to 50%) over 15 min to afford tert-butyl 4-(((2-(2,6-dioxopiperidin-3-yl)-1-oxoisoindolin-4-yl)amino)methyl)piperidine-1-carboxylate (450 mg, yield 49%) as a white solid. LC-MS: m/z [M+H-100]⁺ 357.2.

A solution of tert-butyl 4-((2-(2,6-dioxopiperidin-3-yl)-1-oxoisoindolin-4-yl)amino)methyl)piperidine-1-carboxylate (100 mg, 0.219 mmol) in HCl/1,4-dioxane (5.0 mL) was stirred at 25 °C for 1 h. The mixture was concentrated under reduced pressure to afford 3-(1-oxo-4-((piperidin-4-ylmethyl)amino)isoindol-2-yl)piperidine-2,6-dione (100 mg, yield 90%) as a yellow oil. LC-MS: m/z [M+H]⁺ 357.3.

To a solution of 3-(1-oxo-4-((piperidin-4-ylmethyl)amino)isoindol-2-yl)piperidine-2,6-dione (100 mg, 0.280 mmol) in DMF (5.0 mL) were added 1-{6-[1-(2-chloroethyl)piperidin-4-yl]-4-[7-(difluoromethyl)-6-(1-methylpyrazol-4-yl)-3,4-dihydro-2H-quinolin-1-yl]-1,3-dihydroisoindol-2-yl}ethanone (159 mg, 0.280 mmol), Et₃N (85.0 mg, 0.841 mmol) and KI (9.0 mg, 0.056 mmol). The reaction solution was heated to 80 °C and stirred at this temperature for 12 h. The reaction mixture was cooled to 25 °C, diluted with water (25 mL) and extracted with EtOAc (20 mL×2). The combined organic layers were washed with brine (50 mL), dried over Na₂SO₄, filtered and concentrated under reduced pressure. The residue was purified by Prep-HPLC (ACN from 30% to 60% over 7 min) to afford 3-(4-((1-(2-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)ethyl)piperidin-4-yl)methyl)amino)-1-oxoisoindolin-2-yl)piperidine-2,6-dione (3.0 mg, yield 1%) as a white solid.

¹H NMR (400 MHz, DMSO-*d₆*) δ 11.01 (s, 1H), 7.74 (s, 1H), 7.49 (s, 1H), 7.29 - 7.26 (m, 1H), 7.21 - 7.08 (m, 3H), 6.92 (d, *J =* 8.0 Hz, 1H), 6.88 - 6.60 (m, 2H), 6.41 (d, *J =* 28.0 Hz, 1H), 5.67 (s, 1H), 5.32 (s, 1H), 5.14 - 5.09 (m, 1H), 4.85 (s, 1H), 4.65 (s, 1H), 4.23 (d, *J =* 16.0 Hz, 2H), 4.12 (d, *J* = 16.0 Hz, 1H), 3.86 (s, 3H), 3.54 (s, 2H), 3.03 (s, 6H), 2.87 (s, 4H), 2.68 - 2.57 (m, 4H), 2.33 - 2.27 (m, 2H), 2.16 (s, 2H), 2.06 - 1.94 (m, 9H), 1.78 - 1.68 (m, 8H).¹⁹F NMR (400MHz, DMSO-*d₆*) δ -108.10. LC-MS: m/z [M+H]⁺ 888.4.

### Example 2-43 (Synthesis of 2062)

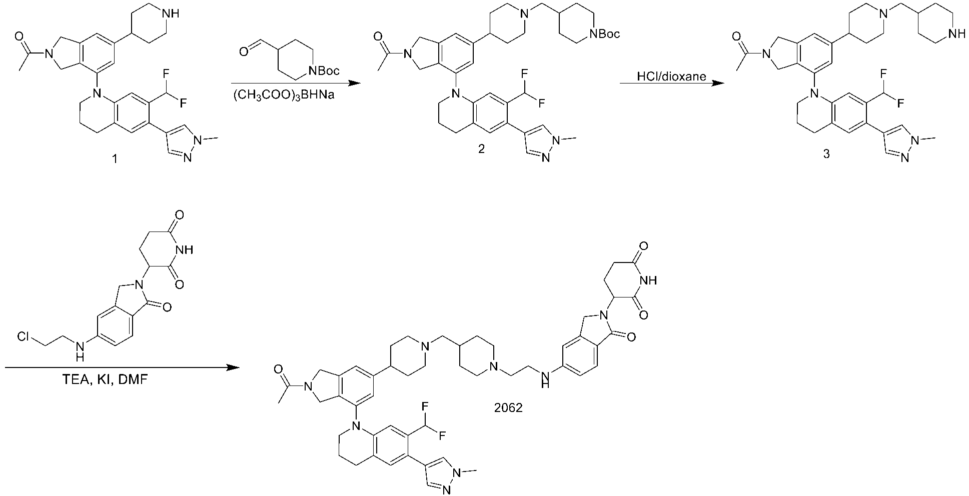

To a solution of 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (300 mg, 0.593 mmol) in DCM (2 mL) were added tert-butyl 4-formylpiperidine-1-carboxylate (253 mg, 1.19 mmol) and AcOH (0.01 mL), and the mixture was stirred at 25 °C for 0.5 h. NaBH(OAc)₃ (252 mg, 1.19 mmol) was added to the reaction mixture. The reaction mixture was stirred at 25 °C for 2 h. LCMS showed formation of the desired product. The reaction mixture was quenched with water, extracted with DCM, and the organic layer was dried over anhydrous Na₂SO₄, filtered and concentrated under reduced pressure. The residue was purified by flash column chromatography, eluting with 0% to 6% methanol in DCM to afford tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidine-1-carboxylate (400 mg, yield 96%) as a yellow solid. LC-MS: (ESI) m/z [M+H] ⁺ 703.4.

tert-Butyl 4-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidine-1-carboxylate (400 mg, 0.569 mmol) was dissolved in HCl/1,4-dioxane (2 mL) and stirred at 25 °C for 2 h. LCMS showed formation of the desired product. The solvent was removed under reduced pressure to afford 1-(4-(7-(difluoromethyl)-6-(1-methyl-1H-pyrazol-4-yl)-3,4-dihydroquinolin-1(2H)-yl)-6-(1-(piperidin-4-ylmethyl)piperidin-4-yl)isoindol-2-yl)ethan-1-one (340 mg, crude) as a white solid, which was used directly without further purification. LC-MS: (ESI) m/z [M+H] ⁺ 603.4.

To a solution of 1-(4-(7-(difluoromethyl)-6-(1-methyl-1H-pyrazol-4-yl)-3,4-dihydroquinolin-1(2H)-yl)-6-(1-(piperidin-4-ylmethyl)piperidin-4-yl)isoindol-2-yl)ethan-1-one (150 mg, 0.25 mmol) in DMF (5 mL) were added 3-(5-((2-chloroethyl)amino)-1-oxoisoindol-2-yl)piperidine-2,6-dione (80 mg, 0.25 mmol), TEA (151 mg, 1.50 mmol) and KI (4 mg, 0.025 mmol). The reaction mixture was stirred at 100 °C for 8 h. LCMS showed formation of the desired product. The solvent was removed under reduced pressure, and the residue was purified by prep-HPLC (column: Xbridge-C18 150 mm×19 mm, 5 µm; mobile phase: ACN-H₂O (0.1% NH₄HCO₃)) to afford 3-(5-((2-((4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidin-1-yl)ethyl)amino)-1-oxoisoindol-2-yl)piperidine-2,6-dione (11.6 mg, yield 5%) as a yellow solid.

¹H NMR (400 MHz, DMSO- *d₆*) *δ* 10.92 (s, 1H), 7.74 (s, 1H), 7.49 (s, 1H), 7.38 (d, *J =* 8.0 Hz, 1H), 7.18 - 7.07 (m, 3H), 6.88 (s, 0.25H), 6.72 (s, 0.5H), 6.70 - 6.62 (m, 2H), 6.60 (s, 0.25H), 6.45 - 6.35 (m, 1H), 6.23 - 6.13 (m, 1H), 5.01 (dd, *J =* 13.6, 5.2 Hz, 1H), 4.85 (s, 1H), 4.64 (s, 2H), 4.27 (d, *J* = 16.8 Hz, 2H), 4.16 (d, *J* = 16.8 Hz, 1H), 3.86 (s, 3H), 3.55 (s, 2H), 3.19 (d, *J =* 6.0 Hz, 2H), 2.97 - 2.81 (m, 8H), 2.69 - 2.59 (m, 2H), 2.37 - 2.32 (m, 1H), 2.13 (s, 2H), 2.10 - 1.89 (m, 12H), 1.80 - 1.73 (m, 2H), 1.71- 1.62 (m, 4H), 1.17- 1.07 (m, 2H). ¹⁹F NMR (377 MHz, DMSO-*d₆*) δ 108.10. LC-MS: (ESI) m/z [M+H] ⁺ 888.8.

### Example 2-44 (Synthesis of 063)

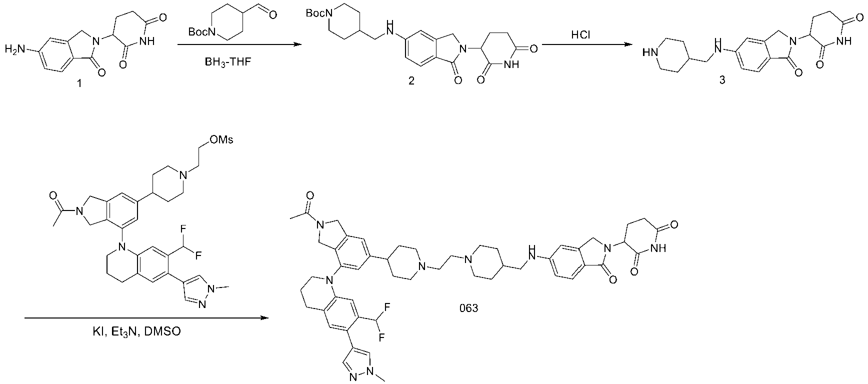

To a solution of 3-(5-amino-1-oxo-3H-isoindol-2-yl)piperidine-2,6-dione (200 mg, 0.77 mmol) in DCM (15.0 mL) and AcOH (3 mL) was added tert-butyl 4-formylpiperidine-1-carboxylate (329 mg, 1.54 mmol) at 0 °C. The mixture was stirred at 0 °C for 0.5 h. Then BH₃-THF (0.8 mL, 1 M solution in THF) was added and the mixture was stirred at 25 °C for 12 h. The mixture was quenched with H₂O (50 mL) and extracted with EA (50 mL×2). The combined organic phases were dried over Na₂SO₄, filtered and concentrated under reduced pressure. The residue was purified by silica gel chromatography, eluting with DCM/THF (THF from 0% to 50%) over 20 min to afford tert-butyl 4-((2-(2,6-dioxopiperidin-3-yl)-1-oxoisoindolin-5-yl)amino)methyl)piperidine-1-carboxylate (330 mg, yield 84%) as a white solid. LC-MS: (ESI) m/z [M+Na] ⁺ 479.2.

To a solution of tert-butyl 4-({[2-(2,6-dioxopiperidin-3-yl)-1-oxo-3H-isoindol-5-yl]amino}methyl)piperidine-1-carboxylate (300 mg, 0.65 mmol) in 1,4-dioxane (1 mL) was added HCl/1,4-dioxane (4 N in 1,4-dioxane) (3 mL) at 25 °C. The mixture was then stirred at 25 °C for 1 h. LCMS showed consumption of starting material and detection of the desired product. The resulting mixture was concentrated under reduced pressure to afford 3-(1-oxo-5-((piperidin-4-ylmethyl)amino)isoindol-2-yl)piperidine-2,6-dione (200 mg, yield 85%) as a white solid. LC-MS: (ESI) m/z [M+H] ⁺ 357.3.

To a solution of ethyl 2-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)methanesulfonate (50 mg, 0.079 mmol) in DMSO (3 mL) were added DIEA (31 mg, 0.239 mmol), potassium iodide (26 mg, 0.159 mmol) and 3-{1-oxo-5-[(piperidin-4-yl)methyl)amino]-3H-isoindol-2-yl}piperidine-2,6-dione (56.8 mg, 0.159 mmol). The solution was stirred at 50 °C for 12 h. LCMS showed consumption of starting material and formation of the desired product. The mixture was concentrated. The residue was purified by Prep HPLC (column: Xbridge-C18 150×19 mm, 5 µm; mobile phase: ACN-H₂O, 15 to 25% ACN in 0.1% FA/water, 25 mL/min) to afford 3-(5-((1-(2-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)ethyl)piperidin-4-yl)methyl)amino)-1-oxoisoindol-2-yl)piperidine-2,6-dione (1.6 mg, yield 2%) as a white solid.

¹H NMR (400 MHz, DMSO) δ 10.92 (s, 1H), 8.30 (s, 2H), 7.74 (s, 1H), 7.49 (s, 1H), 7.37 (d, *J* = 8.4 Hz, 1H), 7.20 - 7.06 (m, 3H), 6.88 (s, 0.23H), 6.74 (s, 0.46H), 6.69 - 6.58 (m, 2.35H), 6.47 - 6.34 (m, 2H), 5.01 (dd, *J =* 13.2, 5.2 Hz, 1H), 4.85 (s, 1H), 4.70 - 4.50 (m, 2H), 4.36 - 4.22 (m, 2H), 4.18 - 4.07 (m, 1H), 3.86 (s, 3H), 3.63 - 3.51 (m, 2H), 3.03 - 2.80 (m, 10H), 2.46 - 2.40 (m, 4H), 2.37 - 2.30 (m, 1H), 2.08 - 1.86 (m, 10H), 1.78 - 1.48 (m, 8H), 1.28 - 1.11 (m, 2H). ¹⁹F NMR (400 MHz, DMSO) δ -108.10. LC-MS: (ESI) m/z [M+H] ⁺ 888.4.

### Example 2-45 (Synthesis of 2064)

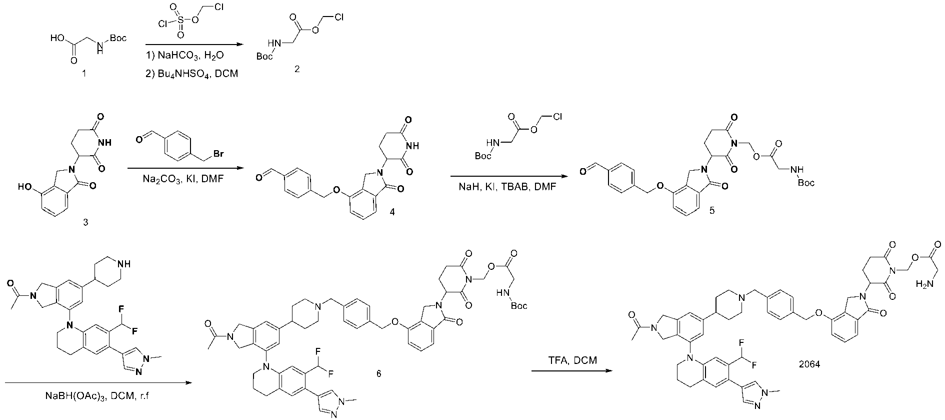

To a suspension of (tert-butoxycarbonyl)glycine (5.00 g, 0.0284 mol) in H₂O (100 mL) was added NaHCO₃ (9.54 g, 0.114 mol). The reaction mixture was stirred at 20 °C for 1 h. Then Bu₄NHSO₄ (0.960 g, 2.80 mmol) and DCM (100 mL) were added to the reaction mixture, the mixture was stirred for 1 h, then chloromethyl sulfonate (5.62 g, 0.0340 mmol) was added to the reaction mixture. The mixture was stirred at 20 °C for 12 h. The mixture was extracted with DCM (80 mL×2), and the organic layer was dried over anhydrous Na₂SO₄, filtered and concentrated under reduced pressure to afford chloromethyl (tert-butoxycarbonyl)glycinate (6.0 g, yield 85%) as a colorless oil. LC-MS:(ESI)m/[M+H]⁺ 246.3.

To a solution of 3-(4-hydroxy-1-oxo-3H-isoindol-2-yl)piperidine-2,6-dione (2.00 g, 7.70 mmol) in DMF (15 mL) was added Na₂CO₃ (0.900 g, 8.40 mmol), and the mixture was stirred at 25 °C for 10 min. Then 4-(bromomethyl)benzaldehyde (1.53 g, 7.70 mmol) and KI (1.40 g, 8.40 mmol) were added to the reaction mixture. The reaction mixture was stirred at 25 °C for 12 h. The mixture was diluted with EtOAc:H₂O (80 mL:80 mL), and the mixture was stirred at 25 °C for 30 min. The mixture was filtered to afford 4-((2-(2,6-dioxopiperidin-3-yl)-1-oxoisoindolin-4-yl)oxy)methyl)benzaldehyde (1.2 g, yield 42%) as a white solid. LC-MS: (ESI) m/z [M+H]⁺ 379.1.

To a solution of 4-({[2-(2,6-dioxopiperidin-3-yl)-1-oxo-3H-isoindol-4-yl]oxy}methyl)benzaldehyde (300 mg, 0.793 mmol) in DMF (8.0 mL) were added NaH (38.1 mg, 1.586 mmol), KI (132 mg, 0.793 mmol) and tetrabutylammonium bromide (25.6 mg, 0.0792 mmol). Then chloromethyl (tert-butoxycarbonyl)glycinate (891 mg, 3.96 mmol) was added to the reaction mixture, which was stirred at 20 °C for 48 h. The reaction mixture was diluted with DCM (100 mL), washed with brine (80 mL), and the organic layer was dried over anhydrous Na₂SO₄, filtered and concentrated under reduced pressure. The residue was purified by flash column chromatography (40 g silica gel column, petroleum ether/EtOAc and EtOAc from 0-60%) to afford (3-(4-((4-formylbenzyl)oxy)-1-oxoisoindolin-2-yl)-2,6-dioxopiperidin-1-yl)methyl(tert-butoxycarbonyl)glycinate (260 mg, yield 55%) as a pale yellow gum. LC-MS:(ESI)m/[M+Na]⁺ 588.2.

To a solution of (3-(4-((4-formylbenzyl)oxy)-1-oxoisoindolin-2-yl)-2,6-dioxopiperidin-1-yl)methyl(tert-butoxycarbonyl)glycinate (100 mg, 0.177 mmol) and 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethenone (89.2 mg, 0.177) in DCM (15 mL) was added NaBH(OAc)₃ (112 mg, 0.530 mmol), and the mixture was stirred at 50 °C for 12 h. The mixture was diluted with DCM (80 mL), washed with brine (60 mL), and the organic layer was dried over anhydrous Na₂SO₄, filtered and concentrated under reduced pressure. The residue was purified by flash chromatography (25 g silica gel column, petroleum ether/EtOAc and EtOAc from 0% to 70%) to afford (3-(4-((4-((2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)benzyl)oxy)-1-oxoisoindolin-2-yl)-2,6-dioxopiperidin-1-yl)methyl(tert-butoxycarbonyl)glycinate (95 mg, yield 50%) as a white solid. LC-MS: (ESI) m/z [M+H]⁺ 1055.4. C-MS:(ESI)m/[M+H]⁺ 1055.4.

To a solution of {(3-(4-((4-((2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)benzyl)oxy)-1-oxoisoindolin-2-yl)-2,6-dioxopiperidin-1-yl)methyl (tert-butoxycarbonyl)glycinate (90 mg, 0.0852 mmol) in DCM (5.0 mL) was added TFA (97.2 mg, 0.852 mmol). The reaction mixture was stirred at 20 °C for 3 h. The mixture was concentrated under reduced pressure, and the residue was purified by Prep-HPLC (column: Gemini-C18 150×21.2 mm; 5 µm; mobile phase: ACN/H₂O (0.1% FA); gradient: 17 to 47% over 9 min) to afford (3-(4-((4-((2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)benzyl)oxy)-1-oxoisoindolin-2-yl)-2,6-dioxopiperidin-1-yl)methyl glycinate (56 mg, yield 64%) as a white solid.

¹H NMR (400 MHz, DMSO) δ 8.17 (s, 1H), 7.74 (s, 1H), 7.54 - 7.41 (m, 4H), 7.37 - 7.33 (m, 3H), 7.13 (dd, *J =* 19.8, 11.0 Hz, 3H), 6.87 (s, 0.26H), 6.73 (s, 0.51H), 6.60 (s, 0.24H), 6.44-6.37 (m, 1.09H), 5.73-5.65 (m 1.9H), 5.34 - 5.17 (m, 3.0H), 4.85 (s, 1H), 4.73 - 4.54 (m, 2H), 4.49 - 4.37 (m, 1.0H), 4.34 - 4.21 (m, 2.0H), 3.86 (s, 3H), 3.56 - 3.52 (m, 6H), 3.15 - 3.04 (m, 2H), 2.98 - 2.76 (m, 6H), 2.60 - 2.54 (m, 1H), 2.49 - 2.40 (m, 1H), 2.15 - 1.93 (m, 8H), 1.81 - 1.61 (m, 4H). ¹⁹F NMR (376 MHz, DMSO) δ -73.46, -108.11. LC-MS:(ESI)m/[M+H]⁺ 955.3.

### Example 2-46 (Synthesis of 2065)

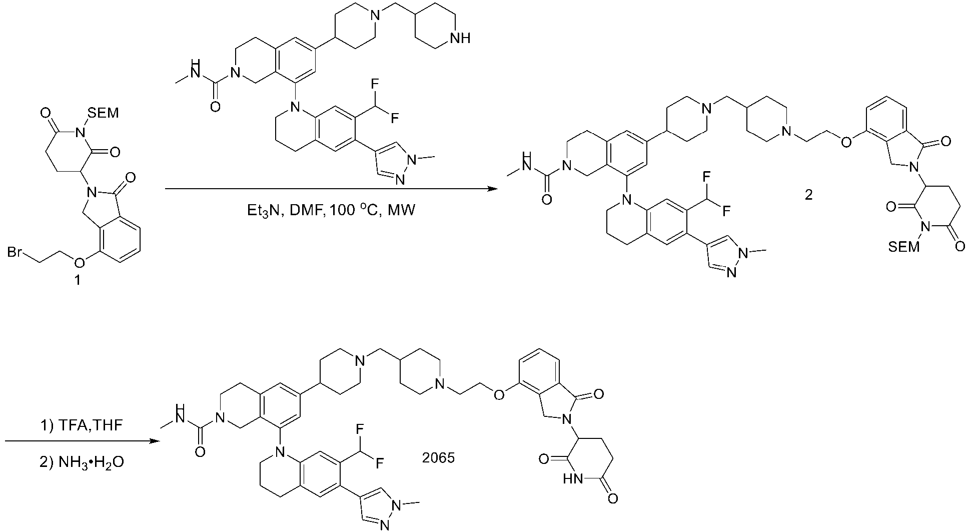

To a solution of 8-[7-(difluoromethyl)-6-(1-methylpyrazol-4-yl)-3,4-dihydro-2H-quinolin-1-yl]-N-methyl-6-[1-(piperidin-4-ylmethyl)piperidin-4-yl]-3,4-dihydro-1H-isoquinoline-2-carboxamide (110 mg, 0.17 mmol) in DMF (3 mL) were added Et₃N (53 mg, 0.52 mmol) and 3-(4-(2-bromoethoxy)-1-oxoisoindol-2-yl)-1-((2-(trimethylsilyl)ethoxy)methyl)piperidine-2,6-dione (130 mg, 0.26 mmol), and the mixture was stirred at 100 °C under microwave for 1 h. LCMS showed consumption of starting material and formation of the desired product. The mixture was diluted with water (30 mL) and extracted with EA (30 mL×3). The combined organic layers were dried over Na₂SO₄ and concentrated. The crude product was purified by prep-TLC (DCM/MeOH=10:1) to afford 8-(7-(difluoromethyl)-6-(1-methyl-1H-pyrazol-4-yl)-3,4-dihydroquinolin-1(2H)-yl)-6-((1-((2-((2,6-dioxo-1-((2-(trimethylsilyl)ethoxy)methyl)piperidin-3-yl)-1-oxoisoindolin-4-yl)oxy)ethyl)piperidin-4-yl)methyl)piperidin-4-yl)-N-methyl-3,4-dihydroisoquinoline-2(1H)-carboxamide (125 mg, yield 68%) as a white solid. LC-MS: (ESI) m/z [M+H] ⁺ 1048.1.

To a solution of 8-(7-(difluoromethyl)-6-(1-methyl-1H-pyrazol-4-yl)-3,4-dihydroquinolin-1(2H)-yl)-6-((1-((2-((2,6-dioxo-1-((2-(trimethylsilyl)ethoxy)methyl)piperidin-3-yl)-1-oxoisoindol-4-yl)oxy)ethyl)piperidin-4-yl)methyl)piperidin-4-yl)-N-methyl-3,4-dihydroisoquinoline-2(1H)-carboxamide (125 mg, 0.12 mmol) in THF (1.0 mL) was added TFA (2 mL) at 0 °C. The mixture was stirred at 25 °C for 0.5 h. Then aqueous ammonia (4 mL) was added and the mixture was stirred at 0 °C for 0.5 h. LCMS showed consumption of starting material and detection of the desired product. The mixture was quenched with H₂O (20 mL) and extracted with EA (30 mL×2). The combined organic layers were washed with brine (50 mL×3), dried over anhydrous Na₂SO₄, filtered and concentrated under reduced pressure. The crude product was purified by prep-HPLC, eluting with 51% to 61% ACN in 0.1% NH₄HCO₃/water over 20 min to afford 8-(7-(difluoromethyl)-6-(1-methyl-1H-pyrazol-4-yl)-3,4-dihydroquinolin-1(2H)-yl)-6-((1-((2-((2,6-dioxopiperidin-3-yl)-1-oxoisoindolin-4-yl)oxy)ethyl)piperidin-4-yl)methyl)piperidin-4-yl)-N-methyl-3,4-dihydroisoquinoline-2(1H)-carboxamide (33 mg, yield 27%) as a yellow solid.

¹H NMR (400 MHz, DMSO-*d₆*) δ 10.97 (s, 1H), 7.71 (s, 1H), 7.53 - 7.43 (m, 2H), 7.35 - 7.21 (m, 2H), 7.11 - 6.96 (m, 3H), 6.83 (s, 0.23H), 6.69 (s, 0.47H), 6.55 (s, 0.23H), 6.50 - 6.42 (m, 1H), 6.18 (s, 1H), 5.22 - 5.03 (m, 1H), 4.51 - 4.32 (m, 2H), 4.27 - 4.18 (m, 3H), 4.14 - 4.04 (m, 1H), 3.86 (s, 3H), 3.57 - 3.38 (m, 4H), 2.98 - 2.77 (m, 9H), 2.75 - 2.65 (m, 2H), 2.58 - 2.52 (m, 4H), 2.48 - 2.38 (m, 2H), 2.19 - 1.84 (m, 9H), 1.76 - 1.55 (m, 6H), 1.45 (s, 1H), 1.16 - 0.99 (m, 2H). ¹⁹F NMR (400 MHz, DMSO) δ -108.06. LC-MS: (ESI) m/z [M+ H] ⁺ 918.3.

### Example 2-47 (Synthesis of 2066)

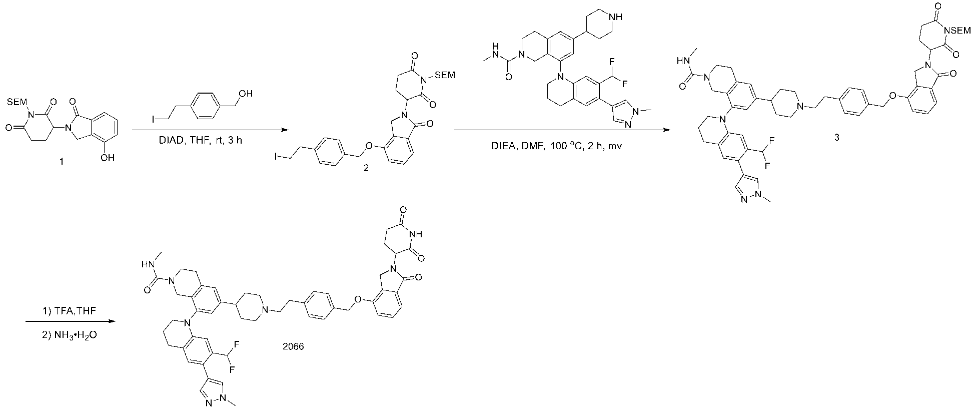

To a solution of 3-(4-hydroxy-1-oxo-3H-isoindol-2-yl)-1-{[2-(trimethylsilyl)ethoxy]methyl}piperidine-2,6-dione (300 mg, 0.76 mmol), (4-(2-iodoethyl)phenyl)methanol (302 mg, 1.15 mmol) and PPh₃ (302 mg, 1.15 mmol) in THF (5 mL) was added DIAD (233 mg, 1.15 mmol). The reaction mixture was stirred at 25 °C for 2 h. LCMS showed formation of the desired product. The reaction mixture was quenched with water and extracted with EtOAc. The organic layer was dried over anhydrous Na₂SO₄, filtered and concentrated in vacuo. The residue was purified by flash column chromatography, eluting with 0% to 50% THF in PE to afford 3-(4-((4-(2-iodoethyl)benzyl)oxy)-1-oxoisoindolin-2-yl)-1-((2-(trimethylsilyl)ethoxy)methyl)piperidine-2,6-dione (360 mg, crude) as a white solid. LC-MS: (ESI) m/z [M+Na] ⁺ 657.1.

To a solution of 8-[7-(difluoromethyl)-6-(1-methylpyrazol-4-yl)-3,4-dihydro-2H-quinolin-1-yl]-N-methyl-6-(piperidin-4-yl)-3,4-dihydro-1H-isoquinoline-2-carboxamide (200 mg, 0.37 mmol) in DMF (5 mL) were added DIEA (97 mg, 0.748 mmol), NaI (56 mg, 0.374 mmol) and 3-(4-((4-(2-iodoethyl)benzyl)oxy)-1-oxoisoindolin-2-yl)-1-((2-(trimethylsilyl)ethoxy)methyl)piperidine-2,6-dione (237 mg, 0.374 mmol). The solution was stirred at 100 °C under microwave for 1 h. LCMS showed consumption of starting material and formation of the desired product. The mixture was quenched with H₂O (10 mL) and extracted with EA (20 mL×2). The combined organic phases were dried over Na₂SO₄, filtered and concentrated under reduced pressure. The residue was purified by prep-TLC (DCM:MeOH=10:1) to afford 8-(7-(difluoromethyl)-6-(1-methyl-1H-pyrazol-4-yl)-3,4-dihydroquinolin-1(2H)-yl)-6-((2-((2,6-dioxo-1-((2-(trimethylsilyl)ethoxy)methyl)piperidin-3-yl)-1-oxoisoindolin-4-yl)oxy)methyl)phenylethyl)piperidin-4-yl)-N-methyl-3,4-dihydroisoquinoline-2(1H)-carboxamide (132 mg, crude) as a yellow solid. LC-MS: (ESI) m/z [M+H] ⁺ 1041.6.

To a solution of 8-(7-(difluoromethyl)-6-(1-methyl-1H-pyrazol-4-yl)-3,4-dihydro-2H-quinolin-1-yl)-6-(1-{2-[4-({[2-(2,6-dioxo-1-{[2-(trimethylsilyl)ethoxy]methyl}piperidin-3-yl)-1-oxo-3H-isoindol-4-yl)oxymethyl)phenyl]ethyl}piperidin-4-yl)-N-methyl-3,4-dihydro-1H-isoquinoline-2-carboxamide (120 mg, 0.115 mmol) in THF (3.0 mL) was added TFA (1 mL) at 0 °C. The mixture was stirred at 25 °C for 2 h. Then aqueous ammonia (5 mL) was added and the mixture was stirred at 0 °C for 0.5 h. LCMS showed consumption of starting material and detection of the desired product. The mixture was quenched with H₂O (20 mL) and extracted with EA (30 mL×2). The combined organic layers were washed with brine (50 mL×3), dried over anhydrous Na₂SO₄, filtered and concentrated under reduced pressure. The crude product was purified by prep-HPLC, eluting with 20% to 50% ACN in 0.1% FA/water over 10 min to afford 8-(7-(difluoromethyl)-6-(1-methyl-1H-pyrazol-4-yl)-3,4-dihydroquinolin-1(2H)-yl)-6-(1-(4-(((2-(2,6-dioxopiperidin-3-yl)-1-oxoisoindolin-4-yl)oxy)methyl)phenylethyl)piperidin-4-yl)-N-methyl-3,4-dihydroisoquinoline-2(1H)-carboxamide (18.4 mg, yield 17%) as a white solid.

¹H NMR (400 MHz, DMSO) δ 10.97 (s, 1H), 8.21 (s, 0.75H), 7.72 (s, 1H), 7.52 - 7.45 (m, 2H), 7.42 - 7.36 (m, 2H), 7.36 - 7.30 (m, 2H), 7.28 - 7.22 (m, 2H), 7.06 (d, *J =* 15.2 Hz, 2H), 7.00 (s, 1H), 6.83 (s, 0.26H), 6.70 (s, 0.51H), 6.56 (s, 0.28H), 6.50 - 6.44 (m, 1H), 6.18 (s, 1H), 5.20 (s, 2H), 5.10 (dd, *J* = 13.2, 5.2 Hz, 1H), 4.41 (dd, *J* = 16.8, 6.0 Hz, 2H), 4.24 (d, *J* = 17.6 Hz, 1H), 4.10 (d, *J =* 16.8 Hz, 1H), 3.86 (s, 3H), 3.59 - 3.38 (m, 6H), 3.08 - 2.99 (m, 2H), 2.96 - 2.71 (m, 8H), 2.60 - 2.52 (m, 5H), 2.14 - 1.93 (m, 5H), 1.81 - 1.70 (m, 2H), 1.68 - 1.54 (m, 2H). ¹⁹F NMR (400 MHz, DMSO) δ -108.06. LC-MS: (ESI) m/z [M+H] ⁺ 911.3.

### Example 2-48 (Synthesis of 2068)

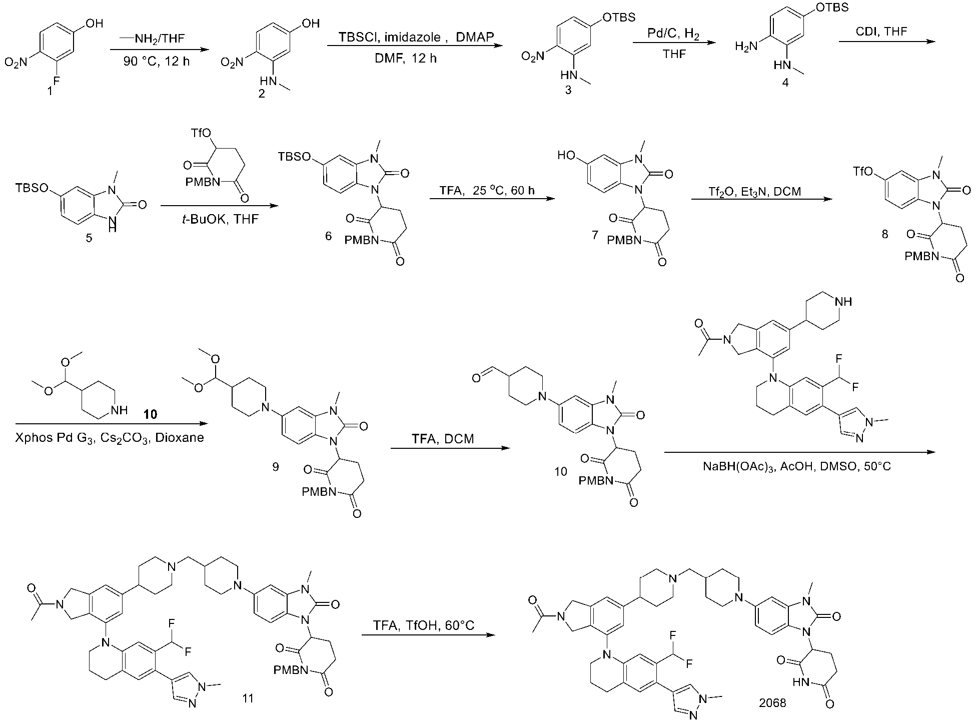

To a solution of 3-fluoro-4-nitrophenol (6.0 g, 0.038 mol) in THF (60.0 mL) was added methylamine (3.56 g, 0.114 mol). The mixture was heated to 80 °C and reacted for 12 h. The mixture was cooled to 25 °C and concentrated under reduced pressure. The residue was purified by silica gel chromatography, eluting with THF/PE (THF from 0% to 50%) over 15 min to afford 3-(methylamino)-4-nitrophenol (6.0 g, yield 89%) as a yellow solid. LC-MS: m/z [M+H]⁺ 169.0.

To a solution of 3-(methylamino)-4-nitrophenol (6.0 g, 0.035 mol) in DMF (60.0 mL) were added imidazole (4.86 g, 0.071 mol) and DMAP (0.22 g, 0.0018 mol). Then TBSCl (8.07 g, 0.053 mol) was added at 0 °C, and the mixture was stirred at 25 °C for 12 h. The mixture was concentrated. The residue was washed with NaHCO₃ solution (50 mL) and extracted with EA (60 mL×2). The combined organic layers were washed with brine (50 mL), dried over Na₂SO₄, filtered and concentrated to dryness. The residue was purified by silica gel chromatography, eluting with THF/PE (THF from 0% to 25%) over 10 min to afford 5-((tertbutyldimethylsilyl)oxy)-N-methyl-2-nitroaniline (8.2 g, yield 77%) as a yellow solid. LC-MS: m/z [M+H]⁺ 283.1.

To a solution of 5-((tert-butyldimethylsilyl)oxy)-N-methyl-2-nitroaniline (8.2 g, 0.029 mol) in THF (80.0 mL) was added Pd/C (1.54 g, 0.014 mol). The solution was stirred at 25 °C under H₂ atmosphere for 12 h. The mixture was filtered to remove solids and concentrated. The residue was purified by silica gel chromatography, eluting with THF/PE (THF from 0% to 30%) over 10 min to afford 5-((tert-butyldimethylsilyl)oxy)-N1-methylbenzene-1,2-diamine (7.0 g, yield 86%) as a yellow solid. LC-MS: m/z [M+H]⁺ 253.1.

To a solution of 5-((tert-butyldimethylsilyl)oxy)-N1-methylbenzene-1,2-diamine (7.0 g, 0.027 mol) in THF (80.0 mL) was added CDI (13.47 g, 0.083 mol). The mixture was stirred at 25 °C for 4 h. The mixture was concentrated under reduced pressure. The residue was purified by silica gel chromatography, eluting with THF/PE (THF from 0% to 30%) over 15 min to afford 6-((tert-butyldimethylsilyl)oxy)-1-methyl-1,3-dihydro-2H-benzo[d]imidazol-2-one (6.0 g, yield 74%) as a red solid. LC-MS: m/z [M+H]⁺ 279.4.

To a solution of 6-((tert-butyldimethylsilyl)oxy)-1-methyl-1,3-dihydro-2H-benzo[d]imidazol-2-one (1.0 g, 0.0036 mol) in THF (10.0 mL) was added t-BuOK (0.81 g, 0.0072 mol). The mixture was stirred at 0 °C for 0.5 h. Then 1-(4-methoxybenzyl)-2,6-dioxopiperidin-3-yl trifluoromethanesulfonate (2.2 g, 0.0057 mol) was added, and the mixture was stirred at 0 °C for 0.5 h. The mixture was concentrated under reduced pressure. The residue was purified by silica gel chromatography, eluting with THF/PE (THF from 0% to 25%) over 15 min to afford 3-(5-((tert-butyldimethylsilyl)oxy)-3-methyl-2-oxo-2,3-dihydro-1H-benzoimidazol-1-yl)-1-(4-methoxybenzyl)piperidine-2,6-dione (1.5 g, yield 72%) as a yellow solid. LC-MS: m/z [M+H]⁺ 510.2.

A solution of 3-(5-((tert-butyldimethylsilyl)oxy)-3-methyl-2-oxo-2,3-dihydro-1H-benzo[d]imidazol-1-yl)-1-(4-methoxybenzyl)piperidine-2,6-dione (800 mg, 1.56 mmol) in TFA (15.0 mL) was stirred at 25 °C for 48 h. The mixture was concentrated under reduced pressure. The residue was purified by silica gel chromatography, eluting with THF/PE (THF from 0% to 25%) over 10 min to afford 3-(5-hydroxy-3-methyl-2-oxo-2,3-dihydro-1H-benzo[d]imidazol-1-yl)-1-(4-methoxybenzyl)piperidine-2,6-dione (450 mg, yield 65%) as a white solid. LC-MS: m/z [M+H]⁺ 396.4.

To a solution of 3-(5-hydroxy-3-methyl-2-oxo-2,3-dihydro-1H-benzo[d]imidazol-1-yl)-1-(4-methoxybenzyl)piperidine-2,6-dione (450 mg, 1.138 mmol) in DCM (10.0 mL) were added TEA (345 mg, 3.41 mmol) and Tf₂O (963 mg, 3.41 mmol) at 0 °C. The mixture was stirred at 25 °C for 2 h. The mixture was concentrated under reduced pressure. The residue was purified by silica gel chromatography, eluting with THF/PE (THF from 0% to 30%) over 10 min to afford 1-(1-(4-methoxybenzyl)-2,6-dioxopiperidin-3-yl)-3-methyl-2-oxo-2,3-dihydro-1H-benzo[d]imidazol-5-yl trifluoromethanesulfonate (450 mg, yield 71%) as a white solid. LC-MS: m/z [M+H]⁺ 528.0.

To a solution of 1-(1-(4-methoxybenzyl)-2,6-dioxopiperidin-3-yl)-3-methyl-2-oxo-2,3-dihydro-1H-benzo[d]imidazol-5-yl trifluoromethanesulfonate (450 mg, 0.853 mmol) in 1,4-dioxane (10.0 mL) were added 4-(dimethoxymethyl)piperidine (272 mg, 1.70 mmol), Cs₂CO₃ (556 mg, 1.70 mmol) and XPhos Pd G3 (72.0 mg, 0.085 mmol). The reaction solution was heated to 110 °C and stirred at this temperature under N₂ protection for 12 h. The mixture was cooled to 25 °C and concentrated under reduced pressure. The residue was purified by silica gel chromatography, eluting with THF/PE (THF from 0% to 40%) over 15 min to afford 3-(5-(4-(dimethoxymethyl)piperidin-1-yl)-3-methyl-2-oxo-2,3-dihydro-1H-benzoimidazol-1-yl)-1-(4-methoxybenzyl)piperidine-2,6-dione (250 mg, yield 49%) as a yellow oil. LC-MS: m/z [M+H]⁺ 537.2.

3-(5-(4-(dimethoxymethyl)piperidin-1-yl)-3-methyl-2-oxo-2,3-dihydro-1H-benzo[d]imidazol-1-yl)-1-(4-methoxybenzyl)piperidine-2,6-dione (150 mg, 0.279 mmol) was dissolved in TFA (1.0 mL) and DCM (4.0 mL), and stirred at 25 °C for 1 h. The mixture was concentrated under reduced pressure to afford 1-(1-(4-methoxybenzyl)-2,6-dioxopiperidin-3-yl)-3-methyl-2-oxo-2,3-dihydro-1H-benzo[d]imidazol-5-yl)piperidine-4-formaldehyde (150 mg, yield 88%) as a yellow oil. LC-MS: m/z [M+H]⁺ 491.1.

To a solution of 1-(1-(4-methoxybenzyl)-2,6-dioxopiperidin-3-yl)-3-methyl-2-oxo-2,3-dihydro-1H-benzo[d]imidazol-5-yl)piperidine-4-formaldehyde (150 mg, 0.305 mmol) in DMSO (5.0 mL) were added 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (124 mg, 0.244 mmol) and AcOH (18 mg, 0.305 mmol) at 25 °C. The mixture was stirred at 25 °C for 1 h. Then NaBH(OAc)₃ (162 mg, 0.764 mmol) was added, and the mixture was stirred at 25 °C for 11 h. The reaction mixture was diluted with water (25 mL) and extracted with EtOAc (20 mL×2). The combined organic layers were washed with brine (50 mL), dried over Na₂SO₄, filtered and concentrated under reduced pressure. The residue was purified by prep-TLC (DCM/MeOH=10:1) to afford 3-(5-((4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)methyl)piperidin-1-yl)-3-methyl-2-oxo-2,3-dihydro-1H-benzo[d]imidazol-1-yl)-1-(4-methoxybenzyl)piperidine-2,6-dione (110 mg, yield 33%) as a yellow solid. LC-MS: m/z [M/2+H]⁺ 490.8.

A solution of 3-(5-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidin-1-yl)-3-methyl-2-oxo-2,3-dihydro-1H-benzo[d]imidazol-1-yl)-1-(4-methoxybenzyl)piperidine-2,6-dione (110 mg, 0.112 mmol) in TFA (3.0 mL) and TfOH (0.3 mL) was heated to 60 °C and stirred at this temperature for 3 h. The reaction mixture was cooled to 25 °C and concentrated under reduced pressure. The residue was purified by Prep-HPLC (ACN from 30% to 60% over 7 min) to afford 3-(5-(4-(2-acetyl-7-(7-(difluoromethyl)-6-(1-methyl-1H-pyrazol-4-yl)-3,4-dihydroquinolin-1(2H)-yl)isoindol-5-yl)piperidin-1-yl)methyl)piperidin-1-yl)-3-methyl-2-oxo-2,3-dihydro-1H-benzo[d]imidazol-1-yl)piperidine-2,6-dione (10.5 mg, yield 11%) as a white solid.

¹H NMR (400 MHz, DMSO-*d₆*) δ 11.06 (s, 1H), 7.75 (s, 1H), 7.49 (s, 1H), 7.17 (d, *J* = 8.0 Hz, 1H), 7.12 (d, *J =* 12.0 Hz, 2H), 6.92 (d, *J =* 8.0 Hz, 1H), 6.82 (s, 1H), 6.76 - 6.60 (m, 2H), 6.42 (d, *J* = 28.0 Hz, 1H), 5.31 - 5.26 (m, 1H), 4.75 (d, *J =* 84.0 Hz, 3H), 4.29 (s, 1H), 3.86 (s, 3H), 3.58 (d, *J* = 16.0 Hz, 4H), 3.30 (s, 3H), 3.06 - 2.83 (m, 6H), 2.65 - 2.59 (m, 6H), 2.31 (d, *J =* 16.0 Hz, 2H), 2.04 - 1.98 (m, 6H), 1.81 (d, *J* = 12.0 Hz, 4H), 1.70 (s, 3H), 1.26 (d, *J* = 9.0 Hz, 2H). ¹⁹F NMR (400MHz, DMSO-*d₆*) δ -108.12. LC-MS: m/z [M+H]⁺ 860.4.

### Example 2-49 (Synthesis of 2069)

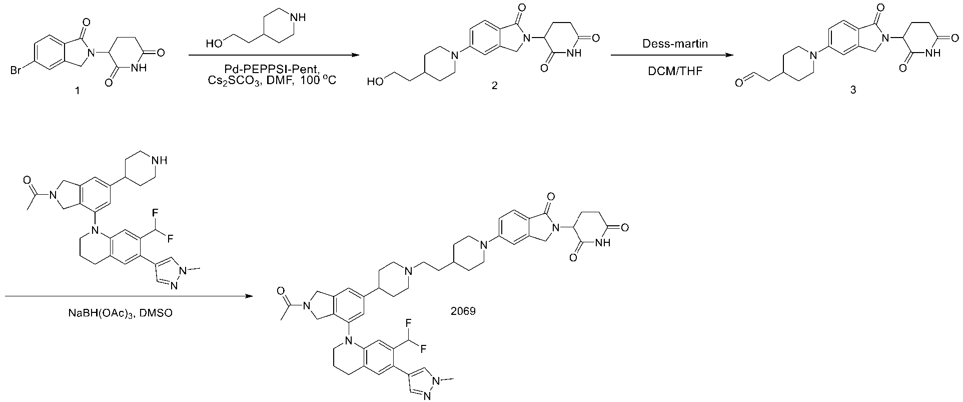

To a solution of 3-(5-bromo-1-oxoisoindolin-2-yl)piperidine-2,6-dione (200 mg, 0.62 mmol) and 2-(piperidin-4-yl)ethan-1-ol (160 mg, 1.24 mmol) in DMF (6 mL) were added Pd-PEPPSI-IPent (52 mg, 0.062 mmol) and Cs₂CO₃ (605 mg, 1.86 mmol). Then the mixture was stirred at 100 °C under N₂ atmosphere for 2 h. After filtration, the filtrate was concentrated under reduced pressure and purified by silica gel chromatography (MeOH/DCM from 0% to 5%) to afford 3-(5-(4-(2-hydroxyethyl)piperidin-1-yl)-1-oxoisoindolin-2-yl)piperidine-2,6-dione (100 mg, yield 44%) as a white solid. LC-MS: m/z 372.2 [M+H]⁺.

To a solution of 3-(5-(4-(2-hydroxyethyl)piperidin-1-yl)-1-oxoisoindolin-2-yl)piperidine-2,6-dione (90 mg, 0.24 mmol) in DCM (2 mL) and DMSO (1 mL) was added Dess-Martin periodinane (123 mg, 0.29 mmol). Then the mixture was stirred at 25 °C for 2 h. The solution was purified by prep-TLC (MeOH/DCM=10%) to afford 2-(1-(2-(2,6-dioxopiperidin-3-yl)-1-oxoisoindolin-5-yl)piperidin-4-yl)acetaldehyde (50 mg, yield 56%) as a yellow solid. LC-MS: m/z 370.2 [M+H]⁺.

To a solution of 2-(1-(2-(2,6-dioxopiperidin-3-yl)-1-oxoisoindolin-5-yl)piperidin-4-yl)acetaldehyde (50 mg, 0.14 mmol) and 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (68 mg, 0.14 mmol) in DMSO (2 mL) was added NaBH(OAc)₃ (43 mg, 0.20 mmol). Then the mixture was stirred at 25 °C for 1 h. The solution was purified by Prep-HPLC (column: WELCH Xtimate C18 21.2*250 mm 10 µm; mobile phase: ACN-H₂O (0.1% TFA); from 32% to 42% over 9 min; flow rate: 25 mL/min) to afford 3-(5-(4-(4-(2-acetyl-7-(difluoromethyl)-6-(1-methyl-1H-pyrazol-4-yl)-3,4-dihydroquinolin-1(2H)-yl)isoindol-5-yl)piperidin-1-yl)ethyl)piperidin-1-yl)-1-oxoisoindolin-2-yl)piperidine-2,6-dione (28.8 mg, yield 24%) as a yellow solid.

¹H NMR (400 MHz, DMSO-*d₆*) δ 10.95 (s, 1H), 7.75 (s, 1H), 7.54 - 7.48 (m, 2H), 7.17 - 7.11 (m, 3H), 7.10 - 7.05 (m, 2H), 6.89 (s, 0.31H), 6.75 (s, 0.63H), 6.61 (s, 0.39H), 6.47 - 6.35 (m, 1H), 5.04 (dd, J = 13.4, 5.2 Hz, 1H), 4.87 (s, 1H), 4.72 - 4.57 (m, 2H), 4.36 - 4.27 (m, 2H), 4.23 - 4.16 (m, 1H), 3.92 - 3.84 (m, 5H), 3.66 - 3.52 (m, 4H), 3.19 - 3.11 (s, 2H), 3.09 - 2.98 (m, 2H), 2.97 - 2.76 (m, 6H), 2.63 - 2.55 (m, 1H), 2.42 - 2.33 (m, 1H), 2.10 - 1.98 (m, 8H), 1.91 - 1.82 (m, 2H), 1.81 - 1.73 (m, 2H), 1.70 - 1.52 (m, 3H), 1.34 - 1.21 (m, 2H). ¹⁹F NMR (400 MHz, DMSO-*d₆*) δ -108.1. LC-MS: m/z 859.4 [M+H]⁺.

### Example 2-50 (Synthesis of 2070)

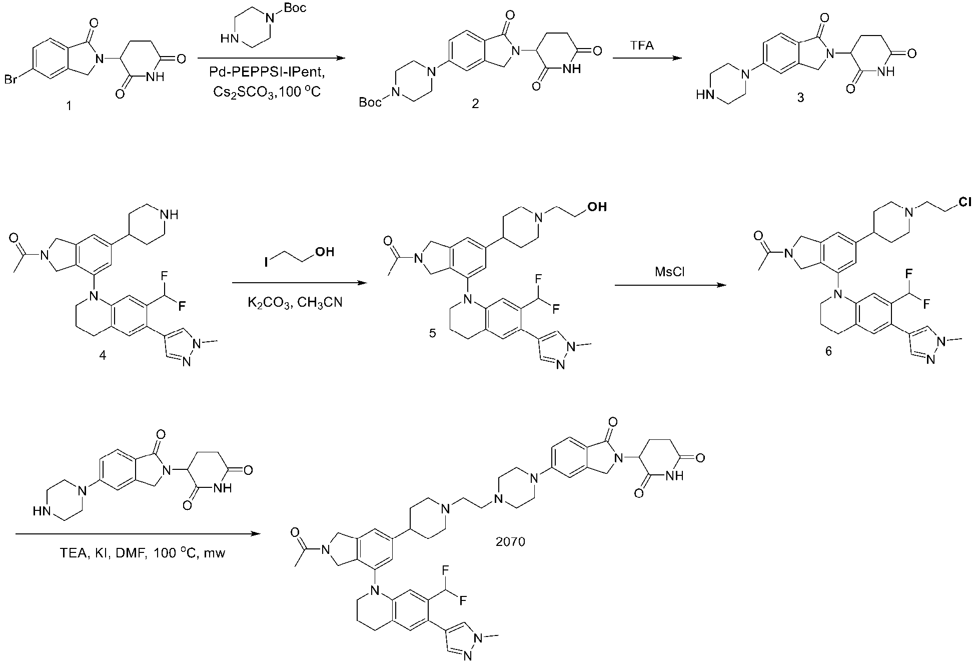

To a solution of 3-(5-bromo-1-oxo-3H-isoindol-2-yl)piperidine-2,6-dione (200 mg, 0.6 mmol) in DMF (2.0 mL) were added tert-butyl piperazine-1-carboxylate (231.8 mg, 1.2 mmol), Pd-PEPPSI-IPent (52.0 mg, 0.06 mol) and Cs₂CO₃ (605.0 mg, 1.86 mmol). Then the mixture was stirred at 100 °C for 2 h. LCMS showed consumption of starting material and detection of the desired product. The residue was purified by flash column chromatography, eluting with 0% to 50% PE in THF, then by flash column chromatography, eluting with 0% to 15% methanol in dichloromethane to afford tert-butyl 4-(2-(2,6-dioxopiperidin-3-yl)-1-oxoisoindolin-5-yl)piperazine-1-carboxylate (250 mg, yield 94%) as a white solid. LC-MS: m/z [M+H]⁺ 429.2.

To a solution of tert-butyl 4-(2-(2,6-dioxopiperidin-3-yl)-1-oxoisoindolin-5-yl)piperazine-1-carboxylate (300 mg, 0.70 mmol) in DCM (2.0 mL) was added TFA (2.0 mL). Then the mixture was stirred at 25 °C for 2 h. LCMS showed consumption of starting material and detection of the desired product. The resulting mixture was concentrated under reduced pressure to give 3-(1-oxo-5-(piperazin-1-yl)isoindol-2-yl)piperidine-2,6-dione (250 mg, yield 98%) as a yellow oil. LC-MS: m/z [M+H]⁺ 329.2.

To a solution of 1-(4-(7-(difluoromethyl)-6-(1-methyl-1H-pyrazol-4-yl)-3,4-dihydroquinolin-1(2H)-yl)-6-(piperidin-4-yl)isoindol-2-yl)ethan-1-one (500 mg, 0.99 mmol) in ACN (4.0 mL) were added 2-iodoethan-1-ol (850.5 mg, 4.9 mmol) and K₂CO₃ (410.0 mg, 2.97 mmol). Then the mixture was stirred at 60 °C for 2 h. The residue was purified by flash column chromatography, eluting with 0% to 50% EtOAc in PE, then by flash column chromatography, eluting with 0% to 10% methanol in dichloromethane to afford 1-(4-(7-(difluoromethyl)-6-(1-methyl-1H-pyrazol-4-yl)-3,4-dihydroquinolin-1(2H)-yl)-6-(1-(2-hydroxyethyl)piperidin-4-yl)isoindolin-2-yl)ethan-1-one (430 mg, yield 79%) as a yellow solid. LC-MS: m/z [M+H]⁺ 550.3.

To a solution of 1-(4-(7-(difluoromethyl)-6-(1-methyl-1H-pyrazol-4-yl)-3,4-dihydroquinolin-1(2H)-yl)-6-(1-(2-hydroxyethyl)piperidin-4-yl)isoindol-2-yl)ethan-1-one (430 mg, 0.78 mmol) in DCM (4.0 mL) were added TEA (158.3 mg, 1.56 mmol) and MsCl (134.4 mg, 1.17 mmol). Then the mixture was stirred at 25 °C for 30 min. The reaction was quenched by addition of saturated NaHCO₃ (30 mL), extracted with DCM (30 mL×3), and dried over anhydrous Na₂SO₄. After filtration, the filtrate was concentrated under reduced pressure. The residue was purified by flash column chromatography, eluting with 0% to 10% EtOAc in PE, then by flash column chromatography, eluting with 0% to 10% DCM in MeOH to afford 1-(6-(1-(2-chloroethyl)piperidin-4-yl)-4-(7-(difluoromethyl)-6-(1-methyl-1H-pyrazol-4-yl)-3,4-dihydroquinolin-1(2H)-yl)isoindolin-2-yl)ethan-1-one (300 mg, yield 61%) as a yellow solid. LC-MS: m/z [M+H]⁺ 568.1.

To a solution of 1-(6-(1-(2-chloroethyl)piperidin-4-yl)-4-(7-(difluoromethyl)-6-(1-methyl-1H-pyrazol-4-yl)-3,4-dihydroquinolin-1(2H)-yl)isoindol-2-yl)ethan-1-one (40.0 mg, 0.07 mmol) in DMF (1.0 mL) were added 3-(1-oxo-5-(piperazin-1-yl)isoindol-2-yl)piperidine-2,6-dione (23.1 mg, 0.07 mmol), TEA (142.5 mg, 1.41 mmol) and KI (23.4 mg, 0.14 mmol) under N₂. The reaction mixture was irradiated at 100 °C in a microwave reactor for 1 h. LCMS showed consumption of starting material and detection of the desired product. The crude product was purified by prep-HPLC, eluting with 35% to 65% ACN in H₂O (0.1% NH₄HCO₃) over 8 min to afford 3-(5-(4-(2-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)piperazin-1-yl)-1-oxoisoindolin-2-yl)piperidine-2,6-dione (2.9 mg, yield 5%) as a white solid.

¹H NMR (400 MHz, DMSO-*d₆*) δ 10.94 (s, 1H), 7.74 (s, 1H), 7.53 - 7.49 (m, 2H), 7.23 - 7.13 (m, 1H), 7.12 - 7.09 (m, 2H), 7.06 - 7.03 (m, 2H), 6.74 (t, *J =* 55.2 Hz, 1H), 6.48 - 6.31 (m, 1H), 5.05 - 5.03 (m, 1H), 4.85 (s, 1H), 4.64 (s, 1H), 4.32 (d, *J* = 16.8 Hz, 1H), 4.20 (d, *J* = 17.2 Hz, 1H), 3.86 (s, 3H), 3.55 (s, 2H), 3.38 - 3.36 (m, 2H), 3.30 - 3.18 (m, 6H), 3.02 - 2.99 (m, 2H), 2.88 - 2.86 (m, 2H), 2.61 - 2.59 (m, 1H), 2.59 - 2.54 (m, 6H), 2.43 - 2.35 (m, 4H), 2.03 - 1.98 (m, 7H), 1.78 - 1.73 (m, 2H), 1.70 - 1.55 (m, 2H). ¹⁹F NMR (376 MHz, DMSO-*d₆*) δ -108.11. LC-MS: m/z [M+H]⁺ 860.3.

### Example 2-51 (Synthesis of 2071)

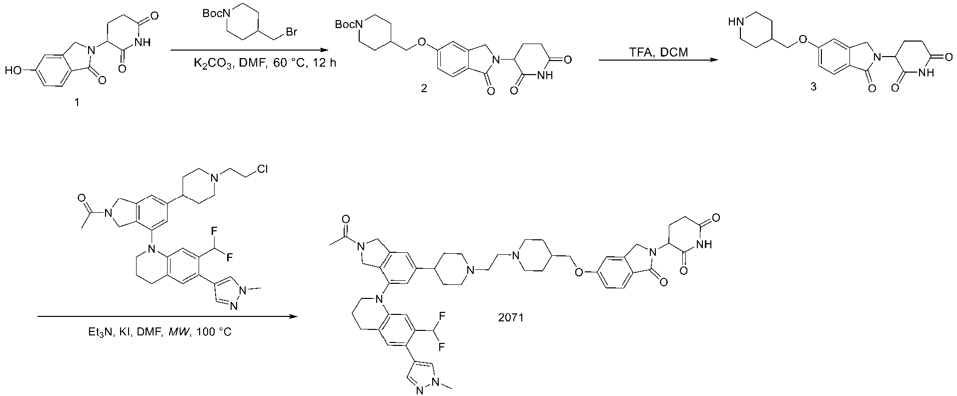

To a solution of 3-(5-hydroxy-1-oxo-3H-isoindol-2-yl)piperidine-2,6-dione (700 mg, 2.69 mmol) in DMF (10.0 mL) were added tert-butyl 4-(bromomethyl)piperidine-1-carboxylate (1122 mg, 4.03 mmol) and K₂CO₃ (744 mg, 5.38 mmol). The reaction solution was heated to 60 °C and stirred at this temperature for 12 h. The reaction mixture was cooled to 25 °C, filtered to remove solids and concentrated. The residue was purified by silica gel chromatography, eluting with MeOH/DCM (MeOH from 0% to 10%) over 15 min to afford tert-butyl 4-((2-(2,6-dioxopiperidin-3-yl)-1-oxoisoindolin-5-yl)oxy)methyl)piperidine-1-carboxylate (200 mg, yield 15%) as a white solid. LC-MS: m/z [M+Na]⁺ 480.0.

A solution of tert-butyl 4-((2-(2,6-dioxopiperidin-3-yl)-1-oxoisoindolin-5-yl)oxy)methyl)piperidine-1-carboxylate (80.0 mg, 0.174 mmol) in DCM (4.0 mL) and TFA (1 mL) was stirred at 25 °C for 1 h. The reaction mixture was concentrated under reduced pressure to afford 3-(1-oxo-5-(piperidin-4-ylmethoxy)isoindolin-2-yl)piperidine-2,6-dione (80.0 mg, yield 89%) as a yellow oil. LC-MS: m/z [M+H]⁺ 358.1.

To a solution of 3-(1-oxo-5-(piperidin-4-ylmethoxy)isoindol-2-yl)piperidine-2,6-dione (80.0 mg, 0.223 mmol) in DMF (2.0 mL) were added 1-(6-(1-(2-chloroethyl)piperidin-4-yl)-4-(7-(difluoromethyl)-6-(1-methyl-1H-pyrazol-4-yl)-3,4-dihydroquinolin-1(2H)-yl)isoindolin-2-yl)ethan-1-one (165 mg, 0.290 mmol), Et₃N (68.0 mg, 0.671 mmol) and KI (7.0 mg, 0.044 mmol). The reaction solution was heated to 100 °C and stirred at this temperature for 1 h. The reaction mixture was cooled to 25 °C, diluted with water (25 mL) and extracted with EtOAc (20 mL×2). The combined organic layers were washed with brine (50 mL), dried over Na₂SO₄, filtered and concentrated under reduced pressure. The residue was purified by Prep-HPLC (ACN from 30% to 60% over 7 min) to afford 3-(5-((1-(2-((4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)ethyl)piperidin-4-yl)methoxy)-1-oxoisoindolin-2-yl)piperidine-2,6-dione (3.9 mg, yield 2%) as a white solid.

¹H NMR (400 MHz, DMSO-*d₆*) δ 10.94 (s, 1H), 7.74 (s, 1H), 7.61 (d, *J* = 8.0 Hz, 1H), 7.49 (s, 1H), 7.16 - 7.09 (m, 4H), 7.04 (d, *J =* 8.0 Hz, 1H), 6.88 - 6.60 (m, 1H), 6.41 (d, *J* = 28.0 Hz, 1H), 5.08 - 5.03 (m, 1H), 4.75 (d, *J =* 84.0 Hz, 3H), 4.38 (d, *J =* 20.0 Hz, 1H), 4.25 (d, *J* = 20.0 Hz, 2H), 3.91 (d, *J =* 8.0 Hz, 2H), 3.86 (s, 3H), 3.54 (s, 2H), 2.96 - 2.88 (m, 7H), 2.68 - 2.53 (m, 2H), 2.42 (s, 4H), 2.36 - 2.30 (m, 1H), 2.05 - 1.90 (m, 10H), 1.74 (s, 4H), 1.68 - 1.55 (m, 2H), 1.36 - 1.22 (m, 2H). ¹⁹F NMR (400MHz, DMSO-*d₆*) δ -108.10. LC-MS: m/z [M+H]⁺ 889.3.

### Example 2-52 (Synthesis of 2072)

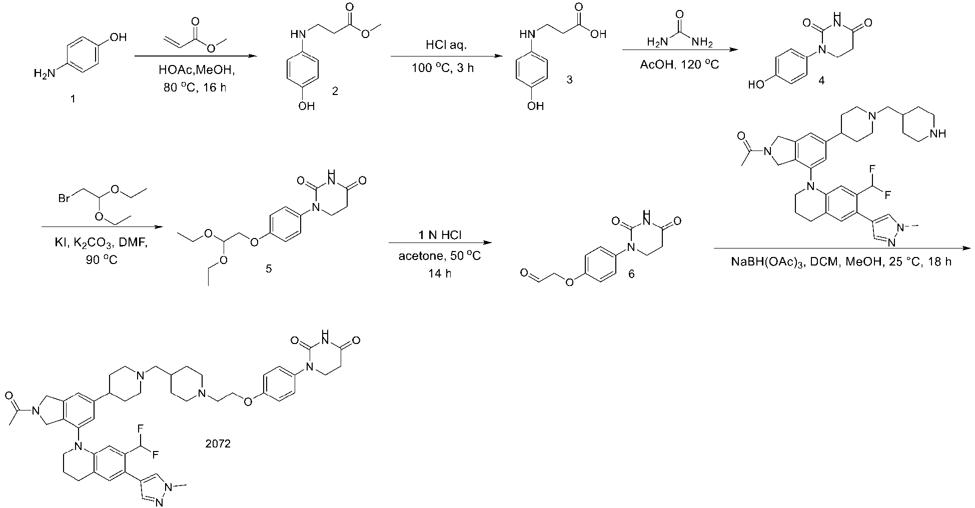

To a solution of 4-aminophenol (5.00 g, 0.0460 mol) in AcOH (0.5 mL) and MeOH (50.0 mL) was added methyl acrylate (4.00 g, 0.0460 mol) at 25 °C. The reaction mixture was stirred at 80 °C for 16 h. The mixture was cooled to room temperature, then concentrated under reduced pressure. The residue was purified by flash chromatography, eluting with 0% to 10% methanol in DCM to afford methyl 3-((4-hydroxyphenyl)amino)propanoate (4.50 g, yield 50%) as a brown solid. LC-MS: (ESI) m/z [M+H] ⁺ 196.3.

A solution of methyl 3-((4-hydroxyphenyl)amino)propanoate (3.90 g, 0.0200 mol) in hydrochloric acid (30.0 mL, 0.180 mol) was stirred at 100 °C for 3 h. The mixture was cooled to room temperature, then concentrated under reduced pressure to afford 3-((4-hydroxyphenyl)amino)propanoic acid (3.90 g, crude) as a black oil. LC-MS: (ESI) m/z [M+H] ⁺ 182.2.

To a solution of 3-((4-hydroxyphenyl)amino)propanoic acid (3.90 g, crude) in AcOH (25.0 mL) was added urea (1.94 g, 0.0322 mol) at 25 °C. The mixture was stirred at 120 °C for 16 h. The mixture was cooled to room temperature, then concentrated under reduced pressure. The residue was purified by silica gel chromatography, eluting with 0% to 15% MeOH/DCM over 30 min to afford 1-(4-hydroxyphenyl)dihydropyrimidine-2,4(1H,3H)-dione (1.34 g, yield 27%) as a brown solid. LC-MS: (ESI) m/z [M+H] ⁺ 207.1.

To a solution of 1-(4-hydroxyphenyl)dihydropyrimidine-2,4(1H,3H)-dione (200 mg, 0.970 mmol) in DMF (15.0 mL) were added K₂CO₃ (402 mg, 2.91 mmol), KI (177 mg, 1.07 mmol) and 2-bromo-1,1-diethoxyethane (382 mg, 1.94 mmol) at 25 °C. The mixture was stirred at 90 °C for 14 h. The mixture was cooled to 0 °C, then diluted with EtOAc (30.0 mL). The mixture was added to aqueous NH₄Cl (60.0 mL), then extracted with EtOAc (30.0 mL×3). The combined organic layers were washed with saturated brine (20.0 mL×3), and dried. The organic layer was concentrated under reduced pressure, and the residue was purified by silica gel chromatography, eluting with 0% to 85% EtOAc in PE over 20 min to afford 1-(4-(2,2-diethoxyethoxy)phenyl)dihydropyrimidine-2,4(1H,3H)-dione (105 mg, yield 30%) as a colorless oil. LC-MS: (ESI) m/z [M+Na] ⁺ 345.1.

To a solution of 1-(4-(2,2-diethoxyethoxy)phenyl)dihydropyrimidine-2,4(1H,3H)-dione (300 mg, 0.310 mmol) in acetone (15.0 mL) was added HCl (15.0 mL, 15.0 mmol) at 25 °C. The mixture was stirred at 50 °C for 14 h. The mixture was cooled to room temperature, then concentrated under reduced pressure to afford 2-(4-(2,4-dioxotetrahydropyrimidin-1(2H)-yl)phenoxy)acetaldehyde (240 mg, crude) as a white solid. LC-MS: (ESI) m/z [M+H] ⁺ 249.1.

To a solution of 2-(4-(2,4-dioxotetrahydropyrimidin-1(2H)-yl)phenoxy)acetaldehyde (49.4 mg, 0.199 mmol) in DCM/MeOH (9.0 mL/1.0 mL) was added 1-(4-(7-(difluoromethyl)-6-(1-methyl-1H-pyrazol-4-yl)-3,4-dihydroquinolin-1(2H)-yl)-6-(1-(piperidin-4-ylmethyl)piperidin-4-yl)isoindolin-2-yl)ethan-1-one (60.0 mg, 0.0995 mmol) at 25 °C. The mixture was stirred at 25 °C for 2 h, then NaBH(OAc)₃ (63.3 mg, 0.299 mmol) was added. The reaction solution was stirred at 25 °C for 16 h. The mixture was concentrated, and the residue was purified by Prep-TLC (DCM:MeOH=85:15) to afford a crude product. The crude product was purified by Prep HPLC (column: WELCH Xtimate C18 21.2*250 mm 10 µm; mobile phase: ACN-H₂O, 45 to 75% ACN in 0.1% NH₄HCO₃/water, 30 mL/min) to afford 1-(4-(2-(4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)methyl)piperidin-1-yl)ethoxy)phenyl)dihydropyrimidine-2,4(1H,3H)-dione (15.6 mg, yield 18%) as a white solid.

¹H NMR (400 MHz, DMSO) δ 10.30 (s, 1H), 7.74 (s, 1H), 7.49 (s, 1H), 7.25-7.08 (m, 5H), 6.94 (d, *J =* 8.8 Hz, 2H), 6.74 (t, *J =* 55.2 Hz, 1H), 6.45-6.36 (m, 1H), 4.85 (s, 1H), 4.64 (s, 1H), 4.28 (s, 1H), 4.09-4.04 (m, 2H), 3.86 (s, 3H), 3.71 (t, *J =* 6.6 Hz, 2H), 3.56-3.52 (m, 2H), 2.99-2.82 (m, 8H), 2.72 - 2.65 (m, 4H), 2.15 - 1.98 (m, 9H), 1.75-1.61 (m, 6H), 1.50 (s, 2H), 1.18-1.05 (m, 3H).

¹⁹F NMR (400 MHz, DMSO) δ -108.09. LC-MS: (ESI) m/z [M+H] ⁺ 835.4.

### Example 2-53 (Synthesis of 2074)

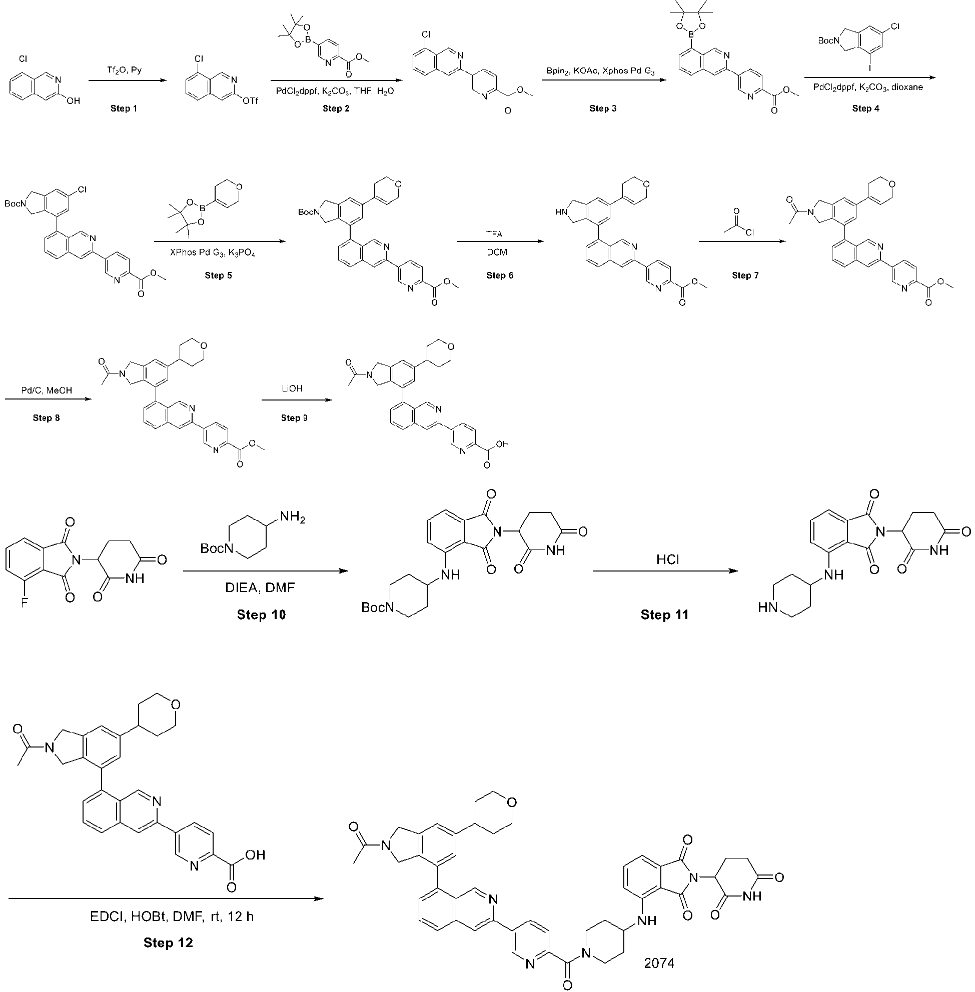

A solution of 8-chloroisoquinolin-3-ol (1.04 g, 5.8 mmol) in dry pyridine (40 mL) was cooled to 0 °C, and added trifluoromethanesulfonic anhydride (2.45 g, 8.7 mmol). The mixture was warmed to 25 °C and reacted for 12 h. The solvent was concentrated under reduced pressure, and the crude product was purified by silica gel chromatography using petroleum ether-THF (3:1) as eluent to afford 8-chloroisoquinolin-3-yl trifluoromethanesulfonate (0.8 g, yield 45%) as a yellow solid. LC-MS: [M+H]⁺ 312.0.

A solution of methyl 8-chloroisoquinolin-3-yl trifluoromethanesulfonate (800 mg, 2.57 mmol), methyl 5-(4,4,5,5-tetramethyl-1,3,2-dioxaborolan-2-yl)pyridine-2-carboxylate (743 mg, 2.82 mmol), K₂CO₃ (710 mg, 5.13 mmol) and Pd(dppf)Cl₂ (188 mg, 0.257 mmol) in 1,4-dioxane (30 mL) and H₂O (3.0 mL) was reacted at 80 °C for 3 h under nitrogen protection. LCMS showed the desired product. The mixture was concentrated under reduced pressure. The residue was purified by SGC (THF/PE=1/1) to afford methyl 5-(8-chloroisoquinolin-3-yl)pyridine carboxylate (660 mg, yield 82%) as a yellow solid. LC-MS: [M+H]⁺ 299.1.

A solution of methyl 5-(8-chloroisoquinolin-3-yl)pyridine carboxylate (400 mg, 1.34 mmol), bis(pinacolato)diboron (442 mg, 1.74 mmol), XPhos-Pd G3 (113 mg, 0.134 mmol) and KOAc (263 mg, 2.68 mmol) in 1,4-dioxane (20 mL) was heated to 90 °C for 5 h. LCMS showed the desired product, which was used directly in the next step. LC-MS: (ESI) m/z [M+H]⁺ 391.1.

A solution of methyl 5-(8-(4,4,5,5-tetramethyl-1,3,2-dioxaborolan-2-yl)isoquinolin-3-yl)pyridine carboxylate (510 mg, 1.31 mmol), tert-butyl 6-chloro-4-iodo-1,3-dihydroisoindole-2-carboxylate (645 mg, 1.70 mmol), Pd(dppf)Cl₂ (96 mg, 0.13 mmol) and K₃PO₄ (832 mg, 3.92 mmol) in 1,4-dioxane (30 mL) and H₂O (3.0 mL) was reacted at 80 °C for 2 h under nitrogen protection. LCMS showed the desired product. The mixture was concentrated under reduced pressure, and the residue was purified by SGC (THF/PE=1/1) to afford tert-butyl 6-chloro-4-(3-(6-(methoxycarbonyl)pyridin-3-yl)isoquinolin-8-yl)isoindoline-2-carboxylate (288 mg, yield 32%) as a yellow solid. LC-MS: (ESI) m/z [M+H]⁺ 516.2.

A solution of tert-butyl 6-chloro-4-(3-(6-(methoxycarbonyl)pyridin-3-yl)isoquinolin-8-yl)isoindoline-2-carboxylate (288 mg, 0.558 mmol), 2-(3,6-dihydro-2H-pyran-4-yl)-4,4,5,5-tetramethyl-1,3,2-dioxaborolane (141 mg, 0.67 mmol), XPhos Pd G3 (47.3 mg, 0.056 mmol) and K₃PO₄ (356 mg, 1.67 mmol) in 1,4-dioxane (10 mL) and H₂O (1.0 mL) was reacted at 80 °C for 5 h under nitrogen protection. The mixture was concentrated under reduced pressure, and the residue was purified by silica gel chromatography (DCM/MeOH=15/1) to afford tert-butyl 6-(3,6-dihydro-2H-pyran-4-yl)-4-(3-(6-(methoxycarbonyl)pyridin-3-yl)isoquinolin-8-yl)isoindoline-2-carboxylate (210 mg, yield 60%) as a yellow solid. LC-MS: (ESI) m/z [M+H]⁺ 564.3.

To a stirred solution of tert-butyl 6-(3,6-dihydro-2H-pyran-4-yl)-4-(3-(6-(methoxycarbonyl)pyridin-3-yl)isoquinolin-8-yl)isoindoline-2-carboxylate (210 mg, 0.373 mmol) in DCM (1.0 mL) was added TFA (1.0 mL) at 25 °C. After addition, the mixture was stirred at this temperature for 30 min. LCMS showed complete conversion. The mixture was concentrated under reduced pressure to afford methyl 5-(8-(6-(3,6-dihydro-2H-pyran-4-yl)isoindolin-4-yl)isoquinolin-3-yl)pyridine carboxylate (180 mg, yield 99%) as a brown solid, which was used directly in the next step without further purification. LC-MS: (ESI) m/z [M+H]⁺ 464.2.

To a solution of methyl 5-(8-(6-(3,6-dihydro-2H-pyran-4-yl)isoindolin-4-yl)isoquinolin-3-yl)pyridine carboxylate (180 mg, 0.388 mmol) in DCM (10 mL) was added TEA (197 mg, 1.94 mmol) at 0 °C, followed by AcCl (45.7 mg, 0.582 mmol), and the mixture was stirred at 25 °C for 30 min. The mixture was concentrated under reduced pressure, and the residue was purified by silica gel chromatography (DCM/MeOH=15/1) to afford methyl 5-(8-(2-acetyl-6-(3,6-dihydro-2H-pyran-4-yl)isoindolin-4-yl)isoquinolin-3-yl)pyridine carboxylate (140 mg, yield 68%) as a yellow solid. LC-MS: (ESI) m/z [M+H]⁺ 506.2.

A solution of methyl 5-(8-(2-acetyl-6-(3,6-dihydro-2H-pyran-4-yl)isoindolin-4-yl)isoquinolin-3-yl)pyridine carboxylate (140 mg, 0.277 mmol) and Pd/C (40.0 mg) in MeOH (10.0 mL) was stirred at 25 °C under H₂ atmosphere for 48 h. The mixture was filtered, and the filtrate was concentrated under reduced pressure to afford methyl 5-(8-(2-acetyl-6-(tetrahydro-2H-pyran-4-yl)isoindolin-4-yl)isoquinolin-3-yl)pyridine carboxylate (110 mg, yield 70%) as a yellow solid. LC-MS: (ESI) m/z [M+H]⁺ 508.2.

To a solution of methyl 5-(8-(2-acetyl-6-(tetrahydro-2H-pyran-4-yl)isoindol-4-yl)isoquinolin-3-yl)pyridine carboxylate (110 mg, 0.217 mmol) in THF (2.0 mL) and H₂O (2.0 mL) was added LiOH·H₂O (18.0 mg, 0.433 mmol). The mixture was stirred at 25 °C for 0.5 h. LCMS showed complete conversion. The mixture was concentrated under reduced pressure and dried in vacuo to afford 5-(8-(2-acetyl-6-(tetrahydro-2H-pyran-4-yl)isoindol-4-yl)isoquinolin-3-yl)pyridine carboxylic acid(78 mg, yield 66%) as a yellow solid, which was used directly in the next step without further purification. LC-MS: (ESI) m/z [M+H]⁺ 494.2.

A solution of 2-(2,6-dioxopiperidin-3-yl)-4-fluoroisoindole-1,3-dione (500 mg, 1.81 mmol), tert-butyl 4-aminopiperidine-1-carboxylate (435 mg, 2.1722 mmol) and DIEA (468 mg, 3.62 mmol) in DMF (8.0 mL) was heated to 90 °C for 17 h. LCMS showed the desired product. The mixture was concentrated under reduced pressure to afford tert-butyl 4-((2-(2,6-dioxopiperidin-3-yl)-1,3-dioxoisoindolin-4-yl)amino)piperidine-1-carboxylate (1 g, yield 73%) as a green oil. LC-MS: (ESI) m/z [M+H]⁺ 457.1.

A solution of tert-butyl 4-((2-(2,6-dioxopiperidin-3-yl)-1,3-dioxoisoindolin-4-yl)amino)piperidine-1-carboxylate (300 mg, 0.657 mmol) in HCl/1,4-dioxane (4 M, 5.0 mL) was stirred at 25 °C for 1 h. LCMS showed consumption of starting material and detection of the desired compound. The reaction mixture was concentrated under reduced pressure and purified by reverse-phase chromatography (ACN-water (0.1% NH₄HCO₃); from 5% to 50%) to afford 2-(2,6-dioxopiperidin-3-yl)-4-(piperidin-4-ylamino)isoindoline-1,3-dione (179 mg, yield 73%) as a yellow solid. LC-MS: m/z [M+H]⁺ 357.2.

To a solution of 5-(8-(2-acetyl-6-(tetrahydro-2H-pyran-4-yl)isoindol-4-yl)isoquinolin-3-yl)pyridine carboxylic acid (78.0 mg, 0.158 mmol), 2-(2,6-dioxopiperidin-3-yl)-4-(piperidin-4-ylamino)isoindoline-1,3-dione (67.6 mg, 0.190 mmol), HOBt (10.7 mg, 0.079 mmol) and DIEA (102 mg, 0.79 mmol) in DMF (3.0 mL) was added EDCI (60.6 mg, 0.316 mmol) at 25 °C. The mixture was stirred at this temperature for 17 h. LCMS showed complete conversion. The mixture was purified by prep-HPLC (column: WELCH Xtimate C18 21.2×250 mm 10 µm; mobile phase: [H₂O (0.1% FA)-ACN]; B%: 40% to 70%, 10 min) to afford 4-((1-(5-(8-(2-acetyl-6-(tetrahydro-2H-pyran-4-yl)isoindolin-4-yl)isoquinolin-3-yl)pyridinecarbonyl)piperidin-4-yl)amino)-2-(2,6-dioxopiperidin-3-yl)isoindoline-1,3-dione (20.6 mg, yield 16%) as a yellow solid.

¹H NMR (400 MHz, DMSO-*d₆*) δ 11.11 (s, 1H), 9.41 (s, 1H), 9.02 (d, *J* = 13.6 Hz, 1H), 8.73 (d, *J =* 2.8 Hz, 1H), 8.68 (dt, *J =* 8.4 Hz 2.0 Hz, 1H), 8.13 (d, *J* = 8.0 Hz, 1H), 7.92 (t, *J* = 7.6 Hz, 1H), 7.75 (dd, *J* = 8.0 Hz 2.8 Hz, 1H), 7.70-7.66 (m, 1H), 7.63-7.59 (m, 1H), 7.42 (d, *J* = 8.0 Hz, 1H), 7.30-7.25 (m, 2H), 7.06 (d, *J* = 7.2 Hz, 1H), 6.33 (d, *J* = 7.6 Hz, 1H), 5.06 (dd, *J* = 12.8 Hz 5.2 Hz, 1H), 4.95 (d, *J* = 7.2 Hz, 1H), 4.79-4.74 (m, 1.5H), 4.50-4.41 (m, 2H), 4.22-4.18 (m, 0.5H), 3.97-3.95 (m, 3H), 3.80 (d, *J* = 12.8 Hz, 1H), 3.47-3.42 (m, 3H), 3.09 (t, *J* = 12.0 Hz, 1H), 2.93-2.85 (m, 2H), 2.61-2.52 (m, 2H), 2.11-2.01 (m, 4H), 1.96-1.88 (m, 2H), 1.78-1.72 (m, 4H), 1.61-1.51 (m, 2H). LC-MS: (ESI) m/z [M+H]⁺ 832.3.

### Example 2-54 (Synthesis of 2079)

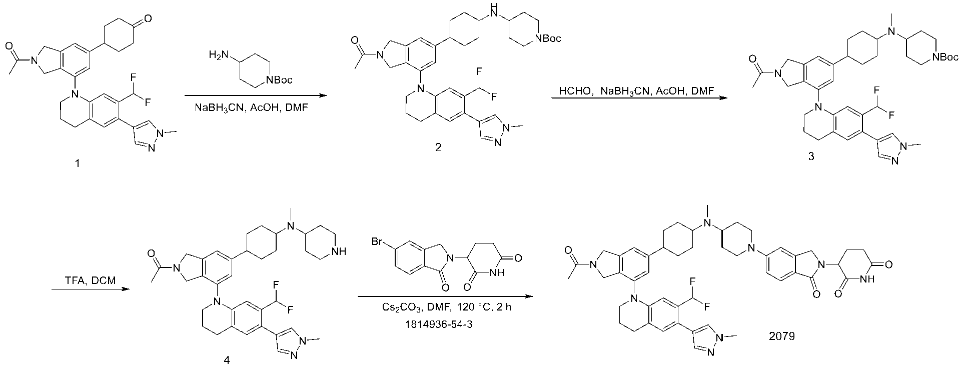

To a solution of 4-(2-acetyl-7-(7-(difluoromethyl)-6-(1-methyl-1H-pyrazol-4-yl)-3,4-dihydroquinolin-1(2H)-yl)isoindol-5-yl)cyclohexanone (170 mg, 0.328 mmol) in DMF (6.0 mL) were added tert-butyl 4-aminopiperidine-1-carboxylate (131 mg, 0.656 mmol) and AcOH (20.0 mg, 0.328 mmol). The reaction solution was stirred at 25 °C for 1 h. Then NaBH₃CN (52.0 mg, 0.820 mmol) was added, and the mixture was stirred at 25 °C for 11 h. The reaction mixture was diluted with water (25 mL) and extracted with EtOAc (20 mL×2). The combined organic layers were washed with brine (50 mL), dried over Na₂SO₄, filtered and concentrated under reduced pressure. The residue was purified by silica gel chromatography, eluting with MeOH/DCM (MeOH from 0% to 5%) over 10 min to afford tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)cyclohexyl)amino)piperidine-1-carboxylate (120 mg, yield 49%) as a yellow solid. LC-MS: m/z [M+H]⁺ 703.4.

To a solution of tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)cyclohexyl)amino)piperidine-1-carboxylate (110 mg, 0.157 mmol) in DMF (6.0 mL) were added HCHO (9.40 mg, 0.313 mmol) and AcOH (9.40 mg, 0.157 mmol). The reaction solution was stirred at 25 °C for 1 h. Then NaBH₃CN (24.6 mg, 0.391 mmol) was added, and the mixture was stirred at 25 °C for 3 h. The reaction mixture was diluted with water (25 mL) and extracted with EtOAc (20 mL×2). The combined organic layers were washed with brine (50 mL), dried over Na₂SO₄, filtered and concentrated under reduced pressure. The residue was purified by silica gel chromatography, eluting with MeOH/DCM (MeOH from 0% to 5%) over 10 min to afford tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)cyclohexyl)(methyl)amino)piperidine-1-carboxylate (75.0 mg, yield 64%) as a yellow solid. LC-MS: m/z [M+H]⁺ 717.4.

To a solution of tert-butyl 4-((4-(2-acetyl-7-(7-(difluoromethyl)-6-(1-methyl-1H-pyrazol-4-yl)-3,4-dihydroquinolin-1(2H)-yl)isoindol-5-yl)cyclohexyl)(methyl)amino)piperidine-1-carboxylate (70.0 mg, 0.098 mmol) in DCM (4.0 mL) was added TFA (2.0 mL). The mixture was stirred at 25 °C for 1 h. The mixture was concentrated under reduced pressure. The residue was washed with NaHCO₃ solution (50 mL) and extracted with DCM (30 mL×2). The combined organic layers were washed with brine (50 mL), dried over Na₂SO₄, filtered and concentrated to dryness. The residue was purified by prep-TLC (DCM:MeOH=10:1) to afford 1-(4-(7-(difluoromethyl)-6-(1-methyl-1H-pyrazol-4-yl)-3,4-dihydroquinolin-1(2H)-yl)-6-(4-(methyl(piperidin-4-yl)amino)cyclohexyl)isoindol-2-yl)ethan-1-one (30.0 mg, yield 45%) as a yellow solid. LC-MS: m/z [M+H]⁺ 617.4.

To a solution of 1-(4-(7-(difluoromethyl)-6-(1-methyl-1H-pyrazol-4-yl)-3,4-dihydroquinolin-1(2H)-yl)-6-(4-(methyl(piperidin-4-yl)amino)cyclohexyl)isoindol-2-yl)ethan-1-one (25.0 mg, 0.041 mmol) in DMF (2.0 mL) were added Cs₂CO₃ (33.0 mg, 0.101 mmol), 3-(5-bromo-1-oxoisoindolin-2-yl)piperidine-2,6-dione (13.1 mg, 0.041 mmol) and Ruphos Pd G4 (6.90 mg, 0.008 mmol). The solution was stirred at 120 °C for 2 h. The mixture was concentrated, and the residue was purified by Prep-HPLC (ACN from 40% to 55% over 9 min) to afford 3-(5-((4-((2-acetyl-7-(7-(difluoromethyl)-6-(1-methyl-1H-pyrazol-4-yl)-3,4-dihydroquinolin-1(2H)-yl)isoindolin-5-yl)cyclohexyl)(methyl)amino)piperidin-1-yl)-1-oxoisoindolin-2-yl)piperidine-2,6-dione (2.7 mg, yield 7%) as a white solid.

¹H NMR (400 MHz, DMSO-*d₆*) δ 10.94 (s, 1H), 7.74 (s, 1H), 7.49 (d, *J* = 4.8 Hz, 2H), 7.17 - 7.10 (m, 2H), 7.08 - 7.02 (m, 3H), 6.87 - 6.60 (m, 1H), 6.43 (d, *J* = 31.8 Hz, 1H), 5.06 - 5.02 (m, 1H), 4.87 (s, 1H), 4.66 (s, 1H), 4.32 (d, *J =* 16.8 Hz, 2H), 4.19 (d, *J* = 17.2 Hz, 1H), 3.92 (s, 2H), 3.86 (s, 3H), 3.54 (s, 2H), 2.93 - 2.82 (m, 6H), 2.71 - 2.58 (m, 3H), 2.39 - 2.29 (m, 2H), 2.08 (d, *J =* 6.0 Hz, 3H), 2.03 - 1.95 (m, 8H), 1.88 - 1.79 (m, 2H), 1.55 (d, *J =* 35.6 Hz, 8H). ¹⁹F NMR (400MHz, DMSO-*d₆*) δ -108.11. LC-MS: m/z [M+H]⁺ 859.4.

### Example 2-55 (Synthesis of 2080)

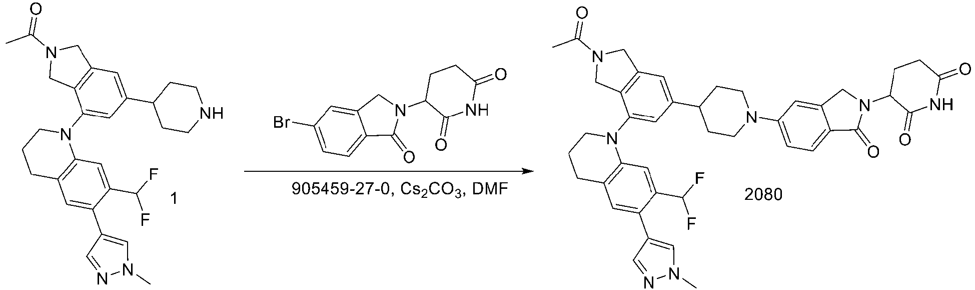

To a solution of 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethene (40 mg, 0.079 mmol) and 3-(4-bromo-1-oxo-3H-isoindol-2-yl)piperidine-2,6-dione (26 mg, 0.079 mmol) in DMF (2 mL) were added Cs₂CO₃ (52 mg, 0.16 mmol) and PEPPSI-IHept-Cl (CAS: 905459-27-0, 15 mg, 0.016 mmol). The mixture was stirred at 130 °C for 2 h. The mixture was purified by prep-HPLC, eluting with 45% to 75% aqueous ACN over 8 min to afford 3-[4-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)-1-oxo-3H-isoindol-2-yl]piperidine-2,6-dione (4.6 mg, yield 8%) as a white solid. MS: m/z [M+H]⁺ 748.3.

¹H NMR (400 MHz, DMSO-*d₆*) δ 10.97 (s, 1H),, 7.75 (d, *J =* 1.6 Hz, 1H), 7.49 (s, 1H), 7.48-7.42 (m, 1H), 7.32 (d, *J* = 7.4 Hz, 1H), 7.25 - 7.12 (m, 4H), 6.88-6.61 (m, 1H), 6.48 - 6.35 (m, 1H), 5.17 - 5.08 (m, 1H), 4.87 (s, 1H), 4.67 (s, 2H), 4.479-4.46(m, 1H), 4.40-4.31 (m, 2H), 3.94 - 3.81 (m, 3H), 3.53 (d, *J* = 27.6 Hz, 4H), 2.95 - 2.73 (m, 6H), 2.58 (d, *J =* 19.6 Hz, 2H), 2.06 - 1.96 (m, 6H), 1.90-1.81 (m, 4H).

### Example 2-56 (Synthesis of 2081)

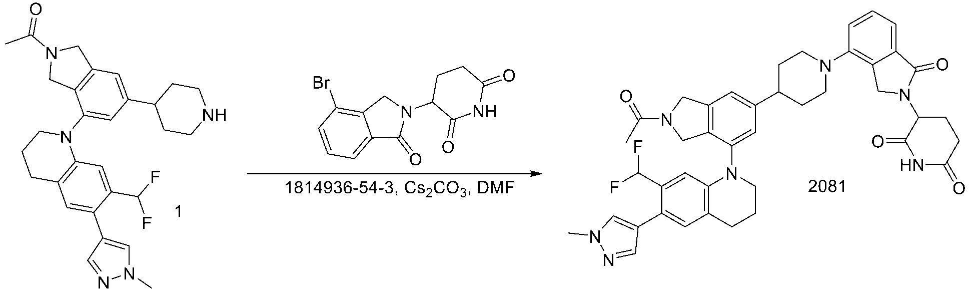

To a solution of 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethene (40 mg, 0.079 mmol) and 3-(5-bromo-1-oxo-3H-isoindol-2-yl)piperidine-2,6-dione (31 mg, 0.095 mmol) in DMF (2 mL) were added Cs₂CO₃ (77 mg, 0.24 mmol) and RuPhos Palladacycle Gen.4 (CAS 1814936-54-3, 20 mg, 0.024 mmol). The mixture was stirred at 130 °C for 2 h. The mixture was purified by prep-HPLC to afford 3-[5-(4-{2-acetyl-7-[7-(difluoromethyl)-6-(1-methylpyrazol-4-yl)-3,4-dihydro-2H-quinolin-1-yl]-1,3-dihydroisoindol-5-yl}piperidin-1-yl)-1-oxo-3H-isoindol-2-yl]piperidine-2,6-dione (6.5 mg, yield 11%) as a white solid. MS: m/z [M+H]⁺ 748.3.

¹H NMR (400 MHz, DMSO-*d₆*) δ 10.95 (s, 1H), 7.74 (d, *J=* 2.4 Hz, 1H), 7.55 - 7.46 (m, 2H), 7.22 - 7.06 (m, 5H), 6.75-6.61 (m, 1H), 6.41 (d, *J* = 28.4 Hz, 1H), 5.07-5.02 (m, 1H), 4.85 (s, 1H), 4.65 (s, 2H), 4.33 (d, *J* = 17.6 Hz, 1H), 4.21 (d, *J* = 16.4 Hz, 1H), 4.02 (d, *J* = 10.6 Hz, 2H), 3.89 - 3.83 (m, 3H), 3.55 (s, 2H), 3.00 - 2.81 (m, 7H), 2.58 (d, *J =* 16.8 Hz, 1H), 2.40 - 2.29 (m, 1H), 2.05 - 1.96 (m, 6H), 1.93-1.86 (m, 2H), 1.75-1.71 (m, 2H).

**Example 2-57 (Synthesis of 2082** 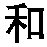 **2083)**

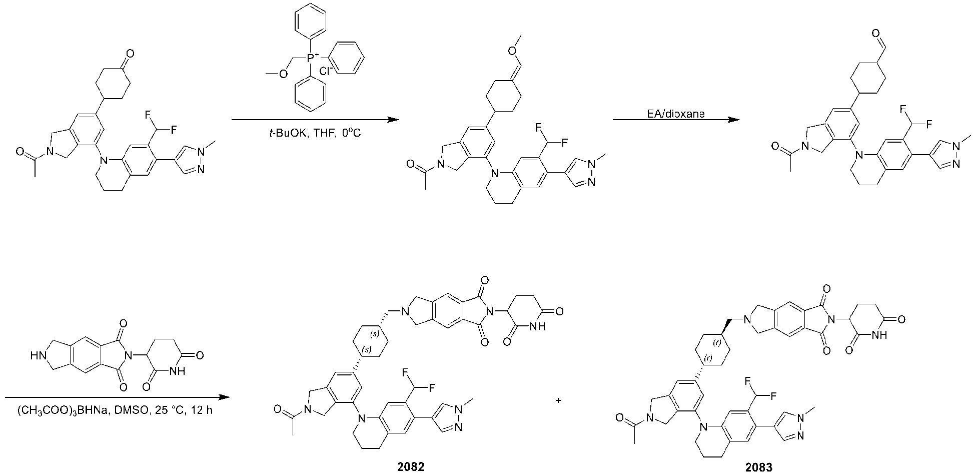

To a solution of (methoxymethyl)triphenylphosphonium chloride (8.23 g, 0.024 mol) in THF (50 mL) was added t-BuOK (2.69 g, 0.024 mol) at 0 °C under N₂ atmosphere. The reaction mixture was stirred at 0 °C for 1 h. 4-(2-acetyl-7-(7-(difluoromethyl)-6-(1-methyl-1H-pyrazol-4-yl)-3,4-dihydroquinolin-1(2H)-yl)isoindolin-5-yl)cyclohexan-1-one (0.6 g, 0.0012 mol) was dissolved in THF (10 mL) and added dropwise to the reaction mixture, which was stirred at 25 °C for 1 h. LCMS showed formation of the desired product. The reaction mixture was quenched with H₂O (50 mL), extracted with EtOAc (50 mL×3), the organic layer was dried over anhydrous Na₂SO₄, filtered and concentrated in vacuo. The residue was purified by pre-HPLC (column: Xbridge-C18 150×19 mm, 5 µm; mobile phase: ACN-H₂O (0.1% FA)) (0 to 37%) to afford 1-(4-(7-(difluoromethyl)-6-(1-methyl-1H-pyrazol-4-yl)-3,4-dihydroquinolin-1(2H)-yl)-6-(4-(methoxymethylene)cyclohexyl)isoindolin-2-yl)ethan-1-one (0.46 g, yield 67%) as a white solid.

1-(4-(7-(difluoromethyl)-6-(1-methyl-1H-pyrazol-4-yl)-3,4-dihydroquinolin-1(2H)-yl)-6-(4-(methoxymethylene)cyclohexyl)isoindolin-2-yl)ethan-1-one (300 mg, 0.5488 mmol) was dissolved in EtOAc/HCl (5 mL). The reaction mixture was stirred at 25 °C for 1 h. LCMS showed formation of the desired product. The solvent was removed under reduced pressure to afford 4-(2-acetyl-7-(7-(difluoromethyl)-6-(1-methyl-1H-pyrazol-4-yl)-3,4-dihydroquinolin-1 (2H)-yl)isoindolin-5-yl)cyclohexane-1-formaldehyde (290 mg, yield 99%) as a crude product, which was used directly without further purification.

To a solution of 4-(2-acetyl-7-(7-(difluoromethyl)-6-(1-methyl-1H-pyrazol-4-yl)-3,4-dihydroquinolin-1(2H)-yl)isoindolin-5-yl)cyclohexane-1-formaldehyde (150 mg, 0.2816 mmol) in DMSO (2 mL) was added 2-(2,6-dioxopiperidin-3-yl)-6,7-dihydropyrrolo[3,4-f]isoindole-1,3(2H,5H)-dione (84.28 mg, 0.2816 mmol), and the reaction mixture was stirred at 25 °C for 2 h. NaBH₄ (12.78 mg, 0.33792 mmol) was added to the reaction mixture, and the mixture was stirred at 25 °C. LCMS showed formation of the desired product. The reaction mixture was purified by pre-HPLC (column: Xbridge-C18 150×19 mm, 5 µm; mobile phase: ACN-H₂O (0.1% FA)) (0 to 40%) to afford 6-(((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)methyl)-2-(2,6-dioxopiperidin-3-yl)-6,7-dihydropyrrolo[3,4-f]isoindole-1,3(2H,5H)-dione (1.3 mg, yield 0.39%) as a white solid, and 6-(((1r,4r)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)methyl)-2-(2,6-dioxopiperidin-3-yl)-6,7-dihydropyrrolo[3,4-f]isoindole-1,3(2H,5H)-dione (12.2 mg, yield 3.98%) as a white solid.

2082: 1H NMR (400 MHz, DMSO-d6) δ 11.19 - 11.04 (m, 1H),7.80 (d, J = 6.4 Hz, 1H), 7.75 (s, 1H), 7.54 (d, J = 33.4 Hz, 1H), 7.26 - 7.10 (m, 2H), 6.74 (t, J = 55.4 Hz, 1H), 6.42 (d, J = 29.2 Hz, 1H), 5.13 (dd, J = 12.6, 5.6 Hz, 1H), 4.90 - 4.46 (m, 3H), 4.30 (s, 1H), 3.99 (s, 2H), 3.86 (s, 3H), 3.56 (s, 2H), 2.89 (s, 3H), 2.82 - 2.51 (m, 9H), 2.07 (t, J = 38.6 Hz, 9H), 1.68 (d, J = 25.6 Hz, 6H).

2083: 1H NMR (400 MHz, DMSO-d6) δ 11.16 - 11.06 (m, 1H), 7.80 (s, 1H), 7.74 (d, J = 4.0 Hz, 1H), 7.49 (d, J = 4.6 Hz, 1H), 7.18 - 7.09 (m, 2H), 6.91 - 6.56 (m, 1H), 6.40 (dd, J = 28.8, 10.0 Hz, 1H), 5.13 (dd, J = 12.8, 5.6 Hz, 1H), 4.92 - 4.48 (m, 3H), 4.29 (s, 1H), 3.98 (s, 2H), 3.86 (s, 3H), 3.55 (s, 2H), 2.88 (s, 3H), 2.69 - 2.51 (m, 9H), 2.09 - 1.82 (m, 9H), 1.69 - 0.96 (m, 6H).

### Example 2-58 (Synthesis of 2085)

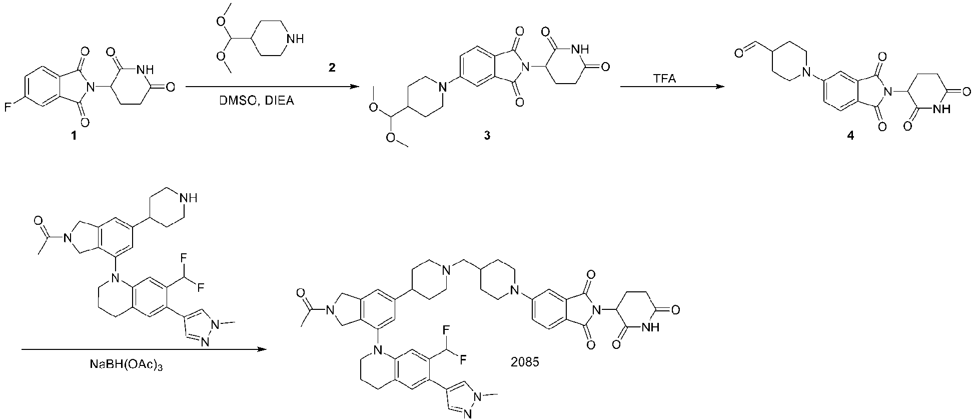

A mixture of 2-(2,6-dioxopiperidin-3-yl)-5-fluoroisoindole-1,3-dione (500 mg, 1.81 mmol), 4-(dimethoxymethyl)piperidine (317 mg, 1.99 mmol) and DIEA (701.85 mg, 5.43 mmol) in DMSO (6 mL) was heated at 100 °C for 1 h. The mixture was concentrated under reduced pressure to afford 5-(4-(dimethoxymethyl)piperidin-1-yl)-2-(2,6-trioxopiperidin-3-yl)isoindole-1,3-dione (720 mg, yield 90.95%) as a yellow oil.

To a solution of 5-(4-(dimethoxymethyl)piperidin-1-yl)-2-(2,6-dioxopiperidin-3-yl)isoindoline-1,3-dione (350 mg, 0.842 mmol) in DCM (3 mL) was added TFA (1 mL). Then the mixture was stirred at 25 °C for 2 h. LCMS showed consumption of starting material and detection of the desired compound. The reactant was concentrated to give 1-(2-(2,6-dioxopiperidin-3-yl)-1,3-dioxoisoindol-5-yl)piperidine-4-formaldehyde (220 mg, yield 67.16%) as a yellow solid.

To a solution of 1-(2-(2,6-dioxopiperidin-3-yl)-1,3-dioxoisoindolin-5-yl)piperidine-4-formaldehyde (90 mg, 0.24 mmol) in DMSO (3 mL) was added 1-{4-[7-(difluoromethyl)-6-(1-methylpyrazol-4-yl)-3,4-dihydro-2H-quinolin-1-yl]-6-(piperidin-4-yl)-1,3-dihydroisoindol-2-yl}ethenone (123.22 mg, 0.24 mmol). Then the mixture was stirred at 0 °C for 10 min. NaBH(OAc)₃ (103.3 mg, 0.487 mmol) was added to the mixture. The mixture was stirred at 25 °C for 1 h. LCMS showed consumption of starting material and detection of the desired product. The crude product was purified by prep-HPLC, eluting with 20% to 50% ACN in 0.1% TFA/water over 20 min to afford 5-(4-(4-(2-acetyl-7-(7-(difluoromethyl)-6-(1-methyl-1H-pyrazol-4-yl)-3,4-dihydroquinolin-1(2H)-yl)isoindolin-5-yl)piperidin-1-yl)methyl)piperidin-1-yl)-2-(2,6-dioxopiperidin-3-yl)isodihydroindoline-1,3-dione (50.3 mg, yield 23.55%) as a yellow solid.

¹H NMR (400 MHz, DMSO) δ 11.08 (s, 1H), 7.75 (s, 1H), 7.68 (d, J = 8.4Hz, 1H), 7.50 (s, 1H), 7.46 - 7.34 (m, 2H), 7.28 (d, J = 8.8 Hz, 1H), 7.19 - 7.08 (m, 2H), 6.89 - 6.61 (m, 1H), 6.48 - 6.34 (m, 1H), 5.07 (dd, J = 13.0, 5.2 Hz, 1H), 4.97 - 4.58 (m, 3H), 4.32 (s, 1H), 4.11 (d, J = 12.0 Hz, 2H), 3.70 - 3.53 (m, 3H), 3.19 - 2.76 (m, 12H), 2.64 - 2.55 (m, 2H), 2.19 (s, 1H), 2.08 - 1.79 (m, 11H), 1.38 - 1.21 (m, 2H).

The compounds shown below were prepared according to the procedures described in Examples 2-1 to 2-58:

| Compound | Characterization Data |
|---|---|
| 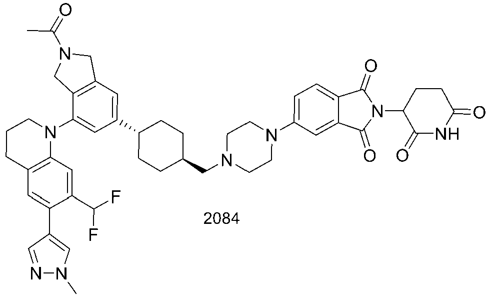 | m/z [M+H] ⁺ =859.4 |
| 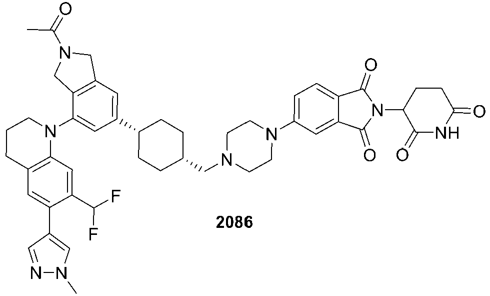 | m/z [M+H] ⁺ =859.4 |

### Test Example 1

22RV1, vCap, MM1S and OPM-2 cells were cultured in RPMI-1640 medium (10% fetal bovine serum and 100 units of penicillin/streptomycin dual-antibody per mL were added to RPMI-1640) in a cell incubator at 37°C with 5% CO₂, respectively. Cells in logarithmic growth phase were seeded into 96-well cell culture plates at 5×10³ cells/100 µL/well and cultured overnight. Active compounds dissolved in DMSO at different concentrations were added with a final concentration of 0.5% DMSO. 1% Tween 20 was used as positive control and 0.5% DMSO was used as negative control. After 72 h of culture, an equal volume of CellTiter-Glo was added to each well, mixed by shaking for 2 min, and left standing at room temperature for 10 min. Fluorescence signal values were read with a microplate reader. Data were analyzed and graphs were generated using Prism 7.0. The values shown are mean ± SEM (N=2). The negative control group was the 0% inhibition group (0% Inhibition), and the positive control group was the 100% inhibition group (100% Inhibition). The formula for inhibition rate is as follows: $% Inhibition = \left(Icompound-IZPE\right) / \left(IHPE-IZPE\right) \times 100$

Icompound is the signal value of the compound group; IHPE is the signal value of the 100% inhibition group; IZPE is the signal value of the negative control group. The X-axis is compound concentration (nM), and the Y-axis is % Inhibition.

| Compound | 22Rv1 Cell IC₅₀ (nM) | vCap Cell IC₅₀ (nM) | MM1S Cell IC₅₀ (nM) | OPM-2 Cell IC₅₀ (nM) |
|---|---|---|---|---|
| 1338 | 3574 | | 165 | 29 |
| 1339 | 2496 | | 48 | 8 |
| 1342 | 3811 | | 104 | 14 |
| 1343 | 205 | | 39 | 8 |
| 1344 | 263 | 33 | 68 | 15 |
| 1345 | 48 | 12 | 15 | 4 |
| 1346 | 81798 | | | |
| 1347 | 155 | | 26 | 2 |
| 1356 | 4268 | | 464 | 53 |
| 1357 | 5424 | | 58 | |
| 1358 | 4935 | | 598 | 39 |
| 1359 | 545 | | | 15 |
| 1361 | 106 | | 2 | 1 |
| 1362 | 51 | 6 | 3 | 2 |
| 1365 | 3354 | | | 30 |
| 1366 | 131 | 28 | 24 | 5 |
| 1367 | 135 | 46 | 33 | 4 |
| 1368 | 1394 | | | 168 |
| 1369 | 4 | 1 | 1 | 0.4 |
| 1370 | 1613 | | | |
| 1371 | 2094 | | | |
| 1373 | 208 | 51 | 44 | 12 |
| 1374 | 11 | 1 | 1 | 1 |
| 1375 | 16 | 2 | 2 | 1 |
| 1376 | 2653 | | | |
| 1377 | 4 | 0.5 | 0.3 | 0.2 |
| 1378 | 10 | 3 | 3 | 2 |
| 1379 | 28 | 8 | 5 | 5 |
| 1383 | 496 | 108 | 122 | 63 |
| 1385 | 358 | 144 | | |
| 1387 | 18 | 3 | 3 | 1 |
| 1388 | 15 | 2 | 3 | 1 |
| 1391 | 438 | 191 | 117 | 33 |
| GNE-781 | 4239 | 2150 | 104 | 8 |
| CCS1477 | 2521 | 140 | 220 | 37 |
| 2001 | 0.06 | 0.154 | / | / |
| 2003 | 0.69 | 0.19 | / | / |
| 2004 | 0.03 | 0.12 | / | / |
| 2005 | 7.7 | / | / | / |
| 2006 | 0.47 | 0.4 | 0.27 | 0.19 |
| 2007 | 0.04 | 0.01 | / | / |
| 2009 | 0.04 | 0.01 | / | / |
| 2010 | 0.08 | 0.193 | / | / |
| 2012 | 0.62 | 2.31 | / | / |
| 2013 | 0.01 | 0.449 | / | / |
| 2014 | nd | 11 | / | / |
| 2015 | 0.05 | 0.01 | / | / |
| 2016 | 0.01 | < 0.005 | / | / |
| 2018 | 0.01 | <0.005 | 0.1 | 0.05 |
| 2019 | 2.43 | 1.25 | / | / |
| 2020 | 1.87 | 0.53 | / | / |
| 2021 | 0.5 | 0.545 | / | / |
| 2022 | 0.3 | 2.12 | / | / |
| 2023 | 434.49 | / | / | / |
| 2024 | 3850 | 4608 | / | / |
| 2025 | 3887 | 7532 | / | / |
| 2026 | 22.39 | / | / | / |
| 2027 | 0.04 | 0.01 | / | / |
| 2028 | 0.1 | 0.26 | / | / |
| 2029 | 0.04 | 0.03 | / | / |
| 2030 | 0.34 | 0.1 | / | / |
| 2031 | 0.04 | 0.21 | / | / |
| 2032 | 0.16 | 0.11 | / | / |
| 2033 | 2.64 | / | / | / |
| 2034 | 0.98 | / | / | / |
| 2035 | 2.6 | / | / | / |
| 2046 | 14 | / | / | / |
| 2047 | >10000 | / | / | / |
| 2048 | 1.84 | 1.85 | / | / |
| 049 | / | / | / | / |
| 2050 | 5.25 | / | / | / |
| 2052 | / | / | / | / |
| 2053 | 1.02 | 0.36 | / | / |
| 2054 | 0.84 | 0.26 | 0.33 | / |
| 2055 | 0.54 | 0.26 | / | / |
| 2058 | 0.31 | 0.29 | 0.3 | 0.23 |
| 2059 | 9.42 | / | / | / |
| 2060 | 6.8 | / | / | / |
| 2061 | 3.48 | / | / | / |
| 2062 | 0.24 | 0.06 | 0.04 | / |
| 2063 | 0.33 | / | 0.28 | / |
| 2064 | 0.23 | 0.35 | | / |
| 2065 | 0.41 | / | 0.28 | / |
| 2066 | 0.08 | / | 0.23 | / |
| 2068 | 0.06 | 0.07 | 0.07 | 0.04 |
| 2069 | 0.38 | / | 0.36 | / |
| 2070 | 0.42 | / | 0.34 | / |
| 2071 | 0.5 | / | 0.31 | / |
| 2072 | 2025 | / | / | / |
| 2074 | >10000 | / | 426 | / |
| 2079 | 3.76 | / | / | / |
| 2080 | 3.97 | 1.17 | 1.16 | 0.52 |
| 2081 | 861 | / | / | / |
| 2082 | 47 | 13 | 25 | 4 |
| 2083 | >10000 | 58 | 35 | 13 |
| 2085 | 4 | 2 | 2 | 2 |

The biochemical activity test of CBP preparations was detected using CBP AlphaLISA^{®} Assay Kit. The detection principle is based on the binding of CBP bromodomain to acetylated histone substrate. The compounds were incubated with CBP and biotinylated substrate for 30 min, followed by the addition of acceptor beads for 15 min and donor beads for 15 min. Alpha counts were read, data were analyzed using Prism 7.0 and IC₅₀ values were calculated. The values shown are mean ± SEM (N=2).

| Compound | CBP | BRD4 |
|---|---|---|
| 1339 | 4.7 | 7432 |
| 1343 | 7.5 | 7876 |
| 1344 | 7.0 | 7915 |
| 1345 | 9.3 | >10000 |
| 1347 | 7.5 | >10000 |
| 1357 | 0.96 | 5714 |
| CCS1477 | 9.9 | 7246 |

### Test Example 2 Protein Degradation Experiment

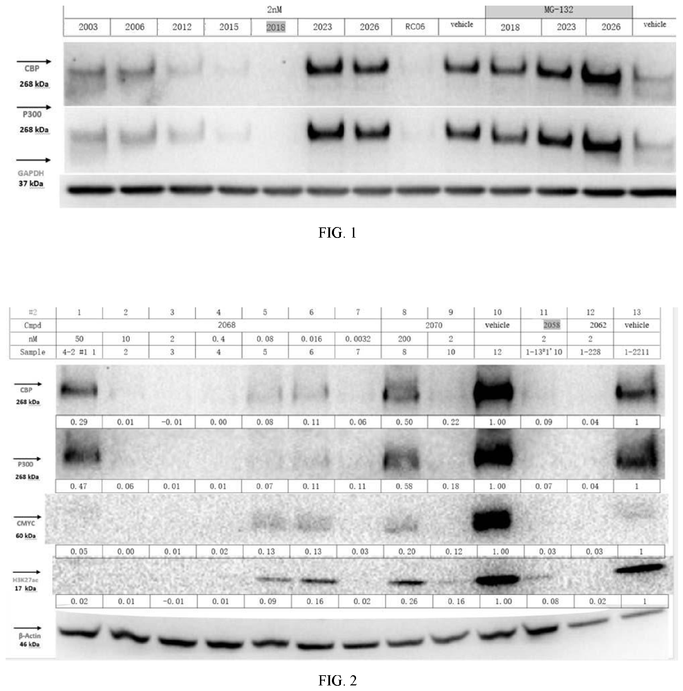

Cells (most commonly 22RV1 and LnCap) were passaged and seeded into 12-well or 24-well plates. The compounds were added after 24 to 26 h. The compounds were diluted with medium first, then added to the culture wells in proportion and mixed thoroughly. The blank control group (DMSO) and the test compound group contained the same concentration of DMSO, usually 1‰. Cell homogenates were prepared with RIPA buffer for sampling. Supernatant was preserved after high-speed centrifugation, and protein concentration was determined by BCA method. Each sample containing 5 µg of total protein was mixed with sample buffer and reducing agent, denatured at 75°C and loaded onto SDS-PAGE Mini-gel. After electrophoresis, proteins were transferred from the gel to PVDF membrane at a constant voltage of 20 V for 60 min using a mini-blot module. PVDF membrane was blocked with 5% milk in TBST for 1 h. It was incubated with primary antibody at 4°C overnight, and was incubated with anti-Rb-IgG-HRP at 1:10,000 for 1.5 h. Membranes were washed three times with TBST for 10 min each between steps. Images were developed, acquired and analyzed on ChemiDoc with chemiluminescent reagents. Quantitative analysis was performed using ChemiDoc's own software. The degradation degree of each test substance was expressed with the blank control group (DMSO) as 1 (or 100%). Dose-response curves were calculated and plotted using GraphPad Prism. Some experiments used Simple Western method instead of conventional western blot. Samples were prepared and loaded into test plates using kits and instructions provided by ProteinSimple. Detection and result collection were performed on Wes or Jess fully automatic protein expression analyzer. Results were edited and quantitatively analyzed using Compass SW software. The experimental results are shown in Figure 1 and Figure 2.

The embodiments of the technical solutions of the present disclosure have been exemplarily described above. It should be understood that the protection scope of the present disclosure is not limited to the above embodiments. Any modifications, equivalent substitutions, improvements, etc. made by those skilled in the art within the spirit and principles of the present disclosure shall be included in the protection scope of the claims of this application.

## Claims

1. A compound represented by formula (I), and a racemate, a stereoisomer, a tautomer, an isotopically labelled derivative, a N-oxide, a solvate, a polymorph, a metabolite, an ester, a prodrug, or a pharmaceutically acceptable salt thereof: 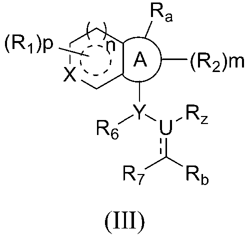 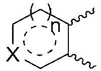
denotes aromatic aryl, heteroaryl, or non-aromatic heterocyclyl;
ring A is selected from phenyl and 5- to 6-membered heteroaryl;
X is selected from NR₁ and CH;
Y is selected from N and CH;
when 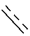 is a single bond, U is Z₁; Z₁ is selected from N and CH; and one of Y and Z₁ is N; when 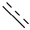 is a double bond, U is C;
each R₁ is the same or different, and is each independently selected from H, oxo (=O), halogen, CN, NH₂, COOH, OH, and the following groups unsubstituted or optionally substituted with 1, 2 or more R₁a: C₁₋₁₂ alkyl, C₁₋₁₂ alkoxy, -(C=O)R, C₆₋₁₄ aryl, 5- to 14- membered heteroaryl; wherein R is selected from C₁₋₁₂ alkyl, C₁₋₁₂ alkoxy, NH₂-, C₁₋₁₂ alkyl-NH-, N,N-di(C₁₋₁₂ alkyl)amino, and C₃₋₁₂ cycloalkyl;
each R₁a is the same or different, and is each independently selected from oxo (=O), halogen, CN, NH₂, COOH, OH, C₁₋₁₂ alkyl, and C₁₋₁₂ alkoxy;
each R₂ is the same or different, and is each independently selected from H, oxo (=O), halogen, CN, NH₂, COOH, OH, C₁₋₁₂ alkyl, and C₁₋₁₂ alkoxy;
Ra is selected from -link-E group, H, halogen, CN, NH₂, COOH, OH, and the following groups unsubstituted or optionally substituted with 1, 2 or more Ra₁: C₆₋₁₄ aryl, 5- to 14- membered heteroaryl, 3- to 14- membered heterocyclyl, C₃₋₁₂ cycloalkyl, and C₁₋₁₂ alkyl;
each Ra₁ is the same or different, and is each independently selected from H, oxo (=O), halogen, COOH, OH, and the following groups unsubstituted or optionally substituted with 1, 2 or more Ra₂: NH₂, C₁₋₁₂ alkyl, C₂₋₁₂ alkenyl, C₁₋₁₂ alkoxy, C₁₋₁₂ alkylthio, C₁₋₁₂ alkyl-NH-, C₂₋₁₂ alkenyl-NH-, N,N-di(C₁₋₁₂ alkyl)amino, C₁₋₁₂alkylC(=O)-, C₂₋₁₂ alkenyl-C(=O)-, C₂₋₁₂ alkynyl-C(=O)-, C₁₋₁₂ alkoxy-C(=O)-, C₁₋₁₂alkyl -NHC(=O)-, N,N-di(C₁₋₁₂alkyl)aminocarbonyl, C₆₋₁₄ aryl, 5- to 14- membered heteroaryl, 3- to 14- membered heterocyclyl, C₃₋₁₂ cycloalkyl, C₆₋₁₄ aryl-C(=O)-, 5- to 14- membered heteroaryl-C(=O)-, 3- to 14- membered heterocyclyl-C(=O)-, C₃₋₁₂ cycloalkyl-C(=O)-, C₁₋₁₂alkyl-S(=O)₂-, C₁₋₁₂alkyl-S(=O)-, C₂₋₁₂ alkenyl-S(=O)-, C₁₋₁₂ alkylS(=O)(=NH)-, C₃₋₁₂ cycloalkyl-S(=O)₂-, C₂₋₁₂alkenyl-S(=O)₂-, C₁₋₁₂alkyl-C(=O)-NH-, C₂₋₁₂ alkenyl-C(=O)-NH-, C₂₋₁₂ alkynyl-C(=O)-NH-, C₁₋₁₂alkyl-S(=O)₂-NH-, and C₂₋₁₂alkenyl-S(=O)₂-NH-;
each Ra₂ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; R₆ is selected from H, halogen, CN, NH₂, COOH, OH, and the following groups unsubstituted or optionally substituted with 1, 2 or more R₆a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl -NHC(=O)-, and N,N-di(C₁₋₁₂ alkyl)aminocarbonyl;
each R₆a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl;
R₇ is selected from H, halogen, CN, NH₂, COOH, OH, and the following groups unsubstituted or optionally substituted with 1, 2 or more R₇a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, and N,N-di(C₁₋₁₂ alkyl)aminocarbonyl;
each R₇a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂alkyl)aminocarbonyl;
alternatively, R₆ and R₇ together with the atoms to which they are attached form the following groups unsubstituted or optionally substituted with 1, 2 or more Rsa, Rc, Rd or Re: C₆₋₁₄ aryl, 5-to 14- membered heteroaryl and 3- to 14- membered heterocyclyl; wherein the 5- to 14-membered heteroaryl and 3- to 14- membered heterocyclyl contains at least one N atom;
each Rsa is the same or different, and is each independently selected from oxo (=O), halogen, CN, NH₂, COOH, OH, and the following groups unsubstituted or optionally substituted with 1, 2 or more Rsb: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂alkyl)aminocarbonyl; each Rsb is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; Rb, Rc, Rd, Re and Rz are the same or different, and are each independently selected from oxo (=O), and the following groups unsubstituted or optionally substituted with 1, 2 or more Rb₁: C₆₋₁₄ aryl and 5- to 14- membered heteroaryl; alternatively, two adjacent groups of Rb, Rc, Rd, Re and Rz together with the atoms to which they are attached form the following groups unsubstituted or optionally substituted by 1, 2 or more Rb₂: C₆₋₁₄ aryl, 5- to 14- membered heteroaryl, and C₃₋₁₂ cycloalkyl;
each of Rb₁ and Rb₂ is the same or different, and is each independently selected from H, halogen, CN, NH₂, COOH, OH, and the following groups unsubstituted or optionally substituted by 1, 2 or more Rb₃: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl; each Rb₃ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
alternatively, Rb and Rz together with the atoms to which they are attached form 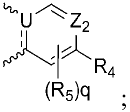 Z₂ is selected from CR₃ and N;
R₃ is selected from H, halogen, CN, NH₂, COOH, OH, and the following groups unsubstituted or optionally substituted by 1, 2 or more R₃a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14-membered heteroaryl, 3- to 14- membered heterocyclyl, and C₃₋₁₂ cycloalkyl;
each R₃a is the same or different, and is each independently selected from oxo (=O), halogen, CN, NH₂, COOH, OH, and the following groups unsubstituted or optionally substituted with 1, 2 or more R₃b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl;
each R₃b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; R₄ is selected from -link-E group, H, halogen, CN, NH₂, COOH, OH, and the following groups unsubstituted or optionally substituted by 1, 2 or more R₄a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl; each R₄a is the same or different, and is each independently selected from oxo (=O), halogen, CN, NH₂, COOH, OH, and the following groups unsubstituted or optionally substituted with 1, 2 or more R₄b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl; each R₄b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; and at least one of Ra and R₄ is a -link-E group, wherein -link-E group in Ra is -link₁-E₁ and - link-E group in R₄ is - link₂-E₂;
link1₁ and link₂ are the same or different, and are each independently selected from -Cy₁-L₁-Cy₂-L₂-Q-;
Cy₁ is selected from the following groups unsubstituted or optionally substituted with 1, 2 or more R_{Cy1}: 3- to 14- membered heterocyclylene, C₃₋₁₂ cycloalkylene, and 5- to 14- membered heteroarylene;
Cy₂ is absent or is selected from the following groups unsubstituted or optionally substituted with 1, 2 or more R_{Cy2}: 3- to 14- membered heterocyclylene, C₃₋₁₂ cycloalkylene, C₆₋₁₄ arylene, and 5- to 14- membered heteroarylene;
L₁ is absent or is selected from N(R_{L}), and the following groups unsubstituted or optionally substituted with 1, 2 or more R_{L1}: C₁₋₁₂ alkylene and C₁₋₁₂ alkyleneoxy; R_{L} is selected from H and C₁₋₁₂ alkyl;
L₂ is absent or is selected from the following groups unsubstituted or optionally substituted with 1, 2 or more R_{L2}: C₁₋₁₂ alkylene and (C₁₋₁₂ alkyleneoxy)ₜ; t is selected from 0, 1, 2, 3, 4, 5, 6, 7, and 8;
Q is absent or is selected from O, N(R_{q}), and ethynylene; R_{q} is selected from H and C₁₋₁₂ alkyl; R_{Cy1} and R_{Cy2} are the same or different, and are each independently selected from halogen, oxo (=O), C₁₋₁₂ alkyl, and C₁₋₁₂ alkoxy;
R_{L1} and R_{L2} are the same or different, and are each independently selected from halogen, oxo (=O), C₁₋₁₂ alkyl, and C₁₋₁₂ alkoxy;
the end at Cy₁ is connected to 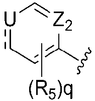
and the end at Q is connected to E₁ or E₂;
E₁ and E₂ are the same or different, and are each independently selected from 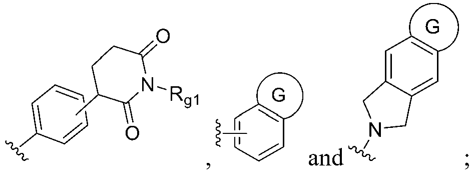
ring G is selected from 5- to 6- membered heterocyclyl containing N and substituted with 1, 2 or more R_{g}; each R_{g} is the same or different,
and is each independently selected from H, oxo (=O), C₁₋₁₂ alkyl, and 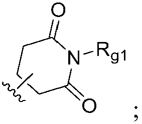 and ring G
contains at least one 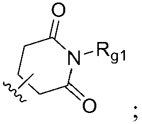
R_{g1} is selected from H, hydroxy C₁₋₁₂ alkyl, and (R_{g11})(R_{g12})-N-C₁₋₁₂ alkyl-C(O)O-C₁₋₁₂ alkyl; R_{g11} and R_{g12} are the same or different, and are each independently selected from H and C₁₋₁₂ alkyl; 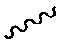 indicates a connection site;
each R₅ is the same or different, and is each independently selected from oxo (=O), halogen, CN, NH₂, COOH, OH, and the following groups unsubstituted or optionally substituted with 1, 2 or more R₅a: C₁₋₁₂ alkyl and C₁₋₁₂ alkoxy;
each R₅a is the same or different, and is each independently selected from oxo (=O), halogen, CN, NH₂, COOH, OH, C₁₋₁₂ alkyl, and C₁₋₁₂ alkoxy;
n is selected from 0, 1, and 2;
m is selected from 0, 1, 2, and 3;
p is selected from 0, 1, 2, 3, 4, and 5;
q is selected from 0, 1, and 2.

2. The compound represented by formula (IIII), and a racemate, a stereoisomer, a tautomer, an isotopically labelled derivative, a N-oxide, a solvate, a polymorph, a metabolite, an ester, a prodrug, or a pharmaceutically acceptable salt thereof according to claim 1, **characterized in that** the compound represented by formula (IIII) has the structure represented by formula (I): 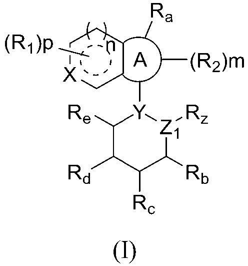 wherein: 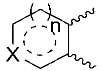
denotes aromatic aryl, heteroaryl, a non-aromatic heterocyclyl;
ring A is selected from phenyl and 5- to 6-membered heteroaryl;
X is selected from NR₁ and CH;
Y is selected from N and CH;
Z₁ is selected from N, C, and CH; and one of Y and Z₁ is N;
each R₁ is the same or different, and is each independently selected from H, oxo (=O), halogen, CN, NH₂, COOH, OH, and the following groups unsubstituted or optionally substituted with 1, 2 or more R₁a: C₁₋₁₂ alkyl, C₁₋₁₂ alkoxy, -(C=O)R, C₆₋₁₄ aryl, 5- to 14- membered heteroaryl; wherein R is selected from C₁₋₁₂ alkyl, C₁₋₁₂ alkoxy, NH₂-, C₁₋₁₂ alkyl-NH-, N,N-di(C₁₋₁₂ alkyl)amino, and C₃₋₁₂ cycloalkyl;
each R₁a is the same or different, and is each independently selected from oxo (=O), halogen, CN, NH₂, COOH, OH, C₁₋₁₂ alkyl, and C₁₋₁₂ alkoxy;
each R₂ is the same or different, and is each independently selected from H, oxo (=O), halogen, CN, NH₂, COOH, OH, C₁₋₁₂ alkyl, and C₁₋₁₂ alkoxy;
Ra is selected from H, halogen, CN, NH₂, COOH, OH, and the following groups unsubstituted or optionally substituted with 1, 2 or more Ra₁: C₆₋₁₄ aryl, 5- to 14- membered heteroaryl, 3- to 14-membered heterocyclyl, C₃₋₁₂ cycloalkyl, and C₁₋₁₂ alkyl;
each Ra₁ is the same or different, and is each independently selected from H, oxo (=O), halogen, COOH, OH, and the following groups unsubstituted or optionally substituted with 1, 2 or more Ra₂: NH₂, C₁₋₁₂ alkyl, C₂₋₁₂ alkenyl, C₁₋₁₂ alkoxy, C₁₋₁₂ alkylthio, C₁₋₁₂ alkyl-NH-, C₂₋₁₂ alkenyl-NH-, N,N-di(C₁₋₁₂ alkyl)amino, C₁₋₁₂ alkyl-C(=O)-, C₂₋₁₂ alkenyl-C(=O)-, C₂₋₁₂ alkynyl-C(=O)-, C₁₋₁₂ alkoxy-C(=O)-, C₁₋₁₂ alkyl-NHC(=O)-, N,N-di(C₁₋₁₂alkyl)aminocarbonyl, C₆₋₁₄ aryl, 5- to 14- membered heteroaryl, 3- to 14- membered heterocyclyl, C₃₋₁₂ cycloalkyl, C₆₋₁₄ aryl-C(=O)-, 5- to 14- membered heteroaryl-C(=O)-, 3- to 14- membered heterocyclyl-C(=O)-, C₃₋₁₂ cycloalkyl-C(=O)-, C₁₋₁₂ alkyl-S(=O)₂-, C₁₋₁₂ alkyl-S(=O)-, C₂₋₁₂ alkenyl-S(=O)-, C₁₋₁₂ alkylS(=O)(=NH)-, C₃₋₁₂ cycloalkyl-S(=O)₂-, C₂₋₁₂ alkenyl-S(=O)₂-, C₁₋₁₂ alkyl-C(=O)-NH-, C₂₋₁₂ alkynyl-C(=O)-NH-, C₁₋₁₂ alkyl-S(=O)₂-NH-, and C₂₋₁₂alkenyl-S(=O)₂-NH-;
each Ra₂ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; Rb, Rc, Rd, Re and Rz are the same or different, and are each independently selected from oxo (=O) and the following groups unsubstituted or optionally substituted with 1, 2 or more Rb₁: C₆₋₁₄ aryl and 5- to 14- membered heteroaryl; alternatively, two adjacent groups of Rb, Rc, Rd, Re and Rz together with the atoms to which they are attached form the following groups unsubstituted or optionally substituted by 1, 2 or more Rb₂: C₆₋₁₄ aryl, 5- to 14- membered heteroaryl, and C₃₋₁₂ cycloalkyl;
each of Rb₁ and Rb₂ is the same or different, and is each independently selected from H, halogen, CN, NH₂, COOH, OH, and the following groups unsubstituted or optionally substituted by 1, 2 or more Rb₃: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl; each Rb₃ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino;
n= 0, 1, 2;
m = 0, 1, 2, 3;
p= 0, 1, 2, 3, 4, 5.

3. The compound represented by formula (IIII), and a racemate, a stereoisomer, a tautomer, an isotopically labelled derivative, a N-oxide, a solvate, a polymorph, a metabolite, an ester, a prodrug, or a pharmaceutically acceptable salt thereof according to claim 1 or 2, **characterized in that**
preferably, Y is N; Z is selected from C and CH;
preferably, Y is CH; Z is N;
preferably, ring A is selected from phenyl, pyrazolyl, and imidazolyl;
preferably, ring A is selected from 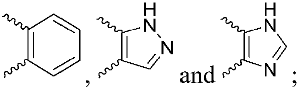
preferably, 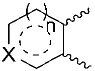
is selected from phenyl and 5- to 6- membered heteroaryl; 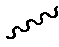 indicates a connection site;
preferably, 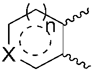
is selected from tetrahydropyrrolyl, piperidinyl, and phenyl;
preferably, 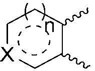
is selected from 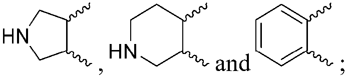
preferably, 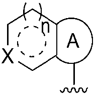
is selected from 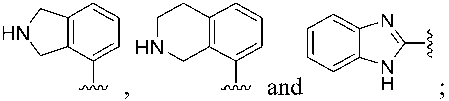 where 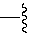
is connected to 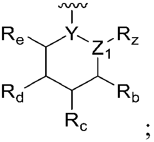
preferably, 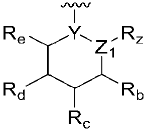
is selected from 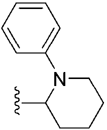
unsubstituted or optionally substituted with 1, 2 or more Rb₁, and 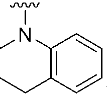 unsubstituted or optionally substituted with 1, 2 or more Rb₂;
preferably, 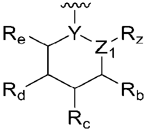
is selected from 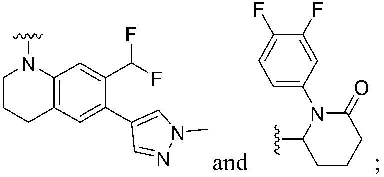 preferably, each R₁ is the same or different, and is each independently selected from -(C=O)C₁₋₆ alkyl, -(C=O)-NH-C₁₋₆ alkyl, and 5- to 6- membered heteroaryl substituted with C₁₋₆ alkyl; preferably, each R₁ is the same or different, and is each independently selected from -(C=O)CH₃,
-(C=O)-NH-CH₃, and 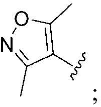 preferably, Ra is selected from the following groups unsubstituted or optionally substituted by 1, 2 or more Ra₁: 3- to 8- membered heterocyclyl and C₃₋₈ cycloalkyl;
preferably, Ra is selected from the following groups unsubstituted or optionally substituted by 1, 2 or more Ra₁: cyclohexyl, piperidinyl, and piperazinyl;
preferably, each Ra₁ is the same or different, and is each independently selected from the following groups unsubstituted or optionally substituted by 1, 2 or more Ra₂: C₁₋₆ alkyl, C₁₋₆ alkyl-C(=O)-, C₂₋₆ alkenyl-C(=O)-, C₂₋₆ alkynyl-C(=O)-, C₁₋₆ alkyl-C(=O)-NH-, C₂₋₆ alkenyl-C(=O)-NH-, C₂₋₆ alkynyl-C(=O)-NH-, C₁₋₆ alkyl-S(=O)₂-, C₂₋₆ alkenyl-S(=O)₂-, C₁₋₆ alkylS(=O)₂-NH-, C₂₋₆ alkenyl-S(=O)₂-NH-, and 3- to 6- membered heterocyclyl;
preferably, each Ra₂ is the same or different, and is each independently selected from halogen, oxo (=O), and (C₁₋₁₂ alkyl)₂-NH-;
preferably, each Ra₁ is the same or different, and is each independently selected from methyl, 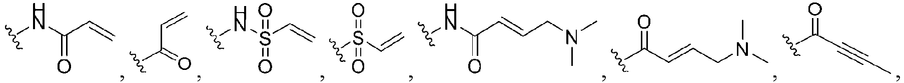 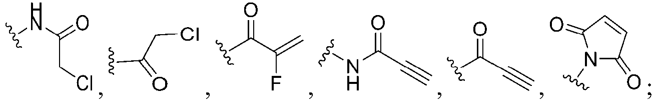
preferably, each Rb is the same or different, and is each independently selected from oxo (=O) and C₆₋₁₀ aryl unsubstituted or substituted optionally by 1, 2 or more Rb₁; alternatively, the two Rb together with the atoms to which they are attached form C₆₋₁₀ aryl unsubstituted or optionally substituted by 1, 2 or more Rb₂;
preferably, each Rb is the same or different, and is each independently selected from oxo (=O), phenyl unsubstituted or optionally substituted by 1, 2 or more Rb₁; alternatively, the two Rb together with the atoms to which they are attached form phenyl unsubstituted or optionally substituted by 1, 2 or more Rb₂;
preferably, each Rb₁ is the same or different, and is each independently selected from halogen, e.g., F;
preferably, each Rb₂ is the same or different, and is each independently selected from CN, halogenated C₁₋₆ alkyl, C₃₋₆ alkyl, and C₁₋₆ alkyl-5- to 6- membered heteroaryl;
preferably, each Rb₂ is the same or different, and is each independently selected from CN, difluoromethyl, cyclopropyl, and

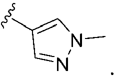

4. The compound represented by formula (IIII), and a racemate, a stereoisomer, a tautomer, an isotopically labelled derivative, a N-oxide, a solvate, a polymorph, a metabolite, an ester, a prodrug, or pharmaceutically acceptable salt thereof according to any one of claims 1-3, **characterized in that** formula (IIII) is further selected from the following structures: 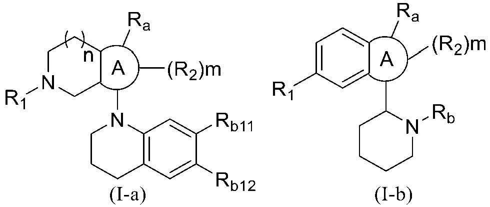
wherein ring A, R₁, R₂, Ra, Rb, m, and n are each independently as defined in any one of claims 1-3; Rb₁₁ and Rb₁₂ are the same or different, and are each independently as defined for Rb₂; preferably, formula (III) is further selected from the following structures: 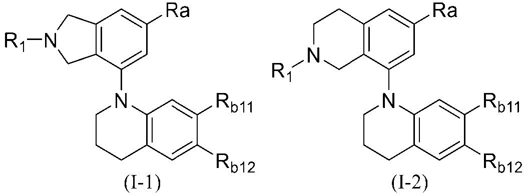 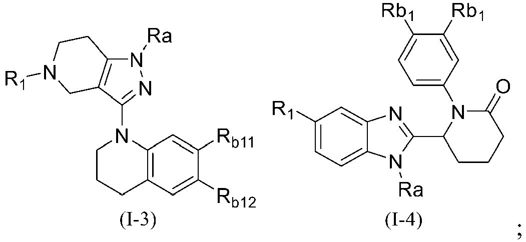
wherein R₁, Ra and Rb₁ are each independently as defined in any one of claims 1-3; Rb₁₁ and Rb₁₂ are the same or different, and are each independently as defined for Rb₂.

5. The compound represented by formula (III), and a racemate, a stereoisomer, a tautomer, an isotopically labelled derivative, a N-oxide, a solvate, a polymorph, a metabolite, an ester, a prodrug, or a pharmaceutically acceptable salt thereof according to any one of claims 1-4, **characterized in that** the compound represented by formula (III) has the structure represented by formula (II): 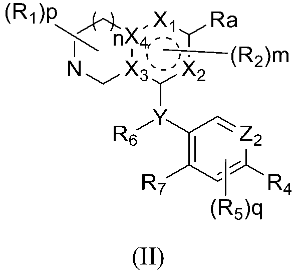 wherein:
n= 0, 1, 2;
m = 0, 1, 2, 3;
p= 0, 1, 2, 3, 4, 5;
q= 0, 1, 2;
X₁, X₂, X₃ and X₄ are the same or different, and is each independently selected from C and N; Y is selected from C and N;
Z₂ is selected from CR₃ and N;
each R₁ is the same or different, and is each independently selected from oxo (=O), halogen, CN, NH₂, COOH, OH, and the following groups unsubstituted or optionally substituted with 1, 2 or more R₁a: C₁₋₁₂ alkyl, C₁₋₁₂ alkoxy, and -(C=O)R;
wherein R is selected from C₁₋₁₂ alkyl, C₁₋₁₂ alkoxy, NH₂-, C₁₋₁₂ alkyl-NH-, N,N-di(C₁₋₁₂ alkyl)amino, and C₃₋₁₂ cycloalkyl;
each R₁a is the same or different, and is each independently selected from oxo (=O), halogen, CN, NH₂, COOH, OH, C₁₋₁₂ alkyl, and C₁₋₁₂ alkoxy;
each R₂ is the same or different, and is each independently selected from oxo (=O), halogen, CN, NH₂, COOH, OH, C₁₋₁₂ alkyl, and C₁₋₁₂ alkoxy;
Ra is selected from -link-E group, H, halogen, CN, NH₂, COOH, OH, and the following groups unsubstituted or optionally substituted with 1, 2 or more Ra₁: C₆₋₁₄ aryl, 5- to 14- membered heteroaryl, 3- to 14- membered heterocyclyl, C₃₋₁₂ cycloalkyl, and C₁₋₁₂ alkyl;
each Rₐ₁ is the same or different, and is each independently selected from H, oxo (=O), halogen, COOH, OH, and the following groups unsubstituted or optionally substituted with 1, 2 or more Ra₂: NH₂, C₁₋₁₂ alkyl, C₁₋₁₂ alkoxy, C₁₋₁₂ alkylthio, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, C₃₋₁₂ cycloalkyl, C₆₋₁₄ aryl-C(=O)-, 5- to 14- membered heteroaryl-C(=O)-, 3- to 14- membered heterocyclyl-C(=O)-, C₃₋₁₂ cycloalkyl-C(=O)-, C₁₋₁₂ alkyl-S(=O)₂-, C₁₋₁₂alkylS(=O)-, C₁₋₁₂ alkyl-S(=O)(=NH)-, C₃₋₁₂ cycloalkyl-S(=O)₂-, C₁₋₁₂ alkyl-C(=O)-NH-, and C₁₋₁₂ alkyl-S(=O)₂-NH-;
each Ra₂ is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; R₃ is selected from H, halogen, CN, NH₂, COOH, OH, and the following groups unsubstituted or optionally substituted by 1, 2 or more R₃a: C₁₋₁₂ alkyl, C₁₋₁₂ alkoxy, C₁₋₁₂ alkyl-NH-, N,N-di(C₁₋₁₂ alkyl)amino, C_{1 -12} alkyl -NHC(=O)-, N,N-di(C₁₋₁₂ alkyl)aminocarbonyl, C₆₋₁₄ aryl, 5- to 14-membered heteroaryl, 3- to 14- membered heterocyclyl, and C₃₋₁₂ cycloalkyl;
each R₃a is the same or different, and is each independently selected from oxo (=O), halogen, CN, NH₂, COOH, OH, and the following groups unsubstituted or optionally substituted with 1, 2 or more R₃b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl; each R₃b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; R₄ is selected from -link-E group, H, halogen, CN, NH₂, COOH, OH, and the following groups unsubstituted or optionally substituted by 1, 2 or more R₄a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl -NHC(=O)-, N,N-di(C₁₋₁₂ alkyl)aminocarbonyl, C₆₋₁₄ aryl, 5- to 14- membered heteroaryl, 3- to 14- membered heterocyclyl, and C₃₋₁₂ cycloalkyl; each R₄a is the same or different, and is each independently selected from oxo (=O), halogen, CN, NH₂, COOH, OH, and the following groups unsubstituted or optionally substituted with 1, 2 or more R₄b: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl; each R₄b is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino; and at least one of Ra and R₄ is -link-E group, wherein -link-E group in Ra is -link₁-E₁ and -link-E group in R₄ is - link₂-E₂;
link1₁ and link₂ are the same or different, and are each independently selected from -Cy₁-L₁-Cy₂-L₂-Q-;
Cy₁ is selected from the following groups unsubstituted or optionally substituted with 1, 2 or more R_{Cy1}: 3- to 14- membered heterocyclylene, C₃₋₁₂ cycloalkylene, and 5- to 14- membered heteroarylene;
Cy₂ is absent or is selected from the following groups unsubstituted or optionally substituted with 1, 2 or more R_{Cy2}: 3- to 14- membered heterocyclylene, C₃₋₁₂ cycloalkylene, C₆₋₁₄ arylene, and 5- to 14- membered heteroarylene;
L₁ is absent or is selected from N(R_{L}), and the following groups unsubstituted or optionally substituted with 1, 2 or more R_{L1}: C₁₋₁₂ alkylene and C₁₋₁₂ alkyleneoxy; R_{L} is selected from H and C₁₋₁₂ alkyl;
L₂ is absent or is selected from the following groups unsubstituted or optionally substituted with 1, 2 or more R_{L2}: C₁₋₁₂ alkylene and (C₁₋₁₂ alkyleneoxy)ₜ; t is selected from 0, 1, 2, 3, 4, 5, 6, 7, and 8;
Q is absent or is selected from O, N(R_{q}), and ethynylene; R_{q} is selected from H and C₁₋₁₂ alkyl; R_{Cy1} and R_{Cy2} are the same or different, and are each independently selected from halogen, oxo (=O), C₁₋₁₂ alkyl, and C₁₋₁₂ alkoxy;
R_{L1} and R_{L2} are the same or different, and are each independently selected from halogen, oxo (=O), C₁₋₁₂ alkyl, and C₁₋₁₂ alkoxy;
the end at Cy₁ is connected to 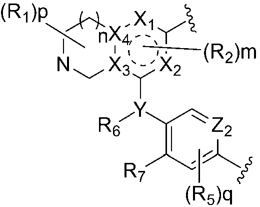
and the end at Q is connected to E₁ or E₂;
E₁ and E₂ are the same or different, and are each independently selected from 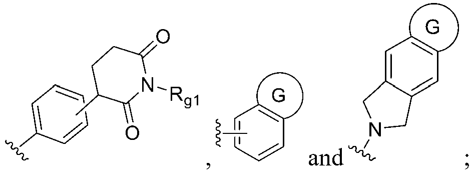
ring G is selected from 5- to 6- membered heterocyclyl containing N substituted with 1, 2 or more R_{g}; each R_{g} is the same or different, and
is each independently selected from H, oxo (=O), C₁₋₁₂ alkyl, and 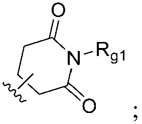
and ring G
contains at least one 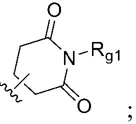
R_{g1} is selected from H, hydroxy C₁₋₁₂ alkyl, (R_{g11})(R_{g12})-N-C₁₋₁₂ alkyl-C(O)O-C₁₋₁₂ alkyl; R_{g11} and R_{g12} are the same or different, and are each independently selected from H and C₁₋₁₂ alkyl; 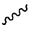 indicates a connection site;
each R₅ is the same or different, and is each independently selected from oxo (=O), halogen, CN, NH₂, COOH, OH, and the following groups unsubstituted or optionally substituted with 1, 2 or more R₅a: C₁₋₁₂ alkyl and C₁₋₁₂ alkoxy;
each R₅a is the same or different, and is each independently selected from oxo (=O), halogen, CN, NH₂, COOH, OH, C₁₋₁₂ alkyl, and C₁₋₁₂ alkoxy;
R₆ is selected from H, halogen, CN, NH₂, COOH, OH, and the following groups unsubstituted or optionally substituted with 1, 2 or more R₆a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl -NHC(=O)-, and N,N-di(C₁₋₁₂ alkyl)aminocarbonyl;
each R₆a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂ alkyl)aminocarbonyl;
R₇ is selected from H, halogen, CN, NH₂, COOH, OH, and the following groups unsubstituted or optionally substituted with 1, 2 or more R₇a: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-NHC(=O)-, and N,N-di(C₁₋₁₂ alkyl)aminocarbonyl;
each R₇a is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂alkyl)aminocarbonyl;
alternatively, R₆ and R₇ together with the atoms to which they are attached form the following groups unsubstituted or optionally substituted with 1, 2 or more Rsa: C₆₋₁₄ aryl, 5- to 14-membered heteroaryl and 3- to 14- membered heterocyclyl; wherein the 5- to 14- membered heteroaryl and 3- to 14- membered heterocyclyl contains at least one N atom;
each Rsa is the same or different, and is each independently selected from oxo (=O), halogen, CN, NH₂, COOH, OH, and the following groups unsubstituted or optionally substituted with 1, 2 or more Rsb: C₁₋₁₂ alkyl, C₁₋₁₂ alkoxy, C₁₋₁₂ alkyl-NH-, N,N-di(C₁₋₁₂ alkyl)amino, C₁₋₁₂ alkyl-C(=O)-, C₁₋₁₂ alkoxy-C(=O)-, C₁₋₁₂ alkyl -NHC(=O)-, and N,N-di(C₁₋₁₂alkyl)aminocarbonyl; each Rsb is the same or different, and is each independently selected from oxo (=O), halogen, CN, NH₂, COOH, OH, C₁₋₁₂ alkyl, C₁₋₁₂ alkoxy, C₁₋₁₂ alkyl-NH-, and N,N-di(C₁₋₁₂ alkyl)amino.

6. The compound represented by formula (III), and a racemate, a stereoisomer, a tautomer, an isotopically labelled derivative, a N-oxide, a solvate, a polymorph, a metabolite, an ester, a prodrug, or a pharmaceutically acceptable salt thereof according to any one of claims 1-5, **characterized in that**: 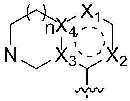
moiety is selected from the following structures: 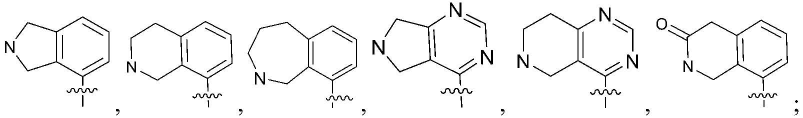
preferably,
each R₁ is the same or different, and is each independently selected from oxo (=O), C₁₋₆ alkyl, C₁₋₆ alkoxy, and -(C=O)R; wherein R is selected from C₁₋₆ alkyl, C₁₋₆ alkoxy, NH₂-, C₁₋₆ alkyl-NH-, and C₃₋₆ cycloalkyl;
preferably, R₁ is -(C=O)CH₃, -C(=O)OCH₃, -C(=O)NH-CH₃;
preferably, Ra is selected from -link₁-E₁ group and 5-to 8- membered heterocyclyl; the 5-to 8-
membered heterocyclyl is, for example, 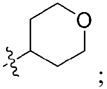 preferably, R₃ is halogenated C₁₋₆ alkyl;
Preferably, R₃ is -CHF₂;
preferably, R₄ is selected from -link₂-E₂ group, and 5- to 6- membered heteroaryl (e.g., pyrazolyl) unsubstituted or optionally substituted with 1, 2 or more R₄a; each R₄a is the same or different,
and is each independently selected from C₁₋₆ alkyl and C₁₋₆ alkoxy; preferably, R₄ is 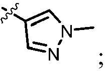 preferably, R₆ and R₇ together with the atoms to which they are attached form the following groups unsubstituted or optionally substituted with 1, 2 or more Rsa: 5- to 6- membered heterocyclyl and C₆₋₁₀ aryl, for example piperidinyl or phenyl;
preferably, R₆ and R₇ together with the atoms to which they are attached form the following structures unsubstituted or optionally substituted with 1, 2 or more Rsa: 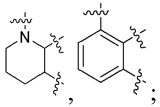
preferably, 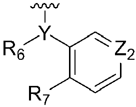
moiety is selected from 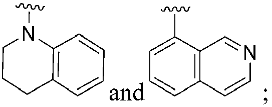 preferably, Cy₁ is selected from the following groups unsubstituted or optionally substituted with 1, 2 or more R_{Cy1}: 5- to 8- membered heterocyclylene, C₃₋₈ cycloalkylene, and 5- to 10-membered heteroarylene;
preferably, Cy₁ is selected from piperidinylene, cyclohexylene, and pyridinylene; for example 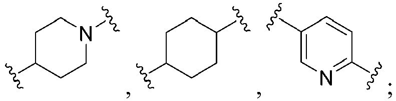
preferably, Cy₂ is absent or is selected from the following groups unsubstituted or optionally substituted with 1, 2 or more R_{Cy2}: 5-to 8- membered heterocyclylene, C₃₋₈ cycloalkylene, C₆₋₁₀ arylene, and 5- to 10- membered heteroarylene;
preferably, Cy₂ is absent or is selected from piperidinylene, morpholinylene, piperazinylene, phenylene, pyridinylene, and triazolylene; for example 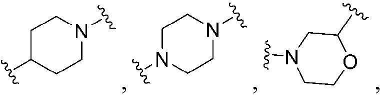 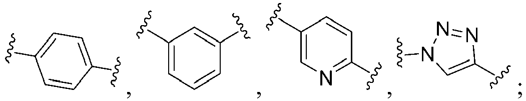
preferably, L₁ is absent or is C₁₋₁₀ alkylene unsubstituted or optionally substituted with 1, 2, or more R_{L1};
preferably, L₁ is absent or is selected from NH, N(CH₃), methylene, ethylene, 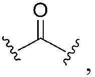 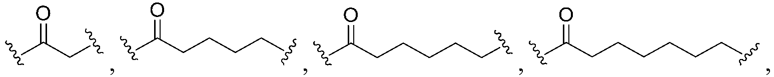 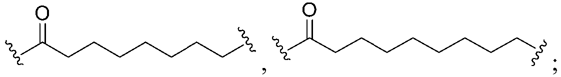 preferably, L₂ is absent or is C₁₋₆ alkylene, (C₁₋₆ alkyleneoxy)ₜ; t is selected from 1, 2, 3, and 4;
preferably, L₂ is absent or is selected from methylene, ethylene, propylene, ethyleneoxy, 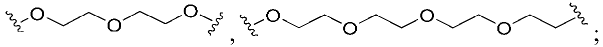
preferably, Q is absent or is selected from O, NH, ethynylene;
preferably, link₁ and link₂ are the same or different, and is each independently selected from 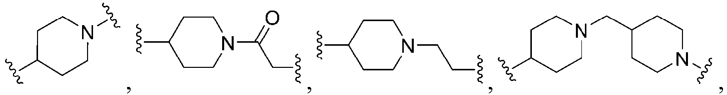 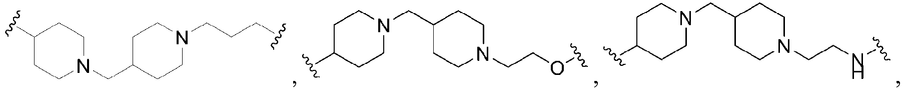 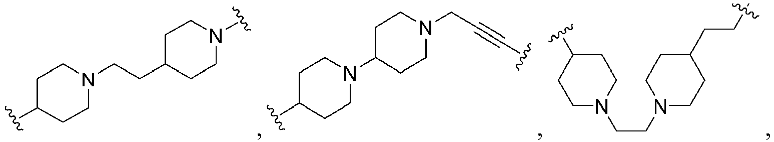 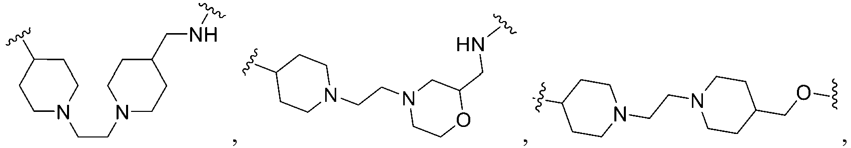 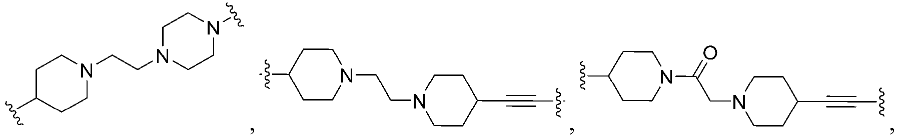 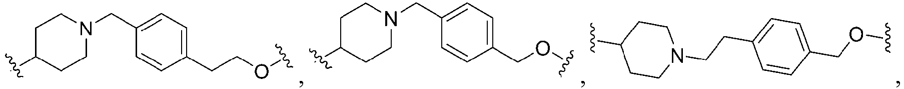 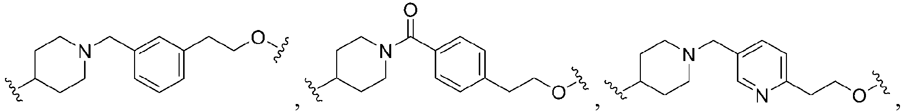 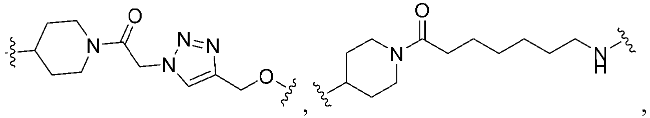 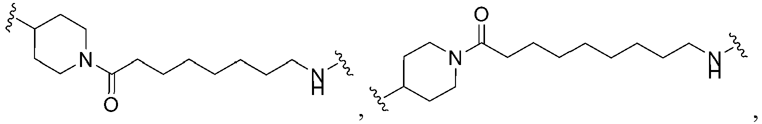 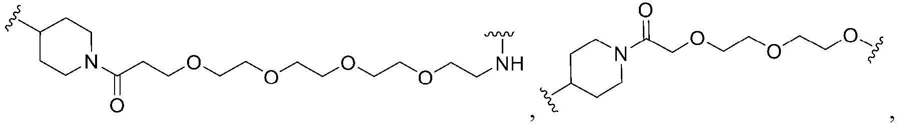 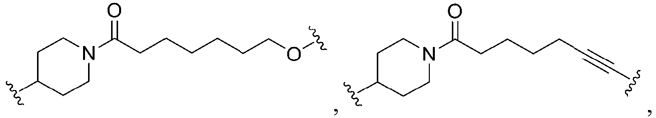 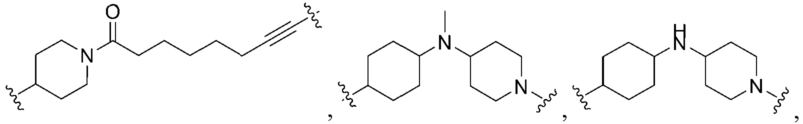 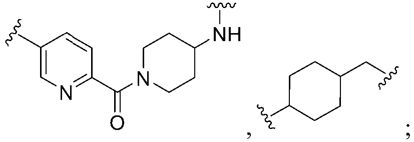 preferably, ring G is selected from tetrahydropyrrolyl and imidazolidinyl substituted with 1, 2 or
more R_{g}; for example 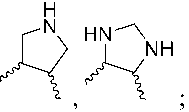
preferably, ring G is selected from 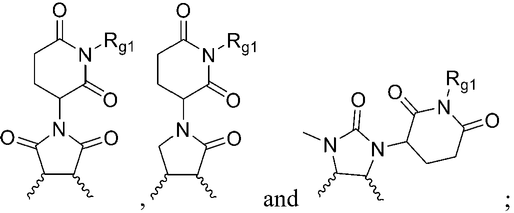 preferably, each R_{g} is the same or different, and is each independently selected from H, oxo
(=O), C₁₋₆ alkyl, and 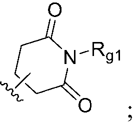
preferably, R_{g1} is selected from H, hydroxy C₁₋₆ alkyl, (C₁₋₆ alkyl)(C₁₋₆ alkyl)-N-C₁₋₆ alkylene-C(O)O-C₁₋₆ alkylene, H₂N-C₁₋₆ alkylene-C(O)O-C₁₋₆ alkylene;
preferably, R_{g1} is selected from H, hydroxymethyl, 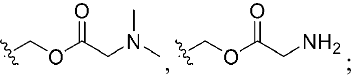 preferably, E₁ and E₂ are the same or different, and are each independently selected from: 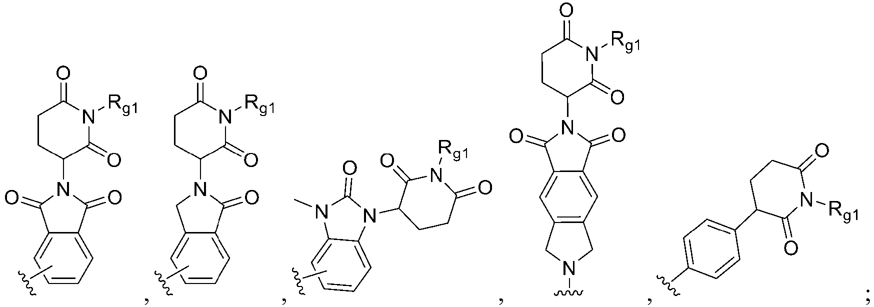
preferably, E₁ and E₂ are the same or different, and are each independently selected from:

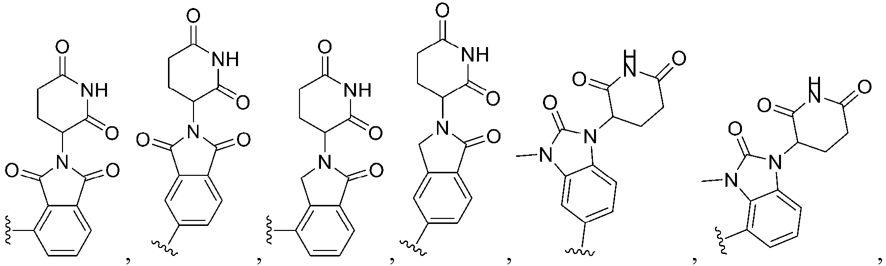

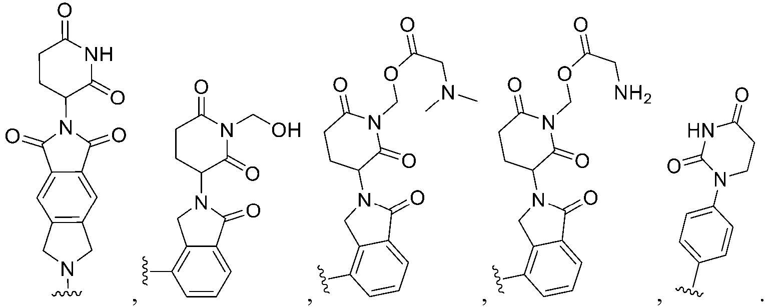

7. The compound represented by formula (III), and a racemate, a stereoisomer, a tautomer, an isotopically labelled derivative, a N-oxide, a solvate, a polymorph, a metabolite, an ester, a prodrug, or a pharmaceutically acceptable salt thereof according to any one of claims 1-6, **characterized in that**:
formula (III) is further selected from the following structures: 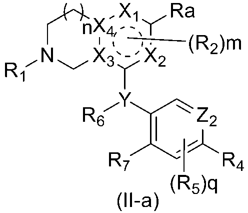
wherein R₁, R₂, Ra, R₄, R₅, R₆, R₇, X₁, X₂, X₃, X₄, Y, Z, m, n and q are as defined in any one of claims 1-6;
preferably, formula (III) is further selected from the following structures: 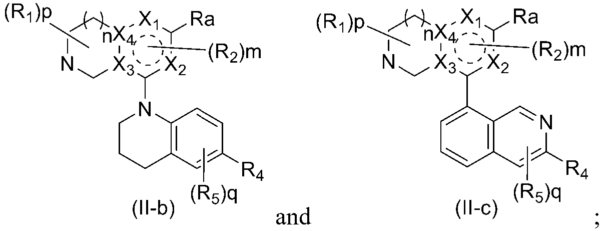
wherein R₁, R₂, Ra, R₄, R₅, X₁, X₂, X₃, X₄, lm, n, p and q are as defined in any one of claims 1-6;
preferably, formula (III) is further selected from the following structures: 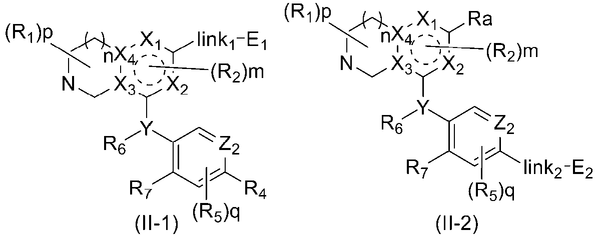 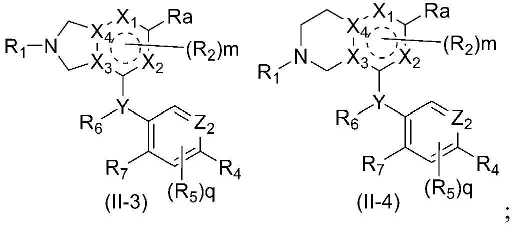 wherein R₁, R₂, Ra, R₄, R₅, R₆, R₇, X₁, X₂, X₃, X₄, Y, Z, link₁, link₂, E₁, E₂, m, n, p, q are as defined in any one of claims 1-6.

8. The compound represented by formula (III), and a racemate, a stereoisomer, a tautomer, an isotopically labelled derivative, a N-oxide, a solvate, a polymorph, a metabolite, an ester, a prodrug, or a pharmaceutically acceptable salt thereof according to any one of claims 1-7, **characterized in that**: exemplary specific compounds of the compound represented by formula (III) are as follows:

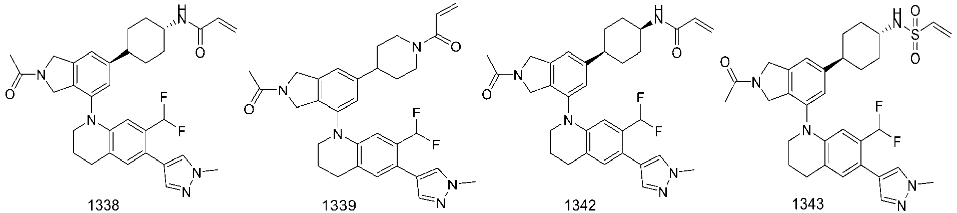

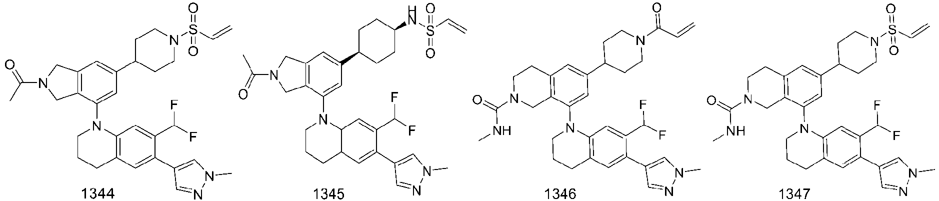

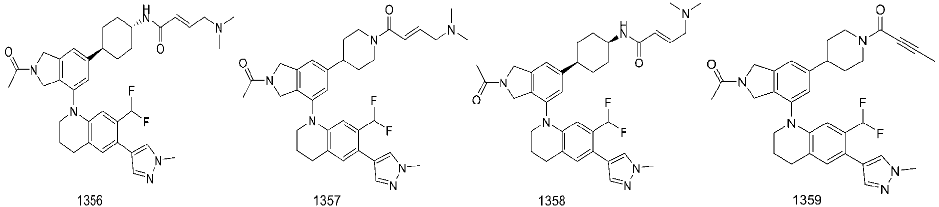

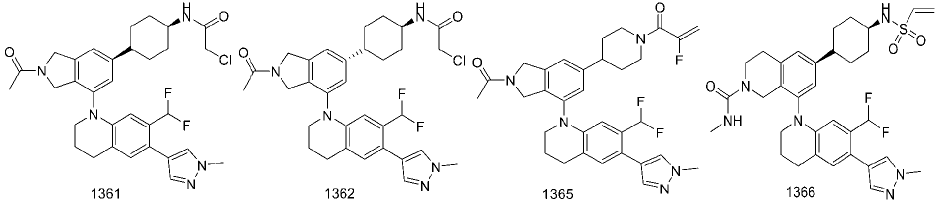

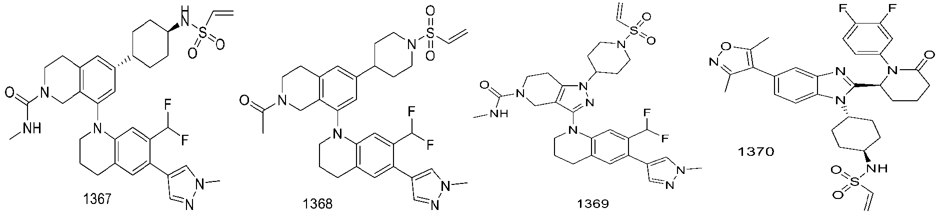

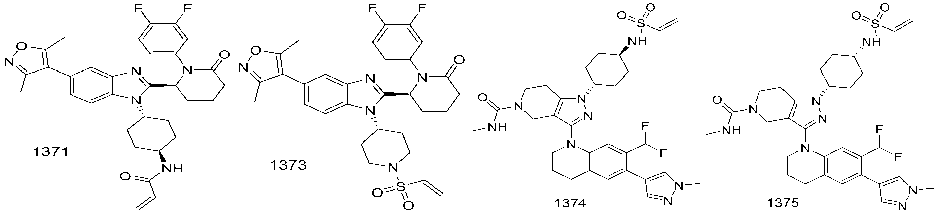

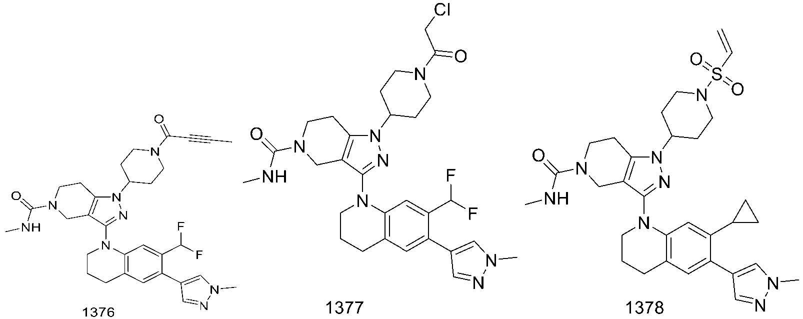

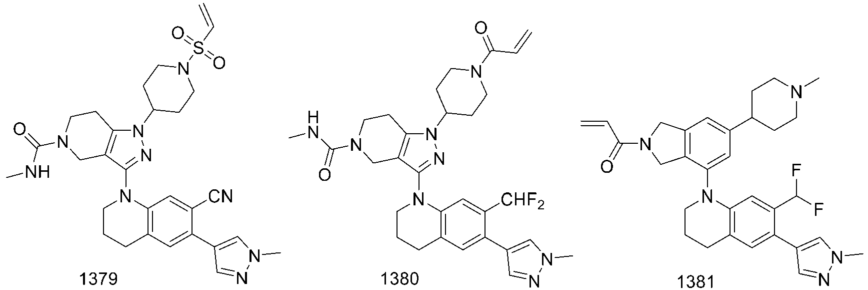

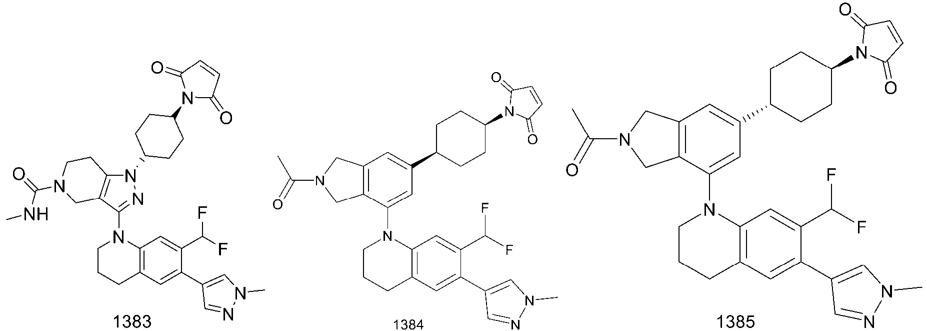

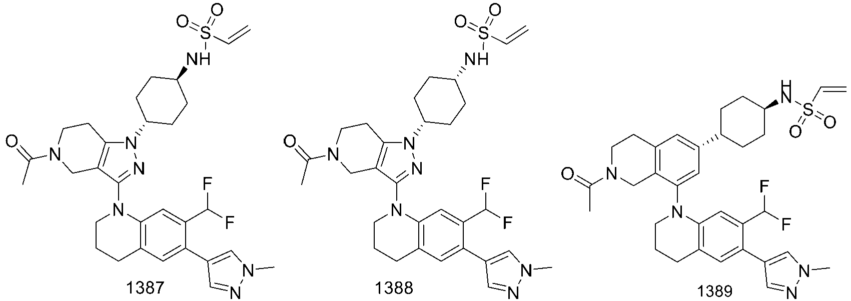

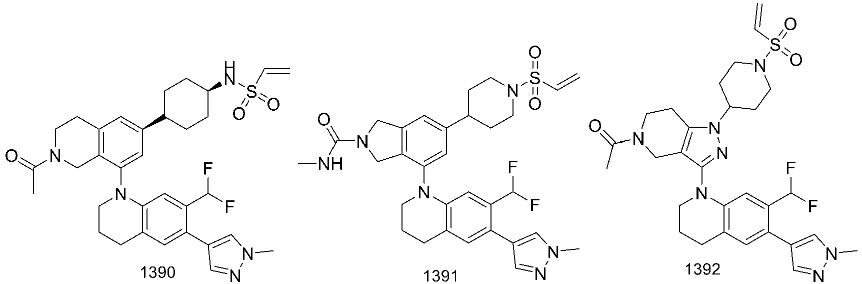

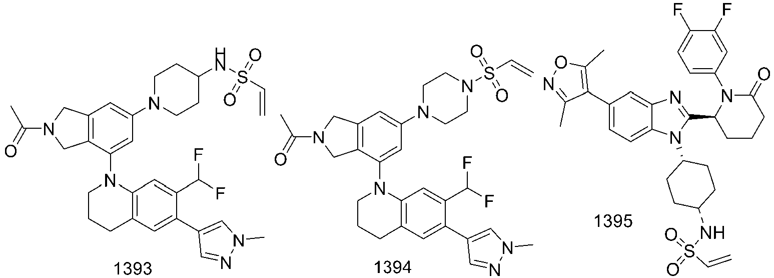

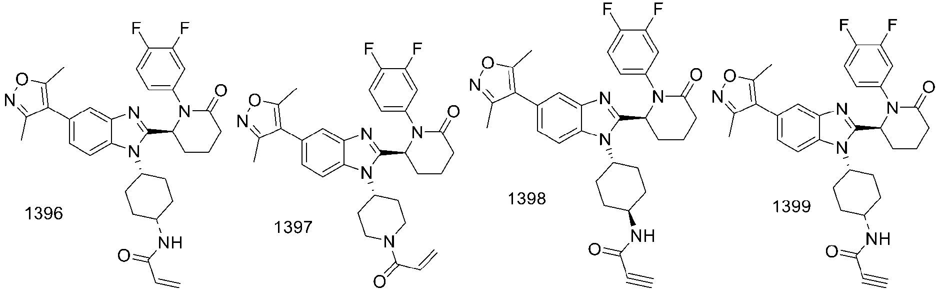

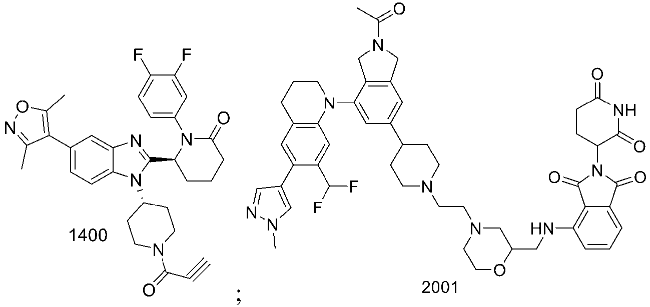

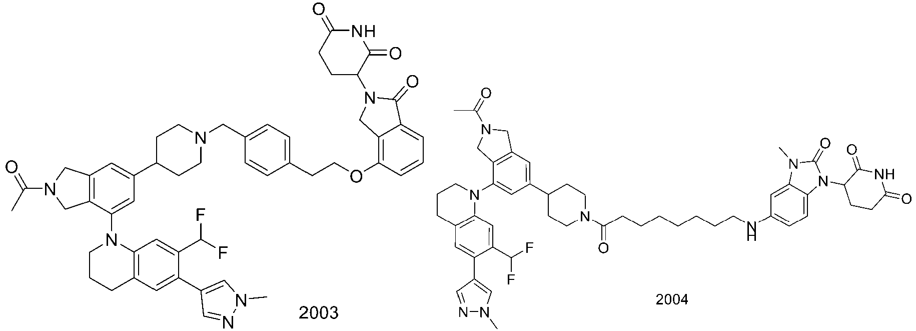

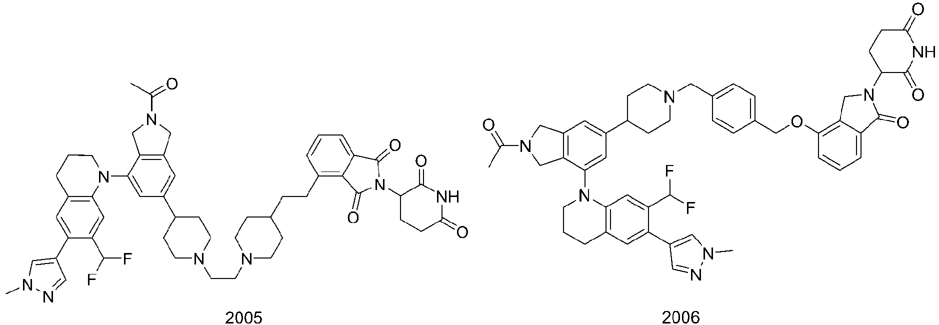

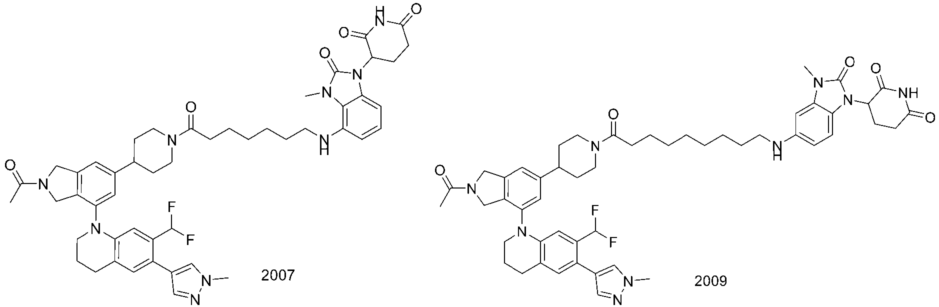

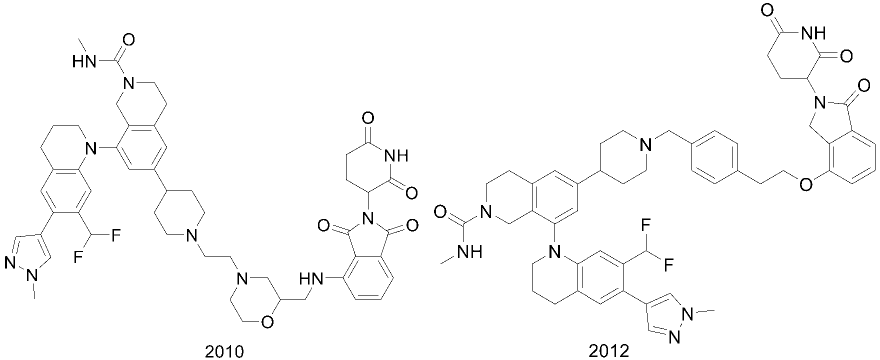

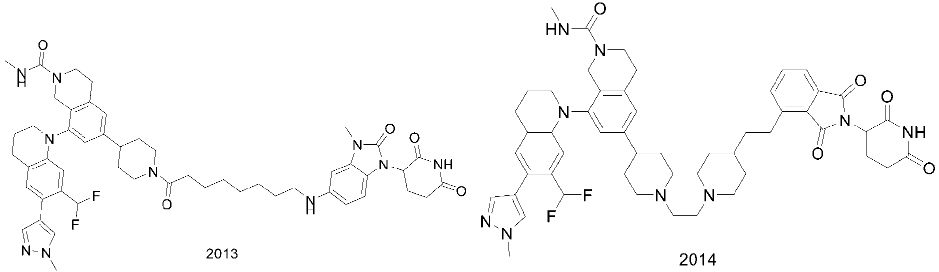

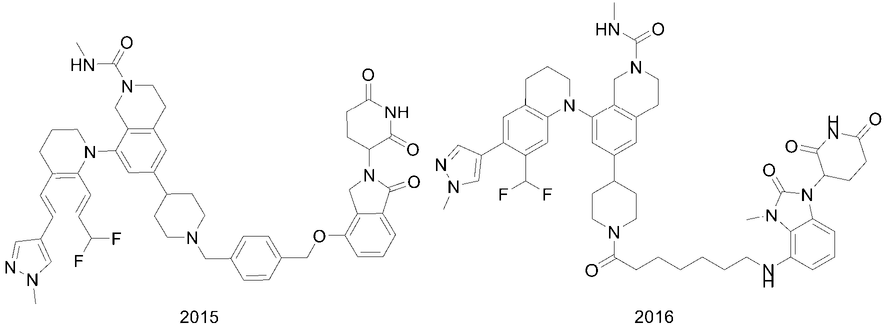

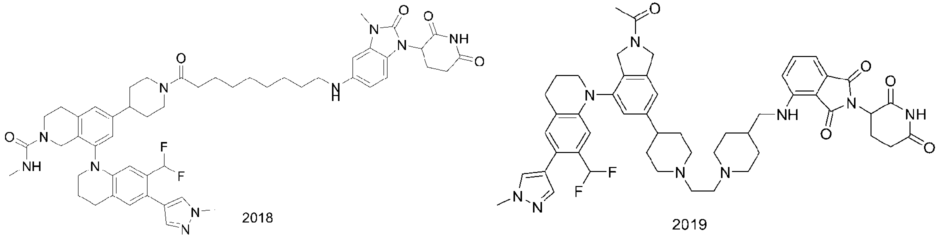

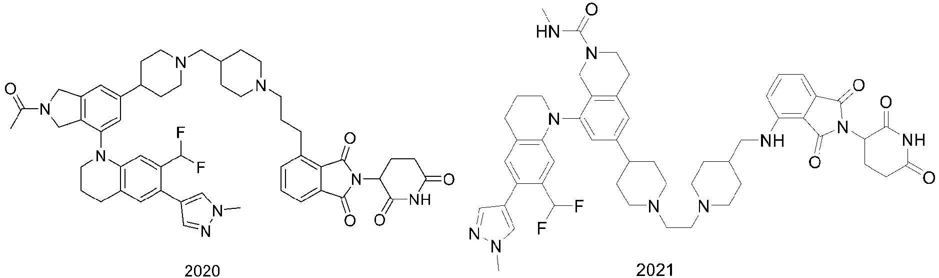

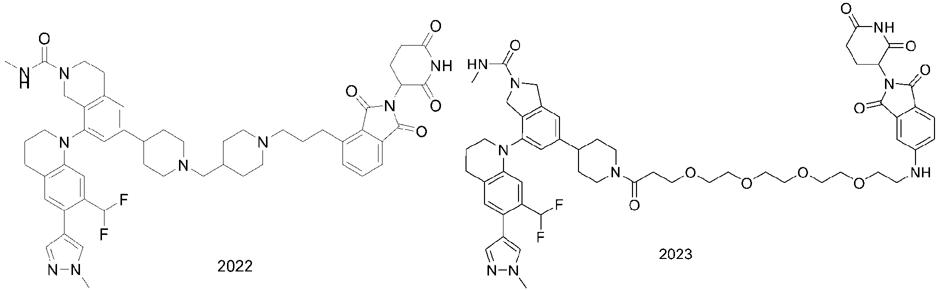

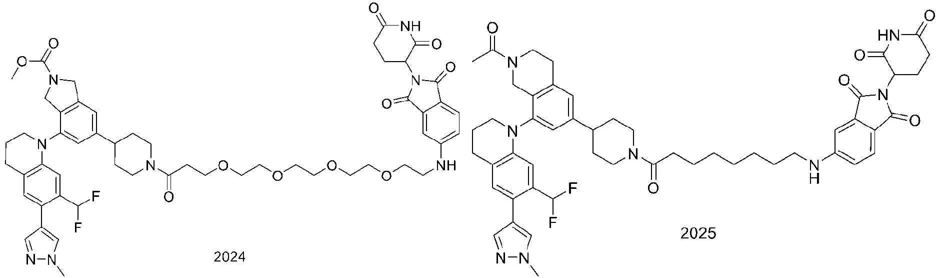

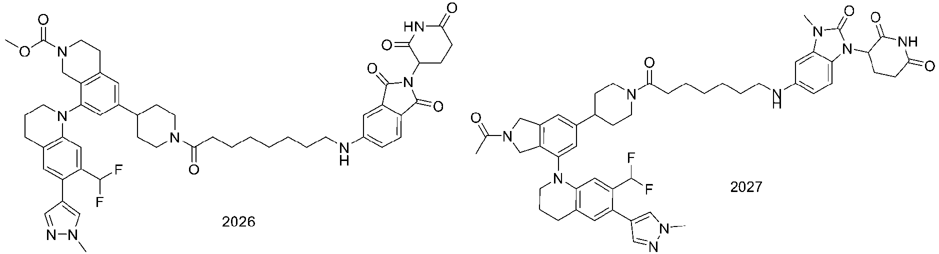

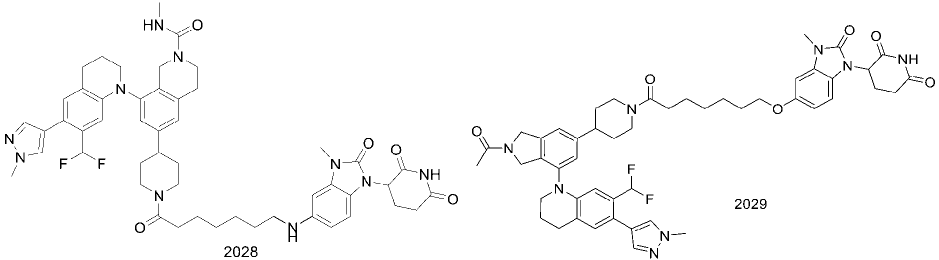

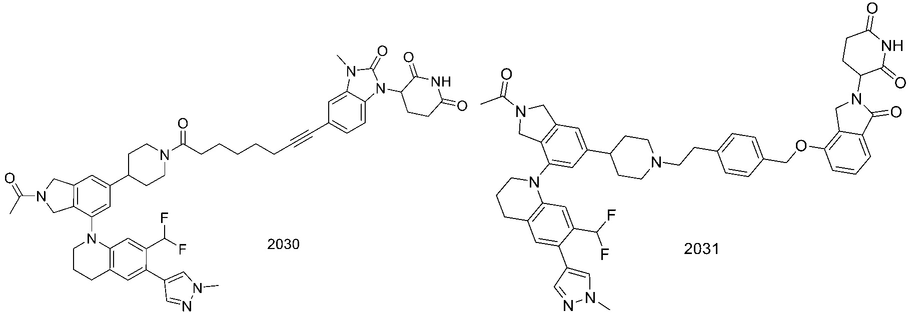

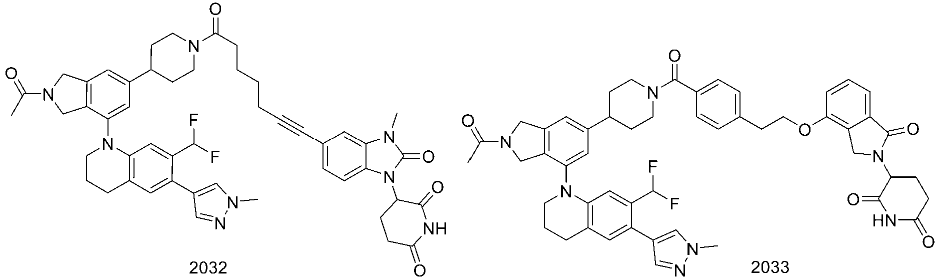

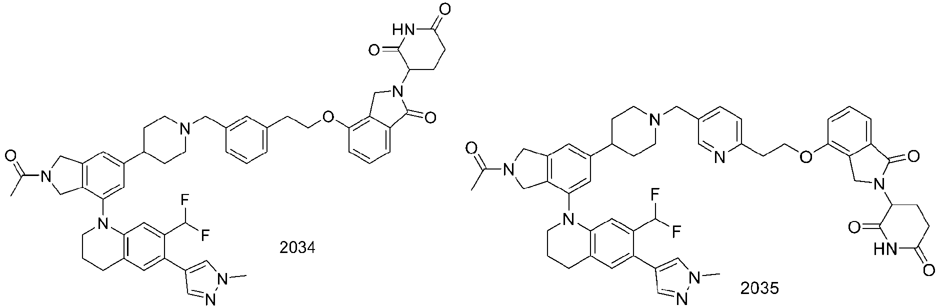

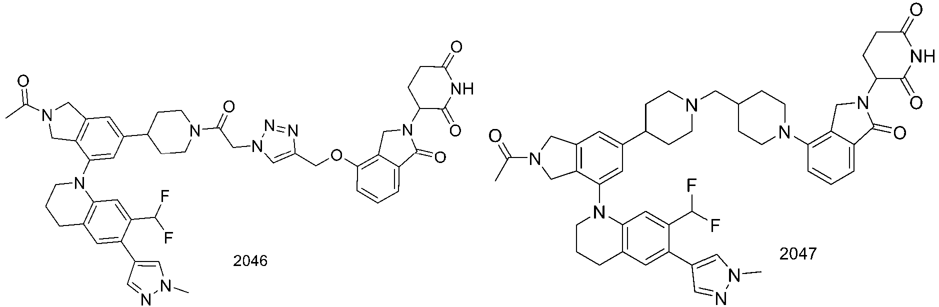

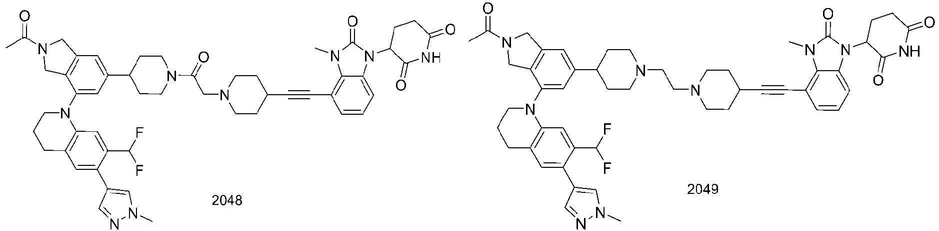

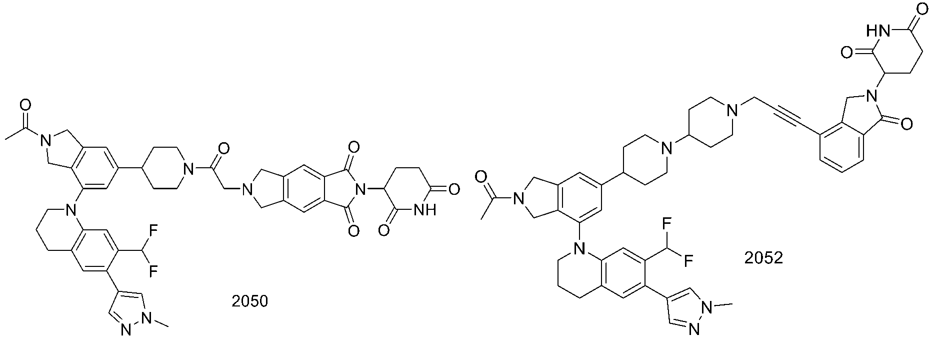

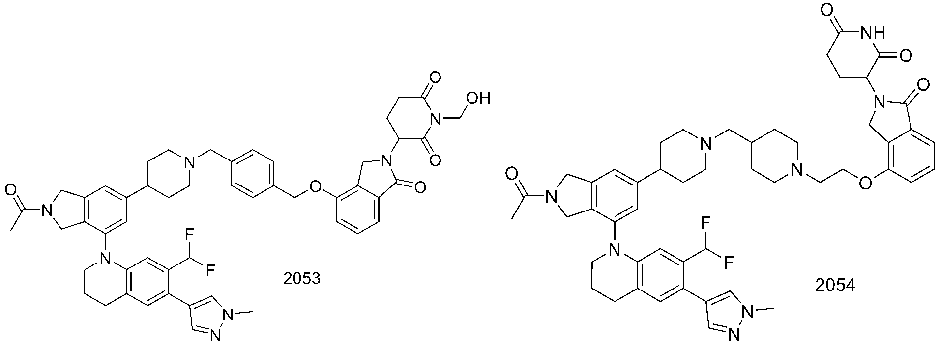

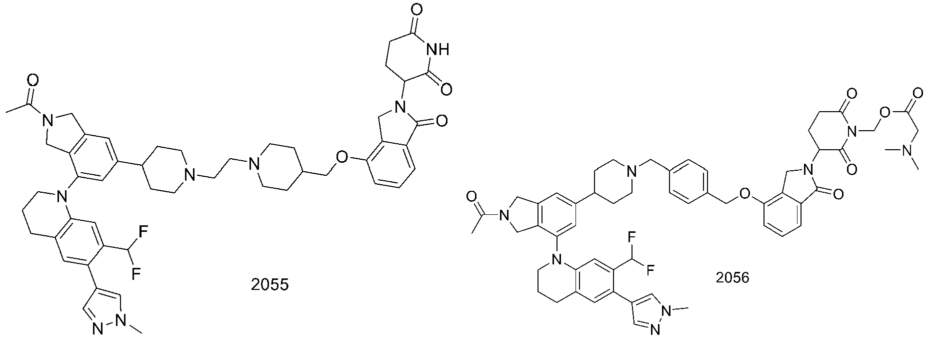

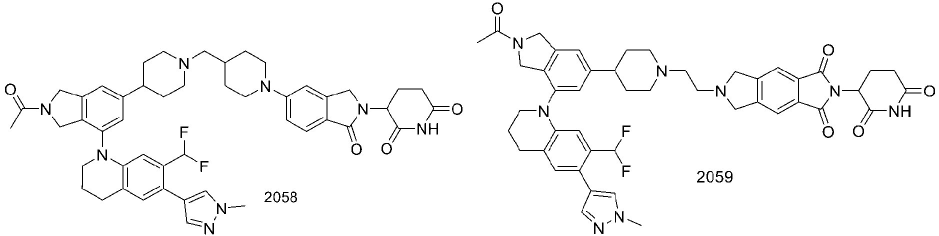

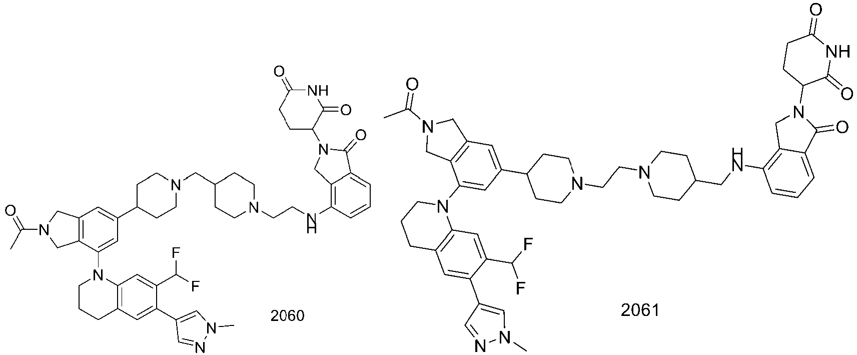

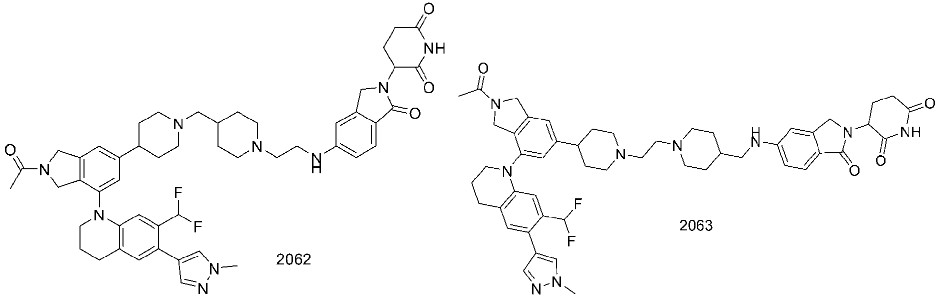

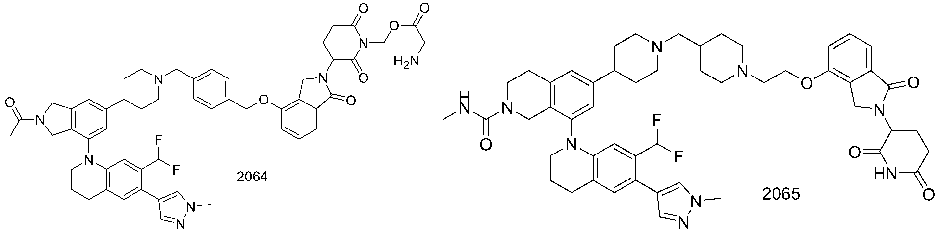

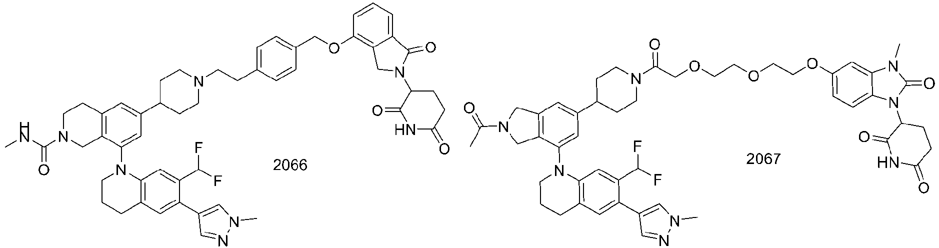

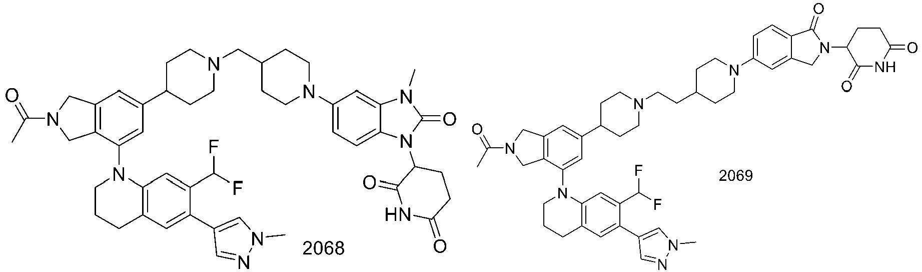

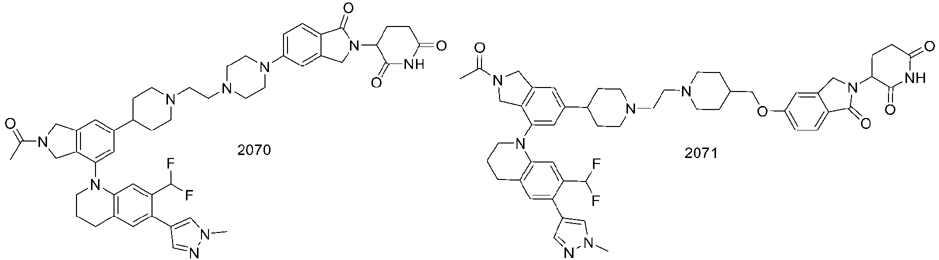

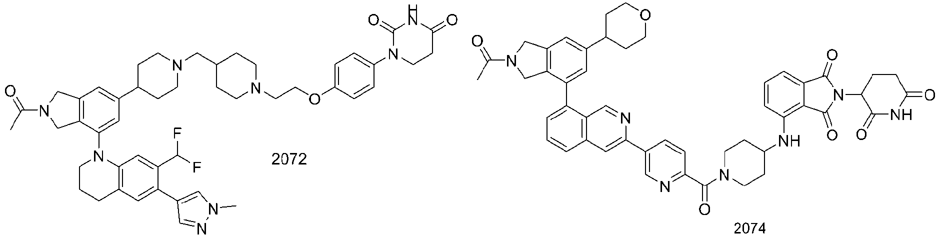

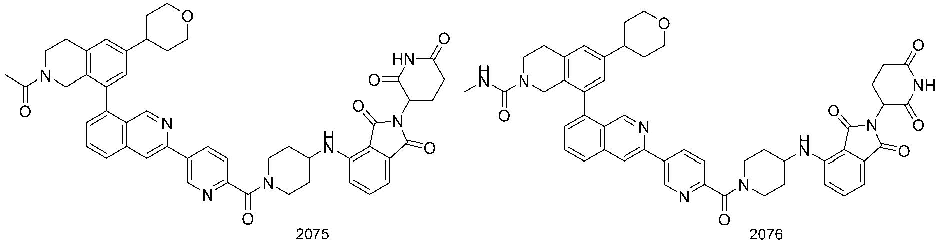

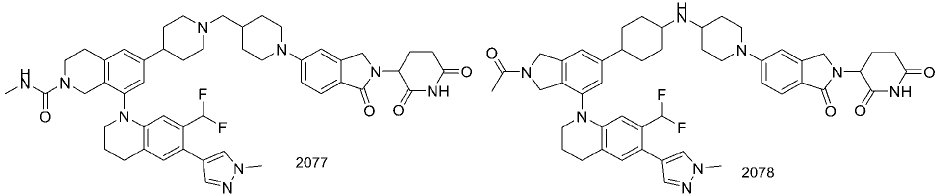

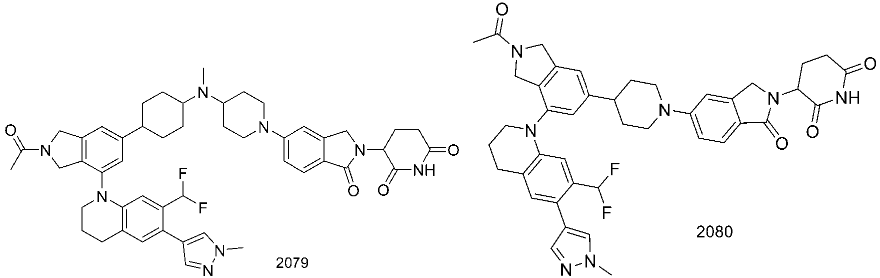

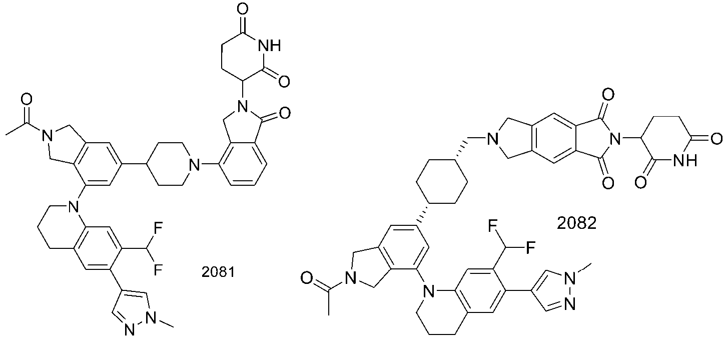

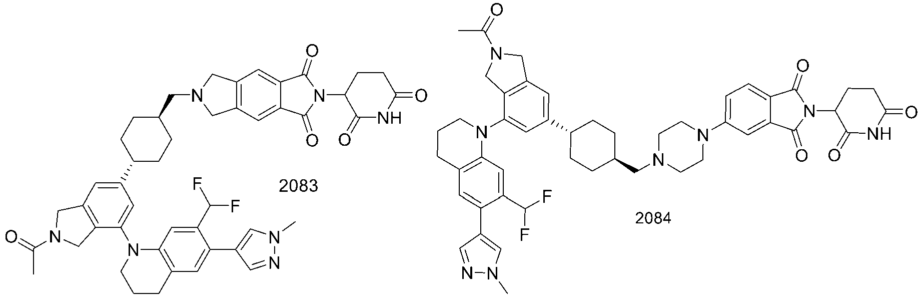

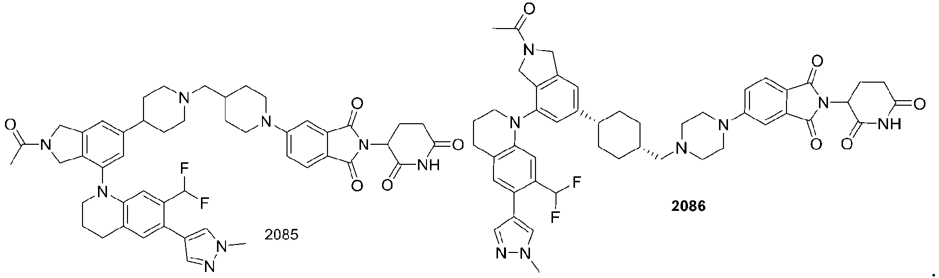

9. A pharmaceutical composition, **characterized in that** the pharmaceutical composition comprises the compound represented by formula (III), and a racemate, a stereoisomer, a tautomer, an isotopically labelled derivative, a N-oxide, solvate, a polymorph, a metabolite, an ester, a prodrug, or a pharmaceutically acceptable salt thereof according to any one of claims 1-8, preferably, the pharmaceutical composition comprises a second therapeutic agent, preferably, the second therapeutic agent is selected from drugs conventionally used for the treatment of cancer, heart diseases, metabolic diseases, inflammatory diseases, fibrotic diseases and viral infections; preferably, the classes of the second therapeutic agent may include androgen receptor antagonists, such as enzalutamide, and inhibitors of CYP17A1 (17α-hydroxylase/C17,20-lyase), such as abiraterone; cytotoxic chemotherapeutic agents, such as docetaxel; the classes of the therapeutic agents for the treatment of lung cancer include cytotoxic chemotherapeutic agents, such as cisplatin, carboplatin, docetaxel; the classes of therapeutic agents for the treatment of bladder cancer include cytotoxic chemotherapeutic agents, such as gemcitabine, cisplatin, or immunotherapy, such as Bacillus Calmette-Guérin (BCG); the classes of the second therapeutic agent may further be selected from immune checkpoint inhibitors, such as pembrolizumab, nivolumab, atezolizumab, ipilimumab; PARP (poly(ADP-ribose) polymerase) inhibitors, such as olaparib; and CDK4/6 (cyclin-dependent kinase 4 and 6) inhibitors; preferably, the second therapeutic agent may be selected for use in combination with, for example, a KRAS inhibitor.

10. Use of the compound represented by formula (III), and a racemate, a stereoisomer, a tautomer, an isotopic derivative, a N-oxide, a solvate, a polymorph, a metabolite, an ester, a prodrug, or a pharmaceutically acceptable salt according to any one of claims 1-8, or a pharmaceutical composition according to claim 9, in the preparation of a medicine for preventing and/or treating an EP300 and/or CBP-mediated disease or condition;
preferably, the EP300 and/or CBP-mediated disease or condition is selected from cancer, heart diseases, metabolic diseases, inflammatory diseases, fibrotic diseases, and viral infections; the cancer includes, but is not limited to, prostate cancer, breast cancer, bladder cancer, lung cancer, melanoma, colorectal cancer, gastric cancer, ovarian cancer, cervical cancer, bladder cancer, laryngeal cancer, multiple myeloma, liver cancer, lymphoma, and leukemia; the prostate cancer may be, for example, castration-resistant prostate cancer (CRPC); the lung cancer may be, for example, non-small cell lung cancer or small cell lung cancer; the lymphoma may be selected from non-Hodgkin's lymphoma, diffuse large B-cell lymphoma, and others.
